# EUROPEAN PATENT APPLICATION

(11) **EP 4 194 451 A1**
(43) Date of publication of application: **14.06.2023**
(21) Application number: 21854197.7
(22) Date of filing: 05.08.2021
(51) Int. Cl.: C07D 401/14, C07D 413/14, C07D 209/02, A61K 31/454, A61K 31/4545, A61P 27/00, A61P 37/00

(54) **COMPLEMENT FACTOR B INHIBITOR, AND PHARMACEUTICAL COMPOSITION THEREOF, PREPARATION METHOD THEREFOR AND USE THEREOF**

(30) Priority: 07.08.2020 CN 202010790872
(71) Applicant: Shanghai Meiyue Biotech Development Co., Ltd., Shanghai 200120 (CN)
(72) Inventor: LUAN, Linbo, Shanghai 200120 (CN); CHEN, Yongkai, Shanghai 200120 (CN); WANG, Chaodong, Shanghai 200120 (CN)
(74) Representative: Gille Hrabal Partnerschaftsgesellschaft mbB Patentanwälte
(86) International application number: PCT/CN2021/110859
(87) International publication number: WO 2022/028527

(57) **Abstract**

A piperidinyl-containing heterocyclic compound represented by formula (I). The compound can be used for treating a condition or disease related to complement alternative pathway activation by inhibiting/regulating complement factor B.

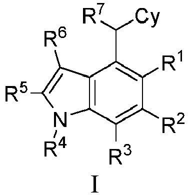

## Description

The present application claims priority to Chinese Patent Application No. 202010790872.8 filed before China National Intellectual Property Administration on Aug. 7, 2020 and entitled "COMPLEMENT FACTOR B INHIBITOR, AND PHARMACEUTICAL COMPOSITION, PREPARATION METHOD AND USE THEREOF", which is incorporated herein by reference in its entirety.

### TECHNICAL FIELD

The present disclosure belongs to the field of pharmaceuticals, and particularly relates to a complement factor B inhibitor and a pharmaceutical composition, a preparation method and an use thereof.

### BACKGROUND

Complements are a class of soluble pattern recognition molecules in the immune system that can perform multiple effector functions. Under natural conditions, complement components are present as inactive zymogens, which are broken down through a variety of specific and nonspecific immunological mechanisms to produce large and small active fragments. The large fragments usually reside on the surface of pathogens or cells and lyse the latter or accelerate their clearance. The small fragments leave the cell surface and mediate multiple inflammatory responses. Complement activation consists of a process closely followed by another, and thus a cascade of reactions of complement activation form. Three primary complement activation pathways are known at present: the classical pathway, the lectin pathway, and the alternative pathway. Although the three complement activation pathways are started through different mechanisms and are activated in different orders, they share a common terminal pathway. The activation of the alternative pathway is independent of antigen-antibody complexes, and usually C3b deposited on the cell surface binds to factor B to be in such a state that it is easily decomposed by factor D in serum. In this process, factor B is decomposed into Ba and Bb. Then C3b and Bb form a complex as the C3 convertase C3bBb in the alternative pathway. In this process, complement factor B plays an early and leading role in the activation of the alternative pathway of the complement cascade. In this case, C3b is both a product of the C3 convertase's decomposition of C3 and a component of C3 convertase in the alternative pathway. As a result, there forms a feedback amplification mechanism of the interplay between the classical pathway and the alternative pathway. The current research reveals that many diseases such as blood, autoimmune, inflammatory and neurodegeneration diseases are associated with complement system dysfunction.

Paroxysmal nocturnal hemoglobinuria (PNH) is a chronic disease that causes constant hemolysis. It is a non-malignant clonal disease caused by acquired somatic mutation in the PIG-A gene of one or more hematopoietic stem cells, a very rare disease of the blood *(*Medicine (Baltimore) 1997, 76(2): 63-93). The course of the disease may manifest itself as various degrees of hemolytic exacerbation (paroxysmal), chronic or recurring episodes of acute intravascular hemolysis or subsequent venous/arterial thrombosis which finally leads to progressive end-stage organ damage and death. Typical PNH is mainly characterized by chronic intravascular hemolysis, hemoglobinuria and hemosiderinuria. However, in most patients, the disease is often atypical, insidious and persistent, and varies in severity.

There are more than ten kinds of proteins on the red cell surface that inhibit the activation of the complement pathways. They are all anchored to the cell membrane by glycosylated phosphatidylinositol (GPI) and thus are known collectively as GPI-anchored protein (AP). It is now believed that in the pathogenesis of PNH, hematopoietic stem cells are mutated first under certain conditions and glycosylphosphatidylinositol-deficient PNH clones are produced; then because of some factors (immune factors are mostly believed now to be the cause), hematopoietic impairment or hematopoietic failure is caused, and PNH clones gain an advantage in proliferation over normal clones. The multiple antigens to which GPI is linked also contribute to the complexity of the interpretation of PNH cell biological behaviors. C3 convertase decay accelerating factor CD55 and membrane attack complex (MAC) inhibitor CD59, the most important proteins that inhibit complement pathway activation, are closely related to PNH in pathogenesis, clinical manifestations, diagnosis and treatment *(*Frontiers in Immunology 2019, 10, 1157). CD59 can prevent C9 from being incorporated into C5b-8 complex and thus the formation of membrane attack units, thereby achieving the inhibition of end-stage attack responses of complements. It is now believed that the typical manifestations of PNH-intravascular hemolysis and thrombosis-are due to CD59 deficiency. Congenital CD59 deficiency patients are reported to exhibit numerous typical symptoms of PNH, such as intravascular hemolysis, hemoglobinuria and venous thrombosis and the like. In PNH patients, CD59 is unable to bind to the cell membrane of red blood cells due to GPI synthesis defects and thus loses its function of inhibiting complement pathway activation. Therefore, the complement pathways are abnormally activated and red blood cells are attacked, leading to various clinical manifestations such as intravascular hemolysis, hemoglobinuria and smooth muscle dysfunction and the like. At present, there is no other effective clinical cure for PNH except that it can be cured by reconstitution of normal hematopoietic function through hematopoietic stem cell transplantation. As hematopoietic stem cell transplantation involves an element of risk and PNH is a benign clonal disease, controlling hemolysis episodes remains a major strategy for the clinical treatment of this disease. At present, only eculizumab is approved for the treatment of PNH. However, many patients still experience anemia after being treated with eculizumab, and constant blood transfusion remains necessary for many of them. Moreover, eculizumab has to be intravenously injected when administered. Therefore, the development of novel inhibitors of the complement pathways is of great significance for the treatment of PNH.

IgAN is the most common primary glomerulonephritis. The disease is characterized by IgA deposition in the mesangial region indicated by immunofluorescence. It has diverse clinical manifestations, and usually manifests itself as recurrent microscopic or macroscopic hematuria. Available evidence suggests that the occurrence of IgAN is associated with congenital or acquired immune dysregulation. Due to irritation to the respiratory tract or the digestive tract caused by viruses, bacteria and food proteins and the like, mucosal IgA1 synthesis is increased, or IgA1-containing immune complexes are deposited in the mesangial region, thereby activating the alternative complement pathway and causing glomerular injury. Human IgA molecules are classified into 2 subtypes: IgA1 and IgA2. IgA1 is the major form (about 85%) of blood circulation in healthy individuals. It is also the major component of the deposition in the mesangial region in IgAN patients. IgA molecules can be present in monomeric form and in polymeric form. The IgA1 molecule comprises a unique heavy chain hinge region between the first and second constant regions that can serve as a domain at the linking site for O-linked glycan groups. In recent years, it has been found that IgA molecules deposited in the serum and mesangial region of IgAN patients are mainly glycosylation-deficient IgA1(gd-IgA1). The abnormal increased production of gd-IgA1 is now believed to be the start of the pathogenesis of IgAN.

Complement C3 deposition occurs in the mesangial region of more than 90% of IgAN patients. Co-deposition of properdin, IgA and C3 occurs in kidney tissue of 75% to 100% of IgAN patients. Co-deposition of complement factors H, IgA and C3 occurs in kidney tissue of 30% to 90% of IgAN patients. In addition to deposition in kidney tissue, some studies have also revealed that the marker level of the alternative complement pathway in plasma of IgAN patients is also associated with the activity of IgAN (J Nephrol 2013, 26(4): 708-715). A study has confirmed that C3a in kidney tissue and urine and the C3a receptor in kidney tissue are significantly associated with the activity and severity of renal injury *(*J clin Immunol 2014, 34(2): 224-232). Other studies have confirmed that IgA is able to activate the alternative complement pathway *in vitro.* In this process, the abnormality in the IgA hinge region does not play a decisive role-rather, the IgA polymer formation is a critical step *(*Eur J 1987, 17(3): 321-326). Complement C3 deposition in the glomerular mesangial region has now become a marker that assists in diagnosis of IgAN. In a study, 163 IgAN patients were subjected to immunofluorescence assays for C3c and C3d. The results showed that IgAN patients in which the intensity of C3c deposition was stronger than that of C3d deposition had lower glomerular filtration rates, higher incidence of hyperplasia in the intraglomerular capillaries, and severer hematuria, which suggested that glomerular deposition of C3c was associated with the active pathological changes of IgAN *(*Am J Nephrol. 2000, 20(2):122-128). At present, there is no specific drug for IgAN in clinical practice. General drugs such as renin-angiotensin inhibitors (ACEI or ARB), glucocorticoids and various immunosuppressive drugs and the like are mainly used. In addition, the safety of such drugs is also an important topic. For example, although glucocorticoids can ameliorate proteinuria, STOP-IgAN tests and TESTING-I tests have clearly confirmed the potential side effects of glucocorticoids *(*IgA nephropathy 2019, 95, 4, 750-756).

Arthritis is a common chronic disease that is caused by inflammation, infection, degeneration, trauma or other factors and clinically manifests itself in red, swollen, hot, painful, dysfunctional and deformed joints. It often causes acute pain, decreased range of motion and deformity in people. Severe arthritis can cause disability, affecting the quality of life for patients. It was found that the serum of K/BxN mice could not induce arthritis in mice deficient in complement factor B but induce arthritis disease in wild-type mice *(*Immunity, 2002,16,157-168). This suggests that the complement system plays an important pathogenic role in the K/BxN mouse serum-induced arthritis model, and that complement factor B is a potential target for the treatment of arthritis.

Other diseases associated with the complement cascade comprise membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), hemodialysis complications, hemolytic anemia or hemodialysis, neuromyelitis optica (NMO), liver-related inflammations, inflammatory bowel disease, dermatomyositis and amyotrophic lateral sclerosis, myasthenia gravis (MG), respiratory diseases and cardiovascular diseases and the like.

At present, there is no small-molecule complement factor B inhibitors for clinical treatment. Currently known projects and those under development comprise: an oligonucleotide drug developed by IONIS Pharmaceuticals Inc. is used as a complement factor B (CFB)-specific inhibitor for the treatment, prevention or alleviation of diseases associated with the dysregulation of the complement alternative pathway (WO2015038939). Small-molecule complement factor B inhibitors developed by Novartis AG Inc. are used for the treatment of diseases such as age-related macular degeneration (AMD) and the like (WO2013164802, WO2013192345, WO2014143638, WO2015009616, WO2015066241) and for the treatment of diseases such as C3G and IgAN and the like (WO2019043609A1). A small-molecule complement factor B inhibitor developed by Achillion Pharmaceuticals Inc. is used for the treatment of diseases such as age-related macular degeneration (AMD) and the like (WO2018005552).

The inflammation and immune-related diseases are characterized by diversity and refractoriness. Eculizumab is the only drug available for PNH disease. However, the drug places a heavy burden on patients due to its prize. In addition, many patients still experience anemia after being treated with eculizumab, and constant blood transfusion remains necessary for many of them. Moreover, eculizumab has to be intravenously injected when administered. At present, there is no specific drug for the treatment of some diseases, such as IgAN and the like. There is an unmet clinical need in these areas. New small-molecule drugs need to be developed for medical treatment.

### SUMMARY

In order to alleviate the technical problems described above, the present disclosure provides a compound represented by the following formula (I) or a racemate, a stereoisomer, a tautomer, an isotopically labeled compound, a solvate, a polymorph, a pharmaceutically acceptable salt or a prodrug compound thereof: 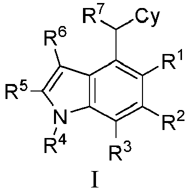
wherein R¹ is selected from halogen, OH, CN, NO₂, and the following groups unsubstituted or optionally substituted with 1, 2 or more R^{a}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R² is selected from H, halogen, OH, CN, NO₂, and the following groups unsubstituted or optionally substituted with 1, 2 or more R^{b}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R³ is selected from halogen, OH, CN, NO₂, and the following groups unsubstituted or optionally substituted with 1, 2 or more R^{c}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R⁴ is selected from H, and the following groups unsubstituted or optionally substituted with 1, 2 or more R^{d}: C₁₋₄₀ alkyl, C₃₋₄₀ cycloalkyl, C₁₋₄₀ alkyl-C(O)-, C₃₋₄₀ cycloalkyl-C(O)-, C₁₋₄₀ alkyl-S(O)₂-, and C₃₋₄₀ cycloalkyl-C(O)₂-;
R⁵ is selected from H, halogen, OH, CN, NO₂, and the following groups unsubstituted or optionally substituted with 1, 2 or more R^{e}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R⁶ is selected from H, halogen, OH, CN, NO₂, and the following groups unsubstituted or optionally substituted with 1, 2 or more R^{f}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R⁷ is selected from hydrogen, OH, CN, and the following groups unsubstituted or optionally substituted with 1, 2 or more R^{g}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
alternatively, R¹ and R⁷, together with atoms to which they are attached, form a 5- to 20-membered cyclic structure that is unsubstituted or optionally substituted with 1, 2 or more R^{h}; wherein the 5- to 20-membered cyclic structure may be selected from, for example, the following groups: C₅₋₂₀ cycloalkenyl, C₆₋₂₀ aryl, 5- to 20-membered heterocyclyl, and 5- to 20-membered heteroaryl;
alternatively, R⁶ and R⁷, together with atoms to which they are attached, form a 5- to 20-membered cyclic structure that is unsubstituted or optionally substituted with 1, 2 or more R¹; wherein the 5- to 20-membered cyclic structure may be selected from, for example, the following groups: C₅₋₂₀ cycloalkenyl, C₆₋₂₀ aryl, 5- to 20-membered heterocyclyl, and 5- to 20-membered heteroaryl;
Cy is selected from the following groups substituted with 1, 2, 3, 4, 5, 6, 7, 8 or more substituents independently selected from R⁸, R⁹, R¹⁰ and R¹¹: C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₃₋₄₀ cycloalkyl-C₁₋₄₀ alkyl-, C₃₋₄₀ cycloalkenyl-C₁₋₄₀ alkyl-, C₃₋₄₀ cycloalkynyl-C₁₋₄₀ alkyl-, C₆₋₂₀ aryl-C₁₋₄₀ alkyl-, 5-to 20-membered heteroaryl-C₁₋₄₀ alkyl-, 3- to 20-membered heterocyclyl-C₁₋₄₀ alkyl-, C₃₋₄₀ cycloalkyl-C₁₋₄₀ alkyl-, C₃₋₄₀ cycloalkenyl-C₁₋₄₀ alkyl-, C₃₋₄₀ cycloalkynyl-C₁₋₄₀ alkyl-, C₆₋₂₀ aryl-C₁₋₄₀ alkyl-, 5- to 20-membered heteroaryl-C₁₋₄₀ alkyl-, and 3- to 20-membered heterocyclyl-C₁₋₄₀ alkyl-, wherein the 3- to 20-membered heterocyclyl in the group Cy comprises 1-5 heteroatoms selected from N, O and S and comprises up to only one N atom;
R⁸ and R⁹ are identical or different, and are each independently selected from H, and the following groups unsubstituted or optionally substituted with 1, 2 or more R^{j}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₃₋₄₀ cycloalkyl-C₁₋₄₀ alkyl-, C₃₋₄₀ cycloalkenyl-C₁₋₄₀ alkyl-, C₃₋₄₀ cycloalkynyl-C₁₋₄₀ alkyl-, C₆₋₂₀ aryl-C₁₋₄₀ alkyl-, 5- to 20-membered heteroaryl-C₁₋₄₀ alkyl-, and 3- to 20-membered heterocyclyl-C₁₋₄₀ alkyl-;
R¹⁰ and R¹¹ are identical or different, and are each independently selected from H, being absent, halogen, OH, CN, NO₂, and the following groups unsubstituted or optionally substituted with 1, 2 or more R^{k}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
alternatively, R⁸ and R⁹, together with atoms to which they are attached, form a 5- to 20-membered cyclic structure that is unsubstituted or optionally substituted with 1, 2 or more R^{j}; wherein the 5- to 20-membered cyclic structure may be selected from, for example, the following groups: C₃₋₂₀ cycloalkyl, C₅₋₂₀ cycloalkenyl, C₆₋₂₀ aryl, 5- to 20-membered heterocyclyl, and 5- to 20-membered heteroaryl;
alternatively, R¹⁰ and R¹¹, together with atoms to which they are attached, form a 5- to 20-membered cyclic structure that is unsubstituted or optionally substituted with 1, 2 or more R^{k}; wherein the 5- to 20-membered cyclic structure may be selected from, for example, the following groups: C₃₋₂₀ cycloalkyl, C₅₋₂₀ cycloalkenyl, C₆₋₂₀ aryl, 5- to 20-membered heterocyclyl, and 5- to 20-membered heteroaryl;
each R^{a}, R^{b}, R^{c}, R^{d}, R^{e}, R^{f}, R^{g}, R^{h}, Rⁱ, R^{j} and R^{k} are identical or different, and are independently selected from H, halogen, OH, CN, NO₂, oxo (=O), thio (=S), and the following groups unsubstituted or optionally substituted with 1, 2 or more R^{p}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5-to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)R¹², -C(O)OR¹³, -OC(O)R¹⁴, -S(O)₂R¹⁵, -S(O)₂OR¹⁶, -OS(O)₂R¹⁷, - B(OR¹⁸)(OR¹⁹), -P(O)(OR²⁰)(OR²¹), and 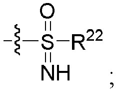
each R^{p} is identical or different, and is independently selected from H, halogen, OH, CN, NO₂, oxo (=O), thio (=S), and the following groups unsubstituted or optionally substituted with 1, 2 or more R^{q}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)R¹²¹, -C(O)OR¹³¹, -OC(O)R¹⁴¹, -S(O)₂R¹⁵¹, - S(O)₂OR¹⁶¹, -OS(O)₂R¹⁷¹, -B(OR¹⁸³)(OR¹⁹¹), -P(O)(OR²⁰¹)(OR²¹¹), and 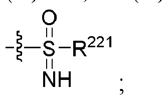
each R^{q} is identical or different, and is independently selected from H, halogen, OH, CN, NO₂, oxo (=O), thio (=S), C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)C₁₋₄₀ alkyl, -C(O)NH₂, - C(O)NHC₁₋₄₀ alkyl, -C(O)-NH-OH, -COOC₁₋₄₀ alkyl, -COOH, -OC(O)C₁₋₄₀ alkyl, -OC(O)H, - S(O)₂C₁₋₄₀ alkyl, S(O)₂H, -S(O)₂OC₁₋₄₀ alkyl, -OS(O)₂C₁₋₄₀ alkyl, -P(O)(OH)₂, -B(OH)₂, and 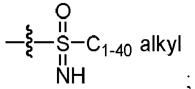
R¹² R¹³ R¹⁴ R¹⁵ R¹⁶ R¹⁷ R¹⁸, R¹⁹, R²⁰, R²¹, R¹²¹ R¹³¹, R¹⁴¹, R¹⁵¹, R¹⁶¹, R¹⁷¹ R¹⁸¹, R¹⁹¹, , , , > > > , , , , > , , , , > , , R²⁰¹, R²¹¹, R¹²², R¹³², R¹⁴², R¹⁵², R¹⁶², R¹⁷², R¹⁸², R¹⁹², R²⁰² and R²¹² are identical or different, and are each independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, and NH₂.

According to an embodiment of the present disclosure, R¹ is selected from halogen, OH, CN, NO₂, and the following groups unsubstituted or optionally substituted with 1, 2 or more R^{a}: C₁₋₆ alkyl, C₃₋₈ cycloalkyl, C₁₋₆ alkyloxy, C₃₋₈ cycloalkyloxy, and NH₂.

According to an embodiment of the present disclosure, R² is selected from H, halogen, OH, CN, NO₂, and the following groups unsubstituted or optionally substituted with 1, 2 or more R^{b}: C₁₋₆ alkyl, C₃₋₈ cycloalkyl, C₁₋₆ alkyloxy, C₃₋₈ cycloalkyloxy, and NH₂.

According to an embodiment of the present disclosure, R³ is selected from halogen, OH, CN, NO₂, and the following groups unsubstituted or optionally substituted with 1, 2 or more R^{c}: C₁₋₆ alkyl, C₃₋₈ cycloalkyl, C₁₋₆ alkyloxy, C₃₋₈ cycloalkyloxy, and NH₂.

According to an embodiment of the present disclosure, R⁴ is selected from H, and C₁₋₆ alkyl unsubstituted or optionally substituted with 1, 2 or more R^{d}.

According to an embodiment of the present disclosure, R⁵ is selected from H, halogen, OH, CN, NO₂, and the following groups unsubstituted or optionally substituted with 1, 2 or more R^{e}: C₁₋₆ alkyl, C₃₋₈ cycloalkyl, C₁₋₆ alkyloxy, C₃₋₈ cycloalkyloxy, and NH₂.

According to an embodiment of the present disclosure, R⁶ is selected from H, halogen, OH, CN, NO₂, and the following groups unsubstituted or optionally substituted with 1, 2 or more R^{f}: C₁₋₆ alkyl, C₃₋₈ cycloalkyl, C₁₋₆ alkyloxy, C₃₋₈ cycloalkyloxy, and NH₂.

R⁷ is selected from hydrogen, OH, CN, and the following groups unsubstituted or optionally substituted with 1, 2 or more R^{g}: C₁₋₆ alkyl, C₃₋₈ cycloalkyl, C₁₋₆ alkyloxy, C₃₋₈ cycloalkyloxy, and NH₂.

Alternatively, according to an embodiment of the present disclosure, R¹ and R⁷, together with atoms to which they are attached, may form the following groups unsubstituted or optionally substituted with 1, 2 or more R^{h}: C₅₋₁₀ cycloalkenyl, C₆₋₁₀ aryl, 5- to 10-membered heterocyclyl, and 5- to 10-membered heteroaryl, e.g., C₅₋₆ cycloalkenyl, C₆ aryl, 5- to 6-membered heterocyclyl, and 5- to 6-membered heteroaryl. Preferably, the 5- to 6-membered heterocyclyl and 5- to 6-membered heteroaryl comprise, for example, 1, 2, 3, 4, 5 or more heteroatoms selected from O, S and N, wherein N and S may optionally be unoxidized or be oxidized to various oxidized forms. By way of example, R¹ and R⁷, together with atoms to which they are attached, may form cyclopentyl, cyclohexyl, tetrahydrofuranyl, tetrahydropyranyl or tetrahydrothiopyranyl (wherein a sulfur atom is unoxidized or oxidized to a -S(O)₂- group) that is fused to the indolyl group in formula (I) and is unsubstituted or optionally substituted with 1, 2 or more R^{h}.

Alternatively, according to an embodiment of the present disclosure, R⁶ and R⁷, together with atoms to which they are attached, may form the following groups unsubstituted or optionally substituted with 1, 2 or more Rⁱ: C₅₋₂₀ cycloalkenyl, C₆₋₂₀ aryl, 5- to 20-membered heterocyclyl, and 5- to 20-membered heteroaryl, e.g., C₅₋₆ cycloalkenyl, C₆ aryl, 5- to 6-membered heterocyclyl, and 5- to 6-membered heteroaryl. Preferably, the 5- to 6-membered heterocyclyl and 5- to 6-membered heteroaryl comprise, for example, 1, 2, 3, 4, 5 or more heteroatoms selected from O, S and N, wherein N and S may optionally be unoxidized or be oxidized to various oxidized forms. By way of example, R⁶ and R⁷, together with atoms to which they are attached, may form cyclopentyl, cyclohexyl, tetrahydrofuranyl, tetrahydropyranyl or tetrahydrothiopyranyl (wherein a sulfur atom is unoxidized or is oxidized to a -S(O)₂- group) that is fused to the indolyl group in formula (I) and is unsubstituted or optionally substituted with 1, 2 or more R^{h}.

According to an embodiment of the present disclosure, Cy may be selected from the following groups substituted with 1, 2, 3, 4, 5, 6, 7, 8 or more substituents independently selected from R⁸, R⁹, R¹⁰ and R¹¹: C₃₋₄₀ cycloalkyl, C₆₋₂₀ aryl, 5- to 20-membered heteroaryl, and 3- to 20-membered heterocyclyl, wherein the 3- to 20-membered heterocyclyl in the group Cy comprises 1-3 heteroatoms selected from N, O and S and comprises up to only one N atom.

According to a preferred embodiment of the present disclosure, Cy may be selected from 3- to 20-membered heterocyclyl substituted with 1, 2, 3, 4, 5, 6, 7 or 8 substituents selected from R⁸, R⁹, R¹⁰ and R¹¹. For example, Cy is selected from 3- to 20-membered heterocyclyl substituted with R⁸, R⁹, R¹⁰ and R¹¹ and optionally further substituted with 1, 2, 3 or 4 substituents independently selected from R⁸, R⁹, R¹⁰ and R¹¹, wherein the 3- to 20-membered heterocyclyl in the groups Cy comprises 1 or 2 heteroatoms selected from N, O and S and comprises up to only one N atom.

According to an illustrative embodiment of the present disclosure, Cy may be selected from the following saturated or unsaturated non-aromatic carbocyclic or heterocyclic ring systems: a 4-, 5-, 6- or 7-membered monocyclic ring system, a 7-, 8-, 9-, 10-, 11- or 12-membered bicyclic (e.g. fused, bridged, or spiro) ring system or a 10-, 11-, 12-, 13-, 14- or 15-membered tricyclic ring system, and the heterocyclic ring systems comprise 1-5 heteroatoms selected from O, S and N and comprise up to only one N atom, wherein the N and S atoms, if present, may optionally be unoxidized or oxidized to various oxidized forms.

According to an illustrative embodiment of the present disclosure, Cy comprises 1 N atom, and optionally comprises 1 or 2 atoms selected from O and S that are present or absent. Preferably, where Cy is selected from a bicyclic ring system, the N atom is in a different cyclic structure in the bicyclic ring than the O or S atom.

According to an illustrative embodiment of the present disclosure, Cy comprises up to 2 heteroatoms, one and only one of which is selected from N atom.

According to an illustrative embodiment of the present disclosure, Cy may be selected from the following cyclic groups:
piperidyl;
piperidyl fused to a ring system selected from cyclopropyl, tetrahydrofuranyl, tetrahydropyranyl and phenyl;
aza and/or oxa spiro[2.4], [3.4], [4.4], [2.5], [3.5], [4.5] or [5.5] cyclic groups; and
aza and/or oxa bicyclo[2.2.1], [2.2.2], [3.2.1], [3.2.2] or [3.3.2] cyclic groups.

According to a preferred embodiment of the present disclosure, the N atom of Cy may be bonded to the C atom shared by the groups Cy and R⁷ of formula (I).

By way of example, Cy may be selected from monocyclic, fused and bridged cyclic groups, e.g., the following groups:
piperidyl; 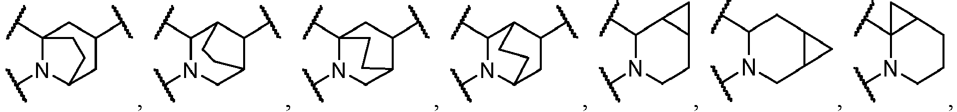 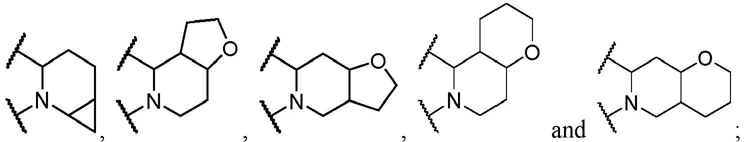 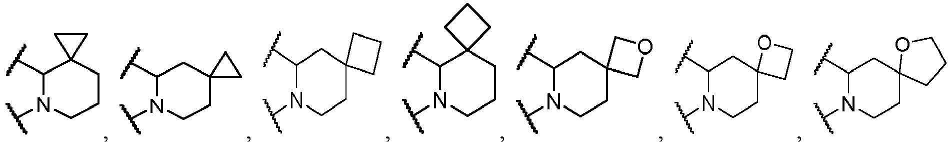 and

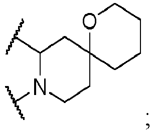

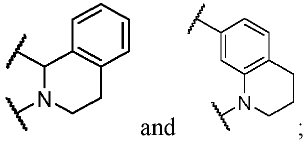

According to an embodiment of the present disclosure, R⁸ may be selected from the following groups optionally substituted with 1, 2 or more R^{j}: C₆₋₁₀ aryl, 5- to 10-membered heteroaryl, and 3- to 20-membered heterocyclyl, e.g., phenyl, pyridinyl, pyrazinyl, furanyl, pyranyl, benzocyclohexyl, benzocyclopentyl, benzofuranyl, and benzotetrahydrofuranyl.

According to an embodiment of the present disclosure, R⁹ is identical or different, and is each independently selected from H, and C₁₋₆ alkyl unsubstituted or optionally substituted with 1, 2 or more R^{j}.

Alternatively, according to an embodiment of the present disclosure, R⁸ and R⁹, together with atoms to which they are attached, may form the following groups that are unsubstituted or optionally substituted with 1, 2 or more R^{j}: C₅₋₁₀ cycloalkenyl, C₆₋₁₀ aryl, 5- to 10-membered heterocyclyl, and 5- to 10-membered heteroaryl.

According to an embodiment of the present disclosure, R¹⁰ and R¹¹ may be identical or different, and are each independently selected from halogen, OH, CN, NO₂, and the following groups unsubstituted or optionally substituted with 1, 2 or more R^{k}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₆ cycloalkyloxy, C₆₋₁₀ aryloxy, 5- to 6-membered heteroaryloxy, 3- to 6-membered heterocyclyloxy, and NH₂.

Alternatively, according to an embodiment of the present disclosure, R¹⁰ and R¹¹, together with atoms to which they are attached, may form the following groups that are unsubstituted or optionally substituted with 1, 2 or more R^{k}: C₅₋₁₀ cycloalkenyl, C₆₋₁₀ aryl, 5- to 10-membered heterocyclyl, and 5- to 10-membered heteroaryl.

According to an embodiment of the present disclosure, each R^{j} is identical or different, and is independently selected from the following groups unsubstituted or optionally substituted with 1, 2 or more R^{p}: C₁₋₆ alkyl, C₃₋₈ cycloalkyl, C₆₋₁₀ aryl, 5- to 10-membered heteroaryl, 3- to 10-membered heterocyclyl, C₁₋₆ alkyloxy, C₃₋₈ cycloalkyloxy, C₆₋₁₀ aryloxy, 5- to 10-membered heteroaryloxy, 3- to 10-membered heterocyclyloxy, NH₂, -C(O)R¹², -C(O)OR¹³, - B(OR¹⁸)(OR¹⁹), -P(O)(OR²⁰)(OR²¹), and

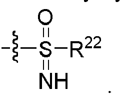

According to an embodiment of the present disclosure, each R^{k} is identical or different, and is independently selected from halogen, OH, CN, NO₂, and the following groups unsubstituted or optionally substituted with 1, 2 or more R^{p}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₆ cycloalkyloxy, C₆₋₁₀ aryloxy, 5- to 6-membered heteroaryloxy, 3- to 6-membered heterocyclyloxy, and NH₂.

According to an embodiment of the present disclosure, each R^{p} is identical or different, and is independently selected from H, halogen, OH, and the following groups unsubstituted or optionally substituted with 1, 2 or more R^{q}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₈ cycloalkyloxy, C₆₋₁₀ aryloxy, 5- to 6-membered heteroaryloxy, 3- to 6-membered heterocyclyloxy, NH₂, -C(O)R¹²¹, -C(O)OR¹³¹, -B(OR¹⁸¹)(OR¹⁹¹), -P(O)(OR²⁰¹)(OR²¹¹), and

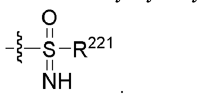

According to an embodiment of the present disclosure, R^{q} is as defined above.

According to an embodiment of the present disclosure, R¹², R¹³, R¹⁸, R¹⁹, R²⁰, R²¹, R¹²¹, R¹³¹, R¹⁸¹, R¹⁹¹, R²⁰¹, R²¹¹ are identical or different, and are each independently selected from H, C₁₋₆ alkyl, C₃₋₈ cycloalkyl, C₆₋₁₀ aryl, 5- to 6-membered heteroaryl, 3- to 6-membered heterocyclyl, and NH₂.

According to an embodiment of the present disclosure, the compound represented by formula (I) may have a structure represented by formula (I-1) or formula (I-2): 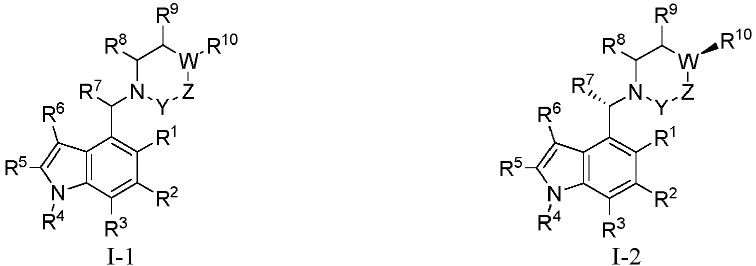
wherein W is selected from CH, O and S;
Y and Z are identical or different, and are each independently selected from CHR¹¹, O and S;
R¹, R², R³, R⁴, R⁵, R⁶, R⁷, R⁸, R⁹, R¹⁰ and R¹¹ are independently as defined in formula (I).

According to an embodiment of the present disclosure, if appropriate, a carbon-carbon single bond or a carbon-carbon double bond may be formed between W and Z or Z and Y.

According to an embodiment of the present disclosure, where W is selected from O and S, R¹⁰ is absent.

According to an embodiment of the present disclosure, where W is selected from CH, R¹⁰ is selected from H, halogen, OH, CN, NO₂, and the following groups unsubstituted or optionally substituted with 1, 2 or more R^{k}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3-to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂, wherein R^{k} is as defined above.

According to an embodiment of the present disclosure, the compound represented by formula (I) may have a structure represented by formula (I-3) or formula (I-4): 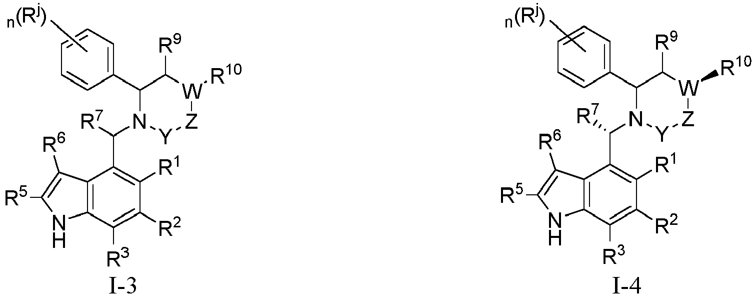
wherein W, Y, Z, R¹, R², R³, R⁵, R⁶, R⁷, R⁹, R¹⁰ and R^{j} are independently as defined above;
n is selected from 1, 2, 3, 4 and 5.

According to an embodiment of the present disclosure, n may be selected from 1, 2 and 3.

According to an embodiment of the present disclosure, each R^{j} may be a substituent at the 2-, 3-, 4- or 5-position of phenyl.

According to an embodiment of the present disclosure, each R^{j} may be independently selected from the following groups unsubstituted or optionally substituted with 1, 2 or more R^{p}: C₁₋₆ alkyl, NH₂, -C(O)R¹², -C(O)OR¹³, -B(OR¹⁸)(OR¹⁹), -P(O)(OR²⁰)(OR²¹), and

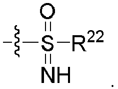

According to an embodiment of the present disclosure, R¹⁰ is selected from halogen, OH, CN, NO₂, and the following groups unsubstituted or optionally substituted with 1, 2 or more R^{k}: C₁₋₆ alkyl (e.g., methyl, ethyl, propyl, isopropyl, butyl, isobutyl, tert-butyl, pentyl, or isopentyl), C₃₋₈ cycloalkyl (e.g., cyclopropyl, cyclobutyl, cyclopentyl, cyclohexyl, cycloheptyl, or cyclooctyl), 3- to 6-membered heterocyclyl (e.g., pyrrolidinyl, imidazolidinyl, piperidyl, piperazinyl, oxetanyl, tetrahydrofuranyl, or tetrahydropyranyl), C₁₋₆ alkyloxy, C₃₋₆ cycloalkyloxy, 3- to 6-membered heterocyclyloxy, and NH₂.

According to an embodiment of the present disclosure, each R^{k} is identical or different, and is independently selected from halogen, OH, CN, NO₂, and the following groups unsubstituted or optionally substituted with 1, 2 or more R^{p}: C₁₋₆ alkyl (e.g., methyl, ethyl, propyl, isopropyl, butyl, isobutyl, tert-butyl, pentyl, or isopentyl), C₃₋₈ cycloalkyl (e.g., cyclopropyl, cyclobutyl, cyclopentyl, cyclohexyl, cycloheptyl, or cyclooctyl), C₆₋₁₀ aryl (e.g., phenyl), 5- to 6-membered heteroaryl (e.g., pyrrolyl, pyridinyl, pyrazinyl, imidazolyl, or triazolyl), 3- to 6-membered heterocyclyl (e.g., pyrrolidinyl, imidazolidinyl, piperidyl, piperazinyl, oxetanyl, tetrahydrofuranyl, or tetrahydropyranyl), C₁₋₆ alkyloxy, C₃₋₆ cycloalkyloxy, C₆₋₁₀ aryloxy, 5- to 6-membered heteroaryloxy, and 3- to 6-membered heterocyclyloxy.

According to an embodiment of the present disclosure, each R^{p} is identical or different, and is independently selected from H, halogen (F, Cl, Br or I), OH, and the following groups unsubstituted or optionally substituted with 1, 2 or more R^{q}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₈ cycloalkyloxy, C₆₋₁₀ aryloxy, 5- to 6-membered heteroaryloxy, 3- to 6-membered heterocyclyloxy, and NH₂.

According to an embodiment of the present disclosure, 1, 2, 3 or more H atoms in said compound and a substituent thereof (e.g., methyl or ethyl) may be optionally replaced with its isotope (e.g., D) to form a group such as CD₃ or C₂D₅.

According to an embodiment of the present disclosure, the compound represented by formula (I) may be selected from the following compounds:

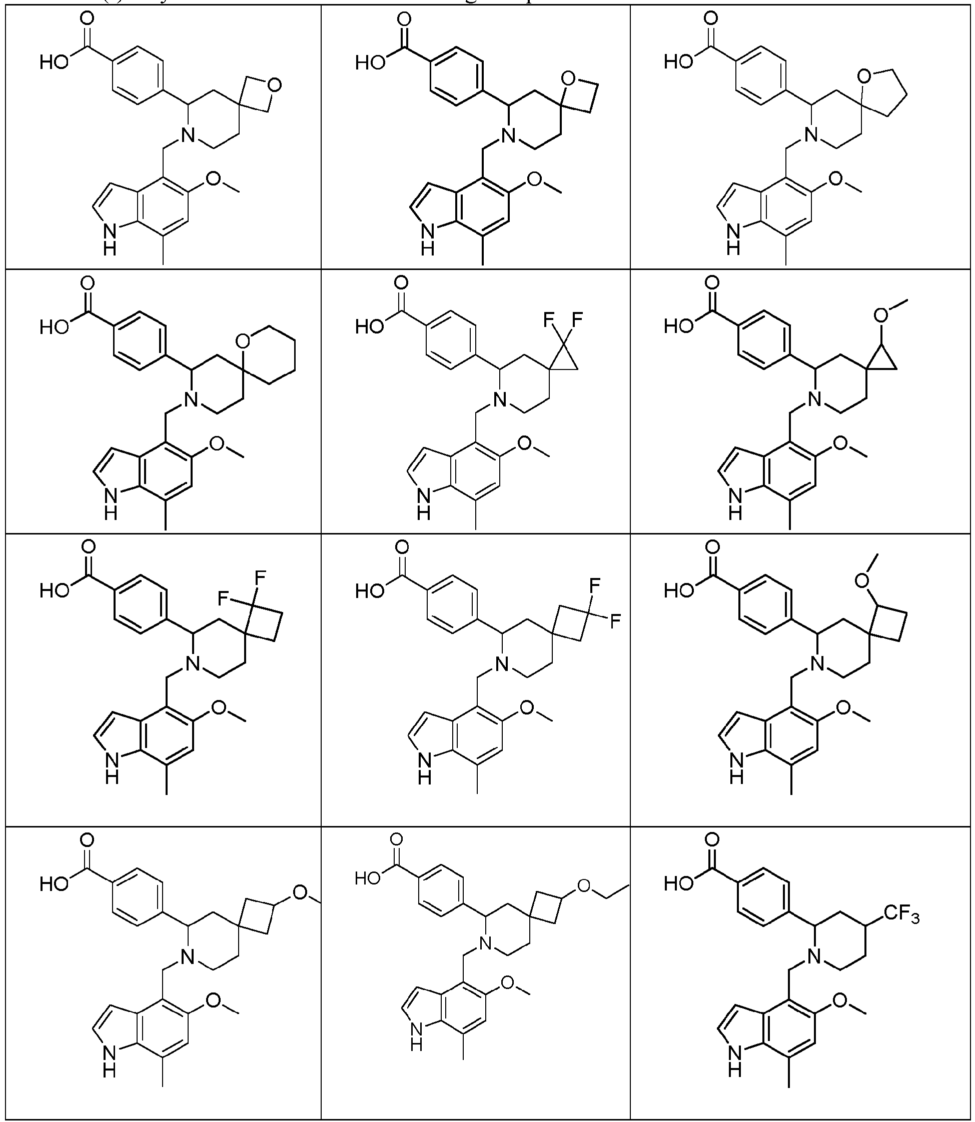

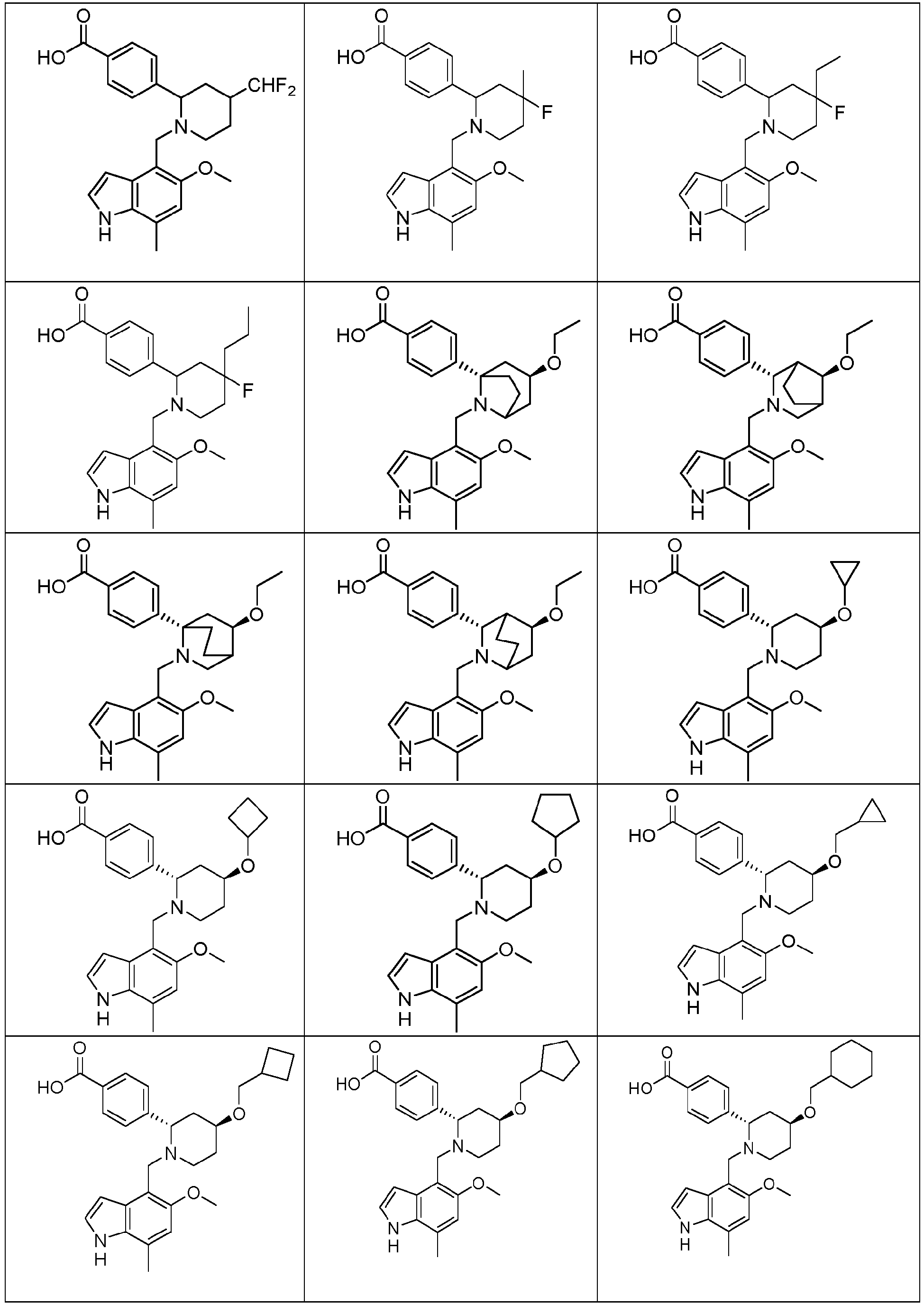

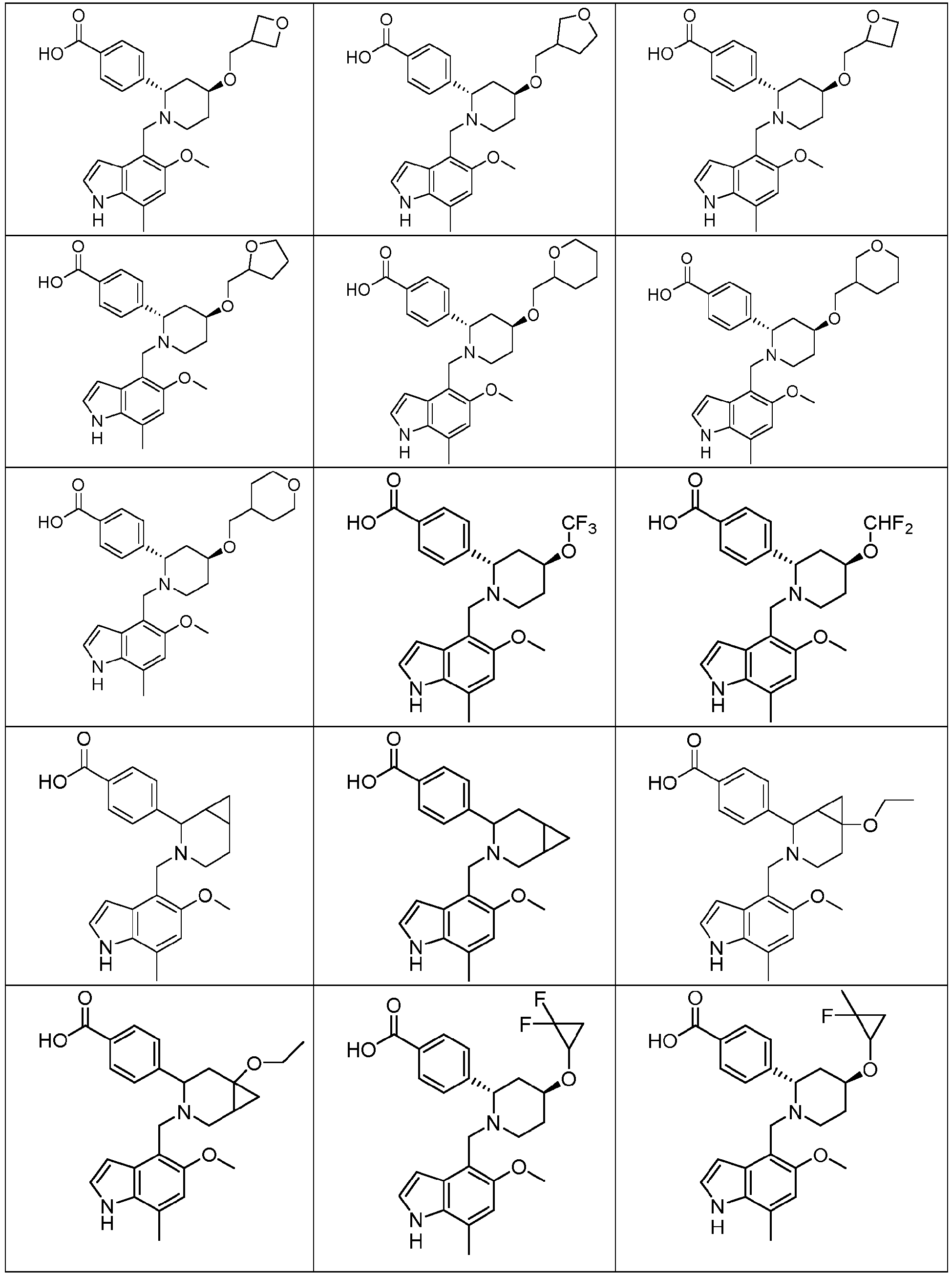

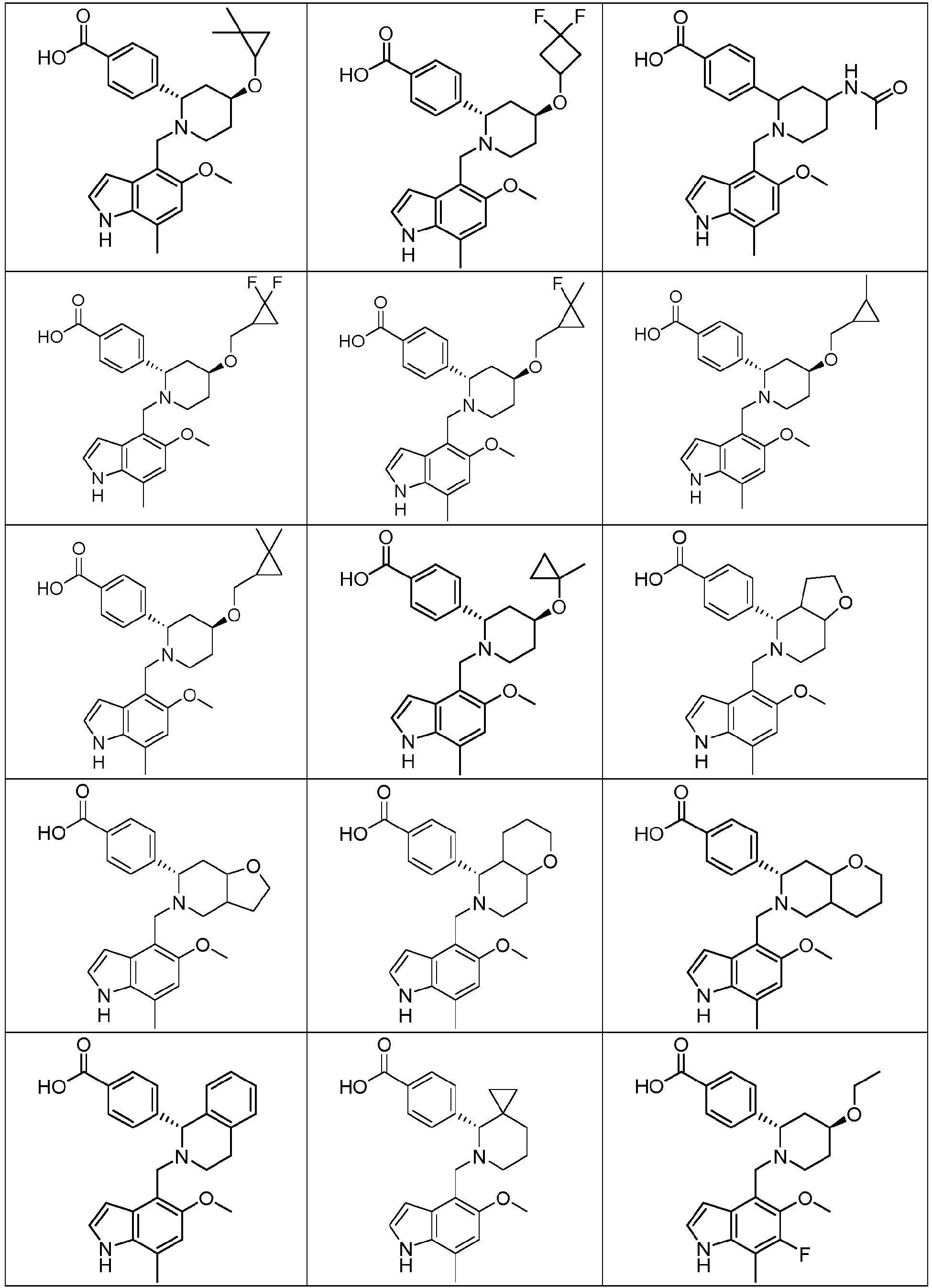

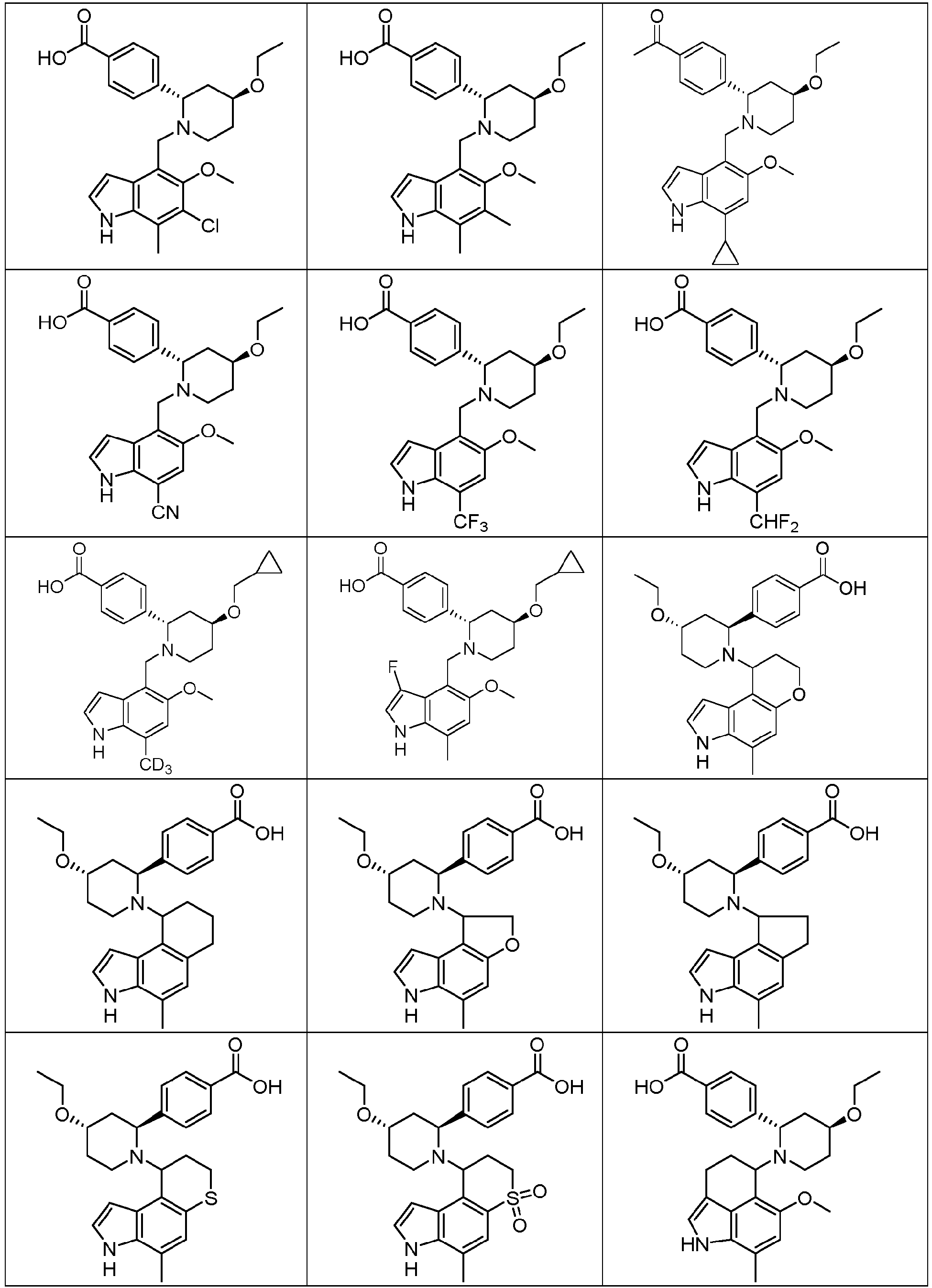

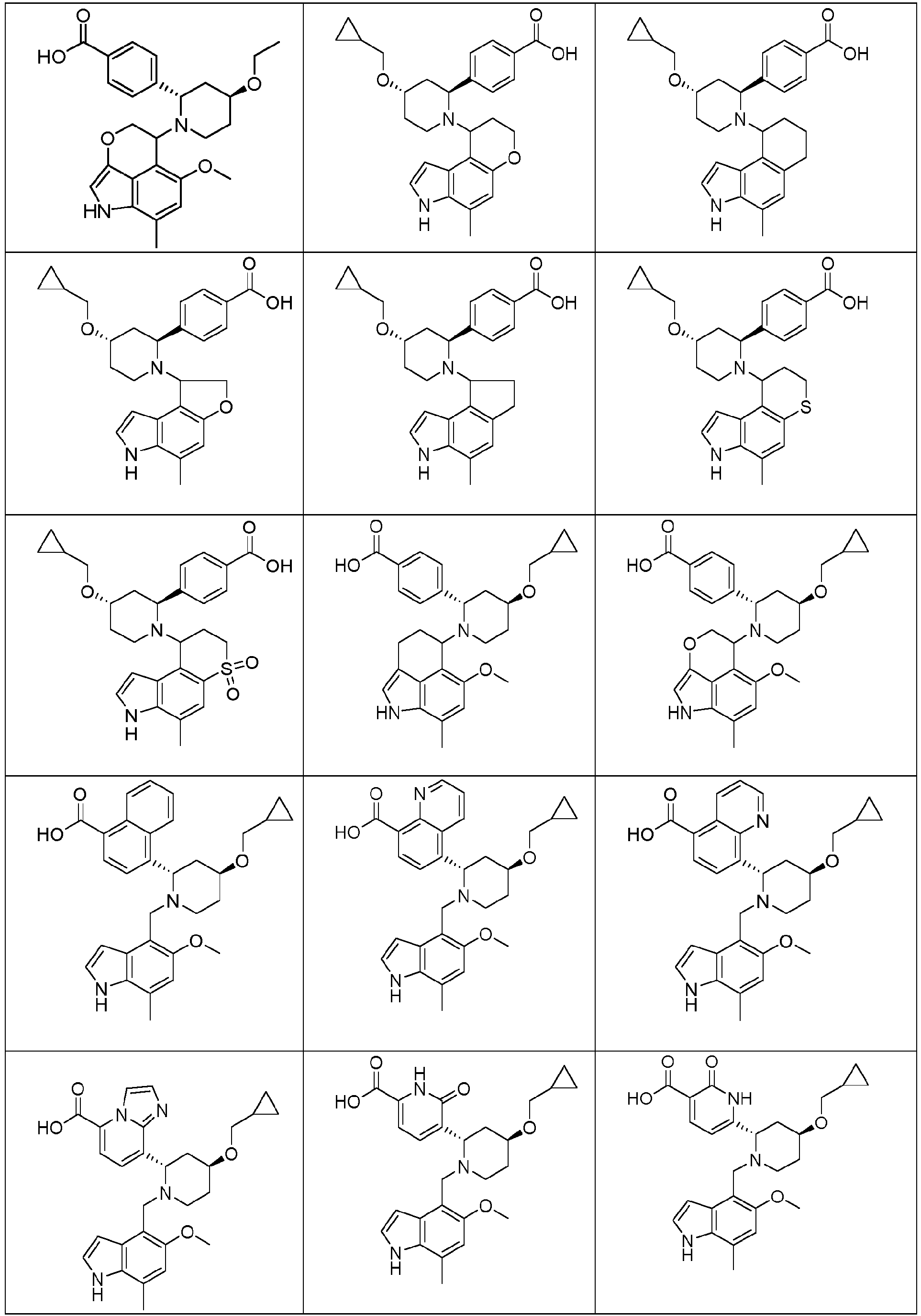

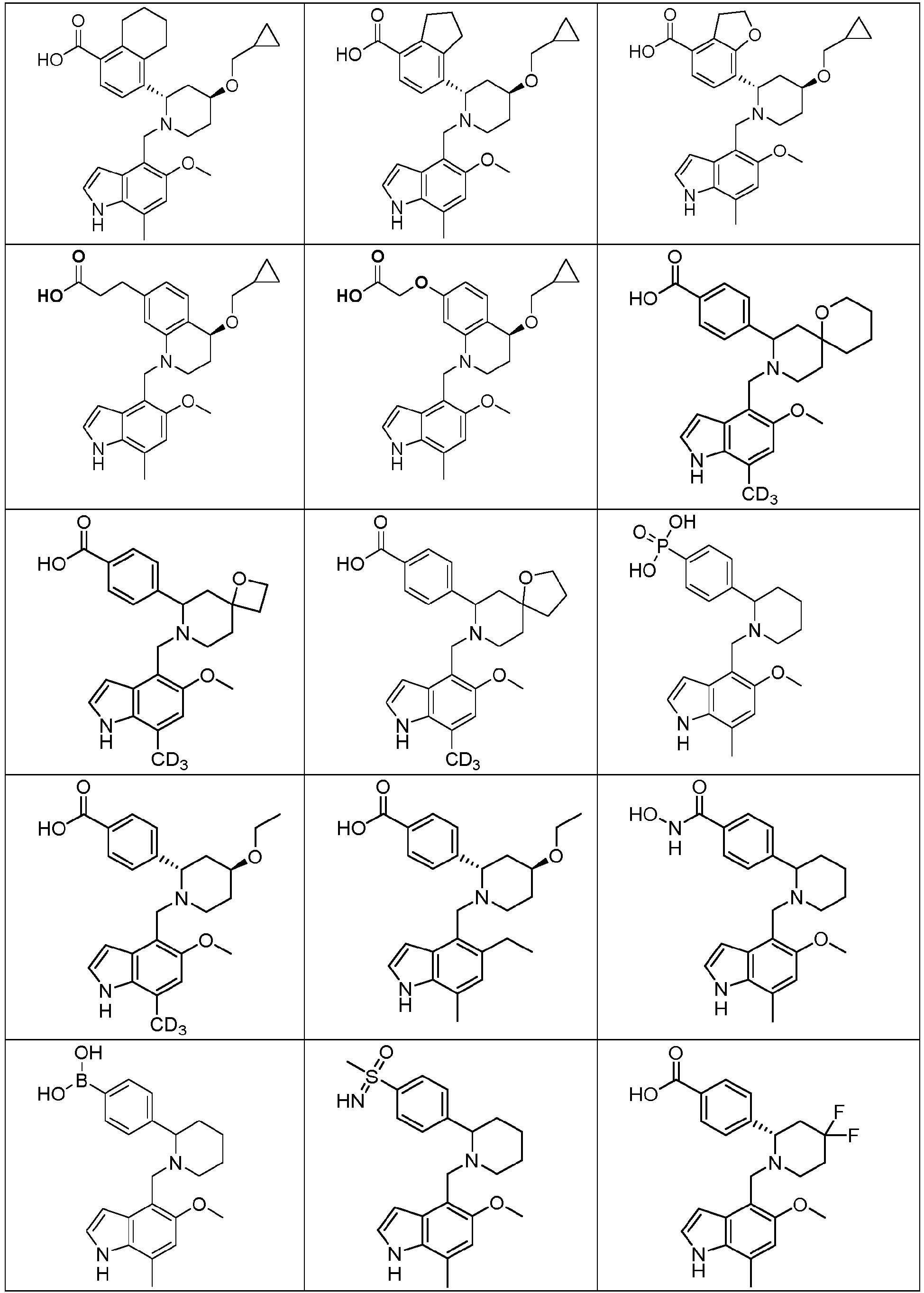

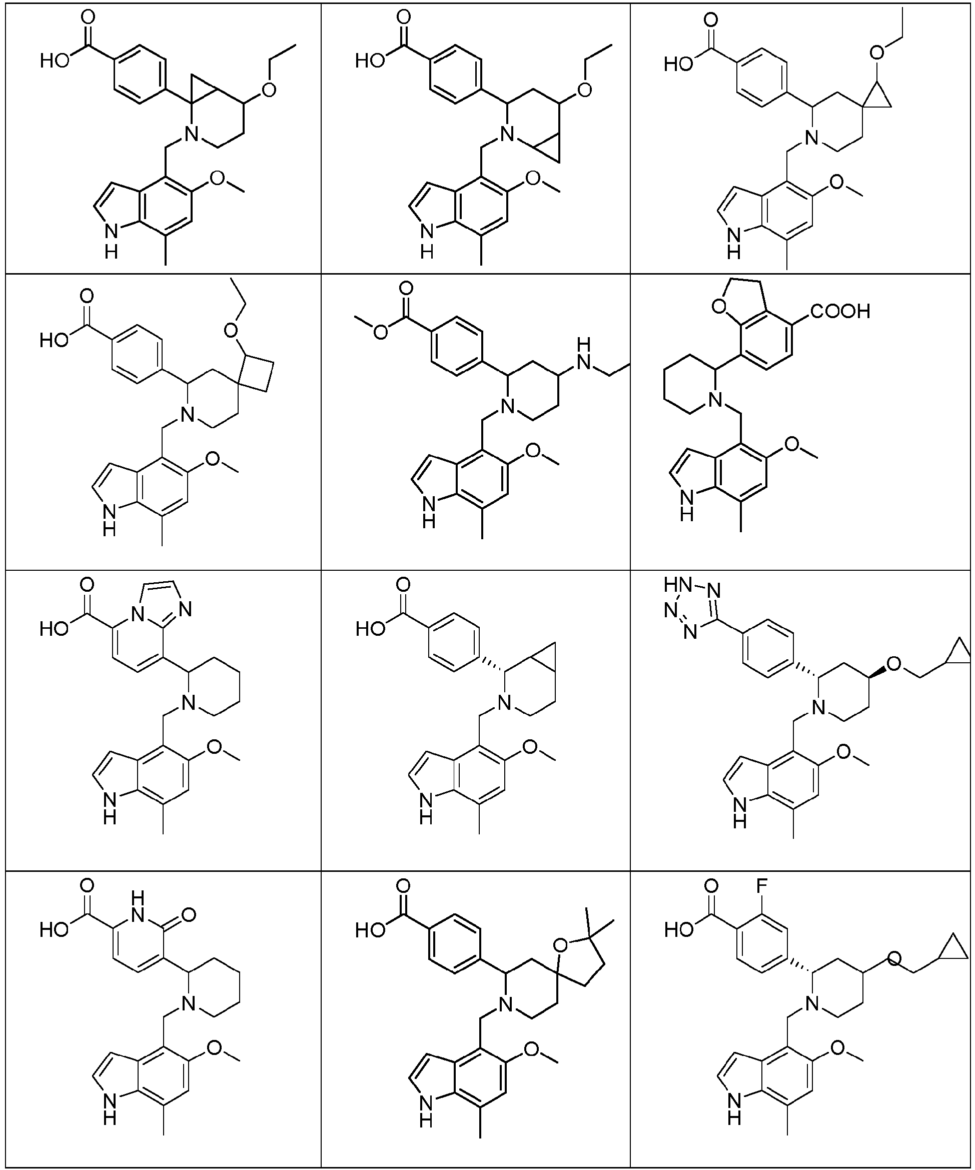

The present disclosure further provides a compound represented by the following formula (IV): 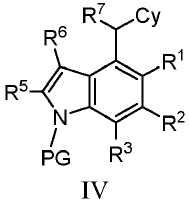
wherein PG is a protective group;
R¹, R², R³, R⁵, R⁶, R⁷ and Cy are independently as defined above.

The present disclosure also provides an use of the compound represented by formula (IV) for preparing the compound represented by formula (I) or the racemate, the stereoisomer, the tautomer, the isotopically labeled compound, the solvate, the polymorph, the pharmaceutically acceptable salt or the prodrug compound thereof.

The present disclosure also provides a preparation method for the compound represented by formula (I), which comprises reacting the compound of formula (IV) as a starting material to give a compound of formula (Ia), i.e. a compound represented by formula (I) in which R⁴ is H: 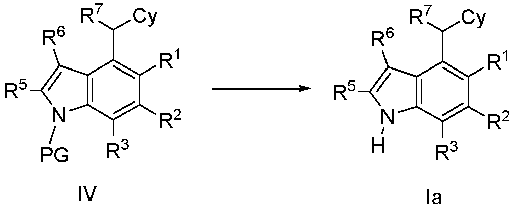
and optionally, further reacting the compound of formula (Ia) with R⁴-L¹ to give a compound represented by formula (I) in which R⁴ is a group defined above other than H;
wherein PG, R¹, R², R³, R⁵, R⁶, R⁷ and Cy are independently as defined above;
L¹ is a leaving group, e.g., OH, F, Cl, Br, I, or halogenated C₁₋₄₀ alkyl.

According to an embodiment of the present disclosure, PG may be selected from amino-protecting groups. Suitable PG may be selected from C₁₋₄₀ alkyl and C₆₋₂₀ aryl C₁₋₄₀ alkyl-, e.g., tert-butyl, isopropyl, benzyl, *tert*-butoxycarbonyl (Boc), 2-biphenyl-2-propoxycarbonyl, benzyloxycarbonyl, fluorenylmethoxycarbonyl (Fmoc), and trifluoroacetyl.

According to an embodiment of the present disclosure, the compound of formula (IV) is reacted under a condition for removing the protective group PG to give the compound of formula (I). The conditions for removing the protective group PG are those known to those skilled in the art.

The present disclosure also provides a preparation method for the compound represented by formula (IV), which comprises reacting a compound of formula (II) with a compound of formula (III) to give the compound represented by formula (IV); 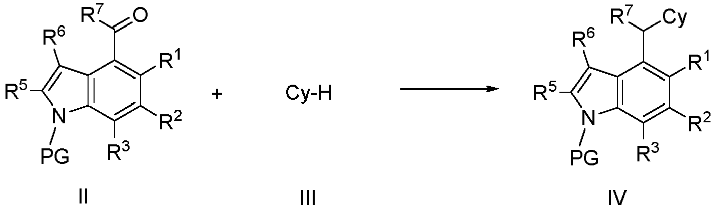 wherein PG, R¹, R², R³, R⁵, R⁶, R⁷ and Cy are independently as defined above.

According to an embodiment of the present disclosure, the preparation method may be carried out in the presence of a solvent such as an organic solvent. For example, the organic solvent may be selected from at least one of the following: alcohols such as methanol, ethanol, isopropanol and n-butanol; ethers such as ethyl propyl ether, n-butyl ether, anisole, phenetole, cyclohexylmethyl ether, dimethyl ether, diethyl ether, dimethyl glycol, diphenyl ether, dipropyl ether, diisopropyl ether, di-n-butyl ether, diisobutyl ether, diisoamyl ether, ethylene glycol dimethyl ether, isopropyl ethyl ether, methyl tert-butyl ether, tetrahydrofuran, methyltetrahydrofuran, dioxane, dichlorodiethyl ether, and polyethers of ethylene oxide and/or propylene oxide; aliphatic, cycloaliphatic or aromatic hydrocarbons such as pentane, hexane, heptane, octane, nonane, and those that may be substituted with a fluorine or chlorine atom, such as methylene chloride, dichloromethane, trichloromethane, tetrachloromethane, fluorobenzene, chlorobenzene or dichlorobenzene; cyclohexane, methylcyclohexane, petroleum ether, octane, benzene, toluene, chlorobenzene, bromobenzene, and xylene; and esters such as methyl acetate, ethyl acetate, butyl acetate, isobutyl acetate and dimethyl carbonate, dibutyl carbonate or ethylene carbonate.

According to an embodiment of the present disclosure, the preparation method may be carried out in the presence of a reducing agent; wherein the reducing agent is used for reducing a carbon-nitrogen double bond, and may be selected from sodium borohydride, potassium borohydride, lithium borohydride, sodium borohydride acetate, sodium cyanoborohydride and lithium aluminum hydride.

The present disclosure also provides a pharmaceutical composition comprising a therapeutically effective amount of at least one selected from the compound represented by formula (I) and the racemate, the stereoisomer, the tautomer, the isotopically labeled compound, the solvate, the polymorph, the pharmaceutically acceptable salt and the prodrug compound thereof.

According to an embodiment of the present disclosure, the pharmaceutical composition further comprises one or more pharmaceutically acceptable auxiliary materials.

According to an embodiment of the present disclosure, the pharmaceutical composition may further comprise one or more additional therapeutic agents.

The present disclosure also provides a method for treating a disease associated with activation of the complement alternative pathway, which comprises administering to a patient a prophylactically or therapeutically effective amount of at least one selected from the compound represented by formula (I) and the racemate, the stereoisomer, the tautomer, the isotopically labeled compound, the solvate, the polymorph, the pharmaceutically acceptable salt and the prodrug compound thereof.

The disease associated with activation of the complement alternative pathway comprises a disease selected from paroxysmal nocturnal hemoglobinuria (PNH), primary glomerulonephritis (IgAN), membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), diabetic retinopathy (DR), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory diseases and cardiovascular diseases and the like.

In some embodiments, the patient is a human.

The present disclosure also provides at least one selected from the compound represented by formula (I) and the racemate, the stereoisomer, the tautomer, the isotopically labeled compound, the solvate, the polymorph, the pharmaceutically acceptable salt and the prodrug compound thereof, or the pharmaceutical composition thereof, for diseases associated with activation of the complement alternative pathway.

The present disclosure also provides use of at least one selected from the compound represented by formula (I) and the racemate, the stereoisomer, the tautomer, the isotopically labeled compound, the solvate, the polymorph, the pharmaceutically acceptable salt and the prodrug compound thereof for the manufacturing of a medicament.

The medicament can be used for a disease associated with activation of the complement alternative pathway.

Where used as a medicament, the compound of the present disclosure can be administered in the form of a pharmaceutical composition. Said composition can be prepared using methods well known in the art of pharmacy and can be administered through a variety of routes, depending on whether topical or systemic treatment is needed and on the area to be treated. The administration may be topical administration (e.g., transdermal, dermal, ocular and mucosal administration, including intranasal, vaginal and rectal delivery), pulmonary administration (e.g., inhalation or insufflation of powders or aerosols, including using a nebulizer; intratracheal or intranasal administration), oral administration or parenteral administration. Parenteral administration comprises intravenous administration, intraarterial administration, subcutaneous administration, intraperitoneal administration or intramuscular injection or infusion; or intracranial administration, e.g., intrathecal or intraventricular administration. Parenteral administration may be performed at a single large dose, or may be performed using, for example, a continuous infusion pump. Pharmaceutical compositions and formulations for topical administration may comprise transdermal patches, ointments, lotions, creams, gels, drops, suppositories, sprays, liquids and powders. Conventional pharmaceutical carriers, water, powdered or oily bases and thickeners and the like may be necessary or desirable.

In preparing the composition of the present disclosure, the active ingredient is typically mixed with an excipient, diluted with an excipient or enclosed within such a carrier as capsules, sachets, paper or other container forms. Where the excipient serves as a diluent, it may be a solid, semi-solid, or liquid substance used as a vehicle, carrier, or medium for the active ingredient. Thus, the composition may be in the following forms: tablets, pills, powders, lozenges, sachets, cachets, elixirs, suspensions, emulsions, solutions, syrups, aerosols (solid or dissolved in a liquid vehicle); ointments, soft and hard gelatin capsules, suppositories, sterile solutions for injection and sterile packaged powders comprising, for example, up to 10% by weight of active compound.

Some examples of suitable excipients comprise lactose, glucose, sucrose, sorbitol, mannitol, starch, acacia, calcium phosphate, alginate, tragacanth, gelatin, calcium silicate, microcrystalline cellulose, polyvinylpyrrolidone, cellulose, water, syrup, and methylcellulose. Formulations may also comprise: lubricants such as talc, magnesium stearate and mineral oil; humectants; emulsifiers and suspending agents; preservatives such as methyl benzoate and hydroxypropyl benzoate; sweeteners and flavoring agents. The composition of the present disclosure can be formulated using known methods in the art so as to provide immediate release, sustained release or delayed release of the active ingredient upon being administered to patients.

The composition may be formulated in unit dosage form. Each dose comprises about 5-1000 mg, more typically about 100-500 mg, of the active ingredient. The term "unit dosage form" refers to physically isolated, single dosage units suitable for human patients and other mammals, each unit comprising a predetermined amount of active substance that can produce the desired therapeutic effects according to calculation and is mixed with a suitable pharmaceutical excipient.

The effective dose of the active compound may range widely. The active compound is generally administered in a pharmaceutically effective amount. It will be understood, however, that the amount of compound actually administered is generally determined by a physician in light of the relevant circumstances, including the condition to be treated, the chosen route of administration, the actual compound administered; the age, weight and response of the individual patient; the severity of symptoms in the patient, etc.

In preparing solid compositions such as tablets, the main active ingredient is mixed with pharmaceutical excipients to form a solid preformulation composition of a homogeneous mixture comprising the compound of the present disclosure. Where referring to these preformulation compositions as being homogeneous, it is meant that the active ingredient is generally distributed evenly throughout the composition so the compositions may be readily divided into equally effective unit dosage forms such as tablets, pills and capsules. The solid preformulation is then divided into unit dosage forms of the type described above comprising, for example, about 0.1-1000 mg of the active ingredient of the present disclosure.

The tablets or pills of the present disclosure may be coated or compounded to give a dosage form affording the advantage of prolonged action. For example, a tablet or pill comprises an inner dosage component and an outer dosage component, the latter being the coated form of the former. The two components can be separated by an enteric coating layer, which serves to resist disintegration in the stomach so that the inner component passes through the duodenum intact or that release is delayed. A variety of substances may be used for such enteric coating layers or coating agents. Such substances comprise various polymeric acids and mixtures of polymeric acids and such substances as shellac, cetyl alcohol and cellulose acetate.

Liquid forms for oral administration or injection administration in which the compound and composition of the present disclosure may be incorporated comprise aqueous solutions, a suitable flavoring syrup, aqueous or oil suspensions; and emulsions flavored with edible oils such as cottonseed oil, sesame oil, coconut oil or peanut oil; as well as elixirs and similar pharmaceutical vehicles.

Compositions for inhalation or insufflation comprise solutions and suspensions in pharmaceutically acceptable water or organic solvents or mixtures thereof, and powders. Liquid or solid compositions may comprise suitable pharmaceutically acceptable excipients as described above. In certain embodiments, the composition is administered through the oral or nasal respiratory route to achieve topical or systemic effects. The composition may be nebulized using an inert gas. Nebulized solution can be inhaled directly via a nebulizing device, or the nebulizing device can be connected to a mask or an intermittent positive pressure ventilator. Solution, suspension or powder compositions can be administered orally, or nasally via a device that delivers a formulation in a suitable manner.

The amount of the compound or composition administered to a patient is not fixed and depends on the drug administered, the purpose of the administration such as prevention or treatment; the condition of the patient, the route of administration, etc. In therapeutic applications, the composition may be administered to a patient that is suffering from a disease in an amount sufficient to cure or at least partially suppress the symptoms of the disease and its complications. The effective dosage should depend on the state of the disease to be treated and the judgment of the attending clinician, and the judgment depends on factors such as the severity of the disease, the age, weight and general condition of the patient, etc.

The composition administered to the patient may be in the form of the pharmaceutical composition described above. These compositions can be sterilized by using conventional sterilization techniques or by filtration. Aqueous solutions can be packaged for use as is, or lyophilized. The lyophilized formulation is mixed with a sterile aqueous carrier prior to administration. The pH of the compound formulation is usually 3-11, more preferably 5-9, and most preferably 7-8. It can be understood that the use of a certain excipient, carrier or stabilizer described above may result in the formation of a pharmaceutical salt.

The therapeutic dosage of the compound of the present disclosure can be determined, for example, according to: the specific use of the treatment, the route of administration of the compound, the health and condition of the patient, and the judgment of the prescriber. The proportion or concentration of the compound of the present disclosure in the pharmaceutical composition may vary and depends on a variety of factors including the dosage, the chemical properties (e.g., hydrophobicity), and the route of administration. For example, the compound of the present disclosure can be provided by a physiological buffered aqueous solution comprising about 0.1-10% w/v of the compound parenteral administration. Certain typical dosage ranges are from about 1 µg/kg to about 1 g/kg of body weight/day. In certain embodiments, the dosage range is from about 0.01 mg/kg to about 100 mg/kg of body weight/day. The dosage is likely to depend upon such variables as the type and extent of progression of the disease or condition, the general health of the particular patient, the relative biological potency of the compound selected, the excipient formulation and its route of administration. The effective dosage can be extrapolated from a dose-response curve derived from an *in vitro* or animal model test system.

### Advantageous Effects

The compound provided by the present disclosure has good regulation/inhibition effects on complement factor B, and can be used for the treatment of conditions or diseases associated with activation of the complement alternative pathway and for the manufacturing of a medicament for such conditions and diseases. In addition, the compound has good pharmacokinetics, liver microsomal stability and other properties.

### BRIEF DESCRIPTION OF THE DRAWINGS

FIG. 1 shows the experimental data (ng/mL) of the cynomolgus monkey plasma concentration curves in the biological examples.
FIG. 2 shows the experimental data (% relative to 0 h) of the cynomolgus monkey serum AP activity curves in the biological examples.
FIG. 3 shows the experimental data for *Streptococcus-induced* rheumatoid arthritis in rats in the biological examples.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be optionally combined and incorporated with each other. The definitions of groups and the structures of the compounds in such combinations and incorporations should be construed as being within the scope of the present specification and/or the claims.

Unless otherwise stated, a numerical range set forth in the description and claims shall be construed as at least including each specific integer within the range. For example, the numerical range "1-40" shall be construed as at least including each integer value in the numerical range "1-10", i.e., 1, 2, 3, 4, 5, 6, 7, 8, 9 and 10, and each integer value in the numerical range "11-40", i.e., 11, 12, 13, 14, 15,..., 35, 36, 37, 38, 39 and 40. Moreover, when certain numerical ranges are defined as "numbers", it shall be construed as including both endpoints of the range, each integer within the range, and each decimal within the range. For example, "numbers of 0-10" shall be construed as including not only each of integers 0, 1, 2, 3, 4, 5, 6, 7, 8, 9 and 10, but also at least the sums of each integer and 0.1, 0.2, 0.3, 0.4, 0.5, 0.6, 0.7, 0.8 or 0.9 respectively.

It should be understood that when one, two or more are described herein, "more" shall mean an integer greater than 2, e.g., an integer greater than or equal to 3, e.g., 3, 4, 5, 6, 7, 8, 9 or 10.

The term "halogen" refers to fluorine, chlorine, bromine and iodine.

The term "C₁₋₄₀ alkyl" preferably refers to a linear or branched saturated monovalent hydrocarbyl group having 1-40 carbon atoms. For example, "C₁₋₁₀ alkyl" refers to a linear or branched alkyl group having 1, 2, 3, 4, 5, 6, 7, 8, 9 or 10 carbon atoms and "C₁₋₆ alkyl" refers to a linear or branched alkyl group having 1, 2, 3, 4, 5 or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "C₂₋₄₀ alkenyl" preferably refers to a linear or branched monovalent hydrocarbyl comprising one or more double bonds and having 2-40 carbon atoms, preferably "C₂₋₁₀ alkenyl". "C₂₋₁₀ alkenyl" preferably refers to a linear or branched monovalent hydrocarbyl comprising one or more double bonds and having 2, 3, 4, 5, 6, 7, 8, 9 or 10 carbon atoms, for example, having 2, 3, 4, 5 or 6 carbon atoms (i.e., C₂₋₆ alkenyl) or having 2 or 3 carbon atoms (i.e., C₂₋₃ alkenyl). It should be understood that in the case that the alkenyl comprises more than one double bond, the double bonds can be separated from one another or conjugated. The alkenyl is, for example, vinyl, allyl, (E)-2-methylvinyl, (Z)-2-methylvinyl, (E)-but-2-enyl, (Z)-but-2-enyl, (*E*)-but-1-enyl, (*Z*)-but-1-enyl, pent-4-enyl, (E)-pent-3-enyl, (Z)-pent-3-enyl, (E)-pent-2-enyl, (Z)-pent-2-enyl, (*E*)-pent-1-enyl, (*Z*)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (*E*)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl or 1-isopropylvinyl.

The term "C₂₋₄₀ alkynyl" refers to a linear or branched monovalent hydrocarbyl comprising one or more triple bonds and having 2-40 carbon atoms, preferably "C₂₋₁₀ alkynyl". The term "C₂₋₁₀ alkynyl" preferably refers to a linear or branched monovalent hydrocarbyl comprising one or more triple bonds and having 2, 3, 4, 5, 6, 7, 8, 9 or 10 carbon atoms, for example, having 2, 3, 4, 5 or 6 carbon atoms (i.e., "C₂₋₆ alkynyl") or having 2 or 3 carbon atoms ("C₂₋₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl or prop-2-ynyl.

The term "C₃₋₄₀ cycloalkyl" refers to a saturated monovalent monocyclic or bicyclic (e.g., fused, bridged or spiro) hydrocarbon ring or tricyclic alkanyl having 3-40 carbon atoms, preferably "C₃₋₁₀ cycloalkyl". The term "C₃₋₁₀ cycloalkyl" refers to a saturated monovalent monocyclic or bicyclic (e.g., fused, bridged or spiro) hydrocarbon ring or tricyclic alkanyl having 3, 4, 5, 6, 7, 8, 9 or 10 carbon atoms. The C₃₋₁₀ cycloalkyl may be monocyclic hydrocarbyl such as cyclopropyl, cyclobutyl, cyclopentyl, cyclohexyl, cycloheptyl, cyclooctyl, cyclononyl or cyclodecyl, or bicyclic hydrocarbyl such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, 2,6-diazaspiro[3,4]octyl, or tricyclic hydrocarbyl such as adamantyl.

Unless otherwise defined, the term "3- to 20-membered heterocyclyl" refers to a saturated or unsaturated non-aromatic ring or ring system; for example, it is a 4-, 5-, 6- or 7-membered monocyclic ring system, a 7-, 8-, 9-, 10-, 11- or 12-membered bicyclic (e.g., fused, bridged or spiro) ring system or a 10-, 11-, 12-, 13-, 14- or 15-membered tricyclic ring system, and comprises at least one, e.g., 1, 2, 3, 4, 5 or more heteroatoms selected from O, S and N, wherein N and S may also be optionally oxidized to various oxidized forms to form nitrogen oxides,-S(O)- or -S(O)₂-. Preferably, the heterocyclyl may be selected from "3- to 10-membered heterocyclyl". The term "3- to 10-membered heterocyclyl" refers to a saturated or unsaturated non-aromatic ring or ring system and comprises at least one heteroatom selected from O, S and N. The heterocyclyl may be connected to the rest of the molecule through any of the carbon atoms or the nitrogen atom (if present). The heterocyclyl may comprise fused or bridged rings as well as spiro rings. In particular, the heterocyclyl may comprise, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. Heterocyclyl may be partially unsaturated, i.e., it may comprise one or more double bonds, for example, but not limited to, dihydrofuranyl, dihydropyranyl, 2,5-dihydro-1*H*-pyrrolyl, 4*H*-[1,3,4]thiadiazinyl, 4,5-dihydrooxazolyl or *4H-*[1,4]thiazinyl, or it may be benzo-fused, for example, but not limited to, dihydroisoquinolyl. Where the 3- to 20-membered heterocyclyl is connected to another group to form the compound of the present disclosure, the group may be connected to the carbon atom on the 3- to 20-membered heterocyclyl, or may be connected to the heteroatom on the 3- to 20-membered heterocyclyl. For example, where the 3- to 20-membered heterocyclyl is selected from piperazinyl, the group may be connected to the nitrogen atom on the piperazinyl. Alternatively, when the 3- to 20-membered heterocyclyl is selected from piperidyl, the group may be connected to the nitrogen atom on the piperidyl or the carbon atom in the para position.

The term "C₆₋₂₀ aryl" preferably refers to an aromatic or partially aromatic monovalent monocyclic, bicyclic (e.g., fused, bridged or spiro) or tricyclic hydrocarbon ring having 6-20 carbon atoms, which may be a single aromatic ring or multiple aromatic rings fused together, preferably "C₆₋₁₄ aryl". The term "C₆₋₁₄ aryl" preferably refers to an aromatic or partially aromatic monovalent monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl or biphenyl, a ring having 9 carbon atoms ("C₉ aryl"), such as indanyl or indenyl, a ring having 10 carbon atoms ("C₁₀ aryl"), such as tetrahydronaphthyl, dihydronaphthyl or naphthyl, a ring having 13 carbon atoms ("C₁₃ aryl"), such as fluorenyl, or a ring having 14 carbon atoms ("C₁₄ aryl"), such as anthryl. When the C₆₋₂₀ aryl is substituted, it may be monosubstituted or polysubstituted. In addition, the substitution site is not limited, and may be, for example, ortho-substitution, para-substitution, or meta-substitution.

The term "5- to 20-membered heteroaryl" refers to a monovalent aromatic monocyclic, bicyclic (e.g., fused, bridged or spiro) or tricyclic ring system which has 5-20 ring atoms and comprises 1-5 heteroatoms independently selected from N, O and S, such as "5- to 14-membered heteroaryl". The term "5- to 14-membered heteroaryl" refers to a monovalent aromatic monocyclic, bicyclic or tricyclic ring system that has 5, 6, 7, 8, 9, 10, 11, 12, 13 or 14 ring atoms, in particular 5, 6, 9 or 10 carbon atoms, comprises 1-5, preferably 1-3 heteroatoms independently selected from N, O and S, and may be benzo-fused in each case. "Heteroaryl" also refers to a group in which a heteroaromatic ring is fused to one or more aryl, alicyclic or heterocyclyl rings, wherein the radical or site of attachment is on the heteroaromatic ring. Non-limiting examples of the term heteroaryl comprise, for example, pyridinyl, pyrazinyl, furanyl, thienyl, pyrimidinyl, isoxazolyl, isothiazolyl, oxazolyl, thiazolyl, pyrazolyl, furazanyl, pyrrolyl, pyrazolyl, triazolyl, tetrazolyl, 1,2,4-thiadiazolyl, and pyridazinyl; as well as 1-, 2-, 3-, 5-, 6-, 7- or 8-indolizinyl, 1-, 3-, 4-, 5-, 6- or 7-isoindolyl, 2-, 3-, 4-, 5-, 6- or 7-indolyl, 2-, 3-, 4-, 5-, 6- or 7-indazolyl, 2-, 4-, 5-, 6-, 7- or 8-purinyl, 1-, 2-, 3-, 4-, 6-, 7-, 8- or 9-quinolizinyl, 2-, 3-, 4-, 5-, 6-, 7- or 8-quinolyl, 1-, 3-, 4-, 5-, 6-, 7- or 8-isoquinolyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6-or 7-pteridinyl, 1-, 2-, 3-, 4-, 5-, 6-, 7- or 8-4a*H*-carbazolyl, 1-, 2-, 3-, 4-, 5-, 6-, 7- or 8-carbazolylcarbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-pyridinyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl, 2-, 3-, 4-, 5-, 6- or 1-, 3-, 4-, 5-, 6-, 7-, 8-, 9- or 10-benzisoquinolyl, 2-, 3-, 4- or thieno[2,3-b]furanyl, 2-, 3-, 5-, 6-, 7-, 8-, 9-, 10- or 11-7*H-*pyrazino[2,3-*c*]carbazolyl, 2-, 3-, 5-, 6- or 7-2*H*-furo[3,2-*b*]-pyranyl, 2-, 3-, 4-, 5-, 7- or 8-5*H*-pyrido[2,3-*d*]-*o*-oxazinyl, 1-, 3- or *5-1H-*pyrazolo[4,3-*d* ]-thiazolyl, 2-, 4- or 5-1*H*-imidazo[4,5-*d*]thiazolyl, 3-, 5- or 8-pyrazino[2,3-d]pyridazinyl, 2-, 3-, 5- or 6-imidazo[2,1-*b*]thiazolyl, 1-, 3-, 6-, 7-, 8- or 9-furo[3,4-c]cinnolinyl, 1-, 2-, 3-, 4-, 5-, 6-, 8-, 9-, 10- or 11-4*H*-pyrido[2,3-*c*]carbazolyl, 2-, 3-, 6- or 7-imidazo[1,2-*b*][1,2,4]triazinyl, 7-benzo[*b*]thienyl, 2-, 4-, 5-, 6- or 7-benzoxazolyl, 2-, 4-, 5-, 6- or 7-benzimidazolyl, 2-, 4-, 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoazinyl, 1-, 2-, 3-, 5-, 6-, 7-, 8-, 9-, 10- or 11*-1H-*pyrrolo[1,2-b][2]benzazapinyl. Typical fused heteroaryl groups comprise, but are not limited to, 2-, 3-, 4-, 5-, 6-, 7- or 8-quinolyl, 1-, 3-, 4-, 5-, 6-, 7- or 8-isoquinolyl, 2-, 3-, 4-, 5-, 6- or 7-indolyl, 2-, 3-, 4-, 5-, 6- or 7-benzo[b]thienyl, 2-, 4-, 5-, 6- or 7-benzoazolyl, 2-, 4-, 5-, 6- or 7-benzimidazolyl and 2-, 4-, 5-, 6- or 7-benzothiazolyl. When the 5- to 20-membered heteroaryl is connected to another group to form the compound of the present disclosure, the group may be connected to the carbon atom on the 5- to 20-membered heteroaryl ring, or may be connected to the heteroatom on the 5- to 20-membered heteroaryl ring. When the 5- to 20-membered heteroaryl is substituted, it may be monosubstituted or polysubstituted. In addition, the substitution site is not limited. For example, hydrogen connected to the carbon atom on the heteroaryl ring may be substituted, or hydrogen connected to the heteroatom on the heteroaryl ring may be substituted.

The term "spiro rings" refers to a ring system in which two rings share 1 ring-forming atom.

The term "fused rings" refers to a ring system in which two rings share 2 ring atoms.

The term "bridged rings" refers to a ring system in which two rings share 3 or more ring-forming atoms.

Unless otherwise stated, the heterocyclyl, heteroaryl or heteroarylene comprises all possible isomeric forms thereof, e.g., positional isomers thereof. Thus, for some illustrative non-limiting examples, forms that involving substitutions at or bonding to other groups at one, two or more of positions 1, 2, 3, 4, 5, 6, 7, 8, 9, 10, 11, 12 and the like (if present) are comprised, including pyridin-2-yl, pyridinylene-2-yl, pyridin-3-yl, pyridinylene-3-yl, pyridin-4-yl and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl and pyrazol-5-yl.

The term "oxo" means that the carbon atom, nitrogen atom or sulfur atom in the substituent is substituted with an oxy group formed after oxidation (=O).

Definitions of terms herein apply equally to groups comprising the term unless otherwise stated. For example, the definition of C₁₋₆ alkyl also applies to C₁₋₆ alkyloxy and C₃₋₈ cycloalkyl-C₁₋₆ alkyl-.

It will be understood by those skilled in the art that the compound represented by formula (I) may be present in the form of various pharmaceutically acceptable salts. If these compounds have basic centers, they can form acid addition salts; if these compounds have acidic centers, they can form base addition salts; if these compounds comprise both acidic centers (e.g., carboxyl) and basic centers (e.g., amino), they can also form internal salts.

The compound disclosed herein may exist in the form of a solvate (e.g., hydrate), and the compound disclosed herein comprises a polar solvent as a structural element of the crystal lattice of the compound, particularly, for example, water, methanol or ethanol. The amount of polar solvent, especially water, can exist in a stoichiometric or non-stoichiometric ratio.

According to the molecular structure, the compounds disclosed herein may be chiral and may therefore exist in various enantiomeric forms. These compounds may therefore exist in racemic or optically active form. The compounds disclosed herein encompass isomers with each chiral carbon in R or S configuration, or mixtures and racemates thereof. The compounds disclosed herein or intermediates thereof may be separated into enantiomers by chemical or physical methods well known to those skilled in the art, or used in this form for synthesis. In the case of racemic amines, diastereoisomers are manufactured from mixtures by reaction with optically active resolving agents. Examples of suitable resolving agents are optically active acids such as R- or S-tartaric acid, diacetyltartaric acid, dibenzoyltartaric acid, mandelic acid, malic acid, lactic acid, suitable N-protected amino acids (e.g., N-benzoylproline or *N-*benzenesulfonylproline) or various optically active camphorsulfonic acids. Enantiomeric resolution by chromatography can be advantageously performed with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvent comprising water or alcohol, for example, hexane/isopropanol/acetonitrile.

The corresponding stable isomers can be separated according to known methods, such as extraction, filtration or column chromatography.

The term "patient" refers to any animal including mammals, preferably mice, rats, other rodents, rabbits, dogs, cats, pigs, cattle, sheep, horses or primates, and the most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition, (i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms).

### DETAILED DESCRIPTION

The technical solution of the present disclosure will be further illustrated in detail with reference to the following specific examples. It should be understood that the following examples are illustrative statements and interpretations of the present disclosure only, and should not be construed as limiting the scope of protection of the present disclosure. All techniques implemented based on the content of the present disclosure described above are encompassed within the protection scope of the present disclosure.

Unless otherwise stated, the starting materials and reagents used in the following examples are all commercially available products or can be prepared by using a known method.

The structures of the following compounds were determined by nuclear magnetic resonance (NMR) and/or mass spectrometry (MS). The NMR shifts (*δ*) were given in 10⁻⁶ (ppm). The NMR measurement was performed by using Bruker ASCEND^{™}-400 NMR spectrometer, with deuterated dimethyl sulfoxide (DMSO-*d*₆), deuterated chloroform (CDCl₃) and deuterated methanol (CD₃OD) as solvents and tetramethylsilane (TMS) as an internal standard.

The MS measurement was performed by using Agilent 6110, Agilent 1100, Agilent 6120 and AgilentG6125B liquid chromatography-mass spectrometers.

The HPLC measurement was performed by using Shimadzu HPLC-2010C high-pressure liquid chromatograph (XBRIDGE 2.1 × 50 mm, 3.5 µm column).

The chiral HPLC analysis was performed by using THARSFC X5.

Yantai Qingdao GF254 silica gel plates were used as thin-layer chromatography silica gel plates: 0.15 mm to 0.2 mm silica gel plates were used for thin-layer chromatography (TLC) analysis, and 0.4 mm to 0.5 mm silica gel plates for thin-layer chromatography product separation and purification.

Qingdao Haiyang 200-300 mesh silica gel was generally used as the carrier in column chromatography.

Waters 2767, Waters 2545 and a Chuangxintongheng LC3000 preparative chromatograph were used in preparative high performance liquid chromatography.

A Beijing Jiawei Kechuang Technology GCD-500G hydrogen generator was used in pressurized hydrogenation reactions.

A Biotage initiator+ microwave reactor was used in microwave reactions.

The reactions were all carried out under argon atmosphere or nitrogen atmosphere unless otherwise specified. The argon atmosphere or nitrogen atmosphere means that the reaction flask was connected to a balloon with about 1 liter of argon or nitrogen gas. The hydrogen atmosphere means that the reaction flask was connected to a balloon with about 1 liter of hydrogen gas.

The reaction temperature was at room temperature and ranged from 20 °C to 30 °C unless otherwise specified.

### Example 1

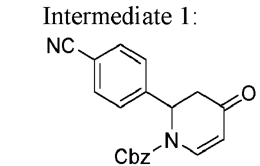

A 3 L three-necked flask was successively added tetrahydrofuran (150 mL) and 4-bromoxynil (50 g). Isopropylmagnesium chloride lithium chloride complex (1.3 M, 210 mL) was slowly added to the reaction system under nitrogen atmosphere. The reaction mixture was reacted at room temperature for 2 h. Then the reaction system was added with anhydrous tetrahydrofuran (500 mL) for dilution and cooled to -5 °C, and 4-methoxypyridine (25 mL) was added, followed by the slow dropwise addition of benzyl chloroformate (35 mL) (the system temperature was maintained at below 0 °C). After the dropwise addition was completed, the reaction mixture was stirred at 0 °C for 2 h, and then warmed to room temperature and successively reacted at room temperature for 16 h. After the reaction was completed, 6 M hydrochloric acid (150 mL) was added. The mixture was stirred for half an hour, added with water (1000 mL) for dilution, and extracted twice with ethyl acetate (500 mL). The combined extract phases were washed with saturated brine (50 mL), dried with anhydrous sodium sulfate and filtered. The filtrate was concentrated, and the resulting crude product was purified by column chromatography (petroleum ether:ethyl acetate = 3:1 to 1:1) to give intermediate 1 (23 g, yield: 23%). MS m/z (ESI): 333.0[M+1]. Intermediate 2:

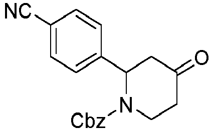

Intermediate 1 (28 g), zinc powder (55 g) and acetic acid (200 mL) were successively added to a 500 mL single-neck flask. The reaction mixture was heated to 100 °C and stirred at that temperature for 16 h. After the reaction was completed, the mixture was filtered. The filtrate was added with water (500 mL) for dilution and extracted with ethyl acetate (500 mL). The extract phase was washed twice with saturated aqueous sodium bicarbonate solution (500 mL), washed once with saturated brine (100 mL), dried over anhydrous sodium sulfate and filtered. The filtrate was concentrated under reduced pressure to give intermediate 2 (26 g, yield: 73%). MS m/z (ESI): 334.8[M+1]. Intermediate 3:

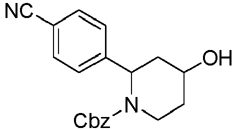

A 500 mL single-neck flask was successively added tetrahydrofuran (100 mL), ethanol (100 mL) and intermediate 2 (26 g), and sodium borohydride (2 g) was added in batches. The reaction mixture was reacted at room temperature for 2 h. After the reaction was completed, the system was cooled to 0 °C, and saturated aqueous ammonium chloride solution (30 mL) was added until the temperature did not increase any more. Water (500 mL) was added for dilution, followed by the extraction twice with ethyl acetate (200 mL). The combined extract phases were washed with saturated brine (500 mL), dried over anhydrous sodium sulfate and filtered. The filtrate was concentrated under reduced pressure to give intermediate 3 (25 g, yield: 76%).

MS m/z (ESI): 336.9[M+1].

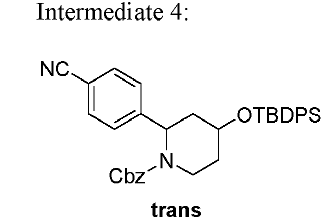

Dichloromethane (200 mL) was added to a 500 mL single-neck flask, and then intermediate 3 (25 g), imidazole (6.6 g) and tert-butyldiphenylchlorosilane (25 g) were successively added. The mixture was reacted at room temperature for 2 h. After the reaction was completed, the reaction liquid was washed with water (500 mL), dried over anhydrous sodium sulfate and filtered. The filtrate was concentrated under reduced pressure. The residue was purified by column chromatography (petroleum ether: ethyl acetate = 10:1) to give intermediate 4 (5.7 g, yield: 13%, R_{f} = 0.55; *trans-isomer* R_{f} = 0.50). MS m/z (ESI): 597.0[M+23].

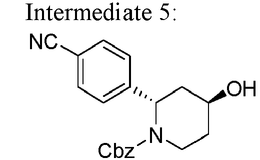

A 250 mL single-neck flask was successively added intermediate 4 (5 g) and a solution of tetrabutylammonium fluoride in tetrahydrofuran (1 M, 30 mL). The reaction mixture was reacted at room temperature for 2 h. After the reaction was completed, water (100 mL) was added for dilution, followed by the extraction with ethyl acetate (50 mL × 3). The combined extract phases were washed with saturated brine (100 mL), dried over anhydrous sodium sulfate and filtered. The filtrate was concentrated under reduced pressure. The residue was purified by column chromatography (petroleum ether: ethyl acetate = 3:1 to 0:1) to give a racemic intermediate. The intermediate was subjected to SFC chiral resolution (Apparatus: SFC Thar prep 80; Column: CHIRALPAK AD-H, 250 mm × 20 mm, 5 µm; Modifier: 35% methanol (0.2% aqueous ammonia); column temperature: 40 °C; column pressure: 60 bar; wavelength: 214/254 nm; flow rate: 40 g/min; Rt = 4.78 min) to give intermediate 5 (1.2 g, yield: 41%). MS m/z (ESI): 358.8[M+23].

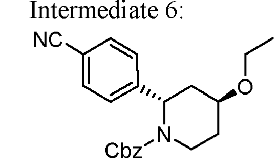

A 100 mL single-neck flask was successively added *N*,*N*-dimethylformamide (15 mL) as a solvent, intermediate 5 (1.2 g) and iodoethane (1.1 g). After the reaction system was cooled to 0 °C, sodium hydride (60%, 243 mg) was added. Then the system was warmed to room temperature and reacted at that temperature for 2 h. After the reaction was completed, water (30 mL) was added for dilution, followed by the extraction with ethyl acetate (50 mL). The extract phase was washed with saturated brine (10 mL), dried over anhydrous sodium sulfate and filtered. The filtrate was concentrated under reduced pressure to give intermediate 6 (1.2 g, yield: 83%). MS m/z (ESI): 386.9[M+23]. Intermediate 7:

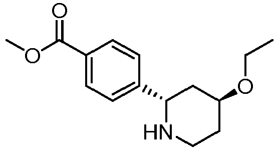

A 100 mL single-neck flask was successively added methanol (10 mL), water (10 mL), concentrated sulfuric acid (10 mL) and intermediate 6 (1.2 g). The reaction mixture was heated to 80 °C and reacted at that temperature for 48 h. After the reaction was completed, the reaction liquid was concentrated to remove methanol. The residue was made neutral with aqueous sodium hydroxide solution (2 M) and extracted with ethyl acetate (10 mL × 3). The combined extract phases were washed with saturated brine (5 mL), dried over anhydrous sodium sulfate and filtered. The filtrate was concentrated under reduced pressure to give intermediate 7 (850 mg, yield: 81%). MS m/z (ESI): 264.1[M+1]. ¹H NMR (400 MHz, CDCl₃) *δ* 8.01 (d, *J=* 8.3 Hz, 2H), 7.49 (d, *J=* 8.3 Hz, 2H), 4.13 (dd, *J=* 11.7, 2.4 Hz, 1H), 3.92 (s, 3H), 3.82-3.70 (m, 1H), 3.62-3.47 (m, 2H), 3.27-3.10 (m, 1H), 3.02-2.88 (m, 1H), 2.07-1.97 (m, 1H), 1.95-1.85 (m, 1H), 1.82-1.62 (m, 2H), 1.27 (t, *J=* 7.0 Hz, 3H).

Alternatively, the intermediate 7 was obtained by using the following method:

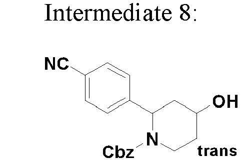

A 2 L three-necked flask was successively added a solution of tetrabutylammonium fluoride in tetrahydrofuran (1 M, 840 mL) and intermediate 4 (140 g). The reaction mixture was reacted at room temperature for 2 h. After the reaction was completed, water (600 mL) was added for dilution, followed by the extraction with ethyl acetate (700 mL × 3). The extract phase was washed with saturated brine (500 mL), dried over anhydrous sodium sulfate and filtered. The filtrate was concentrated under reduced pressure. The residue was purified by column chromatography (petroleum ether:ethyl acetate = 3:1 to 1:1) to give intermediate 8 (77 g, yield: 95%), MS m/z (ESI): 358.8[M+23].

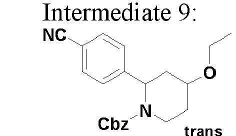

A 2 L three-necked flask was successively added N,N-dimethylformamide (700 mL) as a solvent, intermediate 8 (77 g) and iodoethane (56 g). After the reaction system was cooled to 0 °C, sodium hydride (60%, 14.61 g) was added. Then the system was warmed to room temperature and reacted at that temperature for 2 h. After the reaction was completed, the temperature was lowered to 0 °C, and aqueous ammonium chloride solution was added until the temperature of the reaction mixture did not increase. Ethyl acetate (500 mL) was added for extraction. The extract phase was washed with saturated brine (300 mL), dried over anhydrous sodium sulfate and filtered. The filtrate was concentrated under reduced pressure to give intermediate 9 (75 g, yield: 89%). MS m/z (ESI): 386.9[M+23].

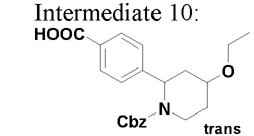

A 2 L three-necked flask was successively added isopropanol (300 mL), water (800 mL), intermediate 9 (75 g) and Ba(OH)₂·8H₂O (233 g). The reaction mixture was heated to 100 °C and reacted at that temperature for 20 h. After the reaction was completed, the reaction liquid was concentrated to remove isopropanol. The residue was adjusted to pH 2-3 with saturated aqueous sodium hydroxide solution and extracted with dichloromethane (300 mL × 3). The extract phase was washed with saturated brine (200 mL), dried over anhydrous sodium sulfate and filtered. The filtrate was concentrated under reduced pressure to give intermediate 10 (67 g, yield: 85%). MS m/z (ESI): 384.1[M+1].

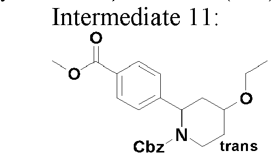

A 2 L three-necked flask was successively added N,N-dimethylformamide (670 mL), potassium carbonate (96.6 g), iodomethane (37.3 g) and intermediate 10 (67 g). The mixture was reacted at room temperature for 2 h. After the reaction was completed, 300 mL of water was added to quench the reaction, followed by the extraction with methyl tert-butyl ether (300 mL × 2). The extract phase was washed with saturated brine (5 mL), dried over anhydrous sodium sulfate and filtered. The filtrate was concentrated under reduced pressure. The residue was purified by column chromatography (petroleum ether:ethyl acetate = 10:1 to 3:1) to give intermediate 11 (54 g, yield: 78%). MS m/z (ESI): 394.1[M+1].

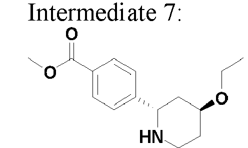

A 1 L single-neck flask was successively added ethyl acetate (500 mL), palladium on carbon (5.4 g, 10% loading) and intermediate 11 (54 g). The reaction mixture was reacted under one bar pressure of hydrogen at room temperature for 16 h. After the reaction was completed, the reaction liquid was added to celite for filtration. The filtrate was concentrated under reduced pressure to give a racemic intermediate. The intermediate was subjected to chiral resolution (Apparatus: Shimadzu LC-20AD; Column: CHIRALPAK AD-H(ADH0CD-SK003), 0.46 cm I.D. × 25 cm L; Modifier: (methanol/diethylamine 0.1%)/CO₂ = 25/75 (V/V); flow rate: 2.0 mL/min; Rt = 3.58 min) to give intermediate 7 (15.7 g, yield: 43%). MS m/z (ESI): 264.0[M+1]. ¹H NMR (400 MHz, DMSO-*d₆*) *δ* 7.89 (d, *J* = 8.26 Hz, 2H),7.50 (d, *J* = 8.26 Hz, 2H), 3.92 (dd, *J* = 11.36, 2.32 Hz, 1H), 3.84 (s, 3H), 3.69-3.64 (m, 1H), 3.51-3.42 (m, 2H), 2.94 (dt, *J* = 12.15, 2.56 Hz, 1H), 2.76 (ddd, *J* = 11.62, 4.24, 2.62 Hz, 1H), 1.85 (dd, *J* = 13.23, 2.16 Hz, 1H), 1.73 (d, *J =* 13.47 Hz, 1H), 1.59-1.41 (m, 2H), 1.16 (t, *J=* 6.98 Hz, 3H).

### Example 2

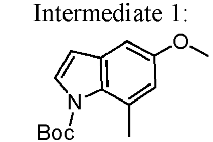

A 250 mL single-neck flask was successively added dichloromethane (50 mL), 5-methoxy-7-methyl-1*H*-indole (3 g), Boc anhydride (5.68 g), 4-dimethylaminopyridine (227 mg) and triethylamine (2.26 g). The reaction mixture was reacted at room temperature for 16 h. After the reaction was completed, the reaction liquid was quenched by adding saturated ammonium chloride solution (5 mL) and extracted three times with dichloromethane (20 mL). The combined organic phases were washed with water (5 mL), dried over anhydrous sodium sulfate and filtered. The filtrate was concentrated. The residue was purified by column chromatography (petroleum ether:ethyl acetate = 10:1) to give intermediate 1 (4.6 g, yield: 94%). MS m/z (ESI): 262.0[M+1].

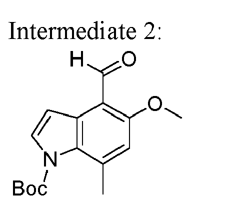

A 250 mL single-neck flask was successively added dichloromethane (80 mL), N-methylformanilide (3.8 g) and oxalyl chloride (3.6 g). The reaction mixture was stirred at room temperature for 3 h. Then the reaction temperature was lowered to -14 °C, and intermediate 1 (2.5 g) was added. The reaction system was naturally warmed to room temperature and stirred at room temperature for 1 h. After the reaction was completed, the reaction liquid was poured into ice water (100 mL) and extracted with dichloromethane (100 mL × 3). The combined extract phases were washed with water (10 mL × 2), dried over anhydrous sodium sulfate and filtered. The filtrate was concentrated. The residue was purified by column chromatography (petroleum ether:ethyl acetate = 20:1) to give intermediate 2 (1.3 g, yield: 47%). MS m/z (ESI): 290.0[M+1]. ¹H NMR (400 MHz, CDCl₃) *δ* 10.65 (s, 1H), 7.65 (d, *J* = 3.4 Hz, 1H), 7.49 (d, *J=* 3.4 Hz, 1H), 6.76 (s, 1H), 3.98 (s, 3H), 2.70 (s, 3H), 1.65 (s, 9H).

### Example 3

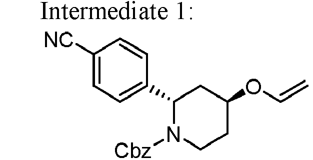

A 100 mL single-neck flask was successively added 1-(vinyloxy)butane (10 mL), triethylamine (300 mg), o-phenanthroline (54 mg), palladium acetate (67 mg) and benzyl (2*S*,4*S*)-2-(4-cyanophenyl)-4-hydroxypiperidine-1-carboxylate (Example 1, intermediate 5) (500 mg). The reaction mixture was heated to 90 °C under nitrogen atmosphere and stirred at that temperature for 16 h. After the reaction was completed, the reaction liquid was directly concentrated under reduced pressure. The residue was purified by column chromatography (petroleum ether:ethyl acetate = 1:1) to give intermediate 1 (360 mg, yield: 63%). MS m/z (ESI): 384.8[M+23].

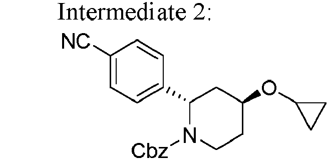

A solution of trifluoroacetic acid (228 mg) in dichloromethane (2 mL) was added to a solution of diethyl zinc (1 M, 2 mL) in dichloromethane (4 mL) with cooling in an ice bath under nitrogen atmosphere. After the reaction mixture was reacted in the ice bath for one hour, a solution of diiodomethane (536 mg) in dichloromethane (2 mL) was added to the reaction system. The mixture was reacted for 1 h. Then a solution of intermediate 5 (362 mg) of Example 1 in dichloromethane (2 mL) was added. The reaction mixture was naturally warmed to room temperature and successively stirred at room temperature for 18 h. After the reaction was completed, it was quenched with diluted hydrochloric acid (0.1 M, 10 mL). Water (20 mL) was added for dilution, followed by the extraction with ethyl acetate (30 mL). The extract phase was washed with saturated brine (20 mL), dried over anhydrous sodium sulfate and filtered. The filtrate was concentrated under reduced pressure. The residue was purified by column chromatography (petroleum ether:ethyl acetate = 1:1) to give intermediate 2 (300 mg, yield: 64%). MS m/z (ESI): 398.8[M+23].

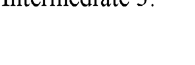

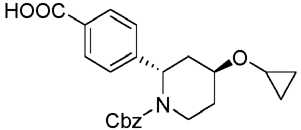

Sodium hydroxide (320 mg) was added to a mixed solution of intermediate 2 (300 mg) in isopropanol (2 mL) and water (5 mL). The reaction system was heated to 100 °C and stirred at that temperature for 48 h. After the reaction was completed, diluted hydrochloric acid (1 M) was added to the reaction liquid with cooling in an ice bath to adjust the pH to 5-6. Water (10 mL) was added for dilution, followed by the extraction with ethyl acetate (10 mL). The extract phase was washed with saturated brine (10 mL), dried over anhydrous sodium sulfate and filtered. The filtrate was concentrated under reduced pressure to give intermediate 3 (180 mg, yield: 45%). MS m/z (ESI): 395.9[M+1].

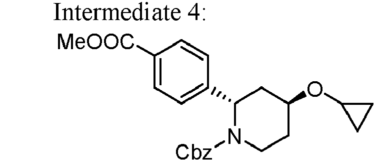

A solution of intermediate 3 (180 mg) in acetonitrile (3 mL) was successively added potassium carbonate (126 mg) and iodomethane (129 mg). The reaction liquid was heated to 50 °C and stirred at that temperature for 16 h. After the reaction was completed, the reaction liquid was concentrated. The residue was purified by column chromatography (petroleum ether: ethyl acetate = 3:1) to give intermediate 4 (130 mg, yield: 62%). MS m/z (ESI): 431.8[M+23].

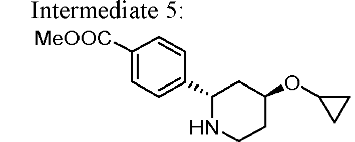

A solution of intermediate 4 (120 mg) in tetrahydrofuran (2 mL) was added palladium/carbon (20 mg). The reaction liquid was subjected to a catalytic hydrogenation reaction under hydrogen atmosphere at room temperature for 16 h. After the reaction was completed, the reaction liquid was directly filtered and concentrated under reduced pressure to give intermediate 5 (70 mg, yield: 79%). MS m/z (ESI): 275.9[M+1].

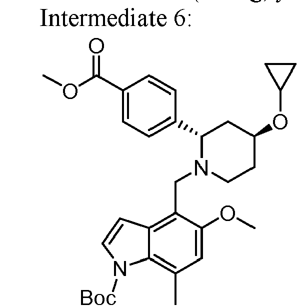

Intermediate 5 (70 mg) was added to a solution of intermediate 2 (88 mg) of Example 2 in 1,2-dichloroethane (5 mL). The reaction mixture was stirred at room temperature for 8 h. Then sodium borohydride acetate (162 mg) was added, and the mixture was successively reacted for 16 h. After the reaction was completed, the reaction liquid was concentrated under reduced pressure. The residue was purified by column chromatography (dichloromethane:methanol = 20:1) to give intermediate 7 (170 mg, yield: 73%). MS m/z (ESI): 548.8[M+1].

### Target compound:

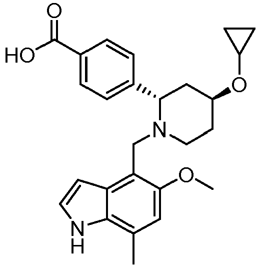

Sodium hydroxide (127 mg) was added to a mixed solution of intermediate 6 (175 mg) in methanol (2 mL) and water (2 mL). The reaction liquid was heated to 75 °C and reacted at that temperature for 3 h. After the reaction was completed, the reaction liquid was made neutral by adding hydrochloric acid (1 M) with cooling in an ice bath. Then the mixture was directly purified by preparative high-pressure liquid chromatography (column: Gemini-C18 150 × 21.2 mm, 5 µm; mobile phase: acetonitrile-water (0.1% formic acid); gradient: 20-40%) to give the target compound (31.5 mg, yield: 22%, comprising 0.5 equivalents of formic acid). MS m/z (ESI): 434.9[M+1]. ¹H NMR (400 MHz, CD₃OD) δ 8.44 (s, 0.5H), 8.16 (d, *J =* 7.9 Hz, 2H), 7.65 (d, *J* = 7.9 Hz, 2H), 7.31 (d, *J* = 3.1 Hz, 1H), 6.75 (s, 1H), 6.33 (s, 1H), 4.80-4.62 (m, 1H), 4.43-4.09 (m, 2H), 4.03-3.86 (m, 1H), 3.74 (s, 3H), 3.54-3.40 (m, 2H), 3.40-3.31 (m, 1H), 2.50 (s, 3H), 2.36-2.17 (m, 2H), 2.14-1.93 (m, 2H), 0.72-0.47 (m, 4H).

### Example 4:

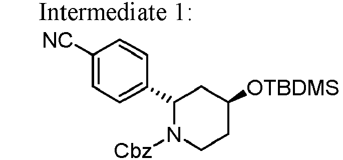

A solution of intermediate 5 (500 mg) of Example 1 in N,N-dimethylformamide (10 mL) was added imidazole (202 mg) and tert-butyldimethylchlorosilane (270 mg). The reaction mixture was stirred at room temperature for 2 h. After the reaction was completed, the reaction liquid was added with water (50 mL) for dilution and extracted with ethyl acetate (20 mL). The organic phase was washed with saturated brine (50 mL), dried over anhydrous sodium sulfate and filtered. The filtrate was directly concentrated to give intermediate 1 (700 mg, yield: 88%). MS m/z (ESI): 472.8[M+23].

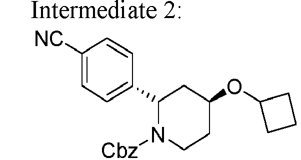

Intermediate 1 (750 mg) was added to dichloromethane (10 mL). The reaction system was cooled to -78 °C under nitrogen atmosphere. Cyclobutanone (117 mg) and trimethylsilyl trifluoromethanesulfonate (37 mg) were successively added. After the reaction mixture was stirred at -78 °C for one hour, triethylsilane (193 mg) was added. Then the reaction mixture was slowly warmed to room temperature and stirred at that temperature for 16 h. After the reaction was completed, the reaction liquid was quenched by adding saturated aqueous sodium bicarbonate solution (10 mL), added with water (10 mL) for dilution and extracted with dichloromethane (10 mL). The extract phase was washed once with water (10 mL), dried over anhydrous sodium sulfate and filtered. The filtrate was concentrated. The residue was purified by column chromatography (petroleum ether:ethyl acetate = 3:1) to give intermediate 2 (700 mg, yield: 86%). MS m/z (ESI): 391.0[M+1].

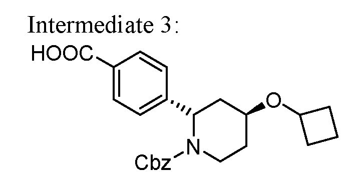

A mixed solution of intermediate 2 (700 mg) in isopropanol (5 mL) and water (10 mL) was added sodium hydroxide (720 mg). The reaction system was heated to 100 °C and stirred at that temperature for 48 h. After the reaction was completed, diluted hydrochloric acid (1 M) was added to the reaction liquid with cooling in an ice bath to adjusted the pH to 5-6. Water (20 mL) was added for dilution, followed by the extraction with ethyl acetate (20 mL). The extract phase was washed once with saturated brine (20 mL), dried over anhydrous sodium sulfate and filtered. The filtrate was concentrated under reduced pressure to give intermediate 3 (700 mg, yield: 76%). MS m/z (ESI): 409.9[M+1].

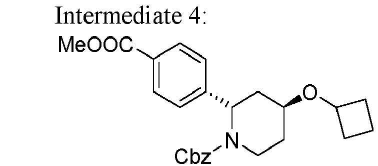

Potassium carbonate (472 mg) and iodomethane (486 mg) were added to a solution of intermediate 3 (700 mg) in acetonitrile (5 mL). The reaction liquid was heated to 50 °C and reacted at that temperature for 16 h. After the reaction was completed, the reaction liquid was directly concentrated. The residue was purified by column chromatography (petroleum ether: ethyl acetate = 3:1) to give intermediate 4 (380 mg, yield: 47%). MS m/z (ESI): 423.9[M+1].

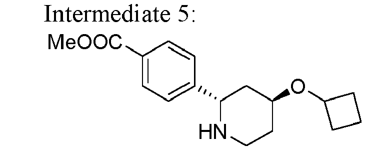

A solution of intermediate 4 (350 mg) in tetrahydrofuran (5 mL) was added palladium/carbon (35 mg). The reaction liquid was subjected to a catalytic hydrogenation reaction under hydrogen atmosphere at room temperature for 2 h. After the reaction was completed, the reaction liquid was directly filtered and concentrated under reduced pressure to give intermediate 5 (200 mg, yield: 75%). MS m/z (ESI): 290.0[M+1].

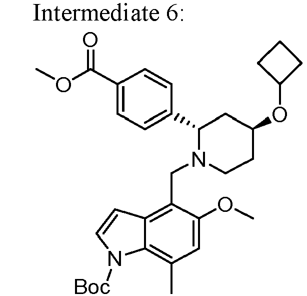

Intermediate 5 (242 mg) was added to a solution of intermediate 2 (242 mg) of Example 2 in 1,2-dichloroethane (5 mL). After the reaction mixture was stirred at room temperature for 8 h, sodium borohydride acetate (532 mg) was added, and the reaction mixture was successively stirred at room temperature for 16 h. After the reaction was completed, the reaction liquid was directly concentrated. The residue was purified by column chromatography (dichloromethane:methanol = 20:1) to give intermediate 6 (350 mg, yield: 63%). MS m/z (ESI): 562.8[M+1].

### Target compound:

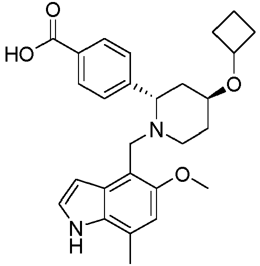

A 50 mL single-neck flask was successively added methanol (3 mL), water (3 mL), intermediate 6 (350 mg) and sodium hydroxide (248 mg). The mixture was heated to 75 °C and reacted at that temperature for 3 h. After the reaction was completed, diluted hydrochloric acid (1 M) was added to the reaction liquid with cooling in an ice bath to adjust the pH to 7. Then the mixture was directly concentrated and purified by preparative high-pressure liquid chromatography (column: Gemini-C18 150 × 21.2 mm, 5 µm; mobile phase: acetonitrile-water (0.1% formic acid); gradient: 20-40%) to give the target compound (85 mg, yield: 30%, comprising 0.4 equivalents of formic acid). MS m/z (ESI): 448.8[M+1]. ¹H NMR (400 MHz, CD₃OD) δ 8.45 (s, 0.4H), 8.15 (d, *J* = 8.0 Hz, 2H), 7.65 (d, *J=* 8.0 Hz, 2H), 7.31 (d, *J=* 2.8 Hz, 1H), 6.73 (s, 1H), 6.32 (s, 1H), 4.80-4.67 (m, 1H), 4.38-4.24 (m, 1H), 4.23-4.13 (m, 1H), 4.14-4.03 (m, 1H), 3.87-3.77 (m, 1H), 3.73 (s, 3H), 3.59-3.45 (m, 1H), 3.40-3.31 (m, 1H), 2.49 (s, 3H), 2.35-1.86 (m, 8H), 1.79-1.67 (m, 1H), 1.64-1.46 (m, 1H).

### Example 5

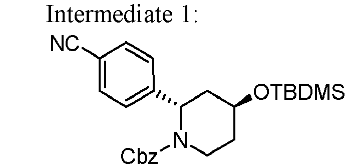

A solution of intermediate 5 (1200 mg) of Example 1 in *N,N*-dimethylformamide (10 mL) was added imidazole (486 mg) and *tert*-butyldimethylchlorosilane (593 mg). The reaction mixture was stirred at room temperature for 2 h. After the reaction was completed, the reaction mixture was added with water (100 mL) for dilution and extracted with ethyl acetate (50 mL). The extract phase was washed once with saturated brine (50 mL), dried over anhydrous sodium sulfate and filtered. The filtrate was directly concentrated to give intermediate 1 (600 mg, yield: 90%). MS m/z (ESI): 472.8[M+23].

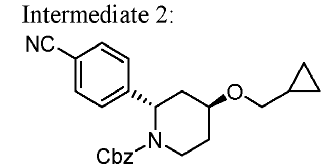

Intermediate 1 (700 mg) was added to dichloromethane (10 mL) at room temperature. Cyclopropanecarboxaldehyde (110 mg) and trimethylsilyl trifluoromethanesulfonate (35 mg) were added to the reaction mixture under nitrogen atmosphere at -78 °C. The reaction system was maintained at -78 °C and stirred for one hour. Then triethylsilane (180 mg) was added. The mixture was naturally warmed to room temperature and successively stirred at that temperature for 16 h. After the reaction was completed, the reaction liquid was quenched by adding saturated aqueous sodium bicarbonate solution (20 mL), added with water (10 mL) for dilution and extracted with dichloromethane (10 mL). The extract phase was washed once with water (10 mL), dried over anhydrous sodium sulfate and filtered. The filtrate was concentrated. The residue was purified by column chromatography (petroleum ether:ethyl acetate = 3:1) to give intermediate 2 (400 mg, yield: 46%). MS m/z (ESI): 390.9[M+1].

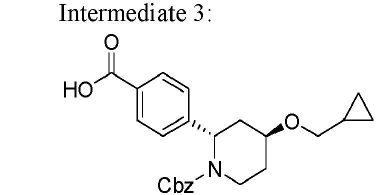

A 50 mL single-neck flask was successively added intermediate 2 (400 mg), isopropanol (2 mL), water (3 mL) and sodium hydroxide (400 mg). The reaction mixture was heated to 100 °C and stirred at that temperature for 16 h. After the reaction was completed, diluted hydrochloric acid (1 M) was added to the reaction mixture to adjust the pH to 5-6 under an ice bath. Water (5 mL) was added for dilution, followed by the extraction with ethyl acetate (5 mL). The extract phase was washed once with saturated brine (5 mL). The organic phase was dried over anhydrous sodium sulfate and filtered. The filtrate was concentrated at 45 °C to give intermediate 3 (200 mg, yield: 33%). MS m/z (ESI): 431.8[M+23].

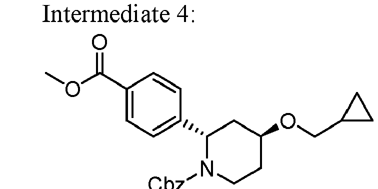

Potassium carbonate (135 mg) and iodomethane (140 mg) were added to a solution of intermediate 3 (200 mg) in acetonitrile (5 mL). The reaction mixture was heated to 50 °C and stirred at that temperature for 16 h. After the reaction was completed, the reaction liquid was directly concentrated. The residue was purified by column chromatography (petroleum ether: ethyl acetate = 3:1) to give intermediate 4 (180 mg, yield: 40%). MS m/z (ESI): 445.8[M+23].

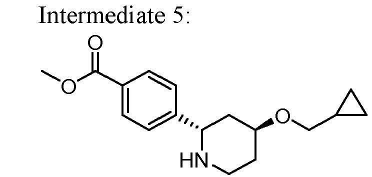

To a solution of intermediate 4 (180 mg) in tetrahydrofuran (3 mL) was added palladium/carbon (50 mg). The reaction liquid was subjected to a catalytic hydrogenation reaction under hydrogen atmosphere at room temperature for 2 h. After the reaction was completed, the reaction mixture was filtered. The filtrate was directly concentrated to give intermediate 5 (120 mg, yield: 54%). MS m/z (ESI): 290.0[M+1].

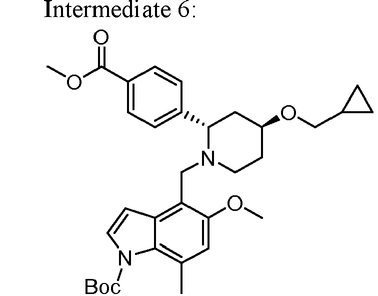

Intermediate 5 (120 mg) was added to a solution of intermediate 2 (119 mg) of Example 2 in 1,2-dichloroethane (5 mL). The reaction mixture was stirred at room temperature for 8 h. Then sodium borohydride acetate (261 mg) was added, and the mixture was successively stirred at room temperature for 16 h. After the reaction was completed, the reaction mixture was directly concentrated. The residue was purified by column chromatography (dichloromethane:methanol = 20:1) to give intermediate 6 (200 mg, yield: 26%). MS m/z (ESI): 562.8[M+1].

### Target compound:

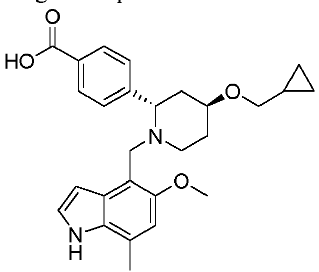

To a 50 mL single-neck flask were successively added methanol (2 mL), water (2 mL), intermediate 6 (200 mg) and sodium hydroxide (150 mg). The reaction mixture was heated to 75 °C and stirred at that temperature for 3 h. After the reaction was completed, diluted hydrochloric acid (1 M) was added to the reaction mixture to adjust the pH to 7 under an ice bath. Then the mixture was directly concentrated under reduced pressure and purified by preparative high-pressure liquid chromatography (column: Gemini-C18 150 × 21.2 mm, 5 µm; mobile phase: acetonitrile-water (0.1% formic acid); gradient: 20-40%) to give the target compound (30.6 mg, yield: 18%; comprising 0.5 equivalents of formic acid). MS m/z (ESI): 448.9[M+1]. ¹H NMR (400 MHz, CD₃OD) δ 8.36 (s, 0.5H), 8.18 (d, *J=* 7.7 Hz, 2H), 7.69 (d, *J* = 7.7 Hz, 2H), 7.32 (s, 1H), 6.76 (s, 1H), 6.34 (s, 1H), 4.88-4.61 (m, 1H), 4.44-4.07 (m, 2H), 3.95-3.81 (m, 1H), 3.75 (s, 3H), 3.63-3.47 (m, 1H), 3.46-3.33 (m, 3H), 2.50 (s, 3H), 2.35-2.14 (m, 2H), 2.13-1.94 (m, 2H), 1.23-1.04 (m, 1H), 0.58 (d, *J* = 7.2 Hz, 2H), 0.28 (d, *J* = 3.8 Hz, 2H).

### Example 6

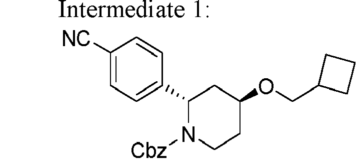

Intermediate 1 (700 mg) of Example 5 was added to dichloromethane (7 mL) under nitrogen atmosphere at -78 °C, and cyclobutanecarboxaldehyde (130 mg) and trimethylsilyl trifluoromethanesulfonate (35 mg) were added. The mixture was maintained at -78 °C and stirred at that temperature for 1 h. Then triethylsilane (180 mg) was added. The reaction mixture was slowly warmed to room temperature and stirred at that temperature for 16 h. After the reaction was completed, the reaction mixture was quenched by adding saturated aqueous sodium bicarbonate solution (20 mL), added with water (10 mL) for dilution and extracted with dichloromethane (10 mL). The extract phase was washed with water (10 mL), dried over anhydrous sodium sulfate and filtered. The filtrate was concentrated. The residue was purified by column chromatography (petroleum ether:ethyl acetate = 3:1) to give intermediate 1 (240 mg, yield: 34%). MS m/z (ESI): 426.8[M+23]. Intermediate 2:

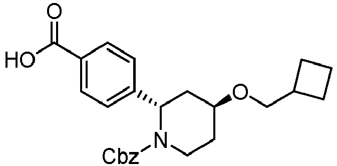

To a 50 mL single-neck flask were successively added isopropanol (1 mL), water (3 mL), intermediate 1 (240 mg) and sodium hydroxide (240 mg). The reaction mixture was heated to 100 °C and reacted at that temperature for 16 h. After the reaction was completed, diluted hydrochloric acid (1 M) was added to the reaction mixture with cooling in an ice bath to adjust the pH to 5-6. Water (5 mL) was added for dilution, followed by the extraction with ethyl acetate (5 mL). The extract phase was washed with brine (5 mL), dried over anhydrous sodium sulfate and filtered. The filtrate was directly concentrated to give intermediate 2 (200 mg, yield: 72%). MS m/z (ESI): 446.1[M+23]. Intermediate 3:

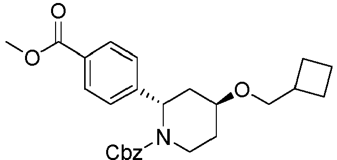

To a solution of intermediate 2 (200 mg) in acetonitrile (5 mL) were added potassium carbonate (130 mg) and iodomethane (134 mg). The reaction mixture was heated to 50 °C and reacted at that temperature for 16 h. After the reaction was completed, the reaction liquid was directly concentrated. The residue was purified through a silica gel column (petroleum ether: ethyl acetate = 3:1) to give intermediate 3 (200 mg, yield: 87%). MS m/z (ESI): 459.8[M+23]. Intermediate 4:

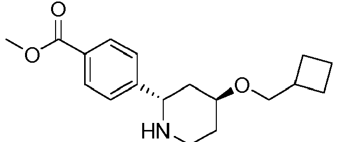

To a solution of intermediate 3 (200 mg) in tetrahydrofuran (3 mL) was added palladium/carbon (50 mg). The reaction mixture was subjected to a catalytic hydrogenation reaction under hydrogen atmosphere at room temperature for 2 h. After the reaction was completed, the reaction mixture was directly filtered and concentrated under reduced pressure to give intermediate 4 (110 mg, yield: 71%). MS m/z (ESI): 303.9[M+1]. Intermediate 5:

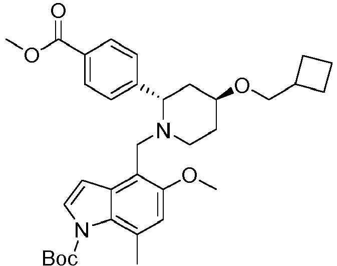

Intermediate 4 (110 mg) was added to a solution of intermediate 2 (105 mg) of Example 2 in 1,2-dichloroethane (5 mL). The reaction mixture was stirred at room temperature for 8 h. Then sodium borohydride acetate (229 mg) was added, and the mixture was successively stirred at room temperature for 16 h. After the reaction was completed, the reaction mixture was directly concentrated. The residue was purified by column chromatography (dichloromethane : methanol = 20:1) to give intermediate 5 (250 mg, yield: 72%). MS m/z (ESI): 576.8[M+1].

### Target compound:

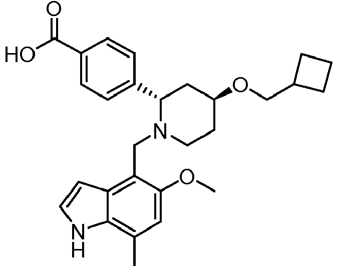

To a 50 mL single-neck flask were successively added methanol (2 mL), water (2 mL), intermediate 5 (250 mg) and sodium hydroxide (175 mg). The reaction mixture was heated to 75 °C and reacted at that temperature for 3 h. After the reaction was completed, diluted hydrochloric acid (1 M) was added to the reaction mixture with cooling in an ice bath to adjust the pH to 7. The mixture was directly concentrated and purified by preparative high-pressure liquid chromatography (column: Gemini-C18 150 × 21.2 mm, 5 µm; mobile phase: acetonitrile-water (0.1% formic acid); gradient: 35-60%) to give the target compound (4.4 mg, yield: 2%). MS m/z (ESI): 462.9[M+1]. ¹H NMR (400 MHz, CD₃OD) δ 8.16 (d, J = 7.5 Hz, 2H), 7.64 (d, J = 7.5 Hz, 2H), 7.31 (d, J = 3.0 Hz, 1H), 6.75 (s, 1H), 6.31 (s, 1H), 4.79-4.55 (m, 1H), 4.43-4.23 (m, 1H), 4.23-4.05 (m, 1H), 3.88-3.65 (m, 4H), 3.59-3.41 (m, 3H), 3.40-3.32 (m, 1H), 2.73-2.58 (m, 1H), 2.50 (s, 3H), 2.28-1.78 (m, 10H).

### Example 7

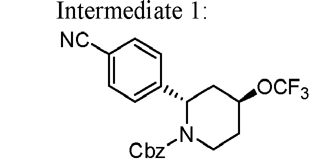

Silver trifluoromethanesulfonate (1600 mg), potassium fluoride (483 mg) and 1-chloromethyl-4-fluoro-1,4-diazobicyclo[2.2.2]octane bis(tetrafluoroborate) (1100 mg) were weighed in a glovebox and added to a 50 mL single-neck flask, and then a solution of intermediate 5 (700 mg) of Example 1 in ethyl acetate (10 mL), 2-fluoropyridine (609 mg) and trifluoromethyltrimethylsilane (889 mg) were added by injection under nitrogen atmosphere. The reaction mixture was stirred at room temperature for 16 h. After the reaction was completed, the reaction mixture was directly purified by column chromatography (petroleum ether: ethyl acetate = 3:1) to give intermediate 1 (300 mg, yield: 32%). MS m/z (ESI): 426.8[M+23].

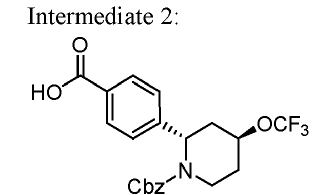

To a 50 mL single-neck flask were successively added isopropanol (2 mL), water (3 mL), intermediate 1 (400 mg) and sodium hydroxide (400 mg). The reaction mixture was heated to 100 °C and reacted at that temperature for 16 h. After the reaction was completed, diluted hydrochloric acid (1 M) was added to the reaction liquid with cooling in an ice bath to adjust the pH to 5-6. Water (5 mL) was added for dilution, followed by the extraction with ethyl acetate (5 mL). The extract phase was washed with saturated brine (5 mL), dried over anhydrous sodium sulfate and filtered. The filtrate was directly concentrated to give intermediate 2 (260 mg, yield: 56%). MS m/z (ESI): 445.7[M+23]. Intermediate 3:

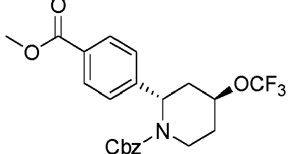

To a solution of intermediate 2 (260 mg) in acetonitrile (5 mL) were added potassium carbonate (170 mg) and iodomethane (175 mg). The reaction mixture was heated to 50 °C and reacted at that temperature for 16 h. After the reaction was completed, the reaction mixture was directly concentrated. The residue was purified by column chromatography (petroleum ether: ethyl acetate = 3:1) to give intermediate 3 (200 mg, yield: 67%). MS m/z (ESI): 459.8[M+23]. Intermediate 4:

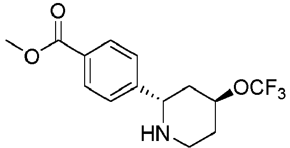

To a solution of intermediate 3 (200 mg) in tetrahydrofuran (3 mL) was added palladium/carbon (50 mg). The reaction mixture was subjected to a catalytic hydrogenation reaction under hydrogen atmosphere at room temperature for 2 h. After the reaction was completed, the reaction mixture was directly filtered and concentrated under reduced pressure to give intermediate 4 (130 mg, yield: 84%). MS m/z (ESI): 303.9[M+1]. Intermediate 5:

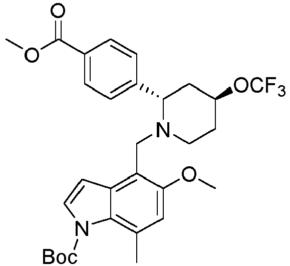

Intermediate 4 (130 mg) was added to a solution of intermediate 2 (125 mg) of Example 2 in 1,2-dichloroethane (5 mL). After the reaction mixture was stirred at room temperature for 8 h, sodium borohydride acetate (273 mg) was added, and the reaction mixture was successively stirred at room temperature for 16 h. After the reaction was completed, the reaction mixture was directly concentrated. The residue was purified by column chromatography (dichloromethane:methanol = 20: 1) to give intermediate 5 (280 mg, yield: 56%). MS m/z (ESI): 576.7[M+1].

### Target compound:

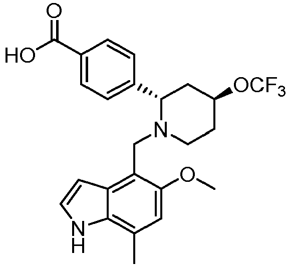

To a 50 mL single-neck flask were successively added methanol (2 mL), water (2 mL), intermediate 5 (280 mg) and sodium hydroxide (194 mg). The reaction mixture was heated to 75 °C and reacted at that temperature for 16 h. After the reaction was completed, diluted hydrochloric acid (1 M) was added to the reaction mixture with cooling in an ice bath to adjust the pH to 7. Then the mixture was directly purified by preparative high-pressure liquid chromatography (column: Gemini-C18 150 × 21.2 mm, 5 µm; mobile phase: acetonitrile-water (0.1% formic acid); gradient: 25-50%) to give the target compound (38.5 mg, yield: 16%, comprising 0.2 equivalents of formic acid). MS m/z (ESI): 462.8[M+1]. ¹H NMR (400 MHz, CD₃OD) δ 8.45 (s, 0.2H), 8.14 (d, *J=* 7.8 Hz, 2H), 7.66 (d, *J=* 7.8 Hz, 2H), 7.29 (d, *J=* 2.9 Hz, 1H), 6.73 (s, 1H), 6.34 (d, *J* = 2.9 Hz, 1H), 4.87-4.78 (m, 1H), 4.64-4.45 (m, 1H), 4.20 (d, *J* = 12.4 Hz, 1H), 4.00 (d, *J=* 12.4 Hz, 1H), 3.75 (s, 3H), 3.38-3.31 (m, 2H), 2.48 (s, 3H), 2.43-2.05 (m, 4H). ¹⁹F NMR (376 MHz, CD₃OD) δ -59.65.

### Example 8

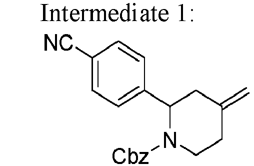

*n*-Butyllithium (6.25 mL) was slowly added dropwise to a solution of methyltriphenylphosphorus bromide (5.35 g) in tetrahydrofuran (100 mL) at -70 °C. The reaction mixture was stirred at -70 °C for 0.5 h. Then a solution of intermediate 2 (3.34 g) of Example 1 in tetrahydrofuran (30 mL) was slowly added dropwise. The reaction mixture was naturally warmed to room temperature and stirred at room temperature for 16 h. After the reaction was completed, it was quenched by adding saturated ammonium chloride (20 mL). Water (100 mL) was added for dilution, followed by the extraction with ethyl acetate (100 mL × 2). The combined extract phases were washed with saturated brine (20 mL), dried over anhydrous sodium sulfate and filtered. The filtrate was concentrated under reduced pressure. The residue was purified by column chromatography (petroleum ether:ethyl acetate = 10:1) to give intermediate 1 (850 mg, yield: 24%). MS m/z (ESI): 333.1[M+1].

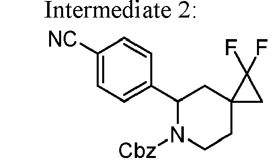

To a solution of intermediate 1 (800 mg) in tetrahydrofuran (10 mL) were added sodium iodide (75 mg) and trifluoromethyltrimethylsilane (1160 mg). The reaction mixture was heated to 70 °C and reacted under closed conditions at this temperature for 16 h. After the reaction was completed, the reaction mixture was directly concentrated. The residue was purified by column chromatography (petroleum ether:ethyl acetate = 5:1) to give intermediate 2 (800 mg, 82%). MS m/z (ESI): 382.9[M+1].

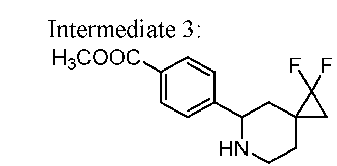

To a 100 mL single-neck flask were successively added methanol (10 mL), a mixed solution of sulfuric acid and water (1:1, 10 mL) and intermediate 2 (480 mg). The reaction mixture was heated to 80 °C and reacted at that temperature for two days. After the reaction was completed, the reaction mixture was naturally cooled to room temperature, poured into ice water and extracted twice with ethyl acetate (50 mL). The combined organic phases were washed once with saturated brine (10 mL), dried over anhydrous sodium sulfate and filtered. The filtrate was concentrated to give intermediate 3 (300 mg, yield: 85%). MS m/z (ESI): 282.0[M+1].

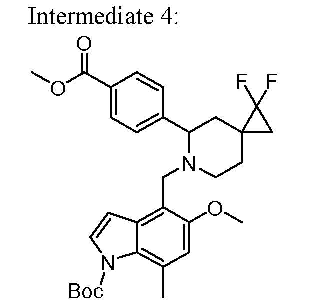

Intermediate 3 (100 mg) was added to a solution of intermediate 2 (100 mg) of Example 2 in 1,2-dichloroethane (3 mL) at room temperature. After the reaction mixture was stirred at room temperature for 8 h, sodium triacetoxyborohydride (220 mg) was added. Then the reaction mixture was successively stirred at room temperature for 16 h. After the reaction was completed, the reaction mixture was directly purified by column chromatography (methanol:dichloromethane = 1:20) to give intermediate 4 (110 mg, 54%). MS m/z (ESI): 554.9[M+1].

### Target compound:

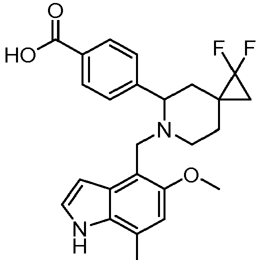

To a 25 mL single-neck flask were successively added methanol (3 mL), water (3 mL), intermediate 4 (110 mg) and sodium hydroxide (40 mg). The reaction mixture was heated to 75 °C and reacted at that temperature for 3 h. After the reaction was completed, the reaction mixture was directly purified by preparative high-pressure liquid chromatography (column: Gemini-C18, 150 × 21.2 mm, 5 µm; mobile phase: acetonitrile-water (0.1% formic acid); gradient: 10-40%). The resulting solution was concentrated. The remaining small amount of aqueous solution was lyophilized to give the target compound (50.6 mg, yield: 75%, comprising 0.5 equivalents of formic acid). MS m/z (ESI): 440.9[M+1]. ¹H NMR (400 MHz, CD₃OD) δ 8.37 (s, 0.5H), 8.16 (d, *J=* 8.1 Hz, 2H), 7.68 (d, *J=* 8.1 Hz, 2H), 7.31 (d, *J=* 2.8 Hz, 1H), 6.76 (s, 1H), 6.34 (d, *J* = 2.8 Hz, 1H), 4.41-4.25 (m, 2H), 4.06 (d, *J* = 12.4 Hz, 1H), 3.77 (s, 3H), 3.56-3.47 (m, 1H), 3.20-3.07 (m, 1H), 2.60-2.45 (m, 4H), 2.35-2.18 (m, 1H), 1.90-1.66 (m, 2H), 1.42-1.28 (m, 2H).

### Example 9

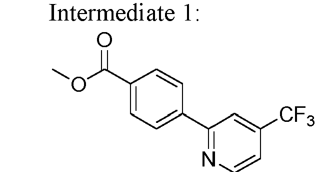

To a 100 mL single-neck flask were successively added 1,4-dioxane (8 mL), water (2 mL), methyl 4-(dihydroxyboranyl)benzoate (500 mg), 2-bromo-4-(trifluoromethyl)pyridine (693 mg), potassium carbonate (413 mg) and tetrakis(triphenylphosphine)palladium(0) (693 mg). The reaction mixture was heated to 90 °C under nitrogen atmosphere and reacted at that temperature for 16 h. After the reaction was completed, the reaction mixture was naturally cooled to room temperature, poured into water (50 mL) and extracted three times with ethyl acetate (100 mL). The combined organic phases were washed with saturated brine (20 mL), dried over anhydrous sodium sulfate and filtered. The filtrate was concentrated under reduced pressure to remove the solvent. The residue was purified by column chromatography (petroleum ether:ethyl acetate = 20:1) to give intermediate 1 (600 mg, yield: 76%). MS m/z (ESI): 282.0[M+1].

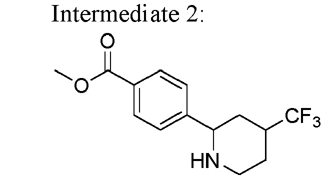

To a 50 mL single-neck flask were successively added methanol (6 mL), intermediate 1 (180 mg), platinum dioxide (14 mg) and a catalytic amount of hydrochloric acid. The reaction mixture was subjected to a catalytic hydrogenation reaction under hydrogen atmosphere at room temperature for 16 h. After the reaction was completed, the reaction mixture was directly filtered. The filtrate was concentrated under reduced pressure. The residue was purified by column chromatography (dichloromethane:methanol = 10:1) to give intermediate 2 (30 mg, yield: 16%). MS m/z (ESI): 288.1[M+1].

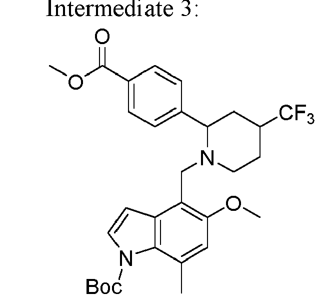

To a 50 mL single-neck flask were successively added 1,2-dichloroethane (4 mL), intermediate 2 (30 mg), intermediate 2 (44 mg) of Example 2 and sodium triacetoxyborohydride (66 mg). The reaction mixture was stirred under nitrogen atmosphere at room temperature for 16 h. After the reaction was completed, methanol was added to the reaction mixture until the solution became clear. Then the mixture was concentrated under reduced pressure. The residue was purified by column chromatography (petroleum ether:ethyl acetate = 20:1) to give intermediate 3 (30 mg, yield: 53%). MS m/z (ESI): 560.7[M+1].

### Target compound:

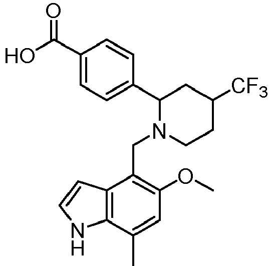

To a 50 mL single-neck flask were successively added methanol (4 mL), water (1 mL), intermediate 3 (65 mg) and sodium hydroxide (92 mg). The reaction mixture was stirred at room temperature for 48 h. After the reaction was completed, the reaction mixture was added with water (3 mL) for dilution, and the pH was adjusted to about 7-8 with diluted hydrochloric acid solution (1 M). Then the mixture was directly concentrated under reduced pressure and purified by preparative high-pressure liquid chromatography (column: Gemini-C18 150 × 21.2 mm, 5 m; mobile phase: acetonitrile-water (0.1% formic acid); gradient: 15-40%; UV: 214 nm) to give the target compound (13.7 mg, yield: 25%, comprising 0.9 equivalents of formic acid). MS m/z (ESI): 447.0[M+1]. ¹H NMR (400 MHz, DMSO-*d₆*) δ 12.89 (s, 1H), 10.85 (s, 1H), 8.13 (s, 0.9H), 7.99 (d, *J* = 8.0 Hz, 2H), 7.69 (d, *J* = 8.0 Hz, 2H), 7.26 (t, *J* = 2.6 Hz, 1H), 6.66 (s, 1H), 6.42 (t, *J* = 2.6 Hz,, 1H), 3.71 (s, 3H), 3.53 (d, *J* = 11.6 Hz, 1H), 3.21 (d, *J* = 11.6 Hz, 1H), 2.84 (d, *J=* 12.0 Hz, 1H), 2.70-2.65 (m, 0.5H), 2.54 (s, 1H), 2.42 (s, 3H), 2.35- 2.30 (m, 0.5H), 2.12-2.02 (m, 1H), 1.86-1.70 (m, 2H), 1.59-1.48 (m, 1H), 1.38-1.29 (m, 1H).

### Example 10

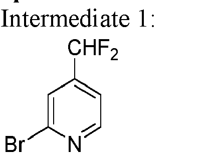

Diethylaminosulfur trifluoride (3.48 g) was slowly added to a solution of 2-bromopyridine-4-carbaldehyde (1 g) in dichloromethane (10 mL) at -78 °C. The reaction mixture was slowly warmed to room temperature and stirred at that temperature for 2 h. After the reaction was completed, the reaction mixture was quenched with saturated sodium bicarbonate (50 mL) and extracted with dichloromethane (50 mL). The extract phase was washed with water (10 mL), dried over anhydrous sodium sulfate and filtered. The filtrate was concentrated under reduced pressure to give intermediate 1 (1 g, yield: 86%). MS m/z(ESI): 207.9[M+1].

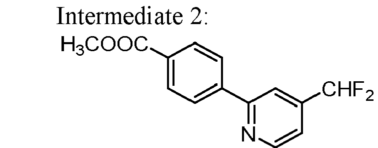

To a 10 mL three-necked flask were successively added 1,4-dioxane (10 mL), water (1 mL), intermediate 1 (1 g), methyl 4-(dihydroxyboranyl)benzoate (0.95 g), sodium carbonate (1.02 g) and tetrakis(triphenylphosphine)palladium(0) (0.166 mg). The reaction mixture was heated to 95 °C under nitrogen atmosphere and stirred at that temperature for 18 h. After the reaction was completed, the reaction mixture was naturally cooled to room temperature, quenched by adding saturated aqueous ammonium chloride solution (2 mL) and extracted three times with ethyl acetate (50 mL). The combined organic substances were dried over anhydrous sodium sulfate and filtered. The filtrate was concentrated under reduced pressure. The residue was purified by column chromatography (petroleum ether:ethyl acetate = 5:1) to give intermediate 2 (0.4 g, yield: 29.17%). MS m/z(ESI): 264.2[M+1].

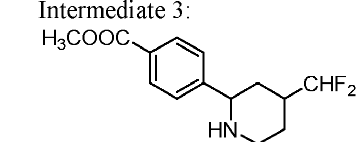

To a 10 mL single-neck flask were successively added methanol (4 mL), concentrated hydrochloric acid (0.2 mL), intermediate 2 (340 mg) and platinum oxide (292.9 mg). The reaction mixture was subjected to a catalytic hydrogenation reaction under hydrogen atmosphere at room temperature for 18 h. After the reaction was completed, the reaction mixture was directly filtered, and the filtrate was concentrated under reduced pressure. The residue was purified by preparative high-pressure liquid chromatography (column: C18 spherical, 100A, 20g, 20-35 µm; acetonitrile-water = 10-70%; UV: 214 nm) to give intermediate 3 (120 mg, yield: 33.51%). MS m/z(ESI): 270.1[M+1].

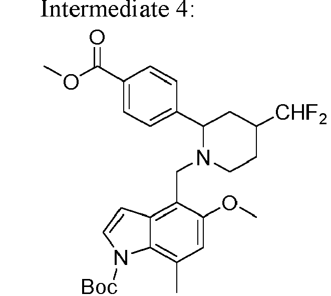

To a 10 mL single-neck flask were successively added 1,2-dichloroethane (2 mL), intermediate 3 (140 mg) and intermediate 2 (196 mg) of Example 2. After the reaction mixture was stirred at room temperature for 8 h, sodium triacetoxyborohydride (330 mg) was added, and the mixture was successively stirred at room temperature for 18 h. After the reaction was completed, dichloromethane (10 mL) was added for dilution, followed by a wash with water (10 mL). The organic phase was dried over anhydrous sodium sulfate and filtered. The filtrate was concentrated. The residue was purified by column chromatography (petroleum ether:ethyl acetate = 3:1) to give intermediate 4 (200 mg, yield: 64.4%). MS m/z(ESI): 542.8[M+1].

### Target compound:

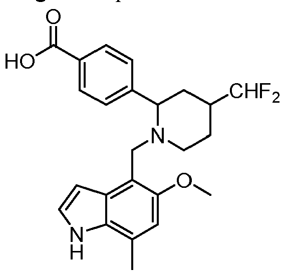

To a 10 mL three-necked flask were successively added methanol (2 mL), tetrahydrofuran (2 mL), water (2 mL), intermediate 4 (180 mg) and sodium hydroxide (132 mg). The reaction mixture was stirred at room temperature for 18 h. After the reaction was completed, the reaction mixture was concentrated under reduced pressure to remove the solvent. The residue was purified by preparative high-pressure liquid chromatography (column: Gemini-C18 150 × 21.2 mm, 5 µm; mobile phase: acetonitrile-water (0.1% formic acid); gradient: 20-25%) to give the target compound (19.5 mg, yield: 13.09%, comprising 0.4 equivalents of formic acid). MS m/z(ESI): 429.2[M+1]. ¹H NMR (400 MHz, CD₃OD) δ 8.36 (s, 0.4H), 8.18 (d, *J=* 8.0 Hz, 2H), 7.68 (d, *J=* 8.0 Hz, 2H), 7.31 (d, *J=* 2.8 Hz, 1H), 6.78-6.72 (m, 1H), 6.30 (s, 1H), 5.81 (td, *J=* 16.4 Hz, 3.6Hz, 1H), 4.55-4.45 (m, 1H), 4.37-4.27 (m, 1H), 4.10-4.03 (m, 1H), 3.78-3.72 (m, 3H), 3.61-3.52 (m, 1H), 3.29-3.24 (m, 1H), 2.50 (s, 3H), 2.45-2.32 (m, 1H), 2.21-2.13 (m, 1H), 2.10-1.92 (m, 2H), 1.88-1.74 (m, 1H).

### Example 11

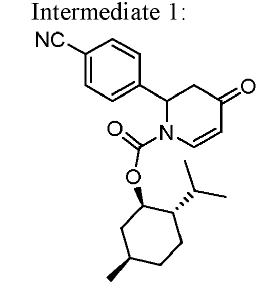

Isopropylmagnesium bromide magnesium chloride complex (85 mL) was added to a solution of 4-bromoxynil (18.2 g) in tetrahydrofuran (100 mL) at room temperature under nitrogen atmosphere. The reaction mixture was stirred at room temperature for 3 h to form a solution of 4-cyanophenylmagnesium bromide (reactant 1).

(1*R*,2*S*,5*R*)-2-Isopropyl-5-methylcyclohexyl chloroformate (20.4 g) was added dropwise to a solution of 4-methoxypyridine (10 g) in tetrahydrofuran (200 mL) at -78 °C under nitrogen atmosphere. After the reaction mixture was stirred at -78 °C for 15 min, the freshly prepared solution of 4-cyanophenylmagnesium bromide (reactant 1) was added. The reaction mixture was successively stirred at -78 °C for 1 h. After the reaction was completed, the reaction mixture was quenched with diluted hydrochloric acid (1 M, 150 mL), naturally warmed to room temperature and successively stirred for 30 min, then added with water (150 mL) for dilution and extracted three times with ethyl acetate (200 mL). The combined organic phases were washed twice with saturated brine (200 mL), dried over anhydrous sodium sulfate and filtered. The filtrate was concentrated under reduced pressure. The residue was purified by column chromatography (petroleum ether:ethyl acetate = 5:1) to give intermediate 1 (16.4 g, yield: 50%).

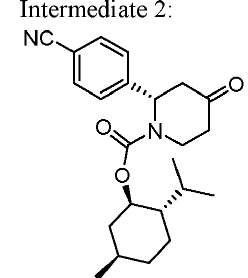

Zinc powder (28 g) was added to a solution of intermediate 1 (16.4 g) in acetic acid (200 mL). The reaction mixture was heated to 100 °C and stirred at that temperature for 5 h. The reaction mixture was filtered. The filtrate was concentrated under reduced pressure. The residue was purified by column chromatography (petroleum ether:ethyl acetate = 2:1) to give intermediate 2 (3 g, yield: 30%).

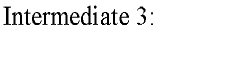

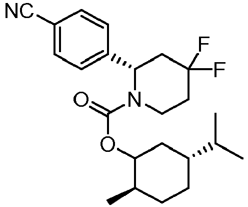

To a 50 mL single-neck flask was added dichloromethane (4 mL) and intermediate 2 (210 mg), followed by diethylaminosulfur trifluoride (177 mg) with cooling in an ice bath. The reaction mixture was heated to 40 °C under nitrogen atmosphere and stirred at that temperature for 16 h. After the reaction was completed, the reaction mixture was poured into ice water (10 mL) and extracted three times with ethyl acetate (50 mL). The combined organic phases were washed with saturated brine (5 mL). The organic phases were dried over anhydrous sodium sulfate and filtered. The filtrate was concentrated under reduced pressure. The residue was purified by column chromatography (petroleum ether:ethyl acetate = 2:1) to give intermediate 3 (144 mg, yield: 54%).

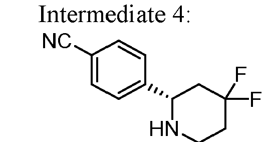

To a 50 mL single-neck flask were added trifluoroacetic acid (4 mL) and intermediate 3 (144 mg). The reaction mixture was heated to 80 °C and stirred at that temperature for 16 h. After the reaction was completed, the reaction mixture was directly concentrated under reduced pressure to give intermediate 4 (80 mg, yield: 60%).

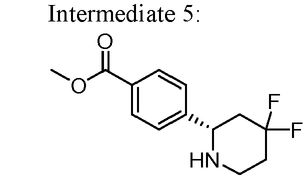

In a 50 mL single-neck flask, intermediate 4 (80 mg) was added to an 80% sulfuric acid/methanol system (1:1, 4 mL). The reaction mixture was heated to 90 °C and stirred at that temperature for 6 h. After the reaction was completed, water (10 mL) was added for dilution and the pH was adjusted to 7-8 with sodium hydroxide solution (2 M) at room temperature, followed by the extraction with ethyl acetate (50 mL × 3). The combined organic phases were dried over anhydrous sodium sulfate and filtered. The filtrate was concentrated under reduced pressure. The residue was purified by thin-layer chromatography (petroleum ether:ethyl acetate = 2:1) to give intermediate 5 (64 mg, yield: 69%).

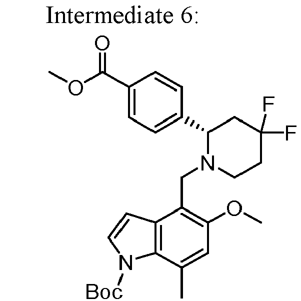

To a 50 mL single-neck flask were successively added 1,2-dichloroethane (2 mL), intermediate 5 (22 mg), intermediate 2 (34 mg) of Example 2 and sodium triacetoxyborohydride (50 mg). The reaction mixture was stirred at room temperature under nitrogen atmosphere for 16 h. After the reaction was completed, methanol was added to the reaction mixture until the solution became clear. Then the mixture was directly concentrated under reduced pressure. The residue was purified by thin-layer chromatography (petroleum ether:ethyl acetate = 5:1) to give intermediate 6 (20 mg, yield: 42%).

### Target compound:

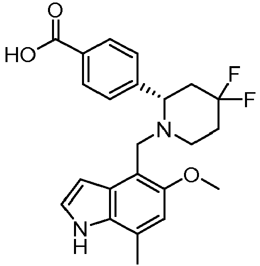

To a 50 mL single-neck flask were successively added methanol (1.5 mL), water (0.5 mL), intermediate 6 (20 mg) and sodium hydroxide (30 mg). The reaction mixture was stirred at room temperature for 48 h. After the reaction was completed, the reaction mixture was poured into water (5 mL), and the pH was adjusted to 7-8 with diluted hydrochloric acid (1 M). The mixture was directly concentrated under reduced pressure. The residue was purified by preparative high-pressure liquid chromatography (column: AZZOTA C18 100A, 10 µm; mobile phase: acetonitrile-water (0.05% aqueous ammonia); gradient: 15-28%). The resulting product was then purified by thin-layer chromatography (dichloromethane:methanol = 10:1) to give the target compound (6 mg, yield: 20%). MS m/z (ESI): 414.45[M+1]. ¹H NMR (400 MHz, CD₃OD) *δ* 8.14 (d, *J* = 8.0 Hz, 2H), 7.71 (d, *J* = 8.0 Hz, 2H), 7.25 (d, *J* = 3.2 Hz, 1H), 6.73 (s, 1H), 6.41 (d, *J=* 3.2 Hz, 1H), 3.92-3.83 (m, 2H), 3.79 (s, 3H), 3.53-3.46 (m, 1H), 3.20-3.12 (m, 1H), 2.68-2.58 (m, 1H), 2.49 (s, 3H), 2.30-2.20 (m, 2H), 2.10-2.00 (m, 2H).

### Example 12

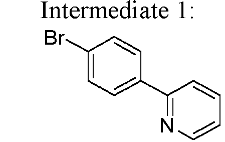

To a solution of 1,4-dioxane (90 mL) and water (15 mL) were successively added at room temperature tetrakis(triphenylphosphine)palladium(0) (1.43 g), 4-bromobenzoic acid (5 g), 2-bromopyridine (4.72 g) and sodium carbonate (6.87g). The reaction mixture was heated to 90 °C under nitrogen atmosphere and reacted at that temperature for 16 h. After the reaction was completed, the reaction mixture was directly concentrated. The residue was purified by column chromatography (petroleum ether:ethyl acetate = 10:1) to give intermediate 1 (5.3 g, yield: 90%). MS m/z (ESI): 233.9[M+1].

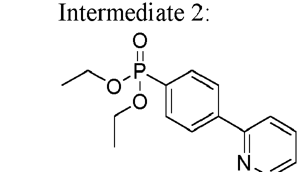

[1,1'-Bis(diphenylphosphino)ferrocene]dichloropalladium(II) (700 mg) was added to a solution of 2-(4-bromophenyl)pyridine (2 g), diethyl phosphite (4.7 g) and a solution of DIEA (2.2 g) in toluene (20 mL). The reaction mixture was heated to 110 °C under nitrogen atmosphere and reacted at that temperature for 16 h. After the reaction was completed, the reaction mixture was directly concentrated under reduced pressure. The residue was purified by column chromatography (petroleum ether:ethyl acetate = 5:1 to 1:1) to give intermediate 2 (1.4 g, yield: 60%). MS m/z (ESI): 291.9[M+1]. Intermediate 3:

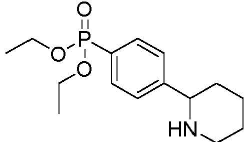

Platinum oxide (280 mg, 20% wt/wt) was added to a solution of intermediate 2 (1.4 g) in EtOH (20 mL) and hydrochloric acid (4 mL). The reaction mixture was subjected to a catalytic hydrogenation reaction under 0.4 MPa for 48 h. After the reaction was completed, the reaction mixture was filtered through celite. The filtrate was concentrated under reduced pressure to give intermediate 3 (1.28 g, yield: 90%). MS m/z (ESI): 297.9[M+1]. Intermediate 4:

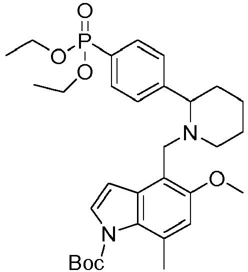

Tetraethyl titanate (226 mg) was added to a solution of intermediate 2 (300 mg) of Example 2 and intermediate 3 (367 mg) in tetrahydrofuran (20 mL). The reaction mixture was heated to 70 °C and stirred at that temperature for 16 h. The reaction system was cooled to room temperature. After sodium triacetoxyborohydride (655 mg) was added, the mixture was heated to 70 °C and successively stirred at that temperature for 1 h. After the reaction was completed, the reaction mixture was concentrated under reduced pressure. The residue was purified by column chromatography (dichloromethane:methanol = 50:1) to give intermediate 4 (700 mg, purity: 80%, yield: 80%). MS m/z (ESI): 571.1[M+1].

### Target compound:

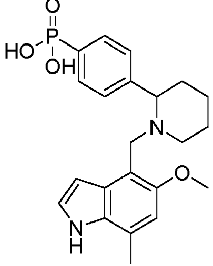

Trimethylbromosilane (2 mL) was added to a solution of intermediate 4 (200 mg) in dichloromethane (6 mL) at 0 °C. The reaction mixture was stirred at room temperature for 16 h. After the reaction was completed, the reaction mixture was concentrated under reduced pressure. The residue was separated and purified by preparative high-pressure liquid chromatography (column: Gemini-C18 150 × 21.2 mm, 5 µm; mobile phase: acetonitrile-water (0.1% formic acid); gradient: 10-40%) to give the target compound (60 mg, yield: 82%). MS m/z (ESI): 414.9[M+1]. ¹H NMR (400 MHz, CD₃OD) δ 8.07 (s, 2H), 8.06-7.95 (m, 2H), 7.66-7.61 (m, 2H), 7.32 (d, *J =* 3.1 Hz, 1H), 6.76 (s, 1H), 6.33 (d, *J =* 3.1 Hz, 1H), 4.49-4.38 (m, 1H), 4.34 (d, *J =* 12.7 Hz, 1H), 4.12 (d, *J =* 12.7 Hz, 1H), 3.76 (s, 3H), 3.51 (d, *J* = 12.8 Hz, 1H), 3.28-3.18 (m, 1H), 2.50 (s, 3H), 2.12-2.04 (m, 2H), 1.96-1.81 (m, 4H).

### Example 13

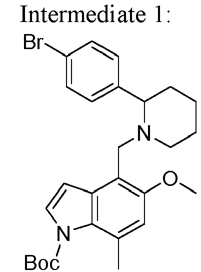

Tetraethyl titanate (151 mg) was added to a solution of intermediate 2 (200 mg) of Example 2 and 2-(4-bromophenyl)piperidine (195 mg) in tetrahydrofuran (15 mL). The reaction system was heated to 70 °C and stirred at that temperature for 16 h. After the reaction was naturally cooled to room temperature, sodium triacetoxyborohydride (438 mg) was added. The mixture was heated to 70 °C and successively stirred at that temperature for 1 h. After the reaction was completed, the reaction mixture was directly concentrated under reduced pressure. The residue was purified by column chromatography (petroleum ether:ethyl acetate = 2:1) to give intermediate 1 as a solid (252 mg, yield: 71%). MS m/z (ESI): 512.7[M+1].

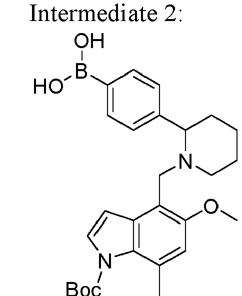

Chloro(2-dicyclohexylphosphino-2',4',6'-triisopropyl-1,1'-biphenyl)[2-(2'-amino-1,1'-biphenyl)]palladium(II) (X-Phos-Pd-G2) (19 mg) was added to a solution of intermediate 1 (252 mg), diborane-1,1,2,2-tetraol (131 mg), 2-dicyclohexylphosphino-2',4',6'-triisopropylbiphenyl (X-Phos) (23 mg) and potassium acetate (144 mg) in ethanol (15 mL). The reaction mixture was heated to 90 °C under nitrogen atmosphere and stirred at that temperature for 16 h. After the reaction was completed, the reaction mixture was directly concentrated under reduced pressure. The residue was purified by column chromatography (dichloromethane:methanol = 5:1) to give intermediate 2 (200 mg, yield: 85%). MS m/z (ESI): 478.9[M+1].

### Target compound:

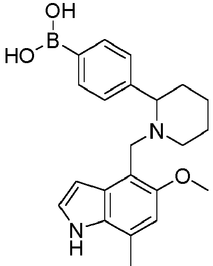

Trimethylbromosilane (3 mL) was added to a solution of intermediate 2 (200 mg) in dichloromethane (9 mL) at 0 °C. The mixture was reacted at room temperature for 16 h. After the reaction was completed, the reaction mixture was directly concentrated under reduced pressure. The residue was separated and purified by preparative high-pressure liquid chromatography (column: Gemini-C18 150 × 21.2 mm, 5 µm; mobile phase: acetonitrile-water (0.1% formic acid); gradient: 10-35%) to give the target compound (42 mg, yield: 33%, comprising 0.8 equivalents of formic acid). MS m/z (ESI): 378.9[M+1]. ¹H NMR (400 MHz, CD₃OD) δ 8.47 (s, 0.8H), 7.83 (s, 2H), 7.55 (d, *J=* 8.0 Hz, 2H), 7.30 (s, 1H), 6.75 (s, 1H), 6.27 (s, 1H), 4.44-4.30 (m, 2H), 4.10 (d, *J=* 12.6 Hz, 1H), 3.75 (s, 3H), 3.55-3.46 (m 1H), 3.29-3.16 (m, 1H), 2.50 (s, 3H), 2.15-2.00 (m, 2H), 2.00-1.70 (m, 4H).

### Example 14

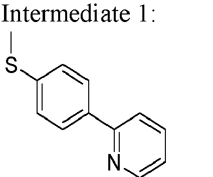

To a mixed solvent of toluene (140 mL), water (140 mL) and ethanol (40 mL) were successively added 4-thiomethyl-phenylboronic acid pinacol ester (13.8 g), 2-bromopyridine (10 g), tetrakis(triphenylphosphine)palladium(0) (2.19 g) and sodium carbonate (50.32 g). The reaction system was heated to 95 °C under nitrogen atmosphere and stirred at that temperature for 8 h. After the reaction was completed, the reaction system was naturally cooled to room temperature, quenched by adding saturated aqueous ammonium chloride solution (50 mL) and extracted three times with ethyl acetate (250 mL). The combined organic phases were dried over anhydrous sodium sulfate and filtered. The filtrate was concentrated. The residue was purified by column chromatography (petroleum ether:ethyl acetate = 5:1) to give intermediate 1 (300 mg, yield: 74.52%). MS m/z(ESI): 202.2[M+1].

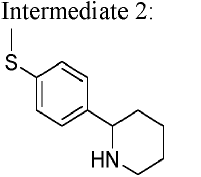

To a 250 mL three-necked flask were successively added toluene (100 mL), intermediate 1 (1 g), diphenylsilane (4.61 g), diphenylamine (3.3844 g) and tris(pentafluorophenyl)boron (0.26 g). The reaction system was warmed to 110 °C under nitrogen atmosphere and stirred at that temperature for 18 h. After the reaction was completed, the reaction system was naturally cooled to room temperature, quenched by adding saturated aqueous ammonium chloride solution (50 mL) and extracted three times with ethyl acetate (50 mL). The combined organic substances were dried over anhydrous sodium sulfate and filtered. The filtrate was concentrated under reduced pressure. The residue was purified by column chromatography (petroleum ether:ethyl acetate = 3:1) to give intermediate 2 (0.9 g, yield: 80%). MS m/z(ESI): 208.2[M+1].

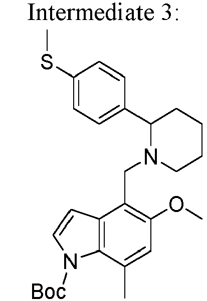

To a 50 mL three-necked flask were successively added 1,2-dichloroethane (10 mL), intermediate 2 (500 mg) and intermediate 2 (909 mg) of Example 2. After the reaction system was stirred at room temperature for 8 h, sodium triacetoxyborohydride (1.53 g) was added, and the mixture was successively reacted at room temperature for 18 h. After the reaction was completed, the reaction mixture was added with dichloromethane (10 mL) for dilution, washed once with water (10 mL), dried over anhydrous sodium sulfate and filtered. The filtrate was concentrated under reduced pressure. The residue was purified by column chromatography (petroleum ether: ethyl acetate = 3:1) to give intermediate 3 (300 mg, yield: 25.6%). MS m/z(ESI): 481.4[M+1].

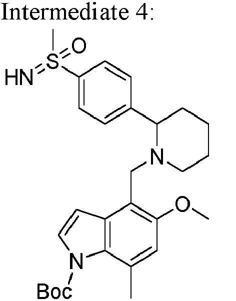

Intermediate 3 (100 mg) was added to a solution of ammonium carbamate (24.36 mg) and phenyliodine diacetate (140.69 mg) in methanol (2 mL). The reaction mixture was stirred at room temperature for 30 min. After the reaction was completed, the reaction mixture was directly separated and purified by preparative high-pressure liquid chromatography (column: C18 spherical, 100A, 20g, 20-35 µm; acetonitrile-water = 10-70%; UV: 214 nm) to give intermediate 4 (30 mg, yield: 17.6%). MS m/z(ESI): 512.3[M+1].

### Target compound:

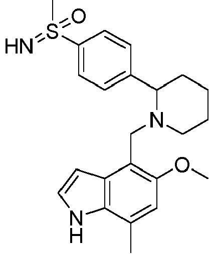

To a 10 mL single-neck flask were successively added dichloromethane (6 mL), intermediate 4 (30 mg) and trimethylbromosilane (0.6 mL). The reaction system was stirred at room temperature for 8 h. After the reaction was completed, the reaction mixture was directly concentrated under reduced pressure. The residue was separated and purified by preparative high-pressure liquid chromatography (column: Gemini-C18 150 × 21.2 mm, 5 µm; mobile phase: acetonitrile-water (0.1% formic acid); gradient: 10-20%) to give the target compound (11.6 mg, yield: 44.67%, comprising 0.6 equivalents of formic acid). MS m/z (ESI): 412.3[M+1]. ¹H NMR (400 MHz, DMSO) δ 10.83 (s, 1H), 8.21 (s, 0.6H), 7.93 (d, *J* = 8.0 Hz, 2H), 7.74 (d, *J=* 8.0 Hz, 2H), 7.25 (t, *J=* 2.4 Hz, 1H), 6.65 (s, 1H), 6.49 (td, *J=* 9.6 Hz, 2.4 Hz, 1H), 4.20 (s, 1H), 3.56 (s, 3H), 3.57-3.52 (m, 1H), 3.25-3.15 (m, 2H), 3.07 (s, 3H), 2.77 (d, *J=* 10.4 Hz, 1H), 2.41 (s, 3H), 1.75-1.64 (m, 2H), 1.57-1.43 (m, 2H), 1.41-1.28 (m, 2H).

### Example 15

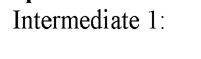

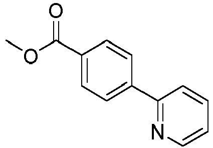

To a 250 mL single-neck flask were successively added a mixed solvent of dioxane and water (8:1, 50 mL), 4-methoxycarbonylphenylboronic acid pinacol ester (5.0 g), 2-bromopyridine (3.3 g), sodium carbonate (3 g) and tetrakis(triphenylphosphine)palladium(0) (662 mg). The reaction system was heated to 80 °C under nitrogen atmosphere and stirred at that temperature for 16 h. After the reaction was completed, the reaction mixture was concentrated under reduced pressure to remove the solvent. The residue was added with 100 mL of water for dilution and extracted three times with ethyl acetate (200 mL). The combined organic phases were washed with saturated brine (50 mL), dried over anhydrous sodium sulfate and filtered. The filtrate was concentrated under reduced pressure. The residue was purified by column chromatography (petroleum ether:ethyl acetate = 5:1) to give intermediate 1 (1.1 g, yield: 27%). MS m/z (ESI): 214.1[M+1].

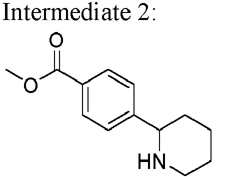

To a 50 mL reactor were successively added methanol (30 mL), intermediate 1 (1.9 g), platinum dioxide (202 mg) and hydrochloric acid (0.5 mL). The mixture was subjected to a catalytic hydrogenation reaction under 0.4 MPa (hydrogen) at room temperature for 20 h. After the reaction was completed, the reaction mixture was filtered. The filtrate was concentrated under reduced pressure. The residue was purified by column chromatography (petroleum ether: ethyl acetate = 5:1) to give intermediate 2 (1.55 g, yield: 79%). MS m/z (ESI): 219.9[M+1].

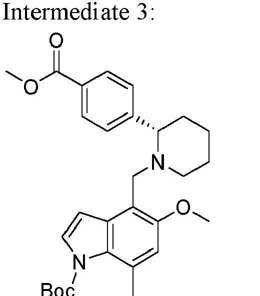

To a 50 mL single-neck flask were successively added tetrahydrofuran (10 mL), intermediate 2 (150 mg) of Example 2, intermediate 2 (150 mg) and tetraethyl titanate (120 mg). The reaction system was heated to 100 °C under nitrogen atmosphere and stirred at that temperature for 8 h. Then the reaction was cool to room temperature. Sodium triacetoxyborohydride (330 mg) was added, and the mixture was successively stirred for 1 h. After the reaction was completed, the reaction mixture was poured into water and extracted three times with ethyl acetate (50 mL). The combined organic phases were dried over anhydrous sodium sulfate and filtered. The filtrate was concentrated under reduced pressure. The residue was purified by column chromatography (petroleum ether:ethyl acetate = 20:1) to give intermediate 3 (135 mg, yield: 40%).

### Target compound:

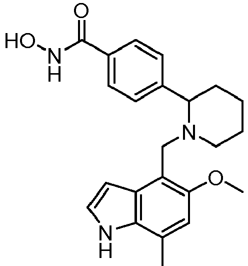

To a 50 mL single-neck flask were successively added *N*,*N*-dimethylformamide (6 mL), intermediate 3 (100 mg), hydroxylamine hydrochloride (55 mg), 2-(7-azabenzotriazole)-*N*,*N*,*N*',*N*'-tetramethyluronium hexafluorophosphate (HATU) (110 mg) and triethylamine (80 mg). The reaction system was stirred at room temperature for 48 h. After the reaction was completed, the reaction mixture was poured into water and extracted three times with ethyl acetate (100 mL). The combined organic phases were washed once with saturated brine (20 mL), dried over anhydrous sodium sulfate and filtered. The filtrate was concentrated under reduced pressure. The residue was separated and purified by preparative high-pressure liquid chromatography (column: Gemini-C18 150 × 21.2 mm, 5 µm; mobile phase: acetonitrile-water (0.1% formic acid); gradient: 10-30%) to give the target compound (11.7 mg, yield: 11%). MS m/z (ESI): 394.1[M+1]. ¹H NMR (400 MHz, CD₃OD) δ 8.51 (s, 1H), 7.94 (d, *J* = 7.8 Hz, 2H), 7.71 (d, *J* = 7.8 Hz, 2H), 7.31 (d, *J* = 2.8 Hz, 1H), 6.76 (s, 1H), 6.34 (s, 1H), 4.45-4.25 (m, 2H), 4.12-4.00 (m, 1H), 3.76 (s, 3H), 3.55-3.42 (m, 1H), 3.22-3.12 (m, 1H), 2.50 (s, 3H), 2.10-1.75 (m, 6H).

### Example 16

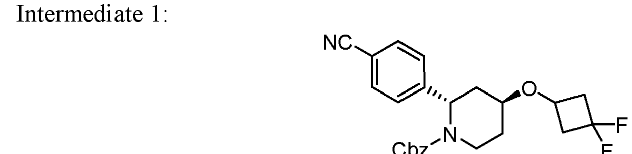

3,3-Difluorocyclobutan-1-one (203 mg) and trimethylsilyl trifluoromethanesulfonate (42 mg) were added to intermediate 1 (860 mg) of Example 6 in dichloromethane (5 mL) at -78 °C under nitrogen atmosphere. The reaction mixture was stirred at -78 °C for one hour. Triethylsilane (222 mg) was added. The reaction mixture was naturally warmed to room temperature and successively stirred at room temperature for 16 h. After the reaction was completed, the reaction mixture was quenched by slowly adding aqueous sodium bicarbonate solution (10 mL), added with water (10 mL) for dilution and extracted with dichloromethane (10 mL). The extract phase was washed once with saturated brine (10 mL), dried over anhydrous sodium sulfate and filtered. The filtrate was concentrated. The residue was purified by column chromatography (petroleum ether:ethyl acetate = 3:1) to give intermediate 1 (80 mg, yield: 8.36%). MS m/z (ESI): 448.8[M+23].

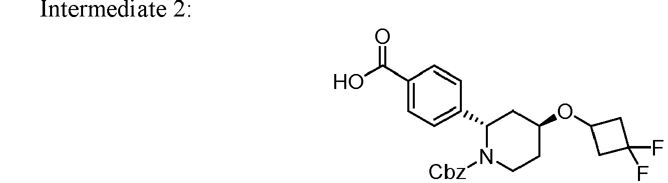

Intermediate 1 (100 mg) and sodium hydroxide (94 mg) were successively added to a mixed solution of isopropanol and water (1 mL/3 mL). The reaction mixture was heated to 100 °C and stirred at that temperature for 16 h. After the reaction was completed, diluted hydrochloric acid (1 M, 2.50 mL) was added to the reaction mixture with cooling in an ice bath to adjust the pH to 5-6. Water (5 mL) was added for dilution, followed by the extraction with ethyl acetate (5 mL). The extract phase was washed with saturated brine (5 mL), dried over anhydrous sodium sulfate and filtered. The filtrate was concentrated under reduced pressure to give intermediate 2 (70 mg, yield: 60%). MS m/z (ESI): 445.9[M+1].

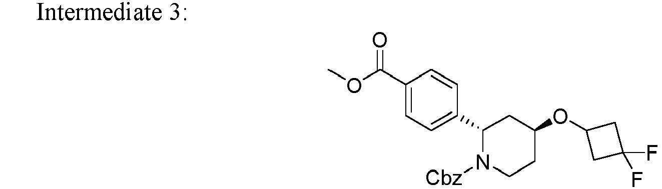

Potassium carbonate (43 mg) and iodomethane (45 mg) were added to a solution of intermediate 2 (70 mg) in acetonitrile (2 mL). The reaction mixture was heated to 50 °C and stirred at that temperature for 2 h. After the reaction was completed, the reaction mixture was directly concentrated. The residue was purified by column chromatography (petroleum ether: ethyl acetate = 3:1) to give intermediate 3 (60 mg, yield: 73%). MS m/z (ESI): 481.7[M+23].

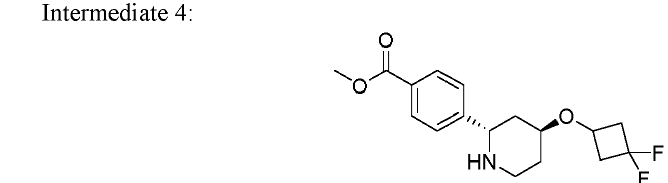

To a solution of intermediate 3 (60 mg) in tetrahydrofuran (1 mL) was added palladium/carbon (10 mg). The reaction mixture was subjected to a catalytic hydrogenation reaction under hydrogen atmosphere at room temperature for 16 h. After the reaction was completed, the reaction mixture was filtered. The filtrate was directly concentrated to give intermediate 4 (35 mg, yield: 74%). MS m/z (ESI): 325.9[M+1].

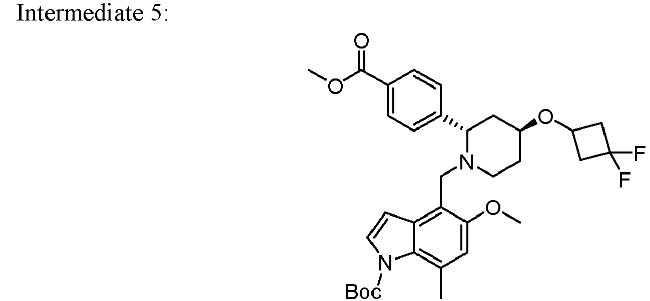

Intermediate 4 (35 mg) was added to a solution of intermediate 2 (32 mg) of Example 2 in 1,2-dichloroethane (2 mL). The reaction mixture was stirred at room temperature for 8 h. Then sodium triacetoxyborohydride (70 mg) was added, and the mixture was successively stirred at room temperature for 16 h. After the reaction was completed, the reaction mixture was directly concentrated. The residue was purified by column chromatography (dichloromethane:methanol = 20:1) to give intermediate 5 (80 mg, yield: 37.5%). MS m/z (ESI): 598.8[M+1].

### Target compound:

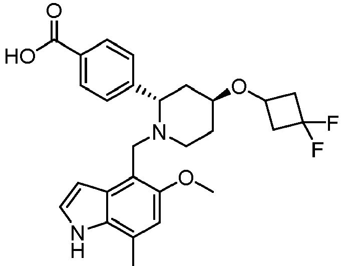

Intermediate 5 (80 mg, 0.134 mmol) and sodium hydroxide (54 mg, 1.35 mmol) were successively added to a mixed solution of methanol and water (1 mL/1 mL) at room temperature. The reaction mixture was heated to 75 °C and stirred at that temperature for 3 h. After the reaction was completed, diluted hydrochloric acid (1 M, 1.35 mL) was added to the reaction mixture with cooling in an ice bath to adjust the pH to about 7. Then the solvent was directly lyophilized. The residue was separated and purified by preparative high-pressure mixture chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.1% formic acid); gradient: 30-50%) to give the target compound (4.7 mg, yield: 7.23%, comprising 0.2 equivalents of formic acid). ¹H NMR (400 MHz, CD₃OD) *δ* 8.44 (s, 0.2H), 8.15 (d, *J =* 8.0 Hz, 2H), 7.63 (d, *J =* 8.0 Hz, 2H), 7.49-7.25 (m, 5H), 6.75 (s, 1H), 6.32 (s, 1H), 4.84-4.71 (m, 1H), 4.64 (q, *J =* 11.8 Hz, 2H), 4.42-4.12 (m, 2H), 4.02-3.86 (m, 1H), 3.74 (s, 3H), 3.64-3.48 (m, 1H), 3.48-3.31 (m, 1H), 2.50 (s, 3H), 2.38-1.97 (m, 4H). MS m/z (ESI): 484.8[M+1].

### Example 17

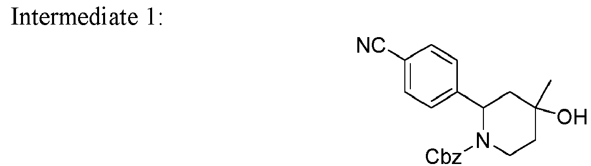

A solution of methylmagnesium bromide (1 M, 6 mL) in tetrahydrofuran was slowly added dropwise (the temperature of the reaction mixture was kept to not exceed -40 °C during the dropwise addition) to a solution of intermediate 2 (2 g) of Example 1 in tetrahydrofuran (50 mL) at -40 °C under nitrogen atmosphere. After the dropwise addition, the mixture was successively stirred and naturally warmed to room temperature, and then successively stirred at room temperature for 2 h. After the reaction was completed, the reaction mixture was quenched by adding saturated aqueous ammonium chloride solution (20 mL) and then added with ethyl acetate (100 mL) and water (100 mL) for dilution. The organic phase was separated, washed with saturated brine (50 mL), dried over anhydrous sodium sulfate and filtered. The filtrate was concentrated under reduced pressure to give intermediate 1 (1.7 g, yield: 40%). MS m/z (ESI): 351.0[M+1].

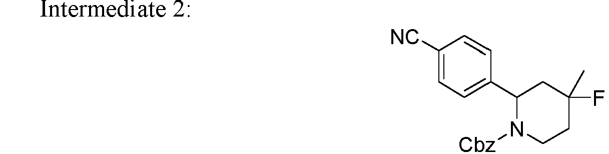

Diethylaminosulfur trifluoride (413 mg) was added dropwise to a solution of intermediate 1 (900 mg) in dichloromethane (20 mL) with cooling in an ice bath. The reaction mixture was stirred at room temperature for 16 h. After the reaction was completed, the reaction mixture was quenched by adding saturated aqueous sodium bicarbonate solution (20 mL), then added with water (100 mL) for dilution and extracted with ethyl acetate (100 mL). The extract phase was washed once with water (100 mL), dried over anhydrous sodium sulfate and filtered. The filtrate was concentrated under reduced pressure. The residue was purified by column chromatography (petroleum ether:ethyl acetate = 5:1) to give intermediate 2 (280 mg, yield: 27%). MS m/z (ESI): 352.9[M+1].

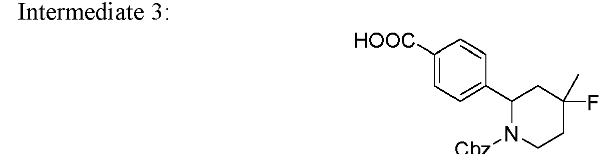

Intermediate 2 (250 mg) and aqueous sodium hydroxide solution (4 M, 3 mL) were successively added to isopropanol (3 mL) at room temperature. The reaction mixture was heated to 100 °C and stirred at that temperature for 30 h. After the reaction was completed, diluted hydrochloric acid (1 M, 13 mL) was slowly added to the reaction mixture with cooling in an ice bath to adjust the pH to 5-6. Water (20 mL) was added for dilution, followed by the extraction with ethyl acetate (20 mL). The extract phase was washed with saturated brine (20 mL), dried over anhydrous sodium sulfate and filtered. The filtrate was concentrated under reduced pressure to give intermediate 3 (200 mg, yield: 64%). MS m/z (ESI): 371.9[M+1].

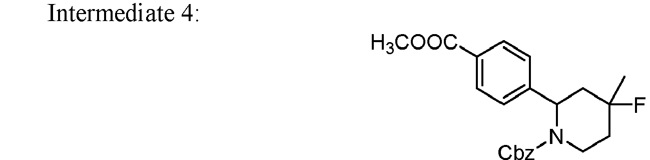

Potassium carbonate (138 mg) and iodomethane (140 mg) were successively added to a solution of intermediate 3 (200 mg) in acetonitrile (5 mL) at room temperature. The reaction mixture was heated to 50 °C and stirred at that temperature for 16 h. After the reaction was completed, the reaction mixture was directly concentrated. The residue was purified by column chromatography (petroleum ether:ethyl acetate = 3:1) to give intermediate 4 (200 mg, yield: 86%). MS m/z (ESI): 385.9[M+1].

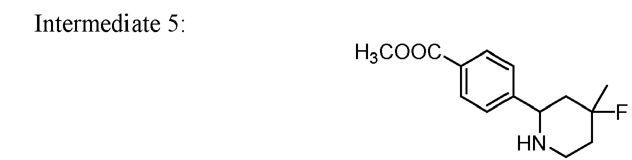

To a solution of intermediate 4 (200 mg) in tetrahydrofuran (5 mL) was added palladium/carbon (50 mg). The reaction mixture was reacted under 1 atmosphere of hydrogen gas at room temperature for 16 h. After the reaction was completed, the reaction mixture was filtered. The filtrate was directly concentrated under reduced pressure to give intermediate 5 (95 mg, yield: 27%). MS m/z (ESI): 251.9[M+1].

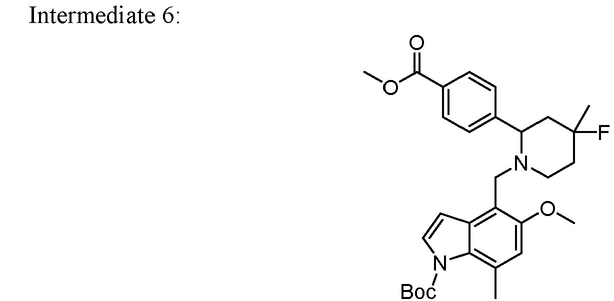

Intermediate 5 (50 mg), 2,8,9-trioxa-5-aza-1-silabicyclo[3.3.3]undecane (150 mg) and acetic acid (30 mg) were successively added to a solution of intermediate 2 (60 mg) of Example 2 in tetrahydrofuran (5 mL) at room temperature. The reaction mixture was heated to 70 °C and stirred at that temperature for 24 h. After the reaction was completed, the reaction mixture was directly concentrated. The residue was purified by column chromatography (dichloromethane:methanol = 20: 1) to give intermediate 6 (40 mg, yield: 26%). MS m/z (ESI): 525.2[M+1].

### Target compound:

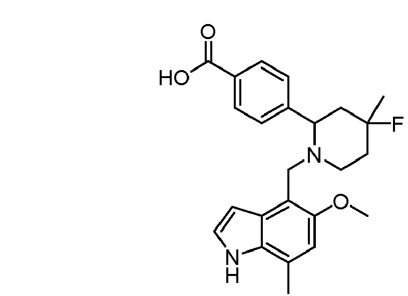

Intermediate 6 (40 mg) and sodium hydroxide (40 mg) were successively added to a mixed solution of methanol/water (2 mL/2 mL) at room temperature. The reaction mixture was heated to 75 °C and stirred at that temperature for 3 h. After the reaction was completed, diluted hydrochloric acid (1 M, 1 mL) was added to the reaction mixture with cooling in an ice bath to adjust the pH to about 8. The mixture was concentrated under reduced pressure. The residue was separated and purified by preparative high-pressure liquid chromatography (column: AQ-C18; 30 × 250 mm, 10 µm; column temperature: 25 °C; flow rate: 45 mL/min; wavelength: 214 nm; column pressure: 19 bar; mobile phase: acetonitrile-water (0.05% NH₃); gradient: 10-40%) to give the target compound (14.0 mg, yield: 17%). MS m/z (ESI): 410.9[M+1]. ¹H NMR (400 MHz, CD₃OD) *δ* 8.16-8.08 (m, 2H), 7.63 (d, *J* = 8.0 Hz, 2H), 7.32-7.25 (m, 1H), 6.77-6.70 (m, 1H), 6.37-6.29 (m, 1H), 4.70-4.51 (m, 1H), 4.35-3.70 (m, 6H), 3.42-3.35 (m, 1H), 2.52-2.46 (m, 3H), 2.30-1.90 (m, 4H), 1.67-1.35 (m, 3H).

### Example 18

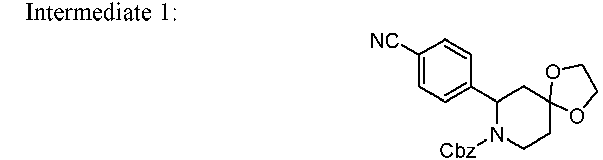

To a solution of intermediate 2 (2 g) of Example 1 in toluene (40 mL) was added ethylene glycol (558 mg) and *p*-toluenesulfonic acid (114 mg) at room temperature. The reaction mixture was heated to 110 °C and stirred for 18 h. After the reaction was completed, the reaction mixture was concentrated under reduced pressure. The residue was purified by column chromatography (petroleum ether:ethyl acetate = 3:1) to give intermediate 1 (1.6 g, yield: 70%). MS m/z (ESI): 379.2[M+1].

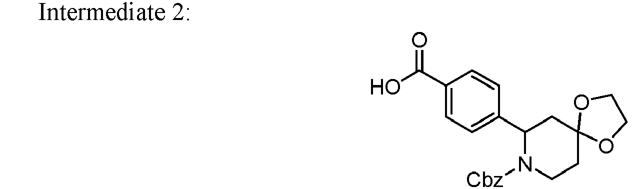

Sodium hydroxide (1.7 g) was added to a mixed solution of intermediate 1 (1.6 g) in methanol and water (20 mL/20 mL) at room temperature. The reaction mixture was heated to 70 °C and stirred at that temperature for 18 h. After the reaction was completed, the reaction system was naturally cooled to room temperature and adjusted to about pH 2 with diluted hydrochloric acid (2 M). The resulting mixture was concentrated under reduced pressure. The resulting residue was separated and purified by preparative high-pressure liquid chromatography (column: -Gemini-C18 150 × 21.2 mm, 5 µm, column temperature: 25 °C; flow rate: 14 mL/min; wavelength: 214 nm; column pressure: 80 bar; mobile phase: acetonitrile-water (0.1% formic acid); gradient: 10-70%) to give intermediate 2 (150 mg, yield: 47.6%). MS m/z (ESI): 398.2[M+1].

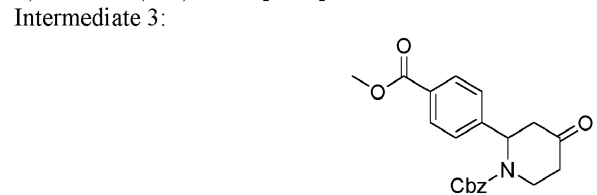

Thionyl chloride (1.34 g) was slowly added dropwise to a solution of intermediate 2 (800 mg) in methanol (10 mL) with cooling in an ice bath. The reaction mixture was stirred at room temperature for 16 h. After the reaction was completed, the reaction mixture was added with water (10 mL) for dilution, then successively stirred for 2 h to quench the reaction, and then directly concentrated under reduced pressure to give intermediate 3 (680 mg, yield: 77.8%). MS m/z (ESI): 368.2[M+1].

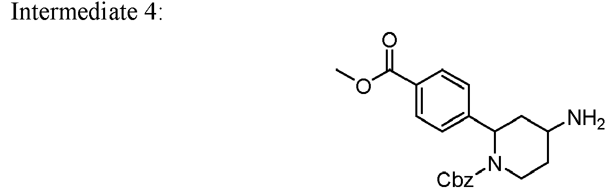

To a solution of intermediate 3 (600 mg) in 1,2-dichloroethane (2 mL) was added a solution of ammonia in methanol (1 M, 5.8 mL) with cooling in an ice bath. After the reaction mixture was stirred at room temperature for 8 h, sodium borohydride acetate (1 g) was added, and the mixture was successively stirred at room temperature for 18 h. The resulting mixture was added with dichloromethane (10 mL) and water (10 mL) for dilution. The organic phase was dried over anhydrous sodium sulfate and filtered. The filtrate was concentrated. The residue was purified by column chromatography (petroleum ether:ethyl acetate = 3:1) to give intermediate 4 (150 mg, yield: 24.98%). MS m/z (ESI): 369.1[M+1].

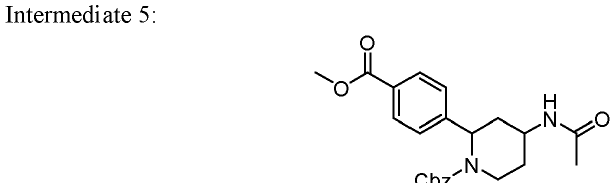

To a solution of intermediate 4 (140 mg) in ethyl acetate (5 mL) was added acetic anhydride (38.79 mg) at room temperature. The reaction mixture was successively stirred at room temperature for 18 h. After the reaction was completed, potassium carbonate (105 mg) was added to the reaction mixture. The mixture was successively stirred for 30 min and filtered. The filtrate was concentrated under reduced pressure. The residue was purified by column chromatography (petroleum ether:ethyl acetate = 3:1) to give intermediate 5 (110 mg, yield: 67%). MS m/z (ESI): 411.3[M+1].

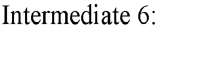

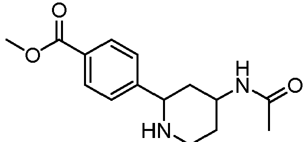

Palladium hydroxide/carbon (10 mg) was added to a solution of intermediate 5 (70 mg) in methanol (5 mL) at room temperature. The reaction mixture was stirred at room temperature under hydrogen atmosphere for 18 h. After the reaction was completed, the reaction mixture was filtered. The filtrate was directly concentrated under reduced pressure to give intermediate 6 (50 mg, yield: 89.41%). MS m/z (ESI): 277.0[M+1]. Intermediate 7:

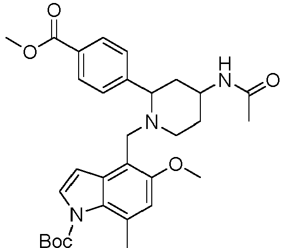

Intermediate 6 (40 mg) and intermediate 2 of Example 2 (52.8 mg) were added to a solution of 1,2-dichloroethane (2 mL) at room temperature. The reaction mixture was stirred at room temperature for 8 h. Then sodium borohydride acetate (89 mg) was added. The reaction mixture was successively stirred at room temperature for 18 h. After the reaction was completed, the reaction mixture was added with dichloromethane (10 mL) for dilution and washed with water (10 mL). The organic phase was dried over anhydrous sodium sulfate and filtered. The filtrate was concentrated. The residue was purified by column chromatography (petroleum ether:ethyl acetate = 3:1) to give intermediate 7 (30 mg, yield: 37%). MS m/z (ESI): 550.1[M+1].

### Target compound:

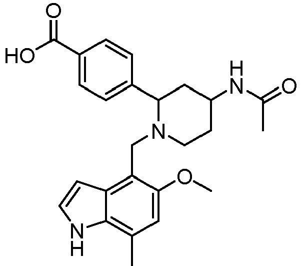

Intermediate 7 (30 mg) and sodium hydroxide (20 mg) were added to a mixed solution of methanol/water/tetrahydrofuran (0.5 mL/0.5 mL/0.5 mL) at room temperature. The reaction mixture was stirred at room temperature for 18 h. After the reaction was completed, diluted hydrochloric acid (2 M, 0.5 mL) was added to the reaction mixture with cooling in an ice bath to adjust the pH to 7. The residue was purified by preparative high-pressure liquid chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.1% formic acid); gradient: 0-20%) to give the target compound (4 mg, yield: 12.86%; comprising 0.3 equivalents of formic acid). MS m/z (ESI): 436[M+1]. ¹H NMR (400 MHz, CD₃OD) *δ*8.40 (s, 0.3H), 8.15 (d, *J* = 8.0 Hz, 2H), 7.64 (d, *J* = 8.0 Hz, 2H), 7.31 (d, *J* = 4.0 Hz, 1H), 6.75 (s, 1H), 6.30 (s, 1H), 4.53-4.41 (m, 1H), 4.34-4.23 (m, 1H), 4.15-3.98 (m, 2H), 3.75 (s, 3H), 3.50-3.43 (m, 1H), 3.27-3.23 (m, 1H), 2.49 (s, 3H), 2.31-2.22 (m, 1H), 2.11-2.03 (m, 2H), 1.91 (s, 3H), 1.83-1.70 (m, 1H).

### Example 19

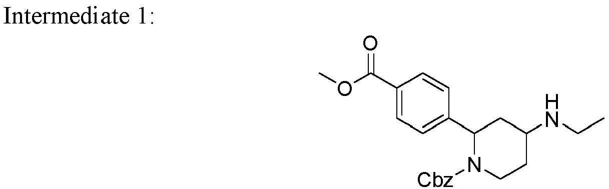

A solution of ethylamine in tetrahydrofuran (0.41 mL) was added to a solution of intermediate 2 (200 mg) of Example 1 in 1,2-dichloroethane (2 mL) at room temperature. After the reaction mixture was stirred at room temperature for 8 h, sodium triacetoxyborohydride (343 mg) was added, and the reaction mixture was successively stirred at room temperature for 18 h. After the reaction was completed, the reaction system was added with dichloromethane (10 mL) for dilution, washed with water (10 mL), dried over anhydrous sodium sulfate and filtered. The filtrate was concentrated under reduced pressure. The residue was purified by column chromatography (petroleum ether:ethyl acetate = 3:1) to give intermediate 1 (100 mg, yield: 44.4%). MS m/z (ESI): 397.3[M+1].

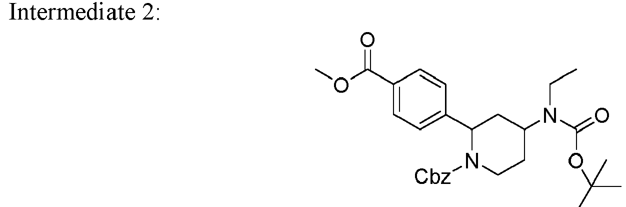

To a solution of intermediate 1 (200 mg) in dichloromethane (2 mL) were added di-*tert-*butyl dicarbonate (131 mg) and triethylamine (76 mg) at room temperature. The reaction mixture was stirred at room temperature for 18 h. After the reaction was completed, the reaction mixture was added with dichloromethane (10 mL) for dilution, washed with water (10 mL), dried over anhydrous sodium sulfate and filtered. The filtrate was concentrated under reduced pressure. The residue was purified by column chromatography (petroleum ether:ethyl acetate = 3:1) to give intermediate 2 (200 mg, yield: 76.5%). MS m/z (ESI): 497.3[M+1].

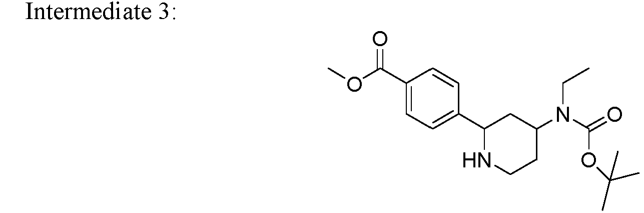

Palladium hydroxide/carbon (20 mg) was added to a solution of intermediate 2 (180 mg) in methanol (5 mL) at room temperature. The reaction mixture was stirred at room temperature under hydrogen atmosphere for 18 h. After the reaction was completed, the reaction mixture was filtered. The filtrate was concentrated under reduced pressure to give intermediate 3 (120 mg, yield: 82.8%). MS m/z (ESI): 363.3[M+1].

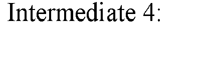

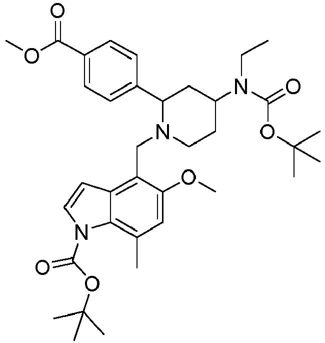

Intermediate 3 (150 mg) was added to a solution of intermediate 2 (142 mg) of Example 2 in 1,2-dichloroethane (2 mL) at room temperature. The reaction mixture was stirred at room temperature for 8 h. Sodium borohydride acetate (261 mg) was added. The reaction mixture was successively stirred at room temperature for 18 h. After the reaction was completed, the reaction mixture was added with dichloromethane (10 mL) for dilution, washed with water (10 mL), dried over anhydrous sodium sulfate and filtered. The filtrate was concentrated. The residue was purified by column chromatography (petroleum ether:ethyl acetate = 2:1) to give intermediate 4 (50 mg, yield: 17.24%). MS m/z (ESI): 636.3[M+1]. Intermediate 5:

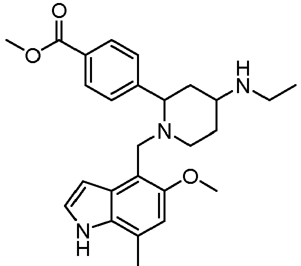

To a solution of intermediate 4 (40 mg) in dichloromethane (1 mL) was added trimethylbromosilane (0.2 mL) at room temperature. The reaction mixture was stirred at room temperature for 18 h. After the reaction was completed, the reaction mixture was directly concentrated under reduced pressure to give intermediate 5 (40 mg, yield: 99.67%). MS m/z (ESI): 436.3[M+1].

### Target compound:

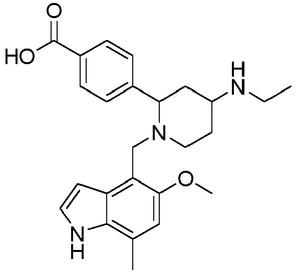

Intermediate 5 (40 mg) and sodium hydroxide (36 mg) were successively added to a mixed solution of methanol/water/tetrahydrofuran (0.5 mL/0.5 mL/0.5 mL) at room temperature. The reaction mixture was stirred at room temperature for 18 h. After the reaction was completed, diluted hydrochloric acid (2 M, 0.5 mL) was added to the reaction mixture with cooling in an ice bath to adjust the pH to about 7. The resulting mixture was concentrated under reduced pressure. The residue was purified by preparative high-pressure liquid chromatography (column: Gemini-C18, 150 × 21.2 mm, 5 µm; mobile phase: acetonitrile-water (0.1% formic acid); column temperature: 25 °C; flow rate: 14 mL/min; wavelength: 214 nm; column pressure: 80 bar; gradient: 0-20%) to give the target compound (4 mg, yield: 9.22%; comprising 2 equivalents of formic acid). MS m/z (ESI): 422[M+1]. ¹H NMR (400 MHz, CD₃OD) *δ* 8.43 (s, 2H), 8.09 (d, *J* = 8.0 Hz, 2H), 7.66 (d, *J* = 8.0 Hz, 2H), 7.21 (d, *J* = 2.8 Hz, 1H), 6.69 (s, 1H), 6.37 (d, *J* = 2.8 Hz, 1H), 3.87-3.81 (m, 1H), 3.75 (s, 3H), 3.62-3.56 (m, 1H), 3.42-3.36 (m, 1H), 3.21-3.13 (m, 1H), 3.07-2.98 (m, 2H), 2.51-2.37 (m, 4H), 2.22-2.16 (m, 1H), 2.06-2.00 (m, 1H), 1.90-1.78 (m, 1H), 1.72-1.55 (m, 2H), 0.91-0.80 (m, 3H).

### Example 20

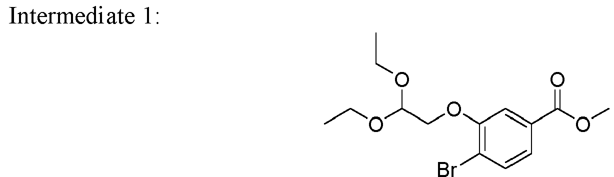

Potassium carbonate (4.47 g) was added to a solution of methyl 4-bromo-3-hydroxybenzoate (5 g) and bromoacetaldehyde diethyl acetal (4.68 g) in DMF (100 mL) at room temperature. The reaction mixture was heated to 100 °C and stirred at that temperature for 16 h. After the reaction was completed, the reaction mixture was poured into water (150 mL) and extracted with ethyl acetate (100 mL × 3). The combined organic phases were washed with saturated brine (100 mL × 3), dried over anhydrous sodium sulfate and filtered. The filtrate was concentrated under reduced pressure to give intermediate 1 (7.9 g, yield: 80%). MS m/z (ESI): 268.7[M+23].

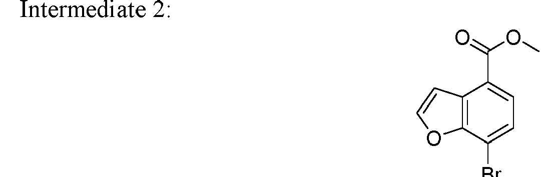

Polyphosphoric acid (3 g) was added to a solution of intermediate 1 (1 g) in chlorobenzene (15 mL) at room temperature. The reaction mixture was heated to 130 °C and stirred at that temperature for 16 h. After the reaction was completed, the reaction mixture was added with water (50 mL) for dilution and extracted with ethyl acetate (30 mL × 3). The combined extract phases were washed with saturated brine (20 mL × 3), dried over anhydrous sodium sulfate and filtered. The filtrate was concentrated under reduced pressure. The residue was purified by column chromatography (petroleum ether:ethyl acetate = 5:1) to give intermediate 2 (300 mg, yield: 40%). GC-MS m/z: 254, 256[M].

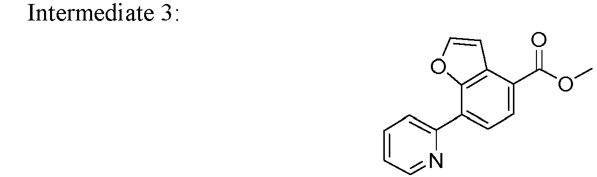

Bis(triphenylphosphine)palladium(II) chloride (54 mg) was added to a solution of intermediate 2 (200 mg), 2-(tributylstannyl)pyridine (344 mg) and copper(I) iodide (15 mg) in dioxane (15 mL) at room temperature. The reaction mixture was heated to 100 °C and stirred at that temperature for 16 h. After the reaction was completed, the reaction mixture was naturally cooled to room temperature and then concentrated under reduced pressure. The residue was purified by column chromatography (petroleum ether:ethyl acetate = 5:1) to give intermediate 3 (140 mg, yield: 70%). MS m/z (ESI): 253.9[M+1].

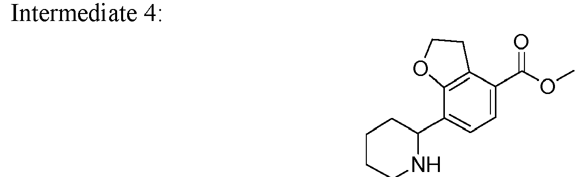

Platinum dioxide (10 mg) was added to a mixed solution of intermediate 3 (20 mg) in methanol/concentrated hydrochloric acid (4 mL/0.5 mL) at room temperature. The reaction mixture was stirred at room temperature in an autoclave with 4 atmospheres of hydrogen gas for 16 h. After the reaction was completed, the reaction mixture was filtered. The filtrate was concentrated under reduced pressure. The residue was purified by column chromatography (dichloromethane:methanol = 20:1) to give intermediate 4 (20 mg, yield: 95%). MS m/z (ESI): 261.9[M+1].

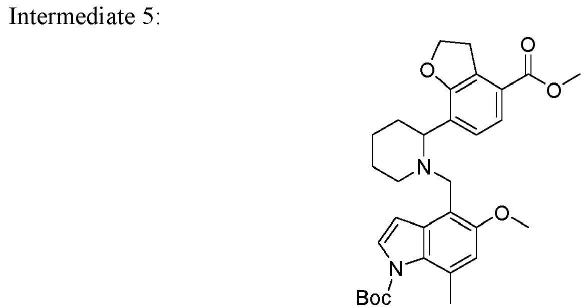

Tetraethyl titanate (83 mg) was added to a solution of intermediate 4 (99 mg) and intermediate 2 (110 mg) of Example 2 in tetrahydrofuran (10 mL) at room temperature. The reaction mixture was heated to 70 °C and stirred at that temperature for 16 h. After the reaction mixture was cooled to room temperature, sodium triacetoxyborohydride (241 mg, 1.14 mmol) was added. The reaction mixture was heated to 70 °C and stirred for 1 h. After the reaction was completed, the reaction mixture was concentrated under reduced pressure. The residue was purified by column chromatography (petroleum ether:ethyl acetate = 20:1) to give intermediate 5 (50 mg, yield: 24%). MS m/z (ESI): 534.8[M+1].

### Target compound:

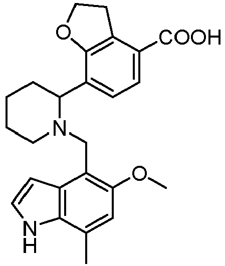

Sodium hydroxide (36 mg) was added to a mixed solution of intermediate 5 (50 mg) in methanol/water (4 mL/1 mL) at room temperature. The reaction mixture was stirred at room temperature for 16 h. After the reaction was completed, the reaction mixture was concentrated under reduced pressure. Hydrochloric acid (5 M, 1 mL) was added to the residue with cooling in an ice bath to adjust the pH to 5-6. The mixture was concentrated under reduced pressure. The resulting crude product was purified by preparative high-pressure liquid chromatography (column: Gemini-C18, 150 × 21.2 mm, 5 µm; mobile phase: acetonitrile-water (0.1% formic acid); gradient: 15-40%, column temperature: 25 °C; flow rate: 14 mL/min; wavelength: 214 nm; column pressure: 80 bar) to give the target compound (12 mg, yield: 31%, comprising 0.9 equivalents of formic acid). ¹H NMR (400 MHz, CD₃OD) *δ* 8.49 (s, 0.9H), 7.54 (d, *J* = 8.0 Hz, 1H), 7.34 (d, *J* = 8.0 Hz, 1H), 7.29 (d, *J* = 2.8 Hz, 1H), 6.76 (s, 1H), 6.29 (d, *J* = 2.8 Hz, 1H), 4.69 (t, *J* = 9.0 Hz, 2H), 4.56 (d, *J* = 10.4 Hz, 1H), 4.44 (d, *J* = 12.7 Hz, 1H), 4.18 (d, *J* = 12.7 Hz, 1H), 3.78 (s, 3H), 3.58 (t, *J* = 8.8 Hz, 2H), 3.51 (d, *J* = 13.2 Hz, 1H), 3.24-3.22 (m, 1H), 2.50 (s, 3H), 2.23-2.17 (m, 1H), 2.10-1.62 (m, 5H). MS m/z (ESI): 421.0[M+1].

### Example 21

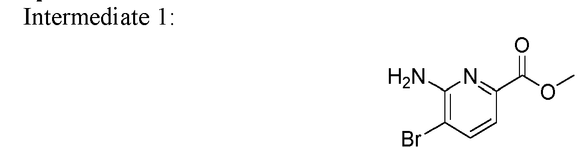

A solution of liquid bromine (10.13 g, 0.0634 mmol) in chloroform (70 mL) was slowly added dropwise to a solution of methyl 6-aminopyridine-2-carboxylate (9.64 g, 0.0634 mol) in chloroform (408 mL) at room temperature over 60 min. After the dropwise addition was completed, the reaction mixture was stirred at room temperature for 18 h. After the reaction was completed, the reaction mixture was quenched by adding saturated sodium thiosulfate solution (150 mL). The phases were separated. The aqueous phase was extracted with dichloromethane (150 mL). The combined organic phases were washed with an aqueous solution (10 mL × 3), dried over anhydrous sodium sulfate and filtered. The filtrate was concentrated. The residue was purified by column chromatography (petroleum ether:ethyl acetate = 2:1) to give intermediate 1 (1.1 g, yield: 7.10%). MS m/z (ESI): 230.8[M+23].

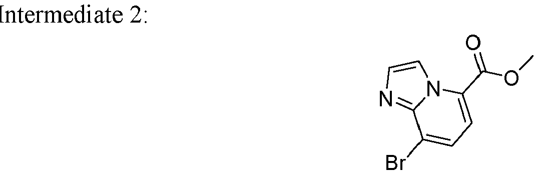

Intermediate 1 (1 g) was added to a 40% solution of chloroacetaldehyde (1.69 g) in isopropanol (20 mL) at room temperature. The reaction mixture was heated to 80 °C and stirred at that temperature for 18 h. After the reaction was completed, the reaction system was naturally cooled to room temperature and concentrated under reduced pressure. The residue was purified by column chromatography (petroleum ether:ethyl acetate = 3:1) to give intermediate 2 (1 g, yield: 86.05%). MS m/z (ESI): 254.8[M+23].

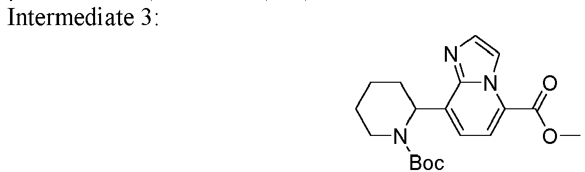

1-(*tert-*Butoxycarbonyl)piperidine-2-carboxylic acid (1.35 g), iridium reagent (Ir[dF(CF₃)ppy]₂(dtbppy))PF₆ (CAS No.: 870987-63-6, 43.98 mg), nickel chloride ethylene glycol dimethyl ether complex (86.25 mg), 4,4'-di-*tert*-butyl-2,2'-bipyridine (157.84 mg) and cesium carbonate (3832.13 mg) were successively added to a solution of intermediate 2 (1 g) in DMF (20 mL) at room temperature. The reaction system was purged three times with nitrogen, then placed in an LED blue light reactor (26W, Compact fluorescent light, 300-400 nM) and reacted for 48 h. After the reaction was completed, the reaction mixture was added with ethyl acetate (200 mL) for dilution, washed with water (200 mL × 5), dried over anhydrous sodium sulfate and filtered. The filtrate was concentrated under reduced pressure. The residue was purified by column chromatography (petroleum ether:ethyl acetate = 3:1) to give intermediate 3 (300 mg, yield: 20.17%). MS m/z (ESI): 360[M+23].

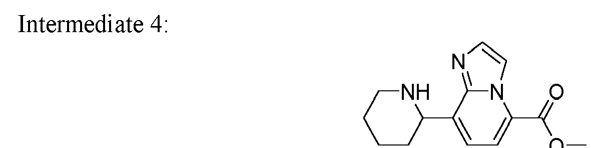

Trifluoroacetic acid (0.6 mL) was added to a solution of intermediate 3 (300 mg, 0.832 mmol) in dichloromethane (3 mL) at room temperature. The reaction mixture was stirred at room temperature for 18 h. After the reaction was completed, the reaction liquid was directly concentrated under reduced pressure to give intermediate 4 (100 mg, yield: 46%). MS m/z (ESI): 260.2[M+23].

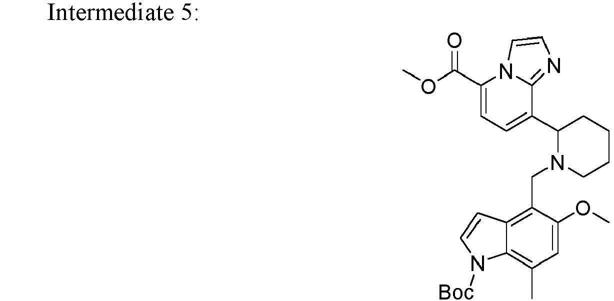

Intermediate 4 (100 mg) was added to a solution of intermediate 2 (134 mg) of Example 2 in 1,2-dichloroethane (2 mL) at room temperature. After the reaction mixture was stirred at room temperature for 8 h, sodium triacetoxyborohydride (245.2 mg, 1.16 mmol) was added, and the reaction mixture was successively stirred at room temperature for 18 h. After the reaction was completed, the reaction mixture was added with dichloromethane (10 mL) for dilution, washed with water (10 mL), dried over anhydrous sodium sulfate and filtered. The filtrate was concentrated under reduced pressure. The residue was purified by column chromatography (petroleum ether:ethyl acetate = 3:1) to give intermediate 5 (100 mg, yield: 46.16%). MS m/z (ESI): 533.3[M+23].

### Target compound:

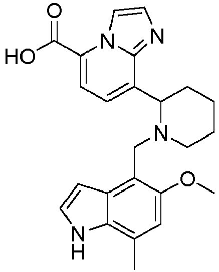

Sodium hydroxide (75 mg) was added to intermediate 5 (100 mg) in tetrahydrofuran/methanol/water (0.5 mL/0.5 mL/0.5 mL) at room temperature. The reaction mixture was stirred at room temperature for 18 h. After the reaction was completed, the reaction mixture was concentrated under reduced pressure to remove the solvent. The residue was purified by preparative high-pressure liquid chromatography (column: Gemini-C18, 150 × 21.2 mm, 5 µm; mobile phase: acetonitrile-water (0.1% formic acid); column temperature: 25 °C; flow rate: 14 mL/min; wavelength: 214 nm; column pressure: 80 bar, gradient: 0-70%) to give the target compound (21 mg, yield: 26.52%). ¹H-NMR (400 MHz, MeOD) *δ* 8.96 (s, 1H), 7.77 (s, 1H), 7.59 (d, *J* = 8.2 Hz, 1H), 7.46 (d, *J* = 8.2 Hz, 1H), 7.26 (d, *J* = 3.2 Hz, 1H), 6.63 (s, 1H), 6.26 (d, *J* = 3.2 Hz, 1H), 4.95-4.92 (m, 1H), 4.42-4.27 (m, 2H), 3.77 (s, 3H), 3.60-3.52 (m, 1H), 3.27-3.20 (m, 1H), 2.65-2.47 (m, 1H), 2.45 (s, 3H), 2.20-2.10 (m, 1H), 2.05-1.88 (m, 3H), 1.86-1.72 (m, 1H). MS m/z (ESI): 419.2[M+23].

### Example 22

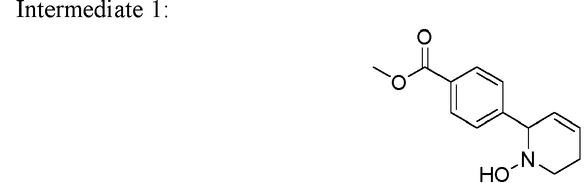

A 1.3 M solution of isopropylmagnesium chloride lithium chloride in tetrahydrofuran (36 mL) was added to a solution of methyl 4-iodo-benzoate (9.9 g) in tetrahydrofuran (100 mL) at - 40 °C under nitrogen atmosphere. The reaction mixture was stirred at -40 °C for 1 h. A solution of pyridine nitroxide (3.0 g) in tetrahydrofuran (50 mL) was added. The reaction mixture was stirred at -40 °C for 1 h. A solution of sodium borohydride (1438 mg, 37.85 mmol) in methanol (50 mL) was added. The reaction mixture was successively stirred at -40 °C for 1 h, slowly warmed to room temperature and successively stirred for 16 h. After the reaction was completed, saturated ammonium chloride solution (100 mL) was added to the reaction mixture. After the mixture was stirred for half an hour, water (400 mL) was added for dilution, followed by the extraction with ethyl acetate (200 mL). The extract phase was washed with saturated brine (400 mL), dried over anhydrous sodium sulfate and filtered. The filtrate was concentrated. The residue was purified by column chromatography (dichloromethane:methanol = 20:1) to give intermediate 1 (2.0 g, yield: 22%). MS m/z (ESI): 234.1[M+1].

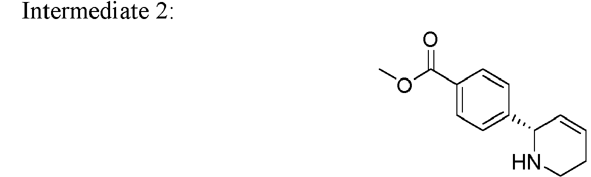

Zinc powder (2.8 g) and water (20 mL) were added to a solution of intermediate 1 (2 g) in acetic acid (20 mL) at room temperature. The reaction mixture was heated to 50 °C and stirred at that temperature for 2 h. After the reaction was completed, the mixture was filtered. The filtrate was concentrated under reduced pressure. The residue was added with water (50 mL) for dilution. A 2 M solution of sodium hydroxide solution was added to the dilution with cooling in an ice bath to adjust the pH to 8-10, followed by the extraction with ethyl acetate (50 mL). The extract phase was washed with saturated brine (100 mL), dried over anhydrous sodium sulfate and filtered. The filtrate was concentrated under reduced pressure. The crude product was resolved by preparative supercritical chiral chromatography (column: chiralpak-AD-H, 250 × 20 mm, 5 µm; column temperature: 40 °C; flow rate: 40 g/min; wavelength: 214 nm; gradient: methanol (0.2% aqueous ammonia)/carbon dioxide = 35/65; back pressure: 100 bar) to give intermediate 2 (670 mg, yield: 35%). MS m/z (ESI): 218.1[M+1].

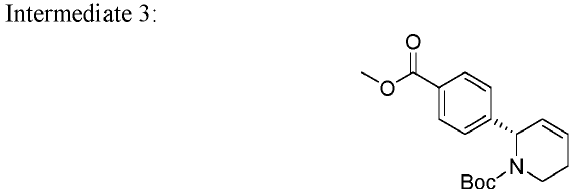

BoczO (2.0 g) and triethylamine (865 mg) were added to a solution of intermediate 2 (930 mg) in dichloromethane (10 mL) at room temperature. The reaction mixture was stirred at room temperature for 16 h. After the reaction was completed, the reaction mixture was directly concentrated. The residue was purified by column chromatography (petroleum ether:ethyl acetate = 10:1) to give intermediate 3 (1.4 g, yield: 92%). MS m/z (ESI): 340.0[M+23].

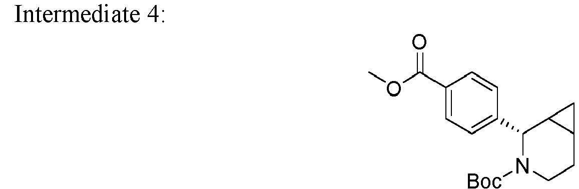

A solution of diazomethane (1 M, 6.3 mL) in ethyl ether was slowly added dropwise to a solution of intermediate 3 (200 mg) and palladium acetate (20 mg, 10%) in ethyl ether (2 mL) with cooling in an ice bath under nitrogen atmosphere. The reaction mixture was stirred for 2 h with cooling in the ice bath under nitrogen atmosphere. After the reaction was completed, the reaction mixture was filtered. The filtrate was directly concentrated to give a crude product. Monitoring showed that only part of the starting materials were converted into the target product. After the reaction procedure described above was repeated four times, all intermediate 3 was substantially converted. The resulting crude product was purified by preparative high-pressure liquid chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.1% formic acid); gradient: 60-80%) to give intermediate 4 (70 mg, yield: 32%). MS m/z (ESI): 354.0[M+23].

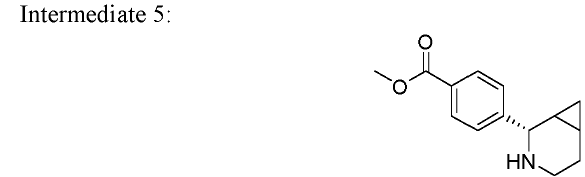

To a solution of intermediate 4 (70 mg) in dichloromethane (1 mL) was added a 4 M solution of hydrochloride in dioxane (0.5 mL) at room temperature. The reaction mixture was stirred at room temperature for 2 h. After the reaction was completed, the reaction mixture was directly concentrated under reduced pressure to give intermediate 5 (70 mg, purity: 50%, yield: 72%). MS m/z (ESI): 232.2[M+1].

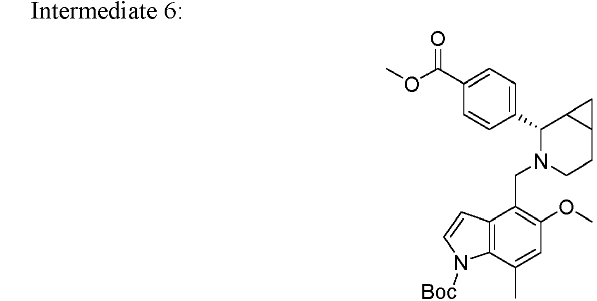

Intermediate 5 (70 mg, 0.30 mmol) was added to a solution of intermediate 2 (88 mg) of Example 2 in 1,2-dichloroethane (2 mL) at room temperature. The reaction mixture was stirred at room temperature for 8 h. Then sodium triacetoxyborohydride (192 mg) was added, and the mixture was successively stirred at room temperature for 16 h. After the reaction was completed, the reaction mixture was directly concentrated under reduced pressure. The residue was purified by column chromatography (dichloromethane:methanol = 20:1) to give intermediate 6 (90 mg, yield: 53%). MS m/z (ESI): 505.1[M+1].

### Target compound:

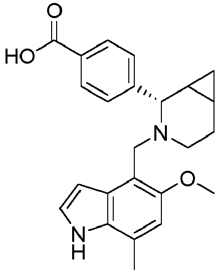

Sodium hydroxide (143 mg) was added to a mixed solution of intermediate 6 (90 mg) in methanol/water (3 mL/3 mL) at room temperature. The reaction mixture was heated to 80 °C and stirred at that temperature for 8 h. After the reaction was completed, diluted hydrochloric acid (2 M) was added to the reaction system with cooling in an ice bath to adjust the pH to about 7. The resulting mixture was directly lyophilized to remove the solvent. The residue was purified by preparative high-pressure liquid chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.1% formic acid); gradient: 15-40%) to give the target compound (30 mg, yield: 43%, comprising 0.3 equivalents of formic acid). ¹H NMR (400 MHz, CD₃OD) δ8.47 (s, 0.3H), 8.20 (d, *J* = 8.4 Hz, 2H), 7.74 (d, *J* = 8.4 Hz, 2H), 7.30 (d, *J* = 3.1 Hz, 1H), 6.74 (s, 1H), 6.15 (d, *J* = 3.1 Hz, 1H), 4.44 (d, *J* = 3.0 Hz, 1H), 4.35 (d, *J* = 12.8 Hz, 1H), 4.07 (d, *J* = 12.8 Hz, 1H), 3.68 (s, 3H), 3.48-3.34 (m, 1H), 3.02 (td, *J* = 13.2, 4.0 Hz, 1H), 2.51 (s, 3H), 2.44-2.30 (m, 1H), 2.11-1.98 (m, 1H), 1.41-1.29 (m, 2H), 1.13-1.02 (m, 1H), 0.87-0.78 (m, 1H). MS m/z (ESI): 391.1[M+1].

### Example 23

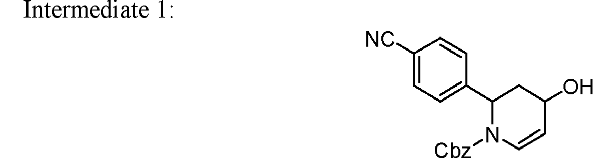

Sodium borohydride (1.1 g) was added to a solution of intermediate 1 (5.0 g) of Example 1 and cerium trichloride (3.7 g) in methanol (80 mL) at room temperature. The reaction mixture was stirred at room temperature for 3 h, supplemented with sodium borohydride (1.1 g) and successively stirred at room temperature for 2 h. After the reaction was completed, the reaction mixture was concentrated under reduced pressure. The residue was dissolved in water (80 mL) and extracted with ethyl acetate (50 mL × 3). The combined organic phases were washed with saturated brine (50 mL × 2), dried over anhydrous sodium sulfate and filtered. The filtrate was concentrated under reduced pressure to give intermediate 1 (4.6 g, yield: 87%). MS m/z (ESI): 356.8[M+23].

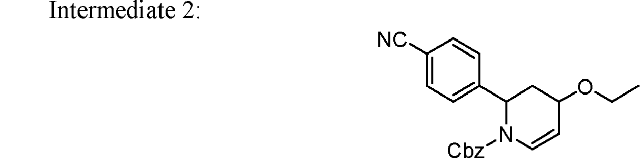

Sodium hydride (660 mg, 60%) was added to a solution of intermediate 1 (4.6 g) in DMF (50 mL) with cooling in an ice bath. After the reaction mixture was stirred for 5 min, iodoethane (3.2 g) was added. The reaction mixture was stirred at room temperature for 3 h. After the reaction was completed, water (0.5 mL) was added for quenching. Then the reaction mixture was concentrated under reduced pressure. The residue was purified by column chromatography (petroleum ether:ethyl acetate = 10:1) to give intermediate 2 (1.5 g, yield: 30%). MS m/z (ESI): 384.9[M+23].

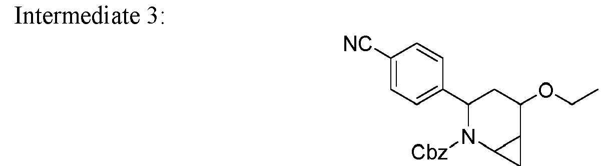

A solution of diethylzinc in hexane (1 mol/L, 3 mL) was added to a solution of intermediate 2 (1.1 g) and diiodomethane (620 mg) in dichloromethane (20 mL) with cooling in an ice bath. The reaction mixture was stirred at room temperature for 16 h. After the reaction was completed, the reaction mixture was directly concentrated under reduced pressure. The residue was purified by column chromatography (petroleum ether:ethyl acetate = 5:1) to give intermediate 3 (950 mg, yield: 29%). MS m/z (ESI): 376.9[M+H].

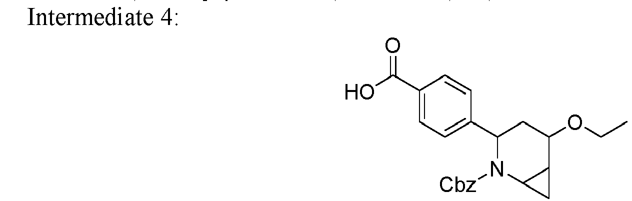

An aqueous solution of sodium hydroxide (1.01 g) was added to a solution of intermediate 3 (950 mg) in ethanol/water (20 mL/7 mL) at room temperature. The reaction mixture was heated to 90 °C and stirred at that temperature for 16 h. After the reaction was completed, the reaction mixture was concentrated under reduced pressure to remove ethanol. Water (15 mL) was added to the residue. The pH was adjusted to 4-5 with a 5 M diluted hydrochloric acid solution, followed by the extraction with ethyl acetate (20 mL × 3). The combined organic phases were washed with saturated brine (20 mL × 2), dried over anhydrous sodium sulfate and filtered. The filtrate was concentrated under reduced pressure to give intermediate 4 (810 mg, yield: 81%). MS m/z (ESI): 395.9[M+H].

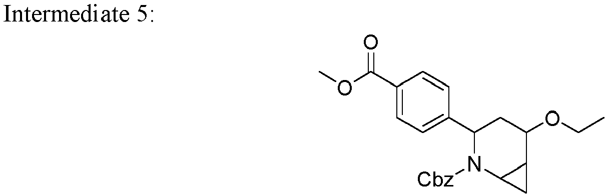

Thionyl chloride (1.5 mL) was added to a solution of intermediate 4 (810 mg) in methanol (15 mL) at room temperature. The reaction mixture was stirred at room temperature for 16 h. The reaction mixture was directly concentrated under reduced pressure to give intermediate 5 (810 mg, yield 81%). MS m/z (ESI): 409.9[M+H].

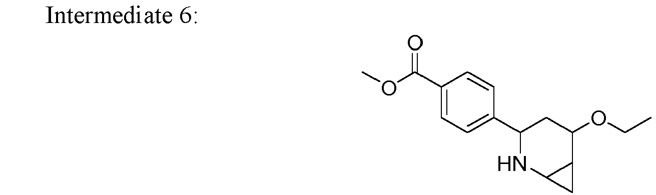

Palladium acetate (109 mg) was added to a solution of intermediate 5 (400 mg), triethylamine (493 mg) and triethylsilane (1.76 g) in dichloromethane (15 mL) at room temperature. The reaction mixture was stirred at room temperature for 16 h. After the reaction was completed, the reaction mixture was concentrated under reduced pressure. The residue was purified by column chromatography (petroleum ether:ethyl acetate = 2:1) to give intermediate 6 (400 mg, yield: 74%). MS m/z (ESI): 276.0[M+H].

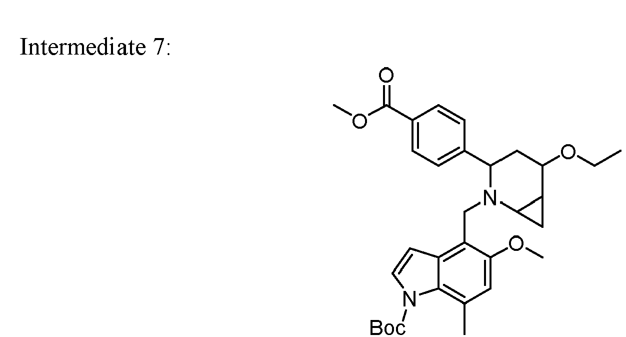

Sodium triacetoxyborohydride (219 mg) was added to a solution of intermediate 6 (94 mg, 0.344 mmol) and intermediate 2 (100 mg) of Example 2 in 1,2-dichloroethane (10 mL) at room temperature. The reaction mixture was stirred at room temperature for 3 h. After the reaction was completed, the reaction mixture was concentrated under reduced pressure. The residue was purified by column chromatography (petroleum ether:ethyl acetate = 2:1) to give intermediate 7 (130 mg, yield: 68%). MS m/z (ESI): 548.8[M+H].

### Target compound:

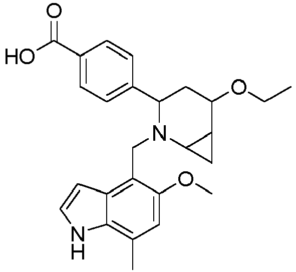

Sodium hydroxide (102 mg) was added to a mixed solution of intermediate 7 (130 mg) in methanol/water (8 mL/2 mL) at room temperature. The reaction mixture was stirred at room temperature for 48 h. After the reaction was completed, the reaction mixture was directly concentrated under reduced pressure to remove the solvent. The residue was purified by preparative high-pressure liquid chromatography (column: Gemini-C18, 150 × 21.2 mm, 5 µm; mobile phase: acetonitrile-water (0.1% formic acid); gradient: 20-40%, column temperature: 25 °C; flow rate: 14 mL/min; wavelength: 214 nm; column pressure: 80 bar) to give the target compound (30 mg, yield: 27%). MS m/z (ESI): 434.9[M+1].¹H NMR (400 MHz, MeOD) *δ* 8.12 (d, *J* = 7.6 Hz, 2H), 7.64 (d, *J* = 7.6 Hz, 2H), 7.29 (d, *J* = 3.1 Hz, 2H), 6.73 (s, 1H), 6.45 (d, *J* = 3.1 Hz, 1H), 4.33 (d, *J* = 12.0 Hz, 1H), 4.27-4.17 (m, 2H), 4.16-4.08 (m, 1H), 3.83-3.67 (m, 4H), 3.52-3.42 (m, 1H), 2.77-2.67 (m, 1H), 2.49 (s, 3H), 2.34-2.21 (m, 1H), 1.84 (d, *J* = 15.0 Hz, 1H), 1.79-1.61 (m, 2H), 1.26 (t, *J* = 7.0 Hz, 3H), 1.00-0.90 (m, 1H).

### Example 24

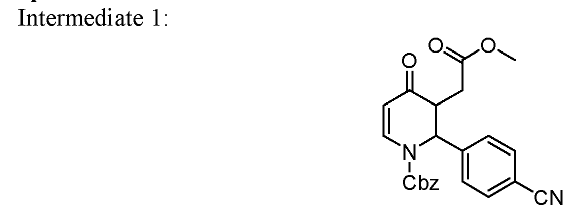

Lithium bis(trimethylsilyl)amide (4.5 mL, 4.5 mmol) was slowly added dropwise to a solution of intermediate 1 (1 g, 3.0 mmol) of Example 1 in THF (4 mL) at -78 °C. After the reaction was carried out at -78 °C for 1 h, methyl bromoacetate (1.4 g, 9.0 mmol) was slowly added to the reaction system at that temperature. After being stirred at that temperature for 1 h, the reaction system was naturally warmed to room temperature and stirred at room temperature overnight. After the reaction was completed, the reaction mixture was added with water (30 mL) for dilution and extracted with ethyl acetate (30 mL × 3). The combined extract phases were washed with saturated brine (100 mL), dried over anhydrous sodium sulfate and filtered. The filtrate was concentrated. The residue was purified by column chromatography (dichloromethane:methanol = 5:1) to give intermediate 1 (330 mg, yield: 28%). MS m/z (ESI): 405.2[M+1].

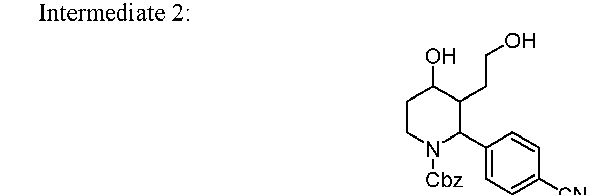

Sodium borohydride (1.2 g, 32 mmol) was slowly added to a solution of intermediate 1 (1.6 g, 4.0 mmol) in methanol (30 mL) at 0 °C. The reaction mixture was stirred at room temperature for 16 h. After the reaction was completed, the reaction system was quenched by adding water (100 mL) and extracted with ethyl acetate (100 mL × 3). The combined extract phases were washed with saturated brine (200 mL), dried over anhydrous sodium sulfate and filtered. The filtrate was concentrated. The residue was purified by column chromatography (dichloromethane:methanol = 2:1) to give intermediate 2 (900 mg, yield: 60%). MS m/z (ESI): 381.1[M+1].

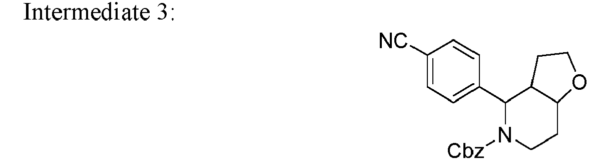

*p*-Toluenesulfonyl chloride (1.8 g, 9.5 mmol) was slowly added to intermediate 2 (1.2 g, 3.2 mmol), 4-dimethylaminopyridine (77 mg, 0.6 mmol) and triethylamine (957 mg, 9.5 mmol) in dichloromethane (40 mL) at room temperature. The reaction mixture was stirred at room temperature overnight. After the reaction was completed, the reaction mixture was washed with water (10 mL), dried over anhydrous sodium sulfate and filtered. The filtrate was concentrated. The residue was purified by column chromatography (dichloromethane:methanol = 3:1) to give intermediate 3 (500 mg, yield: 44%). MS m/z (ESI): 386.1[M+23].

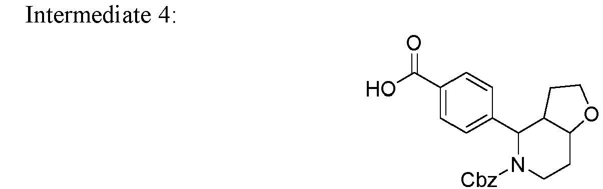

Intermediate 3 (500 mg, 1.4 mmol) and barium hydroxide (1.6 g, 5.0 mmol) were added to a mixed system of isopropanol/water (5 mL/12.5 mL) at room temperature. The reaction mixture was heated to 100 °C and stirred at that temperature for 16 h. After the reaction was completed, a diluted aqueous hydrochloric acid solution (2 M) was added to the reaction mixture to adjust the pH to about 2, followed by the extraction with ethyl acetate (30 mL × 3). The combined extract phases were washed with saturated brine (50 mL), dried over anhydrous sodium sulfate and filtered. The filtrate was concentrated to give intermediate 4 (300 mg, yield: 57%). MS m/z (ESI): 404.1[M+23].

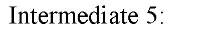

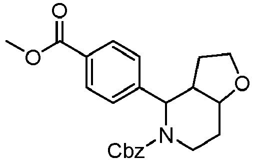

Iodomethane (224 mg, 1.6 mmol) was added to a solution of intermediate 4 (300 mg, 0.8 mmol) and potassium carbonate (217 mg, 1.6 mmol) in *N*,*N*-dimethylformamide (5 mL) at room temperature. The reaction mixture was stirred at room temperature overnight. After the reaction was completed, the reaction mixture was added with water (20 mL) for dilution and extracted with ethyl acetate (20 mL × 3). After being combined, the organic phases of the extraction were washed with saturated brine (30 mL × 4), dried over anhydrous sodium sulfate and filtered. The filtrate was concentrated to give intermediate 5 (300 mg, yield: 96%). MS m/z (ESI): 418.1[M+23].

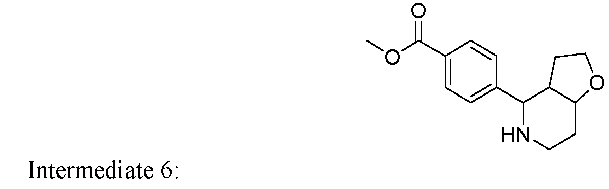

Palladium acetate (85 mg, 0.4 mmol) was added to a solution of intermediate 5 (300 mg, 0.8 mmol), triethylsilane (881 mg, 7.6 mmol) and triethylamine (384 mg, 3.8 mmol) in dichloromethane (10 mL) at room temperature. The reaction mixture was stirred at room temperature for 3 h. After the reaction was completed, the reaction mixture was filtered. The filtrate was concentrated. The residue was purified by reversed-phase C18 column chromatography (acetonitrile:methanol = 10:1) to give intermediate 6 (200 mg, yield: 77%). MS m/z (ESI): 262.1[M+1].

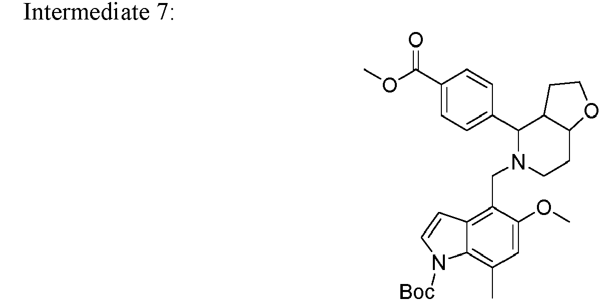

Intermediate 6 (100 mg, 0.383 mmol) was added to a solution of intermediate 2 (110 mg, 1.91 mmol) of Example 2, silatrane (200 mg, 1.53 mmol) and glacial acetic acid (4 drops) in tetrahydrofuran (5 mL) at room temperature. The reaction mixture was heated to 75 °C and stirred at that temperature for 24 h. After the reaction was completed, the reaction mixture was added with water (50 mL) for dilution and extracted with ethyl acetate (50 mL × 3). The combined extract phases were washed with saturated brine (50 mL), dried over anhydrous sodium sulfate and filtered. The filtrate was concentrated. The residue was purified by column chromatography (petroleum ether:ethyl acetate = 5:1) to give intermediate 7 (110 mg, yield: 53%). MS m/z (ESI): 534.7[M+1].

### Target compound:

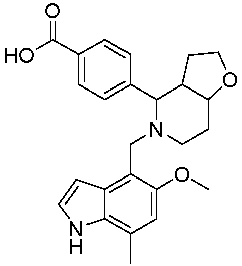

Sodium hydroxide (150 mg, 3.73 mmol) was added to a mixed solution of intermediate 7 (100 mg, 0.19 mmol) in methanol and water (2 mL/2 mL) at room temperature. The reaction mixture was stirred at room temperature for 48 h. After the reaction was completed, diluted hydrochloric acid (2 M) was added dropwise to the reaction mixture to adjust the pH to about 5-7. The resulting mixture was concentrated under reduced pressure to remove the solvent. The residue was purified by preparative high-pressure liquid chromatography (column: AQ-C18; 150 × 21.2 mm, 5 µm; column temperature: 25 °C; flow rate: 20 mL/min; wavelength: 214 nm; column pressure: 80 bar; mobile phase: acetonitrile-water (0.1% formic acid); gradient: 10-30%) to give the target compound (29.2 mg, yield: 37%, comprising 0.4 equivalents of formic acid). MS m/z (ESI): 420.8[M+1]. ¹H NMR (400 MHz, CD₃OD): *δ*8.32 (s, 0.4H), 8.17 (d, *J* = 7.9 Hz, 2H), 7.74-7.62 (m, 2H), 7.29 (d, *J* = 3.1 Hz, 1H), 6.72 (s, 1H), 6.26 (d, *J* = 2.8 Hz, 1H), 4.55-4.04 (m, 3H), 4.01-3.85 (m, 2H), 3.74-3.69 (m, 3H), 3.62-3.53 (m, 1H), 3.49-3.32 (m, 1H), 2.48 (s, 3H), 2.27-2.10 (m, 2H), 2.09-1.81 (m, 1H), 1.79-1.42 (m, 2H).

### Example 25

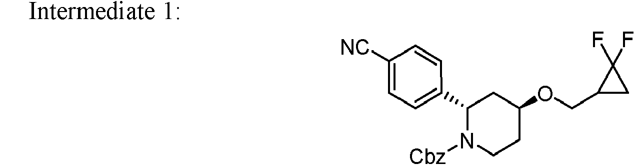

2,2-Difluorocyclopropane-1-carbaldehyde (320 mg) and trimethylsilyl trifluoromethanesulfonate (168 mg) were successively added to a solution of intermediate 1 (680 mg) of Example 6 in dichloromethane (5 mL) at -78 °C under nitrogen atmosphere. After the reaction mixture was stirred at -78 °C for one hour, triethylsilane (351 mg) was added. The reaction mixture was naturally warmed to room temperature and successively reacted at room temperature for 16 h. After the reaction was completed, the reaction mixture was concentrated under reduced pressure. The resulting residue was purified by column chromatography (petroleum ether:ethyl acetate = 3:1) to give intermediate 1 (100 mg, yield: 14%). MS m/z (ESI): 426.8[M+1].

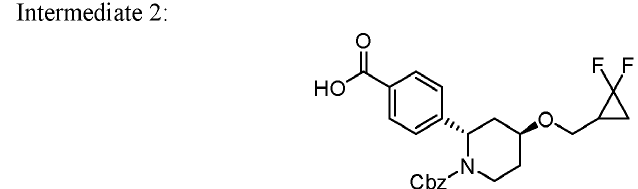

Intermediate 1 (300 mg, 0.70 mmol) and sodium hydroxide (563 mg, 14.07 mmol) were added to a mixed solution of isopropanol/water (2.5 mL/2.5 mL) at room temperature. The reaction mixture was heated to 100 °C and stirred at that temperature for 24 h. After the reaction was completed, diluted hydrochloric acid (2 M, 7.5 mL) was slowly added to the reaction mixture with cooling in an ice bath to adjust the pH to 5-6. Water (20 mL) was added for dilution, followed by the extraction with ethyl acetate (10 mL). The extract phase was washed with saturated brine (20 mL), dried over anhydrous sodium sulfate and filtered. The filtrate was concentrated under reduced pressure to give intermediate 2 (270 mg, yield: 77%). MS m/z (ESI): 445.8[M+1].

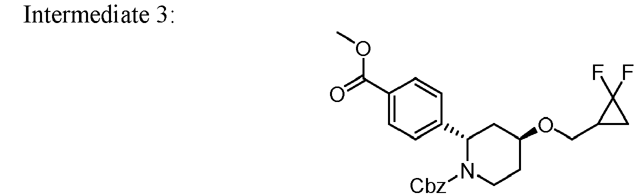

Potassium carbonate (167 mg) and iodomethane (172 mg) were added to a solution of intermediate 2 (270 mg) in acetonitrile (3 mL) at room temperature. The reaction mixture was heated to 50 °C and stirred at that temperature for 2 h. After the reaction was completed, the reaction mixture was directly concentrated under reduced pressure. The residue was purified by column chromatography (petroleum ether:ethyl acetate = 3:1) to give intermediate 3 (190 mg, yield: 61%). MS m/z (ESI): 481.8[M+23].

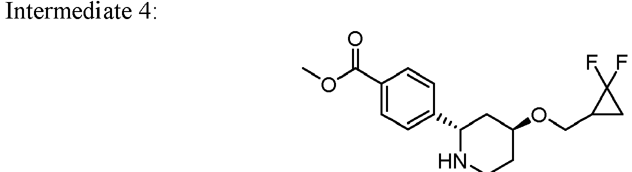

To a solution of intermediate 3 (190 mg) in tetrahydrofuran (3 mL) was added palladium/carbon (50 mg) at room temperature under nitrogen atmosphere. The reaction mixture was subjected to a catalytic hydrogenation reaction under hydrogen atmosphere at room temperature for 16 h. After the reaction was completed, the reaction mixture was filtered. The filtrate was directly concentrated to give intermediate 4 (135 mg, yield: 90%). MS m/z (ESI): 325.9[M+1].

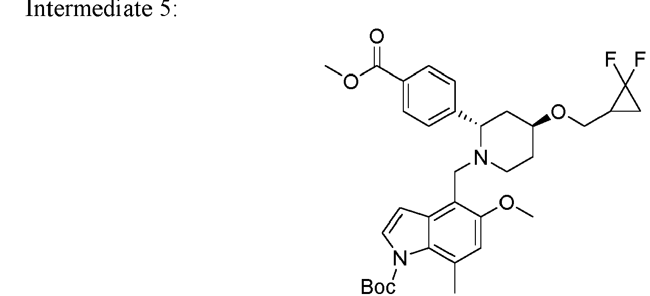

Intermediate 4 (135 mg) was added to a solution of intermediate 2 (132 mg) of Example 2 in 1,2-dichloroethane (3 mL) at room temperature. The reaction mixture was stirred at room temperature for 8 h. Then sodium triacetoxyborohydride (264 mg) was added, and the reaction mixture was successively reacted at room temperature for 16 h. After the reaction was completed, the reaction mixture was directly concentrated under reduced pressure. The residue was purified by column chromatography (dichloromethane:methanol = 20:1) to give intermediate 5 (160 mg, yield: 52%). MS m/z (ESI): 598.7[M+1].

### Target compound:

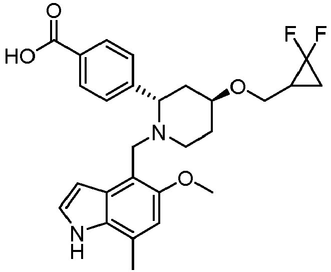

Sodium hydroxide (214 mg) was added to a mixed solution of intermediate 5 (160 mg) in methanol/water (2 mL/2 mL) at room temperature. The reaction mixture was heated to 80 °C and stirred at that temperature for 24 h. After the reaction was completed, diluted hydrochloric acid (2 M, 2.7 mL) was added to the reaction mixture with cooling in an ice bath to adjust the pH to about 7. The resulting mixture was directly lyophilized to remove the solvent. The residue was purified by preparative high-pressure liquid chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.1% formic acid); gradient: 20-40%) to give the target compound (50.7 mg, yield: 39%, comprising 0.2 equivalents of formic acid). MS m/z (ESI): 484.8[M+1]. ¹H NMR (400 MHz, MeOD) δ8.41 (s, 0.2H), 8.16 (d, *J* = 8.2 Hz, 2H), 7.72-7.60 (m, 2H), 7.31 (d, J = 3.1 Hz, 1H), 6.75 (s, 1H), 6.42-6.20 (m, 1H), 4.82-4.66 (m, 1H), 4.37-4.12 (m, 2H), 3.97-3.82 (m, 1H), 3.81-3.68 (m, 4H), 3.58-3.45 (m, 2H), 3.44-3.32 (m, 1H), 2.50 (s, 3H), 2.34-2.17 (m, 2H), 2.10-1.95 (m, 3H), 1.62-1.51 (m, 1H), 1.28-1.19 (m, 1H).

### Example 26

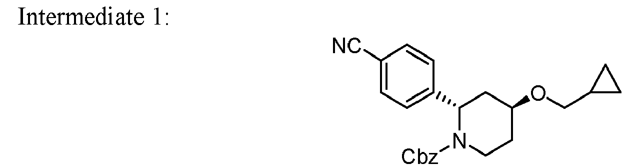

A solution of intermediate 1 (930 mg) of Example 5 in dichloromethane (20 mL) was cooled to -78 °C under nitrogen atmosphere. Then cyclopropanecarboxaldehyde (289 mg) and trimethylsilyl trifluoromethanesulfonate (229 mg) were successively added. After the reaction mixture was stirred at -78 °C for one hour, triethylsilane (479 mg) was added, and then the reaction mixture was naturally warmed to room temperature and successively stirred at room temperature for 16 h. After the reaction was completed, the reaction mixture was concentrated under reduced pressure. The resulting residue was purified by column chromatography (petroleum ether:ethyl acetate = 3:1) to give intermediate 1 (600 mg, yield: 67%). MS m/z (ESI): 390.9[M+1]. Intermediate 2:

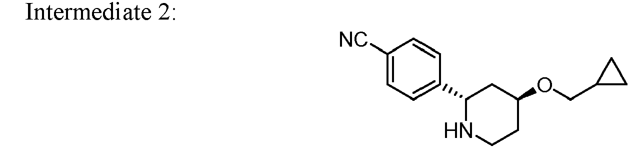

To a solution of intermediate 1 (340 mg) in tetrahydrofuran (3 mL) was added palladium/carbon (50 mg) at room temperature under nitrogen atmosphere. The reaction mixture was subjected to a catalytic hydrogenation reaction at room temperature under hydrogen atmosphere for 16 h. After the reaction was completed, the reaction mixture was filtered. The filtrate was directly concentrated under reduced pressure to give intermediate 2 (220 mg, yield: 89%). MS m/z (ESI): 257.0[M+1].

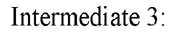

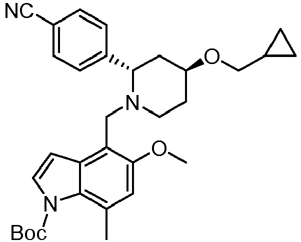

Intermediate 2 (220 mg) was added to a solution of intermediate 2 (248 mg) of Example 2 in 1,2-dichloroethane (5 mL) at room temperature. The reaction mixture was stirred at room temperature for 8 h. Then sodium triacetoxyborohydride (546 mg) was added, and the mixture was successively stirred at room temperature for 16 h. After the reaction was completed, the reaction mixture was directly concentrated under reduced pressure. The residue was purified by column chromatography (dichloromethane:methanol = 20:1) to give intermediate 3 (400 mg, yield: 61%). MS m/z (ESI): 529.8[M+1].

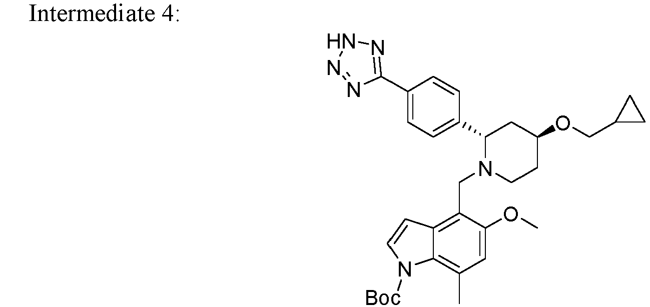

Trimethylsilyl azide (174 mg) and dibutyltin diacetate (265 mg) were added to a solution of intermediate 3 (400 mg) in toluene (5 mL) at room temperature. The reaction mixture was heated to 90 °C and stirred at that temperature for 24 h. After the reaction was completed, the reaction mixture was directly concentrated under reduced pressure. The residue was purified by column chromatography (dichloromethane:methanol = 20:1) to give intermediate 4 (800 mg, purity: 50%, yield: 92%). MS m/z (ESI): 572.8[M+1].

### Target compound:

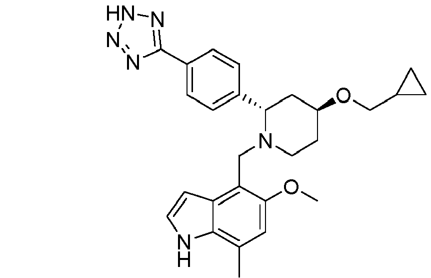

Sodium hydroxide (1.1 g) was added to a mixed solution of intermediate 4 (780 mg) in methanol/water (5 mL/5 mL) at room temperature. The reaction mixture was heated to 80 °C and stirred at that temperature for 16 h. After the reaction was completed, diluted hydrochloric acid (6 M, 4.6 mL) was added to the reaction mixture with cooling in an ice bath to adjust the pH to about 7. The resulting mixture was directly lyophilized to remove the solvent. The resulting residue was purified by preparative high-pressure liquid chromatography (column: Xbridge-C18, 150 × 19 mm, 5 µm; column temperature: 25 °C; flow rate: 20 mL/min; wavelength: 214 nm; column pressure: 93 bar; mobile phase: acetonitrile-water (0.05% aqueous ammonia); gradient: 25-35%) to give the target compound (103.8 mg, yield: 16%). MS m/z (ESI): 472.9[M+1]. ¹H NMR (400 MHz, MeOD) δ 8.25 (d, *J* = 8.0 Hz, 2H), 7.72 (d, *J* = 8.0 Hz, 2H), 7.31 (d, *J* = 2.4 Hz, 1H), 6.71 (s, 1H), 6.34 (d, *J* = 2.4 Hz, 1H), 4.79-4.65 (m, 1H), 4.41-4.28 (m, 1H), 4.25-4.12 (m, 1H), 3.84-3.77 (m, 1H), 3.72 (s, 3H), 3.59-3.50 (m, 1H), 3.42-3.34 (m, 3H), 2.48 (s, 3H), 2.28-2.18 (m, 2H), 2.07-1.94 (m, 2H), 1.18-1.07 (m, 1H), 0.63-0.53 (m, 2H), 0.31-0.23 (m, 2H).

### Example 27

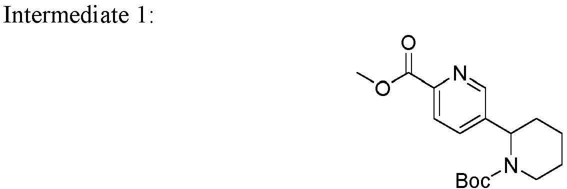

Iridium reagent (Ir[dF(CF₃)ppy]₂(dtbppy))PF₆ (CAS No.: 870987-63-6, 26 mg) was added to a solution of methyl 5-bromopyridine-2-carboxylate (500 mg), 1-(*tert-*butoxycarbonyl)piperidine-2-carboxylic acid (799 mg), nickel chloride ethylene glycol dimethyl ether complex (57 mg), 4'-di-*tert*-butyl-2,2'-bipyridine (310 mg) and cesium carbonate (1.5 g, 4.63 mmol) in *N*,*N*-dimethylformamide (6 mL) at room temperature. The reaction system was purged three times with nitrogen, then placed in an LED blue light reactor (26W, Compact fluorescent light, 300-400 nM) and reacted for 16 h. After the reaction was completed, the reaction mixture was poured into water (30 mL) and extracted with ethyl acetate (30 mL × 3). The combined organic phases were washed with saturated brine (20 mL × 3), dried over anhydrous sodium sulfate and filtered. The filtrate was concentrated under reduced pressure. The resulting residue was purified by column chromatography (petroleum ether:ethyl acetate = 2:1) to give intermediate 1 (500 mg, yield: 33%). MS m/z (ESI): 321.0[M+H].

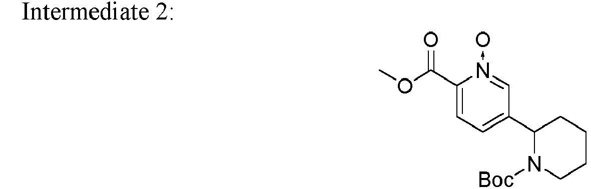

*m*-Chloroperoxybenzoic acid (2.7 g) was added to a solution of intermediate 1 (1.0 g) in dichloromethane (20 mL) at room temperature. The reaction mixture was stirred at room temperature for 5 h. After the reaction was completed, the reaction system was added with dichloromethane (30 mL) for dilution, washed successively with saturated sodium bicarbonate solution (15 mL × 2) and saturated brine (15 mL), dried over anhydrous sodium sulfate and filtered. The filtrate was concentrated under reduced pressure to give intermediate 2 (900 mg, yield: 86%). MS m/z (ESI): 336.0[M+H].

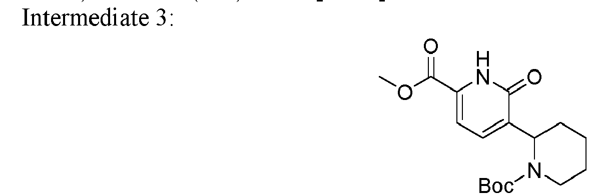

Trifluoroacetic anhydride (5.6 g) was added to a solution of intermediate 2 (900 mg) in DMF (20 mL) with cooling in an ice bath. The reaction mixture was stirred at room temperature for 16 h. After the reaction was completed, the reaction mixture was poured into water (30 mL) and extracted with ethyl acetate (20 mL × 3). The combined organic phases were washed successively with saturated aqueous sodium bicarbonate solution (20 mL) and saturated brine (20 mL × 3), dried over anhydrous sodium sulfate and filtered. The filtrate was concentrated under reduced pressure. The resulting residue was purified by thin-layer chromatography (petroleum ether:ethyl acetate = 2:1) to give intermediate 3 (350 mg, yield: 38%). MS m/z (ESI): 336.9[M+1].

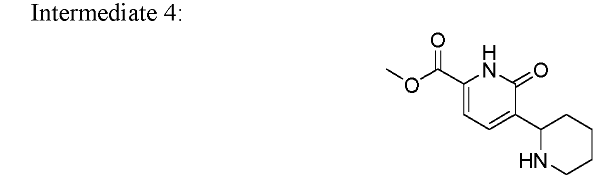

Intermediate 3 (350 mg) was dissolved in a solution of hydrogen chloride in dioxane (15 mL) at room temperature. The reaction mixture was stirred at room temperature for 16 h. After the reaction was completed, the reaction mixture was directly concentrated under reduced pressure to give intermediate 4 (300 mg, yield: 98%). MS m/z (ESI): 236.9[M+1].

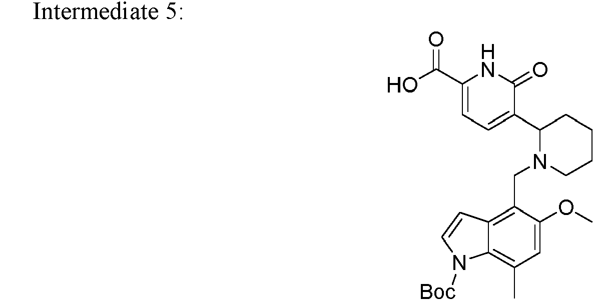

Silatrane (371 mg) was added to a solution of intermediate 2 (245 mg, 0.84 mmol) of Example 2, intermediate 4 (200 mg, 0.84 mmol) and acetic acid (0.5 mL) in 1,2-dichloroethane (15 mL) at room temperature. The reaction mixture was heated to 90 °C and stirred at that temperature for 16 h. After the reaction was completed, the reaction mixture was concentrated under reduced pressure. The resulting residue was purified by column chromatography (dichloromethane:methanol = 10:1) to give intermediate 5 (130 mg, yield: 52%). MS m/z (ESI): 495.8[M+1].

### Target compound:

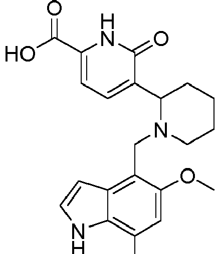

Trimethylbromosilane (1 mL) was added to a solution of intermediate 5 (130 mg) in dichloromethane (4 mL) and water (1 mL) with cooling in an ice bath. The reaction mixture was stirred at room temperature for 16 h. After the reaction was completed, the reaction mixture was directly concentrated under reduced pressure. The residue was purified by preparative high-pressure liquid chromatography (column: Gemini-C18, 150 × 21.2 mm, 5 µm; mobile phase: acetonitrile-water (0.1% formic acid); gradient: 10-30%, column temperature: 25 °C; flow rate: 14 mL/min; wavelength: 214 nm; column pressure: 80 bar) to give the target compound ( 43 mg, yield: 41%, comprising 1 equivalent of formic acid). MS m/z (ESI): 812.5[M+23]. ¹H NMR (400 MHz, CD₃OD) *δ* 8.55 (s, 1H), 8.31-8.26 (m, 1H), 7.86 (d, *J* = 7.9 Hz, 1H), 7.72 (d, *J* = 7.9 Hz, 1H), 7.30 (d, *J* = 3.2 Hz, 1H), 6.68 (s, 1H), 6.44 (s, 1H), 4.22-4.05 (m, 2H), 3.97-3.89 (m, 1H), 3.88-3.76 (m, 3H), 3.46-3.38 (m, 1H), 3.03-2.87 (m, 1H), 2.46 (s, 3H), 1.98-1.60 (m, 6H).

### Example 28

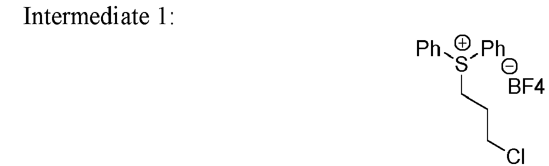

Silver tetrafluoroborate (26 g) was slowly added to a solution of diphenyl sulfide (8 g) and 1-chloro-3-iodopropane (8 g) in nitromethane (15 mL) at room temperature. The reaction mixture was stirred at room temperature for 18 h. After the reaction was completed, dichloromethane (100 mL) was added for dilution. The mixture was filtered. The filtrate was concentrated under reduced pressure. The residual phase was stirred in methyl *tert*-butyl ether (100 mL) for 30 min, and a white solid precipitated. The mixture was filtered. The filter cake was collected to give intermediate 1 (8 g, yield: 50%). MS m/z (ESI): 263.1[M+1].

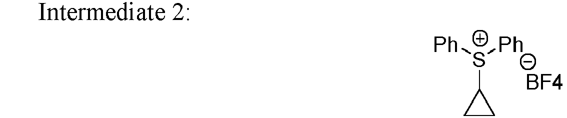

Potassium *tert*-butoxide (4 g) in *N*,*N*-dimethylformamide (24 mL) was added to a solution of intermediate 1 (12 g) in tetrahydrofuran (120 mL) at room temperature. The reaction mixture was stirred at room temperature for 1 h. After the reaction was completed, the reaction system was added with dichloromethane (400 mL) for dilution and washed with water (150 mL). The organic phase was dried over anhydrous sodium sulfate and then filtered. The filtrate was concentrated under reduced pressure. The resulting residue was added to methyl *tert*-butyl ether (300 mL). The mixture was stirred for one hour. Methyl *tert*-butyl ether phase was removed by pouring. The remaining oil was recrystallized from ethanol and methyl *tert*-butyl ether (1:10) to give intermediate 2 (3 g, yield: 26.6%). MS m/z (ESI): 226.8[M+1].

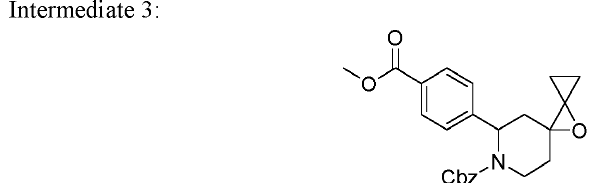

Potassium hexamethyldisilazide (18 mL, 10 mmol) was slowly added dropwise to a solution of intermediate 2 (3 g) in tetrahydrofuran (30 mL) at -40 °C. After the dropwise addition was completed, the reaction mixture was stirred at -40 °C for 10 min. Then intermediate 2 (3 g) of Example 1 was added dropwise. The reaction mixture was successively stirred at -40 °C for 30 min, then naturally warmed to room temperature and stirred at room temperature for 18 h. After the reaction was completed, the reaction system was quenched by adding water (20 mL) and extracted with ethyl acetate (100 mL × 2). The combined extract phases were dried over anhydrous sodium sulfate and filtered. The filtrate was directly concentrated. The resulting residue was purified by column chromatography (petroleum ether: ethyl acetate = 3:1) to give intermediate 3 (1.5 g, yield: 44.4%). MS m/z (ESI): 407.9[M+H].

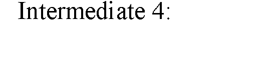

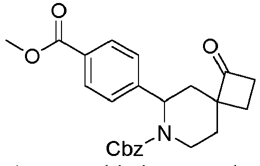

Lithium tetrafluoroborate (0.02 g) was added to a solution of intermediate 3 (1.5 g) in toluene (10 mL) at room temperature. The reaction mixture was heated to 70 °C and stirred at that temperature for 3 h. After the reaction was completed, the reaction mixture was directly concentrated under reduced pressure. The resulting residue was purified by column chromatography (petroleum ether: ethyl acetate = 3:1) to give intermediate 4 (1.2 g, yield: 47.5%). MS m/z (ESI): 408.2[M+1].

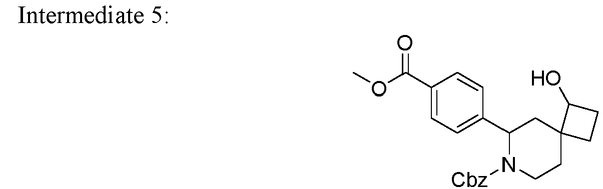

A solution of lithium tri-sec-butylborohydride in tetrahydrofuran (1 M, 4.3 mL) was slowly added dropwise to a solution of intermediate 4 (1.6 g, 3.93 mmol) in tetrahydrofuran (20 mL) with cooling in an ice bath. The reaction mixture was naturally warmed to room temperature and stirred at room temperature for 18 h. After the reaction was completed, methanol (5 mL) was added to the reaction mixture to quench the reaction. The resulting mixture was directly concentrated under reduced pressure. The resulting residue was purified by column chromatography (petroleum ether:ethyl acetate = 3:1) to give intermediate 5 (0.8 g, yield: 80%). MS m/z (ESI): 410.1[M+H].

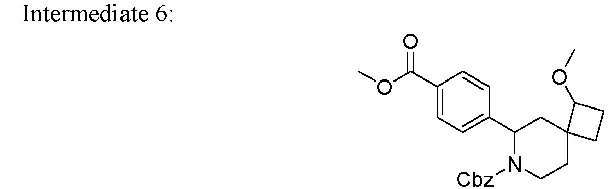

A solution of potassium *tert*-butoxide in tetrahydrofuran (1 M, 1 mL) was added to a solution of intermediate 5 (200 mg) in tetrahydrofuran (2 mL) with cooling in an ice bath. After the reaction mixture was stirred at room temperature for 1 h, iodomethane (347 mg) was added, and the reaction mixture was successively stirred at room temperature for 18 h. After the reaction was completed, methanol (2 mL) was added to quench the reaction. The resulting mixture was directly concentrated under reduced pressure. The residue was purified by column chromatography (petroleum ether:ethyl acetate = 3:1) to give intermediate 6 (100 mg, yield: 45.9%). MS m/z (ESI): 424.2[M+H].

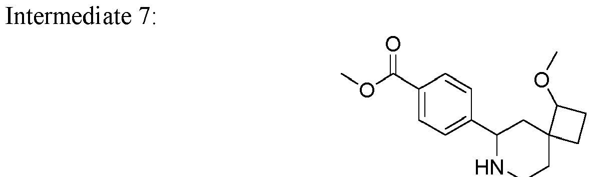

Palladium hydroxide/carbon (10%, 13 mg) was added to a solution of intermediate 6 (100 mg) in methanol (5 mL) at room temperature under nitrogen atmosphere. The reaction system was purged three times with hydrogen and then stirred at room temperature for 18 h. After the reaction was completed, the reaction mixture was filtered. The filtrate was concentrated under reduced pressure. The residue was purified by column chromatography (petroleum ether:ethyl acetate = 3:1) to give intermediate 7 (50 mg, yield: 69.5%). MS m/z (ESI): 290.2[M+1].

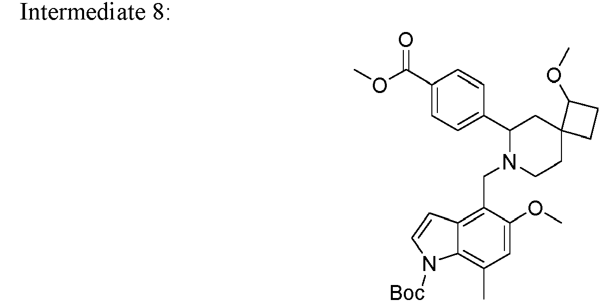

Intermediate 7 (50 mg) was added to a solution of intermediate 2 (60 mg) of Example 2 in 1,2-dichloroethane (2 mL) at room temperature. After the reaction mixture was stirred at room temperature for 8 h, sodium triacetoxyborohydride (110 mg) was added, and the reaction mixture was successively stirred at room temperature for 18 h. After the reaction was completed, the reaction mixture was added with dichloromethane (10 mL) for dilution, washed with water (10 mL), dried over anhydrous sodium sulfate and filtered. The filtrate was concentrated under reduced pressure. The residue was purified by column chromatography (dichloromethane:methanol = 10:1) to give intermediate 8 (50 mg, yield: 48.73%). MS m/z (ESI): 563.3[M+1].

### Target compound:

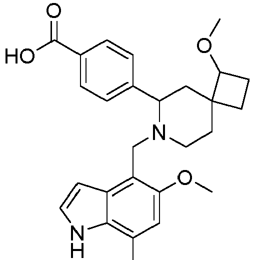

Sodium hydroxide (35.5 mg) was added to a mixed solution of intermediate 8 (50 mg) in tetrahydrofuran/methanol/water (0.5 mL/0.5 mL/0.5 mL) at room temperature. The reaction mixture was heated to 70 °C and stirred at that temperature for 18 h. After the reaction was completed, the reaction mixture was directly concentrated under reduced pressure. The resulting residue was purified by preparative high-pressure liquid chromatography (column: Gemini-C18, 150 × 21.2 mm, 5 µm; mobile phase: acetonitrile-water (0.1% formic acid); column temperature: 25 °C; flow rate: 14 mL/min; wavelength: 214 nm; column pressure: 80 bar, gradient: 0-70%) to give component 1 referred to as Example 28-P1 (13.3 mg, yield: 31.79%). MS m/z (ESI): 448.9[M+1]. ¹H NMR (400 MHz, MeOD) *δ* 8.25-8.15 (m, 2H), 7.75-7.56 (m 2H), 7.35-7.27 (m, 1H), 6.74 (s, 1H), 6.31 (s, 1H), 4.60-4.25 (m, 2H), 4.15-4.07 (m, 1H), 3.74 (s, 3H), 3.67-3.57 (m, 1H), 3.53-3.34 (m, 2H), 3.24 (s, 3H), 2.49 (s, 3H), 2.30-2.17 (m, 2H), 2.15-2.05 (m, 2H), 2.02-1.84 (m, 2H), 1.80-1.53 (m, 2H); and component 2 referred to as Example 28-P2 (5.4 mg, yield: 12.85%): MS m/z (ESI): 448.9[M+1]. ¹H NMR (400 MHz, MeOD) *δ* 8.13 (d, *J* = 8.0 Hz, 2H), 7.73-7.56 (m, 2H), 7.29 (s, 1H), 6.73 (s, 1H), 6.35-6.15 (m, 1H), 4.75-4.25 (m, 2H), 4.15-4.03 (m, 1H), 3.96-3.82 (m, 1H), 3.73 (s, 3H), 3.57-3.42 (m, 1H), 3.34 (s, 3H), 2.47 (s, 3H), 2.40-2.30 (m, 1H), 2.27-2.12 (m, 2H), 2.08-1.77 (m, 3H), 1.75-1.40 (m, 3H).

### Example 29

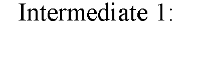

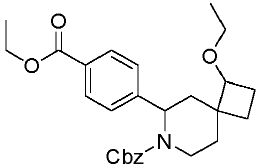

A solution of potassium *tert*-butoxide in tetrahydrofuran (1 M, 1.5 mL) was added to a solution of intermediate 5 (200 mg) of Example 28 in tetrahydrofuran (4 mL) with cooling in an ice bath. The mixture was naturally warmed and stirred at room temperature for 1 h. Iodoethane (381 mg) was added. The mixture was successively stirred at room temperature for 18 h. After the reaction was completed, methanol (2 mL) was added to quench the reaction. The resulting mixture was directly concentrated under reduced pressure. The residue was purified by column chromatography (petroleum ether:ethyl acetate = 3:1) to give intermediate 1 (80 mg, yield: 35.6%). MS m/z (ESI): 451.8[M+1].

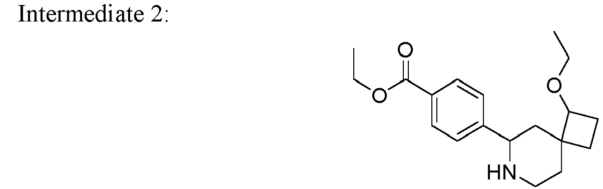

Palladium hydroxide/carbon (10%, 10 mg) was added to a solution of intermediate 1 (80 mg) in methanol (5 mL) at room temperature under nitrogen atmosphere. The reaction system was purged three times with hydrogen and then stirred at room temperature for 18 h. After the reaction was completed, the reaction mixture was filtered. The filtrate was concentrated under reduced pressure. The residue was purified by column chromatography (petroleum ether:ethyl acetate = 3:1) to give intermediate 2 (50 mg, yield: 84.4%). MS m/z (ESI): 317.9[M+1].

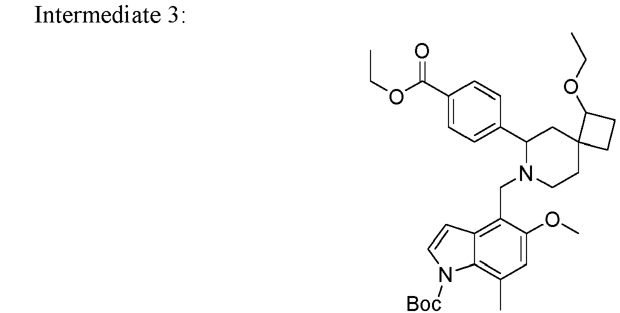

Intermediate 2 (50 mg) was added to a solution of intermediate 2 (55 mg) of Example 2 in 1,2-dichloroethane (2 mL) at room temperature. After the reaction mixture was stirred at room temperature for 8 h, sodium triacetoxyborohydride (100 mg) was added, and the reaction mixture was successively stirred at room temperature for 18 h. After the reaction was completed, the reaction mixture was added with dichloromethane (10 mL) for dilution, washed with water (10 mL), dried over anhydrous sodium sulfate and filtered. The filtrate was concentrated under reduced pressure. The residue was purified by column chromatography (dichloromethane:methanol = 10:1) to give intermediate 3 (50 mg, yield: 48.7%). MS m/z (ESI): 563.3[M+1].

### Target compound:

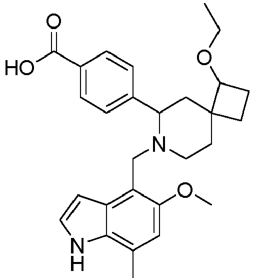

Sodium hydroxide (34 mg) was added to a mixed solution of intermediate 3 (50 mg) in tetrahydrofuran/methanol/water (0.5 mL/0.5 mL/0.5 mL) at room temperature. The reaction mixture was heated to 65 °C and stirred at that temperature for 18 h. After the reaction was completed, the reaction mixture was directly concentrated under reduced pressure. The resulting residue was purified by preparative high-pressure liquid chromatography (column: Gemini-C18, 150 × 21.2 mm, 5 µm; mobile phase: acetonitrile-water (0.1% formic acid); column temperature: 25 °C; flow rate: 14 mL/min; wavelength: 214 nm; column pressure: 80 bar, gradient: 0-70%) to give component 1 referred to as Example 29-P1 (32.2 mg, yield: 39.1%): MS m/z (ESI): 462.8[M+1]. ¹H NMR (400 MHz, CD₃OD) *δ* 8.20-8.10 (m, 2H), 7.72-7.58 (m, 2H), 7.30 (t, *J* = 3.2 Hz, 1H), 6.77-6.70 (m, 1H), 6.37-6.25 (m, 1H), 4.60-4.20 (m, 2H), 4.14-4.09 (m, 1H), 3.77-3.63 (m, 4H), 3.52-3.32 (m, 3H), 3.23-3.10 (m, 1H), 2.48 (d, *J* = 2.8 Hz, 3H), 2.27-1.83 (m, 6H), 1.78-1.52 (m, 2H), 1.10 (t, *J* = 6.8 Hz, 3H); and component 2 referred to as Example 29-P2 (7.3 mg, yield: 8.9%): MS m/z (ESI): 462.8[M+1]. ¹H NMR (400 MHz, MeOD) *δ* 8.14 (d, *J* = 8.0 Hz, 2H), 7.75-7.55 (m, 2H), 7.30 (s, 1H), 6.74 (s, 1H), 6.35-6.15 (m, 1H), 4.60-4.22 (m, 2H), 4.15-3.90 (m, 2H), 3.74 (s, 3H), 3.63-3.32 (m, 4H), 2.57-2.30 (m, 4H), 2.27-1.61 (m, 5H), 1.60-1.40 (m, 2H), 1.36 -1.15 (m, 3H).

### Example 30

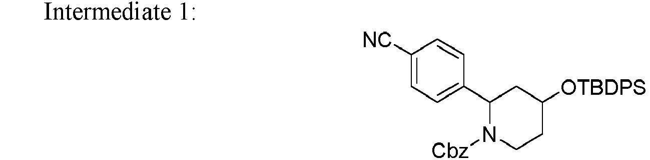

*tert*-Butyldiphenylchlorosilane (25 g) was successively added to intermediate 3 (25 g) of Example 1 and imidazole (6.6 g) in dichloromethane (200 mL) at room temperature. The mixture was reacted at room temperature for 2 h. After the reaction was completed, the reaction mixture was washed with water (500 mL), dried over anhydrous sodium sulfate and filtered. The filtrate was concentrated under reduced pressure. The residue was purified by column chromatography (petroleum ether:ethyl acetate = 5:1) to give intermediate 1 (11.4 g, yield: 26%). MS m/z (ESI): 597.0[M+23].

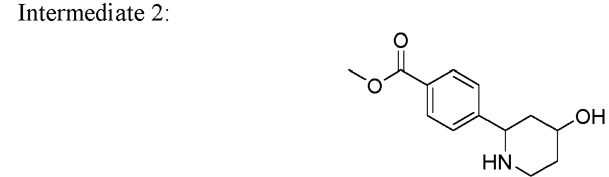

Intermediate 1 (3 g, 6.7 mmol) was added to a mixed solvent of 80% sulfuric acid/methanol = 1/1 (16 mL) at room temperature. The reaction mixture was heated to 100 °C and stirred at that temperature for 16 h. After the reaction was completed, the mixture was naturally cooled to room temperature and added with water (50 mL) for dilution, and the pH was adjusted to 6-7 with a diluted aqueous sodium hydroxide solution (2 M). The resulting mixture was lyophilized to remove the solvent. The residue was purified by column chromatography (dichloromethane:methanol = 5:1) to give intermediate 2 (1.07 g, yield: 67%). MS m/z (ESI): 235.9[M+1].

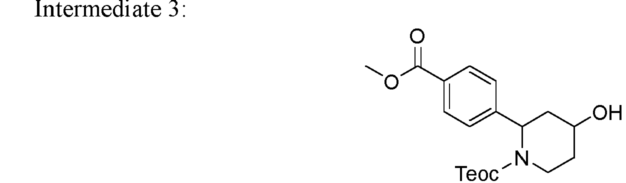

(4-Nitrophenyl) [2-(trimethylsilyl)ethyl]carbonate (1.3 g), triethylamine (552 mg) and 4-dimethylaminopyridine (280 mg) were successively added to a solution of intermediate 2 (1.1 g) in DMF (6 mL) at room temperature. The reaction mixture was stirred at room temperature for 16 h. After the reaction was completed, water (50 mL) was added for dilution, followed by the extraction with ethyl acetate (100 mL × 3). The combined extract phases were washed with saturated brine (50 mL), dried over anhydrous sodium sulfate and filtered. The filtrate was concentrated. The residue was purified by column chromatography (petroleum ether:ethyl acetate = 5:1) to give intermediate 3 (1.13 g, yield: 60%). MS m/z (ESI): 401.8[M+23].

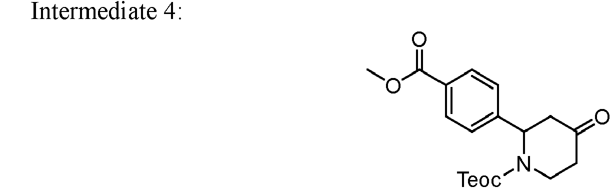

Dess-Martin periodinane (2.4 g) was added to a solution of intermediate 3 (1.1 g) in dichloromethane (8 mL) at room temperature. The reaction mixture was stirred at room temperature for 16 h. After the reaction was completed, the reaction mixture was directly filtered. The filtrate was added with water (50 mL) for dilution and extracted with ethyl acetate (50 mL × 3). The combined extract phases were washed with saturated brine (50 mL), dried over anhydrous sodium sulfate and filtered. The filtrate was concentrated. The residue was purified by column chromatography (petroleum ether:ethyl acetate = 2:1) to give intermediate 4 (560 mg, yield: 48%). MS m/z (ESI): 399.7[M+23].

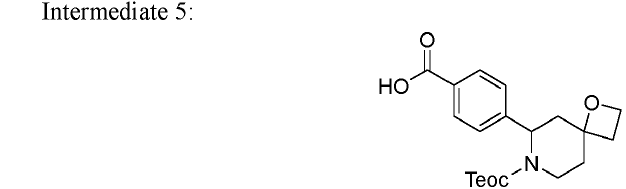

Potassium *tert*-butoxide (613 mg) and *tert*-butanol (6 mL) were successively added to trimethyl sulphoxide iodide (1.63 g) at room temperature. The reaction mixture was heated to 60 °C and stirred at that temperature for 1 h. Then intermediate 4 (560 mg) was added. The reaction mixture was successively stirred at 60 °C for 16 h. After the reaction was completed, water (50 mL) was added for dilution, followed by the extraction with ethyl acetate (100 mL × 3). The combined extract phases were washed with saturated brine (50 mL), dried over anhydrous sodium sulfate and filtered. The filtrate was concentrated. The residue was purified by column chromatography (petroleum ether:ethyl acetate = 2:1) to give intermediate 5 (427 mg, yield: 73%). MS m/z (ESI): 413.7[M+23].

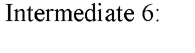

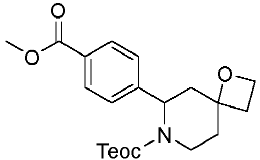

Trimethylsilyldiazomethane (0.7 mL, 2 M) was added to a mixed solution of intermediate 5 (300 mg) in toluene/methanol (4 mL/1 mL) at room temperature. The reaction mixture was stirred at room temperature for 1 h. After the reaction was completed, acetic acid (2 mL) was added to the reaction system to quench the reaction. Water (50 mL) was added for dilution, followed by the extraction with ethyl acetate (50 mL × 3). The combined extract phases were washed with saturated brine (50 mL), dried over anhydrous sodium sulfate and filtered. The filtrate was concentrated. The residue was purified by column chromatography (petroleum ether: ethyl acetate = 5:1) to give intermediate 6 (133 mg, yield: 43%). MS m/z (ESI): 427.8[M+23].

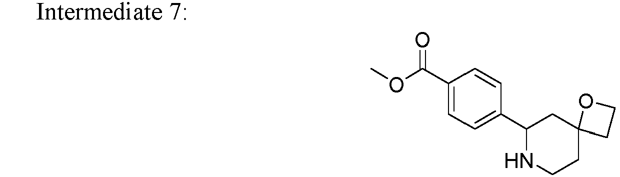

A solution of tetrabutylammonium fluoride in tetrahydrofuran (0.6 mL, 1 M) was added to a solution of intermediate 6 (133 mg) in tetrahydrofuran (4 mL) at room temperature. The reaction mixture was stirred at room temperature for 16 h. After the reaction was completed, the reaction mixture was concentrated under reduced pressure. The residue was purified by thin-layer chromatography (ethyl acetate: tetrahydrofuran = 4:1) to give intermediate 7 (75 mg, yield: 94%). MS m/z (ESI): 261.8[M+1].

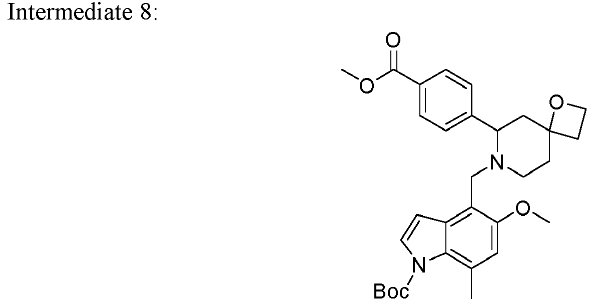

Intermediate 2 of Example 2 (111 mg), silatrane (201 mg) and acetic acid (0.04 mL) were added to a solution of intermediate 7 (100 mg) in tetrahydrofuran (10 mL) at room temperature. The reaction mixture was heated to 70 °C and stirred at that temperature for 16 h. After the reaction was completed, the reaction mixture was concentrated under reduced pressure. The residue was purified by column chromatography (dichloromethane:methanol = 20:1) to give intermediate 8 (40 mg, yield: 20%). MS m/z (ESI): 534.7[M+1].

### Target compound:

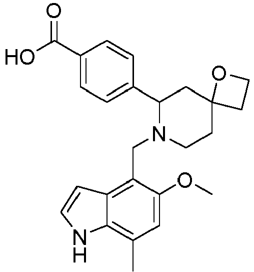

Sodium hydroxide (75 mg) was added to a mixed solution of intermediate 8 (50 mg) in methanol/water (2 mL/2 mL) at room temperature. The reaction mixture was stirred at room temperature for 40 h. After the reaction was completed, the reaction mixture was directly purified by preparative high-pressure liquid chromatography (column: Xbridge-C18; 150 × 19 mm, 5 µm; column temperature: 25 °C; flow rate: 20 mL/min; wavelength: 214 nm; column pressure: 80 bar; mobile phase: acetonitrile-water (0.05% NH₃); gradient: 10-30%) to give the target compound (3.2 mg, yield: 8%). MS m/z (ESI): 411.0[M+1]. ¹H NMR (400 MHz, CD₃OD) *δ* 8.16 (d, *J* = 7.9 Hz, 2H), 7.66 (d, *J* = 7.9 Hz, 2H), 7.30 (d, *J* = 2.8 Hz, 1H), 6.75 (s, 1H), 6.32-6.22 (m, 1H), 4.59 (t, *J* = 7.7 Hz, 2H), 4.44-4.20 (m, 2H), 4.00-3.90 (m, 1H), 3.78-3.71 (m, 3H), 3.50-3.33 (m, 2H), 3.20-3.05 (m, 1H), 2.85-2.65 (m, 2H), 2.53-2.47 (m, 3H), 2.45-2.15 (m, 3H).

### Example 31

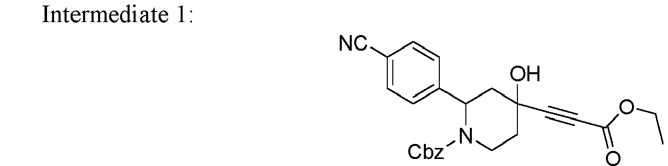

A solution of *n*-butyllithium (2.4 M, 25 mL) was slowly added to a solution of ethyl propiolate (5.89 g) in tetrahydrofuran (200 mL) at -78 °C under nitrogen atmosphere. After the reaction mixture was reacted at that temperature for 0.5 h, intermediate 2 (5 g, 14.95 mmol) of Example 1 was slowly added, and the reaction mixture was successively reacted at -78 °C for 1.5 h. After the reaction was completed, it was quenched by slowly adding saturated aqueous ammonium chloride solution (20 mL). Then water (200 mL) was added for dilution, followed by the extraction with ethyl acetate (200 mL). The extract phase was washed with saturated brine (20 mL), dried over anhydrous sodium sulfate and filtered. The filtrate was concentrated under reduced pressure. The residue was purified by column chromatography (petroleum ether:ethyl acetate = 5:1) to give intermediate 1 (4 g, yield: 59%). MS m/z (ESI): 433.0[M+1].

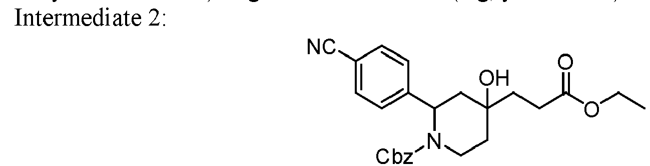

Intermediate 1 (1.9 g) was added to a solution of nickel chloride hexahydrate (300 mg) in ethanol (50 mL) with cooling in an ice bath. After the reaction mixture was stirred at that temperature for 10 min, sodium borohydride (800 mg) was added in batches, and the reaction mixture was successively stirred for 20 min with cooling in an ice bath. After the reaction was completed, water (100 mL) was added for dilution, followed by the extraction with ethyl acetate (100 mL). The extract phase was washed with saturated brine (100 mL), dried over anhydrous sodium sulfate and filtered. The filtrate was concentrated to give intermediate 2 (1.9 g, yield: 90%). MS m/z (ESI): 437.1[M+1].

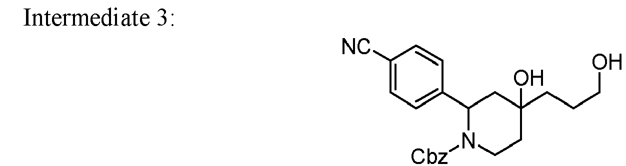

Lithium aluminum hydride (200 mg) was slowly added to a solution of intermediate 2 (1.9 g) in ethanol and tetrahydrofuran (20 mL/20 mL) with cooling in an ice bath. The reaction mixture was stirred at that temperature for 1 h. After the reaction was completed, it was quenched by adding saturated aqueous Na₂SO₄ solution (1 mL). The reaction mixture was filtered. The filtrate was directly concentrated. The residue was purified by column chromatography (dichloromethane:methanol = 20: 1) to give intermediate 3 (1.5 g, yield: 73%). MS m/z (ESI): 395.1[M+1].

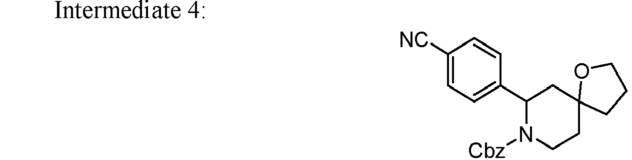

Diisopropyl azodicarboxylate (0.92 g) was slowly added to a solution of intermediate 3 (1.5 g) and triphenylphosphine (1.99 g) in tetrahydrofuran (30 mL) with cooling in an ice bath. The reaction mixture was naturally warmed to room temperature and stirred at that temperature for 16 h. After the reaction was completed, the reaction mixture was added with water (100 mL) for dilution and extracted with ethyl acetate (50 mL). The extract phase was washed with saturated brine (100 mL), dried over anhydrous sodium sulfate and filtered. The filtrate was concentrated. The residue was purified by column chromatography (petroleum ether:ethyl acetate = 5:1) to give intermediate 4 (1.5 g, yield: 89%). MS m/z (ESI): 376.9[M+1].

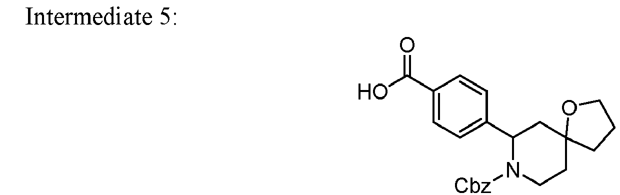

Water (20 mL) and barium hydroxide octahydrate (6.3 g) were successively added to a solution of intermediate 4 (1.5 g) in isopropanol (20 mL) at room temperature. The reaction mixture was heated to 100 °C and stirred at that temperature for 16 h. After the reaction was completed, the reaction mixture was added with water (50 mL) for dilution, adjusted to about pH 3 with diluted hydrochloric acid and extracted with ethyl acetate (100 mL). The extract phase was washed with saturated brine (20 mL), dried over anhydrous sodium sulfate and filtered. The filtrate was concentrated to give intermediate 5 (1.5 g, yield: 85%). MS m/z (ESI): 396.0[M+1].

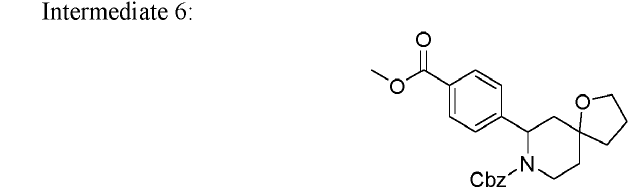

Potassium carbonate (500 mg) and iodomethane (500 mg) were successively added to a solution of intermediate 5 (500 mg) in acetonitrile (20 mL) at room temperature. The reaction mixture was heated to 65 °C and stirred at that temperature for 16 h. After the reaction was completed, water (100 mL) was added for dilution, followed by the extraction with ethyl acetate (100 mL). The extract phase was washed with saturated brine (200 mL), dried over anhydrous sodium sulfate and filtered. The filtrate was concentrated under reduced pressure. The residue was purified by silica gel column chromatography (petroleum ether:ethyl acetate = 15:1) to give intermediate 6 (400 mg, yield: 77%). MS m/z (ESI): 410.1[M+1].

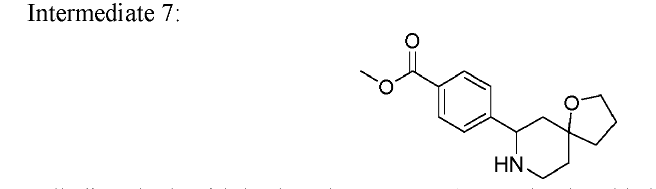

Palladium hydroxide/carbon (10%, 50 mg) was slowly added to a solution of intermediate 6 (200 mg) in tetrahydrofuran (10 mL) at room temperature under nitrogen atmosphere. The reaction mixture was stirred at room temperature under 2 atmospheres of hydrogen gas for 16 h. After the reaction was completed, the reaction mixture was filtered. The filtrate was directly concentrated to give intermediate 7 (150 mg, yield: 84%). MS m/z (ESI): 276.1[M+1].

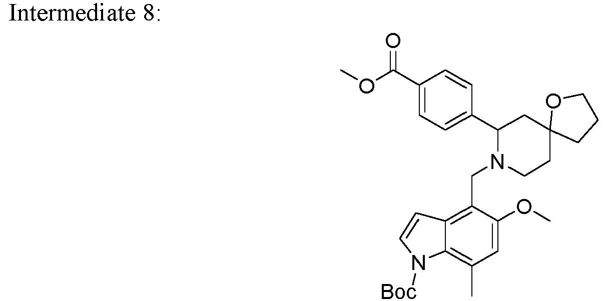

Glacial acetic acid (50 mg) and silatrane (200 mg) were added to a solution of intermediate 7 (150 mg) and intermediate 2 (150 mg) of Example 2 in tetrahydrofuran (5 mL) at room temperature. The reaction mixture was heated to 75 °C and stirred at that temperature for 16 h. After the reaction was completed, the reaction mixture was directly concentrated. The residue was purified by column chromatography (dichloromethane:methanol = 20: 1) to give intermediate 8 (150 mg, yield: 71%). MS m/z (ESI): 549.2[M+1].

### Target compound:

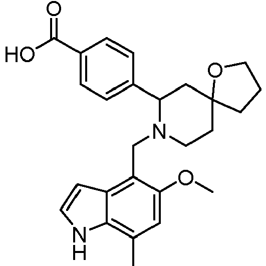

Sodium hydroxide (40 mg) was added to a mixed solution of intermediate 8 (150 mg) in methanol and water (3 mL/3 mL) at room temperature. The reaction mixture was heated to 75 °C and stirred at that temperature for 3 h. After the reaction was completed, the reaction mixture was concentrated. The residue was purified by preparative high-pressure liquid chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.05% NH₃); gradient: 10-40%) to give the target compound (58.0 mg, yield: 48%). MS m/z (ESI): 435.1[M+1]. ¹H NMR (400 MHz, CD₃OD) *δ* 8.13 (d, *J* = 8.0 Hz, 2H), 7.62 (d, *J* = 8.0 Hz, 2H), 7.29 (d, *J=* 3.2 Hz, 1H), 6.74 (s, 1H), 6.30 (s, 1H), 4.45-3.98 (m, 3H), 3.83 (m, 2H), 3.74(s, 3H), 3.52-3.12 (m, 2H), 2.50(s, 3H), 2.32-1.72(m,8H).

### Example 32

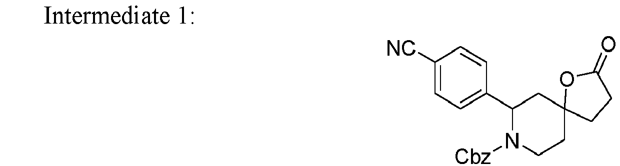

Toluenesulfonic acid (35 mg) was added to a solution of intermediate 2 (800 mg) of Example 31 in toluene (10 mL) at room temperature. The reaction mixture was heated to 110 °C and reacted that temperature for 18 h. After the reaction was completed and naturally cooled to room temperature, the reaction mixture was directly concentrated under reduced pressure. The residue was purified by column chromatography (petroleum ether:ethyl acetate = 3:1) to give intermediate 1 (550 mg, yield: 73.0%). MS m/z (ESI): 391.2[M+1].

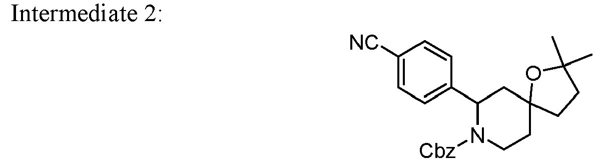

A solution of methylmagnesium chloride in tetrahydrofuran (3 M, 1.4 mL) was slowly added dropwise to a solution of intermediate 1 (550 mg) in tetrahydrofuran (10 mL) with cooling in an ice bath. The reaction mixture was naturally warmed and stirred at that temperature for 18 h. After the reaction was completed, the reaction system was quenched by adding water (10 mL) and extracted with ethyl acetate (10 mL × 2). The combined organic phases were washed with saturated brine (10 mL), dried over anhydrous sodium sulfate and filtered. The filtrate was concentrated under reduced pressure. The residue was purified by column chromatography (petroleum ether:ethyl acetate = 3:1) to give intermediate 2 (150 mg, yield: 25.0%). MS m/z (ESI): 404.8[M+1].

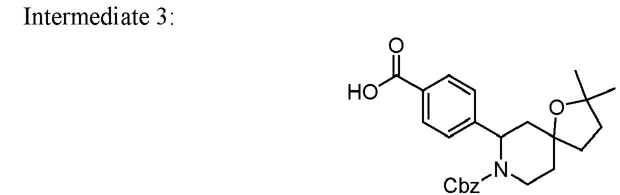

Barium hydroxide octahydrate (584 mg) was added to a mixed solution of intermediate 2 (150 mg) in isopropanol and water (3 mL/6 mL) at room temperature. The reaction mixture was heated to 100 °C and stirred at that temperature for 18 h. After the reaction was completed, the reaction mixture was added with water (10 mL) for dilution and the pH was adjusted to about 5 with diluted hydrochloric acid (1 M). The resulting mixture was extracted with ethyl acetate (20 mL × 2). The combined extract phases were washed with saturated brine (20 mL), dried over anhydrous sodium sulfate and filtered. The filtrate was concentrated under reduced pressure. The residue was purified by column chromatography (dichloroethane:methanol = 10:1) to give intermediate 3 (50 mg, yield: 30.3%). MS m/z (ESI): 423.8[M+1].

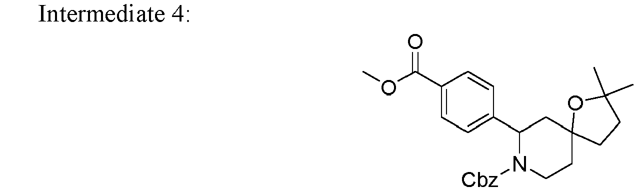

Iodomethane (54 mg) was added to a solution of intermediate 3 (50 mg) and potassium carbonate (52 mg) in acetone (5 mL) at room temperature. The reaction mixture was reacted at room temperature for 18 h. After the reaction was completed, water (5 mL) was added for dilution, followed by the extraction with ethyl acetate (10 mL × 2). The combined extract phases were washed with saturated brine (10 mL), dried over anhydrous sodium sulfate and filtered. The filtrate was concentrated under reduced pressure. The residue was purified by column chromatography (petroleum ether:ethyl acetate = 10:1) to give intermediate 4 (50 mg, yield: 91.77%). MS m/z (ESI): 438.2[M+1].

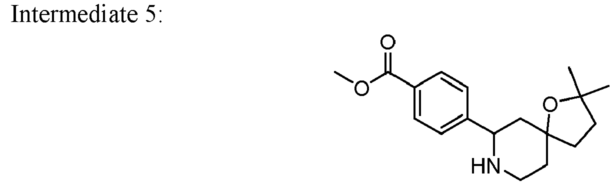

Palladium hydroxide/carbon (3 mg) was added to a solution of intermediate 4 (30 mg) in methanol (5 mL) at room temperature under nitrogen atmosphere. The reaction system was stirred at room temperature under hydrogen atmosphere for 18 h. After the reaction was completed, the reaction mixture was filtered. The filtrate was directly concentrated under reduced pressure to give intermediate 5 (20 mg, purity: 85%, yield: 81.6%).

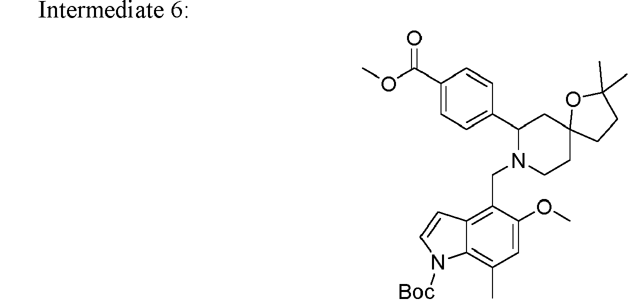

Intermediate 5 (20 mg) was added to a solution of intermediate 2 (23 mg) of Example 2 in 1,2-dichloroethane (2 mL) at room temperature. After the reaction mixture was stirred at room temperature for 8 h, sodium triacetoxyborohydride (42 mg) was added, and the reaction mixture was successively stirred at room temperature for 18 h. After the reaction was completed, the reaction mixture was added with dichloromethane (10 mL) for dilution, washed with water (10 mL), dried over anhydrous sodium sulfate and filtered. The filtrate was concentrated under reduced pressure. The residue was purified by column chromatography (dichloromethane:methanol = 10:1) to give intermediate 6 (20 mg, yield: 49.9%). MS m/z (ESI): 577.1[M+1].

### Target compound:

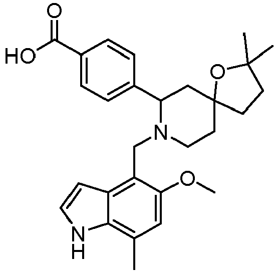

Sodium hydroxide (33.8 mg) was added to a mixed solution of intermediate 6 (20 mg, 0.08 mmol) in tetrahydrofuran/methanol/water (0.5 mL/0.5 mL/0.5 mL) at room temperature. The reaction mixture was heated to 65 °C and stirred at that temperature for 18 h. After the reaction was completed, the reaction mixture was concentrated under reduced pressure. The residue was purified by preparative flash chromatography (column: Gemini-C18, 150 × 21.2 mm, 5 µm; mobile phase: acetonitrile-water (0.1% formic acid); column temperature: 25 °C; flow rate: 14 mL/min; wavelength: 214 nm; column pressure: 80 bar, gradient: 15-40%) to give the target compound (10.1 mg, yield: 59.8%). MS m/z (ESI): 463.1[M+1]. ¹H NMR (400 MHz, MeOD) *δ* 8.14 (d, *J* = 8.0 Hz, 2H), 7.64 (d, *J* = 8.0 Hz, 2H), 7.32-7.26 (m, 1H), 6.76-6.67 (m, 1H), 6.32-6.25 (m, 1H), 4.50 (d, *J* = 12.0 Hz, 1H), 4.27 (d, *J* = 12.0 Hz, 1H), 4.13-4.03 (m, 1H), 3.72 (s, 3H), 3.52-3.42 (m, 1H), 3.35-3.30 (m, 1H), 2.47 (s, 3H), 2.35-2.15 (m, 3H), 2.10-1.96 (m, 2H), 1.95-1.88 (m, 2H), 1.86-1.77 (m, 1H), 1.21 (s, 3H), 1.19 (s,3H).

### Example 33

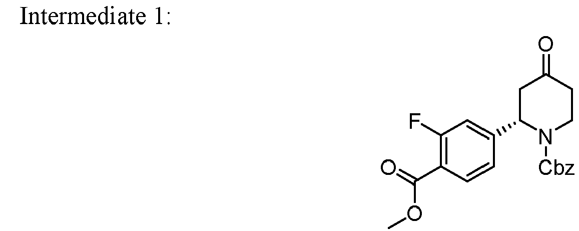

The compounds *S*-(-)-1,1'-binaphthyl-2,2'-bisdiphenylphosphine (CAS No.: 76189-56-5) (404 mg) and bis(dicyclopentadiene)rhodium tetrafluoroborate (CAS No.: 36620-11-8) (202 mg) were added to a solution of 3-fluoro-4-(methoxycarbonyl)benzeneboronic acid (2568 mg) in 1,4-dioxane (7 mL) at room temperature. After the reaction mixture was stirred at room temperature under nitrogen atmosphere for 8 h, benzyl 4-oxo-3,4-dihydropyridine-1(2*H*)-carboxylate (CAS No.: 185847-84-1) (2500 mg), triethylamine (1094 mg) and water (0.7 mL) were successively added. The reaction mixture was warmed to 40 °C under nitrogen atmosphere and successively stirred at that temperature for 16 h. After the reaction was completed, the reaction mixture was directly concentrated. The residue was purified by column chromatography (petroleum ether:ethyl acetate = 3:1) to give intermediate 1 (3000 mg, purity: 30%, yield: 22%). MS m/z (ESI): 385.8[M+1].

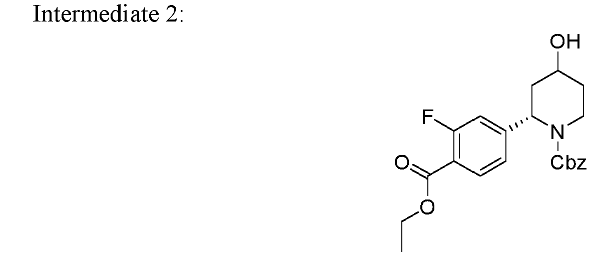

Sodium borohydride (196 mg) was added in batches to a mixed solution of intermediate 1 (3000 mg) in tetrahydrofuran and ethanol (15 mL/15 mL) at room temperature. The reaction mixture was stirred at room temperature for 16 h. After the reaction was completed, the reaction mixture was cooled to below 0 °C, and saturated aqueous ammonium chloride solution (5 mL) was slowly added to quench the reaction. Water (50 mL) was added for dilution, followed by the extraction with ethyl acetate (20 mL). The extract phase was washed with saturated brine (10 mL), dried over anhydrous sodium sulfate and filtered. The filtrate was concentrated under reduced pressure. The residue was purified by column chromatography (petroleum ether:ethyl acetate = 2:1) to give intermediate 2 (1100 mg, purity: 40%, yield: 14%). MS m/z (ESI): 401.8[M+1].

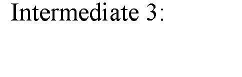

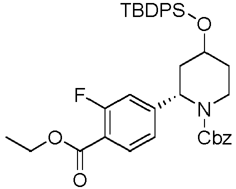

Imidazole (242 mg) and *tert*-butyldiphenylchlorosilane (904 mg) were added to a solution of intermediate 2 (1100 mg) in dichloromethane (20 mL) at room temperature. The reaction mixture was stirred at room temperature for 16 h. After the reaction was completed, the reaction mixture was added with water (20 mL) for dilution and extracted with dichloromethane (50 mL). The extract phase was dried over anhydrous sodium sulfate and filtered. The filtrate was concentrated. The residue was purified by column chromatography (petroleum ether:ethyl acetate = 15:1) to give intermediate 3 (390 mg, yield: 20%). MS m/z (ESI): 661.6[M+23].

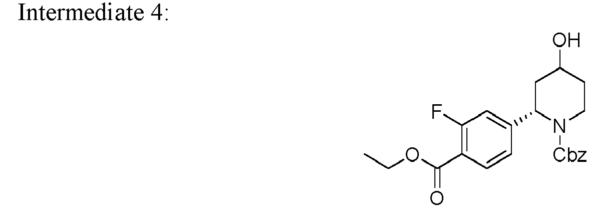

Intermediate 3 (390 mg) was added to a solution of tetrabutylammonium fluoride (1.0 M, 3 mL) at room temperature. The reaction mixture was stirred at room temperature for 2 h. After the reaction was completed, water (20 mL) was added for dilution, followed by the extraction with ethyl acetate (10 mL). The extract phase was washed once with saturated brine (20 mL), dried over anhydrous sodium sulfate and filtered. The filtrate was concentrated. The residue was purified by column chromatography (petroleum ether:ethyl acetate = 2:1) to give intermediate 4 (180 mg, yield: 66%). MS m/z (ESI): 423.8[M+23].

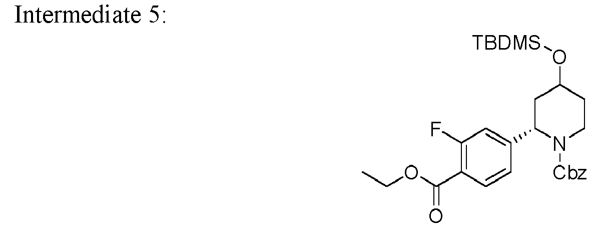

Imidazole (61 mg) and *tert*-butyldimethylchlorosilane (74 mg) were added to a solution of intermediate 4 (180 mg) in DMF (3 mL) at room temperature. The reaction mixture was stirred at room temperature for 2 h. After the reaction was completed, the reaction mixture was added with water (20 mL) for dilution, followed by the extraction with ethyl acetate (10 mL). The extract phase was washed with saturated brine (20 mL), dried over anhydrous sodium sulfate and filtered. The filtrate was concentrated to give intermediate 5 (200 mg, yield: 78%). MS m/z (ESI): 537.8[M+23].

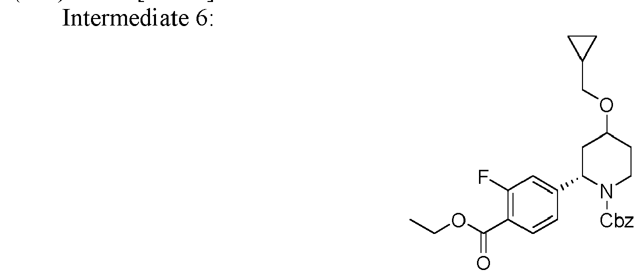

Cyclopropanecarboxaldehyde (147 mg) and trimethylsilyl trifluoromethanesulfonate (168 mg) were successively added to a solution of intermediate 5 (685 mg) in dichloromethane (13 mL) at -78 °C under nitrogen atmosphere. After the reaction mixture was stirred at -78 °C for 1 h, triethylsilane (308 mg) was added. Then the reaction mixture was naturally warmed to room temperature and successively stirred at room temperature for 16 h. After the reaction was completed, the reaction mixture was directly concentrated under reduced pressure. The residue was purified by column chromatography (petroleum ether:ethyl acetate = 3:1) to give intermediate 6 (500 mg, yield: 74%). MS m/z (ESI): 477.7[M+23].

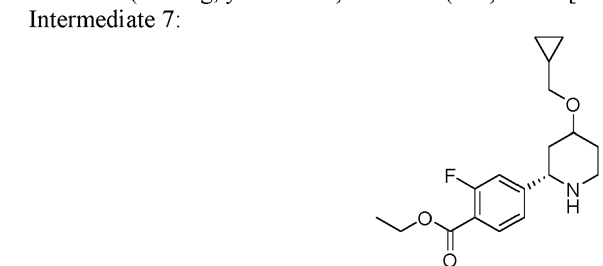

Palladium/carbon (50 mg) was added to a solution of intermediate 6 (500 mg, 1.10 mmol) in tetrahydrofuran (5 mL) at room temperature under nitrogen atmosphere. The reaction mixture was stirred at room temperature under hydrogen atmosphere for 2 h. After the reaction was completed, the reaction mixture was filtered. The filtrate was directly concentrated to give intermediate 7 (330 mg, yield: 84%). MS m/z (ESI): 322.0[M+1].

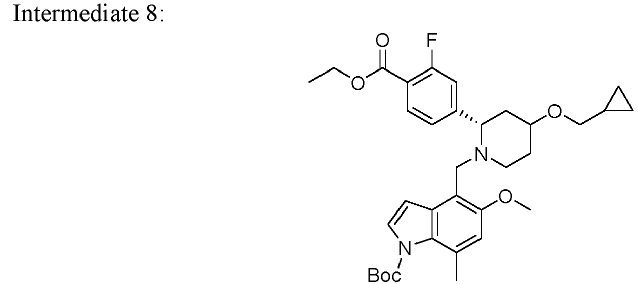

Intermediate 7 (180 mg) was added to a solution of intermediate 2 (178 mg) of Example 2 in 1,2-dichloroethane (5 mL) at room temperature. After the reaction mixture was stirred at room temperature for 8 h, sodium triacetoxyborohydride (356 mg) was added, and the reaction mixture was successively stirred at room temperature for 16 h. After the reaction was completed, the reaction mixture was directly concentrated. The residue was purified by column chromatography (dichloromethane:methanol = 20:1) to give intermediate 8 (350 mg, yield: 84%). MS m/z (ESI): 594.8[M+1].

### Target compound:

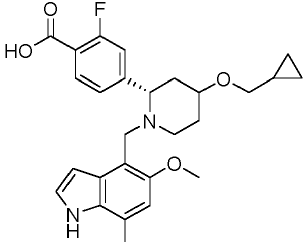

Sodium hydroxide (470 mg) was added to a mixed solution of compound **9** (350 mg) in methanol/water (5 mL/5 mL) at room temperature. The reaction mixture was heated to 80 °C and stirred at that temperature for 16 h. After the reaction was completed, diluted hydrochloric acid (2 M) was added to the reaction mixture with cooling in an ice bath to adjust the pH to about 7. Then the solvent was directly lyophilized. The residue was purified by preparative high-pressure liquid chromatography (column: Gemini-C18, 150 × 21.2 mm, 5 µm; column temperature: 25 °C; flow rate: 14 mL/min; wavelength: 214 nm; column pressure: 80 bar; mobile phase: acetonitrile-water (0.1% formic acid); gradient: 20-40%) to give the target compound (91.7 mg, yield: 32%, comprising 0.6 equivalents of formic acid). ¹H NMR (400 MHz, CD₃OD) *δ*8.29 (s, 0.6H), 7.89 (t, *J* = 7.6 Hz, 1H), 7.44 (t, *J* = 10.1 Hz, 2H), 7.33 (d, *J* = 3.0 Hz, 1H), 6.75 (s, 1H), 6.38 (s, 1H), 4.82-4.68 (m, 1H), 4.43-4.28 (m, 1H), 4.27-4.11 (m, 1H), 3.91-3.81 (m, 1H), 3.78 (s, 3H), 3.60-3.45 (m, 1H), 3.38 (d, *J* = 6.9 Hz, 3H), 2.50 (s, 3H), 2.31-2.14 (m, 2H), 2.10-1.89 (m, 2H), 1.20-1.05 (m, 1H), 0.65-0.51 (m, 2H), 0.32-0.21 (m, 2H). MS m/z (ESI): 467.1[M+1].

According to the methods of Examples 3-33 described above, the following compounds were prepared:

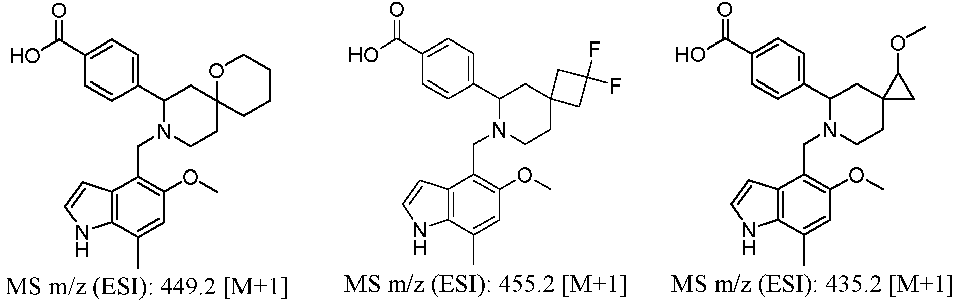

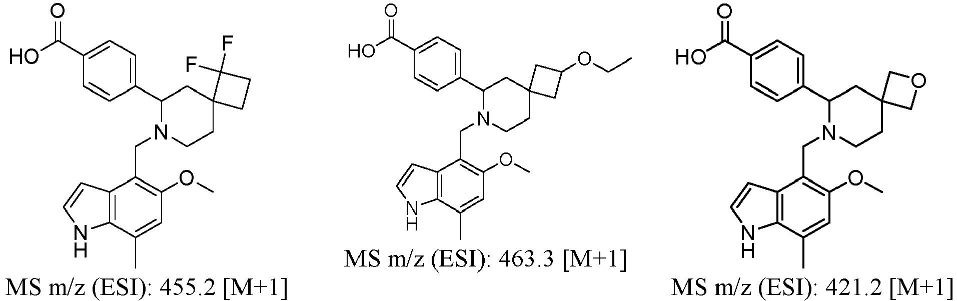

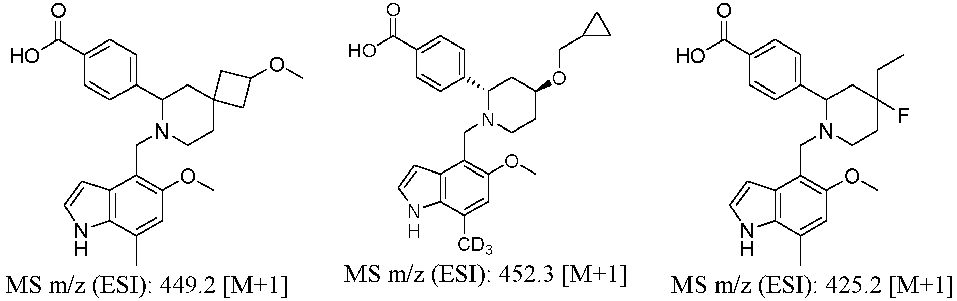

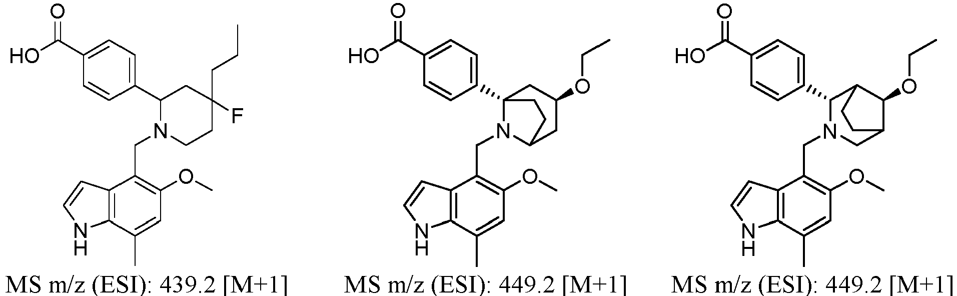

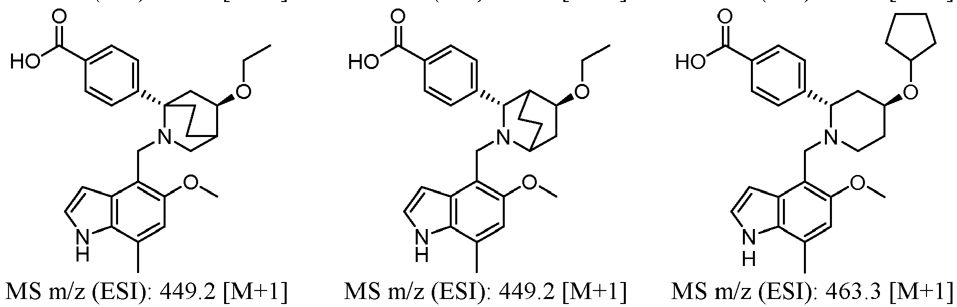

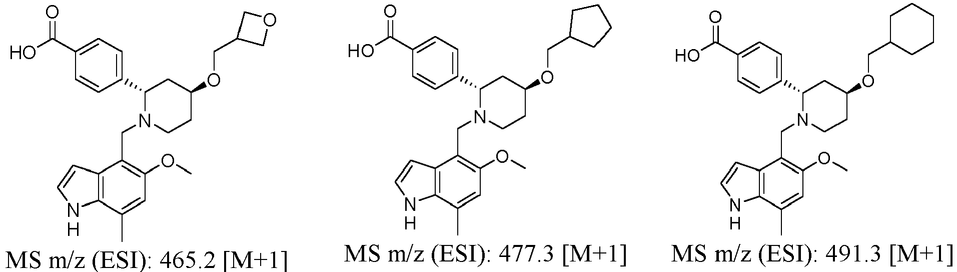

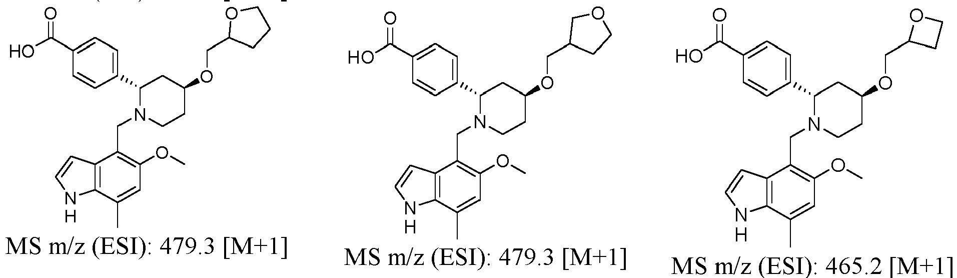

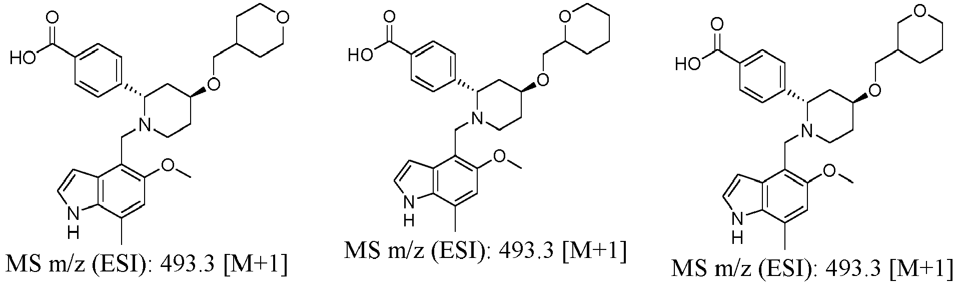

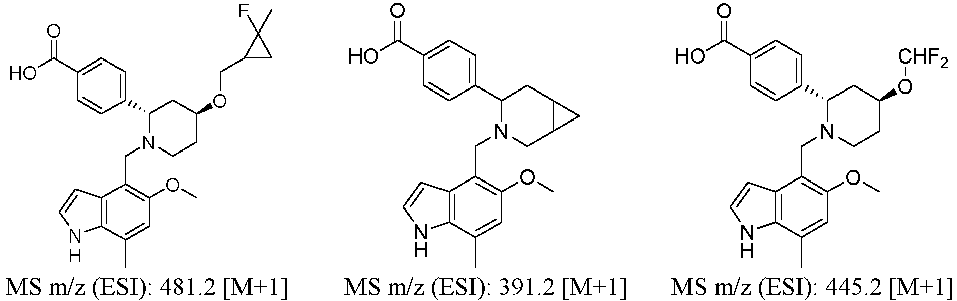

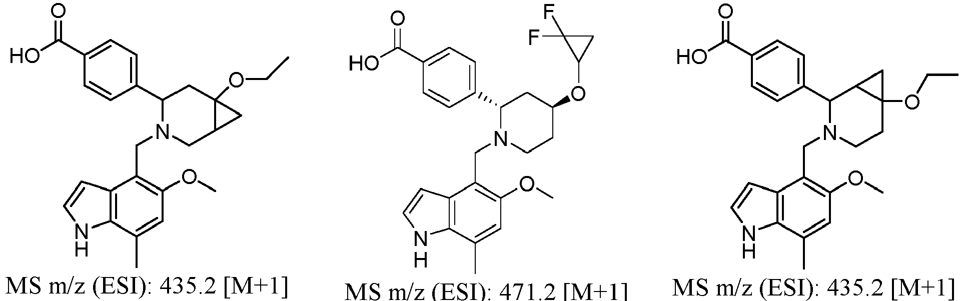

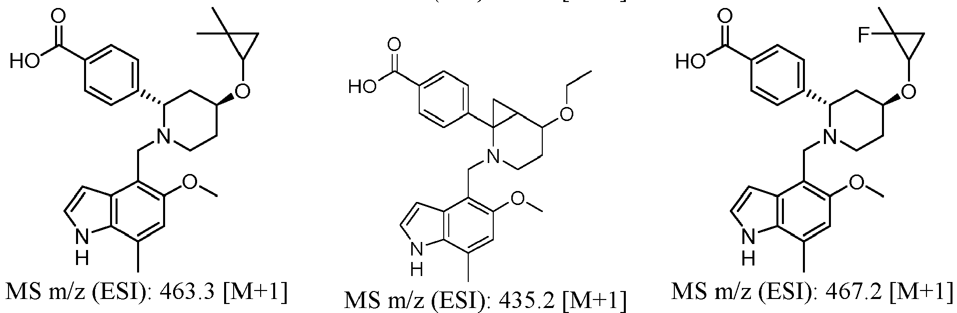

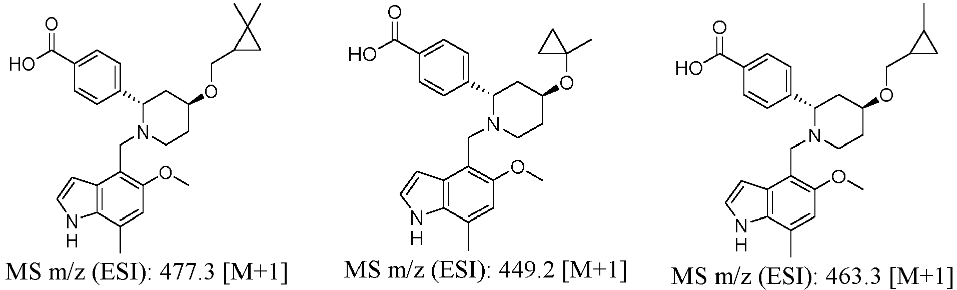

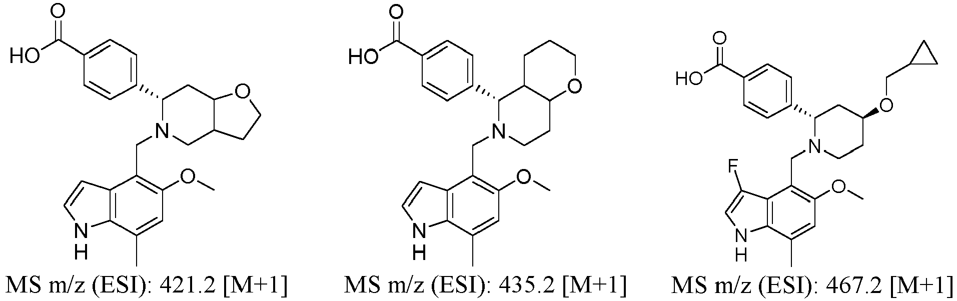

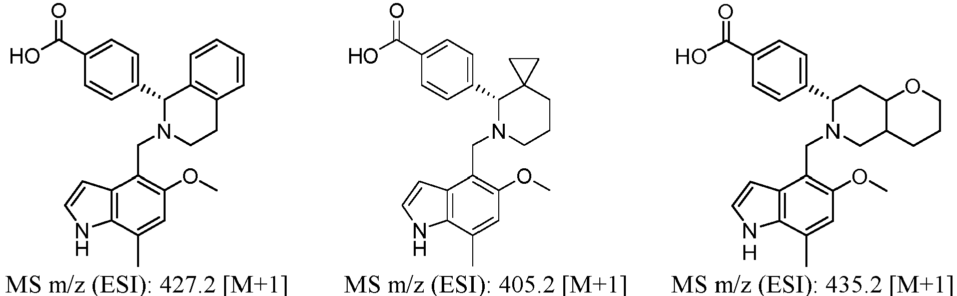

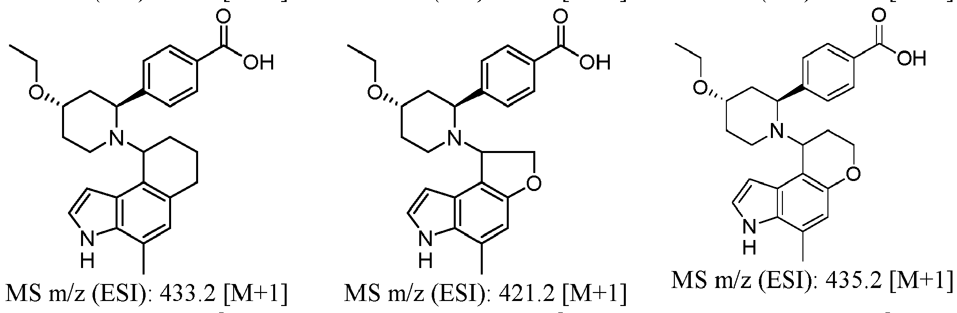

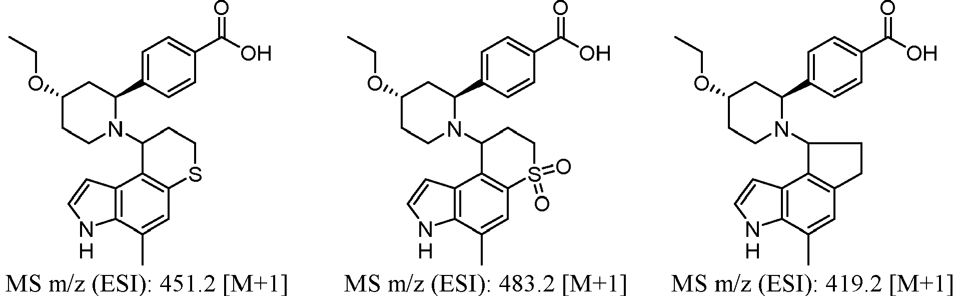

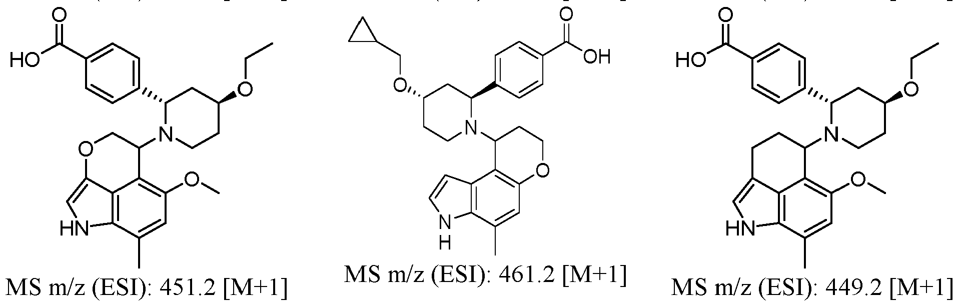

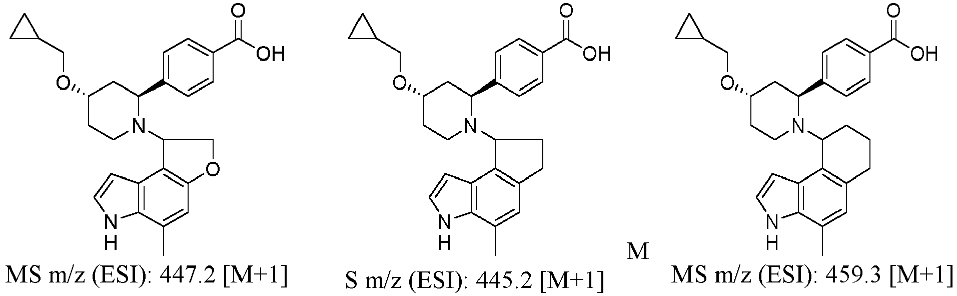

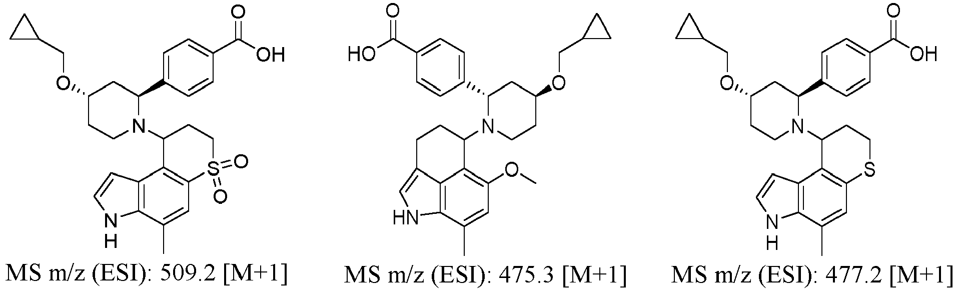

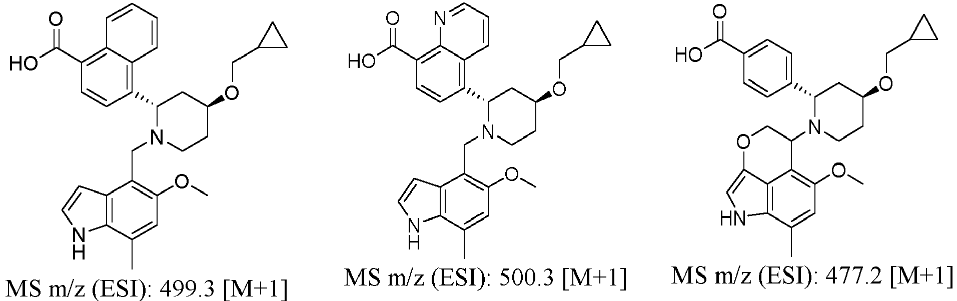

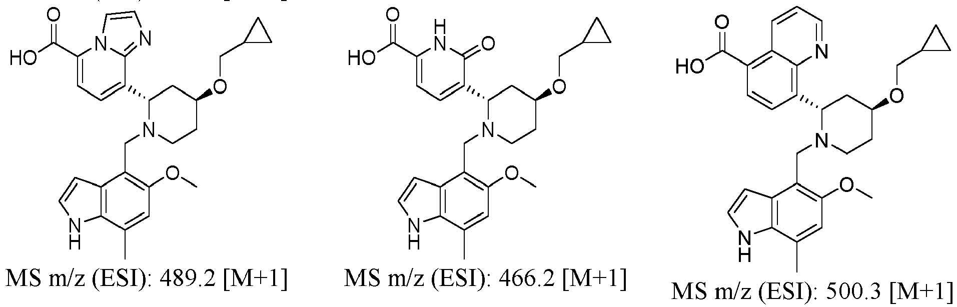

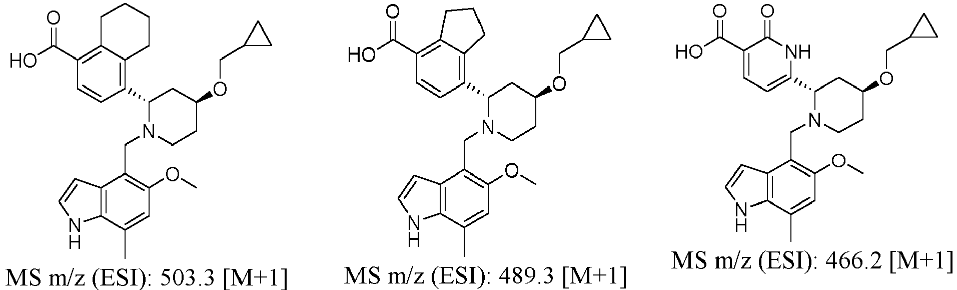

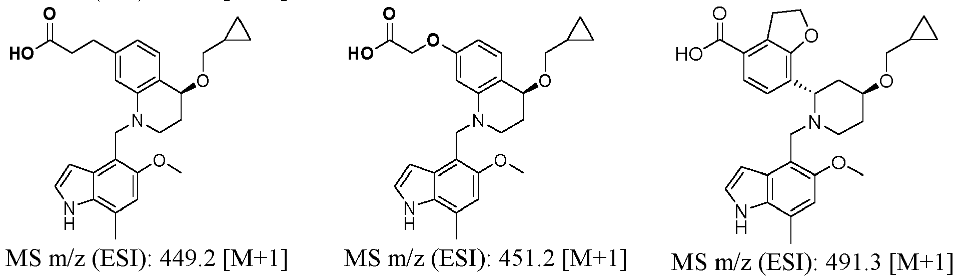

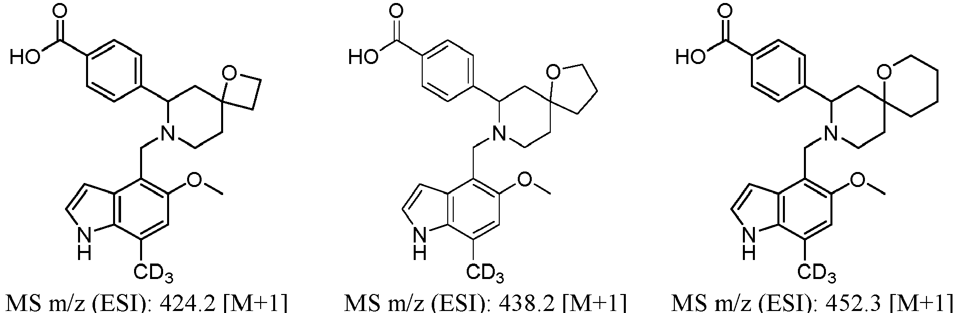

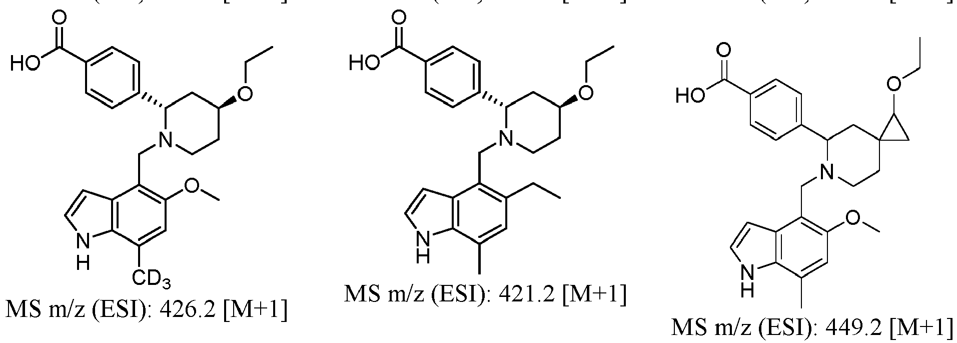

### Preparation of control compound (Example-26c, WO2015009616A1):

### Control intermediate 1:

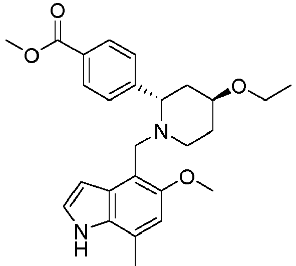

To a 50 mL sealed tube were added tetrahydrofuran (3 mL), intermediate 7 (127 mg) of Example 1, intermediate 2 (130 mg) of Example 2 and tetraethyl titanate (56 mg). The reaction mixture was heated to 70 °C under nitrogen atmosphere and stirred at that temperature for 16 h. The reaction mixture was cooled to room temperature. Then sodium triacetoxyborohydride (52 mg) was added. The reaction mixture was heated to 70 °C and reacted for 1 h. After the reaction mixture was cooled to room temperature, 4 mL of methanol was added to quench the reaction. The reaction mixture was concentrated. The residue was separated and purified by column chromatography (methanol: dichloromethane = 1:10) to give control intermediate 1 (170 mg, yield: 52%).

### Control compound:

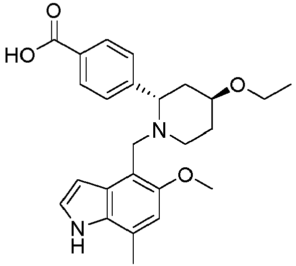

To a 50 mL single-neck flask were added methanol (3 mL), water (1 mL), intermediate 1 (160 mg) and sodium hydroxide (230 mg). The reaction mixture was reacted at room temperature for 16 h. After the reaction was completed, water (10 mL) was added for dilution and the pH was adjusted to 7-8 with a diluted hydrochloric acid solution (1 M). The solvent was removed under reduced pressure (water bath: 45 °C). The residue was purified by preparative high-pressure liquid chromatography (column: Gemini-C18, 150 × 21.2 mm, 5 µm; mobile phase: acetonitrile-water (0.1% formic acid); gradient: 15-30%) to give the target compound (29 mg, yield: 24%). MS m/z (ESI): 423.1[M+1]. ¹H NMR (400 MHz, DMSO-*d₆*) δ 8.17 (d, J = 8.4 Hz, 2H), 7.67 (d, J = 8.4 Hz, 2H), 7.33 (t, J = 2.8 Hz, 1H), 6.78 (s, 1H), 6.35 (s, 1H), 4.82-4.67 (m, 1H), 4.40-4.17 (m, 2H), 3.90-3.81 (m, 1H), 3.77 (s, 3H), 3.62 (q, J = 6.8 Hz, 2H), 3.57-3.50 (m, 1H), 3.45-3.35 (m, 1H), 2.52 (s, 3H), 2.32-2.22 (m, 2H), 2.14-1.96 (m, 2H), 1.32 (t, J = 6.8 Hz, 3H).

### Biological Examples

### 1. Optical surface plasmon resonance (SPR) binding capacity assay

An SPR experiment was carried out at 25 °C. In the experiment, a PBS buffer supplemented with 0.05% (v/v) P20 and 5% DMSO was used as a running buffer, and the analytical instrument Biacore 8K of GE Healthcare was used. A CM7 ship (GE Healthcare) was activated with 400 mM EDC and 100 mM NHS at a flow rate of 30 µL/min for 420 s. Complement factor B was diluted to 50 µg/mL with 10 mM sodium acetate (pH 4.0) and then covalently immobilized to the assay chip by coupling at a flow rate of 10 µL/min for 1200 s (protein immobilization level at 25000 RU). Then the assay chip was treated with 1 M ethanolamine hydrochloride at a flow rate of 10 µL/min for 300 s for chip blocking. The concentration of the test compound was 500 µM, the binding time was 120 s, and the dissociation time was 300 s. Data analysis was performed using a 1:1 binding model (Biacore Insight Evalution Software, Version2.0.15.12933).

### Experimental results:

The experimental results of some of the example compounds are shown in the following Table 1. At a concentration of 500 µM, The compounds of Example 5 and Example 6 have more significant binding capacity to the target protein and are significantly better than the control compound, which indicates that the compounds of the present disclosure have relatively good binding capacity to the target protein.

**Table 1**

| Example compound No. | Rmax (RU) |
|---|---|
| Example 4 | 29.1 |
| Example 5 | 274.2 |
| Example 6 | 106.5 |
| Example 7 | 21.6 |
| Example 8 | 47.7 |
| Example 9 | 23.3 |
| Example 10 | 30.7 |
| Example 11 | ND |
| Control compound | 33.7 |

| | |
|---|---|
| Note: ND means that no SPR binding capacity data was detected. | |

### 2. TR-FRET binding capacity assay

Competitive binding experiments using a small-molecule inhibitor fluorescently labeled with Cy5 as the probe were carried out to screen compounds for inhibitory activity against human complement factor B. After complement factor B and EZ-Link^{™} Sulfo-NHS-LC-LC-Biotin in a ratio of 1:2 were incubated on ice for 1 h, 1 M Tris (pH 7.5) was added to terminate the reaction. The mixture was then purified twice through a 2 mL Zeba^{™} desalt spin column to give a biotin-labeled complement factor B (EZ-LinkTM Sulfo-NHS-LC-Biotin instructions). In the experiments, biotin-labeled complement factor B at a final concentration of 10 nM was pre-incubated with different concentrations of compounds in the buffer at room temperature for 1 h. The reaction was initiated by adding a Cy5 fluorescently labeled probe and europium chelate-labeled streptavidin (petroleum ether rkin Elmer, #AD0060) at final concentrations of 75 nM and 5 nM, respectively. Kinetic readings were taken on a microplate reader (excitation light at 337 nm, emitted light at 665 nm, 70 µs time-gated) and time-resolved fluorescence resonance energy transfer (TR-FRET) data were read to determine IC₅₀.

### 3. Complement system hydrolysis C3 activity assay

Test compounds were 3-fold diluted from a starting concentration of 10 µM to 7 concentrations, and single-well assays were performed. Test compounds were diluted in a 96-well plate with DMSO into solutions with 1000× final concentration and then diluted with Diluent (WIESLAB^{®}COMPLEMENT SYSTEM ALTERNATIVE PATHWAY AP330) into solutions with 5× final concentration. 30 µL was transferred into a 96-well plate, and 120 µL of ready-to-use serum was added. The plate was incubated at room temperature for 15 min. To a positive control well was added 30 µL of 5‰ DMSO and 120 µL of ready-to-use serum, and to a negative control well was added 30 µL of 5‰ DMSO and 120 µL of Diluent. (3) 100 µL was added to the reaction plate, and the plate was incubated at 37 °C for 60 min. The liquids in the wells were discarded, and each well was washed 3 times with 300 µL of washing liquid. To each well was added 100 µL of Conjugate (WIESLAB^{®}COMPLEMENT SYSTEM ALTERNATIVE PATHWAY AP330). The plate was incubated at room temperature for 30 min. The liquids in the wells were discarded, and each well was washed 3 times with 300 µL of washing liquid. Then 100 µL of substrate was added to each well. The plate was incubated at room temperature for 30 min. OD405 values were read using a microplate reader (Perkin Elmer, EnSight).

### 4. Complement hemolytic activity assay

The hemolysis experiment was carried out by referring to the description in Xuan Yuan et al., Haematologica. (2017) 102:466-475. Prior to the experiment, the optimal concentration of normal human serum (NHS) required to achieve 100% lysis of rabbit erythrocytes (REs) was obtained by titration testing. In this experiment, NHS was diluted in a GVB0 buffer (0.1% gelatin, 5 mM Veronal, 145 mM NaCl, 0.025% NaN₃, pH 7.3, Complement technology) comprising 10 mM Mg-EGTA and incubated with various concentration gradients of test compounds at 37 °C for 15 min. REs (collected from healthy Japanese big-ear white rabbits) freshly suspended in a GVB0 buffer comprising 10 mM Mg-EGTA were added to a final concentration of 1 × 10⁸ cells/mL and incubated at 37 °C for 30 min. A GVB0 buffer comprising 10 mM Mg-EGTA and comprising NHS and RE but no test compound was used as a positive control group (100% lysis). A GVB0 buffer comprising 10 mM Mg-EGTA and comprising inactivated NHS (heated at 56 °C for 30 min or at 65 °C for 5 min) and RE but no test compound was used as a negative control group (0% lysis). The samples were centrifuged at 2000 g for 5 min, and the supernatant was collected. Absorbance at 415 nm (A415) was measured using a microplate reader (Molecular Devices, SpectraMax i3X). IC₅₀ values were calculated from percent hemolysis as a function of test compound concentration by non-linear regression.

### Experimental results:

The experimental results of some of the example compounds are shown in the following Table 2. The compound of Example 5 has significantly better inhibitory activity against complement factor B in human serum than the control compound, which indicates that the compound of the present disclosure can relatively good inhibit the activity of complement factor B in human serum and prevent hemolysis caused by its attack on rabbit erythrocytes.

**Table 2**

| Example compound No. | Hemolysis IC₅₀ (nM) |
|---|---|
| Example 3 | 216.3 |
| Example 4 | 280.0 |
| Example 5 | 87.9 |
| Example 6 | 217.3 |
| Example 7 | 228.4 |
| Example 8 | 358.2 |
| Example 9 | 725.0 |
| Example 10 | 610.6 |
| Example 16 | 187.9 |
| Example 17 | 391.0 |
| Example 22 | 184.2 |
| Example 23 | 852.5 |
| Example 25 | 234.0 |
| Example 26 | 319.2 |
| Example 28 | 673.5 |
| Control compound | 379.4 |

### 5. Liver microsomal stability experiment

### (1) Buffer preparation

A 0.1 M solution of potassium dihydrogen phosphate in distilled water (comprising 1 mM ethylenediaminetetraacetic acid) was taken. Then the pH was adjusted to 7.4 with the 0.1 M solution of dipotassium phosphate in distilled water (comprising 1 mM ethylenediaminetetraacetic acid).

### (2) Microsome sources and preparation of working solution

### Microsome sources:

Rat: SD Rat Liver Microsomes, Cat. No.: LM-DS-02M, RILD Research Institute for Liver Diseases (Shanghai) Co. Ltd.
Monkey: Cynomolgus Monkey Liver Microsomes, Cat. No.: LM-SXH-02M, RILD Research Institute for Liver Diseases (Shanghai) Co. Ltd.
Human: Pooled Human Liver Microsomes (Mongolian), Cat. No.: LM-R-02M, RILD Research Institute for Liver Diseases (Shanghai) Co. Ltd.

### Preparation of working solution

A 10 mM solution of each of the control compound and test compounds was prepared in DMSO. Then 10 µL of the solution was added to 190 µL of acetonitrile to form a 0.5 mM mother liquor. 1.5 µL of the 0.5 mM compound mother liquor was measured out, and 18.75 µM of 20 mg/mL liver microsomes and 479.75 µL of the buffer were added. (The actual preparation amount can be adjusted according to use).

### (3) Procedures

A 10 mg/mL solution of reduced coenzyme II (NADPH) was prepared in the buffer. A 96-well plate was placed on ice. Wells corresponding to different time points (0, 10, 30, 60 and 90 min, Non-NADPH) were set for each compound. 30 µL of the working solution was added to each well. For 0 min wells, 155 µL of glacial acetonitrile solution (the internal standard concentration was 1 µM) was added first, and after the mixtures were well mixed using a pipet, 15 µL of NADPH (10 mg/mL) was added. Before the reactions were started, the 96-well plate was pre-incubated on a microplate shaker at 37 °C for 5 min. Then 15 µL of NADPH (10 mg/mL) was added to each well to start the metabolic reactions. After the reactions were carried out for 10, 30, 60 and 90 min, 155 µL of glacial acetonitrile solution (the internal standard concentration was 1 µM) was added to the corresponding wells to terminate the reactions. After 90 min, the reaction in the Non-NADPH system was terminated by adding 155 µL of glacial acetonitrile solution (the internal standard concentration was 1 µM). After the reaction was completed, the 96-well plate was shaken on a microplate shaker (600 rpm) for 10 min and then centrifuged at 4 °C at 4000 g for 15 min. 50 µL of the supernatant was added to a new 2 mL 96-well plate, and 300 µL of deionized water was added. The mixture was analyzed on an AB SCIEX ExionLC-Triple Quad 5500 high performance liquid chromatograph-mass spectrometer. The Analyst 1.6.3 software was used. The assay results are shown in the following Table 3.

**Table 3**

| Example compound No. | MMS (rat) | | MMS (monkey) | | MMS (human) | |
|---|---|---|---|---|---|---|
| | T_{1/2} (min) | Remaining (T=90min) | T_{1/2} (min) | Remaining (T=90min) | T_{1/2} (min) | Remaining (T=90min) |
| Example 4 | 221.058 | 72.23% | 364.082 | 80.68% | 416.398 | 84.79% |
| Example 5 | 547.65 | 89.14% | 770.376 | 93.32% | 672.513 | 91.04% |
| Example 6 | 59.176 | 33.55% | 130.27 | 60.37% | 237.48 | 74.57% |

Experimental results: The data show that the compounds of Example 4, Example 5 and Example 6 all have more significant liver microsomal stability.

### 6. PK experiment of single intragastric administration to rats

### Experimental method:

Six- to nine-week-old male Wistar han rats (from Shanghai Sippe-Bk Lab Animal Co., Ltd.) were fasted overnight. Each group consisted of 3 rats, and they were given the control compound, the compound of Example 5 and the compound of Example 6, respectively, by intragastric administration at 3 mg/kg at a volume of 10 mL/kg. 0.2 mL of blood was collected from the jugular vein at each time point, anticoagulated with EDTA-K2 and immediately centrifuged at 4000 rpm at 4 °C for 5 min. The supernatant was collected. The samples were cryopreserved in a freezer at -80 °C before analysis. Time points of blood collection: before administration, 5 min, 15 min, 30 min, 1 h, 2 h, 4 h, 7 h and 24 h. The states the animals were in were observed at all times after the administration. After blood collection at all of the time points was completed, the animals were euthanized. The plasma samples were analyzed by LC-MS/MS. The data were used to calculate kinetic parameters (Tmax, Cmax, T1/2 and AUC) in the WinNonlin software.

### Experimental results:

The assay results are shown in Table 4.

**Table 4**

| Group | | Tₘₐₓ (hr) | Cₘₐₓ (ng/mL) | T_{1/2} (hr) | AUC₀₋ₜ (hr*ng/mL) | AUC_{inf} (hr*ng/mL) |
|---|---|---|---|---|---|---|
| Example 5 | Mean | 0.25 | 1923.36 | 1.58 | 3120.14 | 3282.35 |
| | S.D. | 0.00 | 602.66 | 0.21 | 721.79 | 692.86 |
| Example 6 | Mean | 0.25 | 1184.20 | 2.28 | 2194.70 | 2257.24 |
| | S.D. | 0.00 | 219.30 | 1.33 | 227.42 | 186.67 |
| Control compound | Mean | 0.33 | 783.97 | 2.42 | 2533.81 | 2726.42 |
| | S.D. | 0.14 | 166.87 | 0.69 | 260.00 | 104.81 |

### 7. PK/PD experiment of single intragastric administration to cynomolgus monkeys

### Experimental method:

Cynomolgus monkeys were used. Each group consisted of 3 cynomolgus monkeys, and they were given the control compound and the compound of Example 5 (3 & 30 mpk) by intragastric administration. Blood was collected at different time points and analyzed for drug concentration and complement activity. The drug concentration in plasma was determined by LC-MS/MS. The complement activity in serum was determined using a wieslab assay (Svar Life Science AB, COMPL AP330 RUO) kit, Normal Human Serum (Complement Technology, NHS).

### Experimental results:

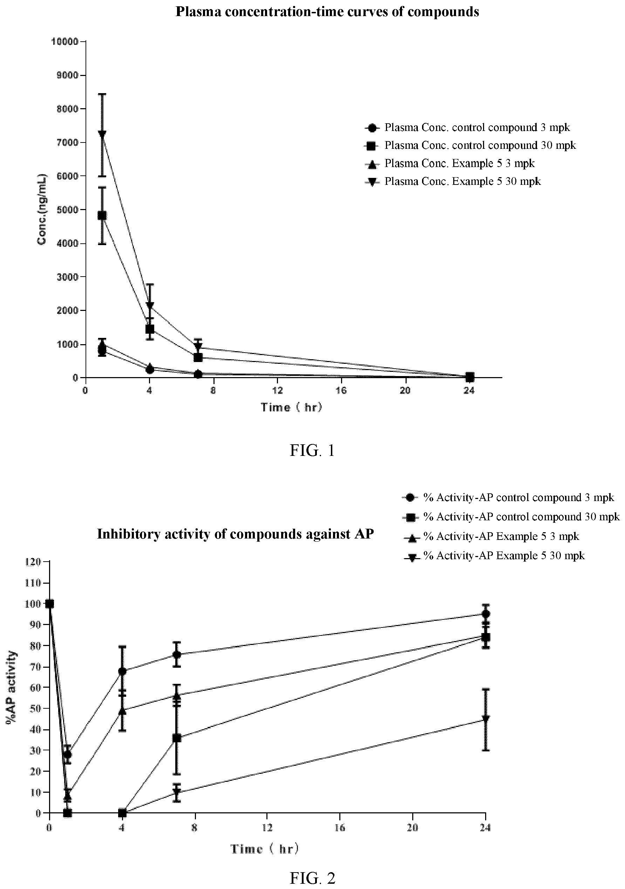

Within the ranges of concentration and time of the assay, the compound of Example 5 has a significantly higher mean plasma concentration than the control compound when administered at the same dose. The plasma concentration curves for cynomolgus monkeys are shown in FIG. 1. The inhibition of cynomolgus monkey serum AP activity is shown in FIG. 2. FIG. 2 shows that the compounds disclosed herein can effectively inhibit the cynomolgus monkey serum AP activity.

### 8. Streptococcus-induced rheumatoid arthritis (RA) model in rats

### Experimental method:

In the experiment, six- to nine-week-old female Lewis rats (Beijing Vital River) were used. Each group consisted of 6 rats, and they were given the cell wall peptidoglycan complexes of *Streptococcus* and several other species of bacteria by intraperitoneal administration (2-3 mg per rat) on D1, and were given the control compound (15 mpk) and Example 5 (15 mpk) by intragastric administration daily for 25 consecutive days. The arthritis in rats was scored in different phases. The scoring criteria are as follows: Scoring according to the degree of lesion (redness and swelling) was performed on a scale of 0-4 points, with a maximum score of 4 for each limb and a total maximum score of 16 for each animal's limbs. The scoring criteria are as follows: 0 points: no redness and swelling; 1 point: 1-2 red and swollen interphalangeal joints; 2 points: 3-4 red and swollen interphalangeal joints; 3 points: more than 4 red and swollen interphalangeal joints; 4 points: severe redness and swelling in toes or fingers to ankle joints or wrist joints.

### Experimental results:

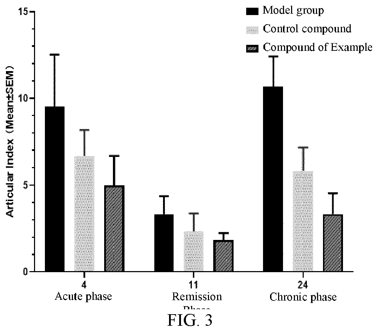

The experimental results are shown in FIG. 3. The data show that both the control compound and the compound of Example 5 can improve the arthritis score of compound, and that the compound of Example 5 has a significantly better effect than the control compound, which demonstrates that the compounds disclosed herein, especially the example compounds, are more effective in ameliorating *Streptococcus*-induced rheumatoid arthritis in rats.

The illustrative examples of the present disclosure have been described above. It should be understood that the protection scope of the present application should not be limited to the illustrative examples described above. Any modification, equivalent replacement and improvement and the like made by those skilled in the art without departing from the spirit and principle of the present disclosure shall fall within the protection scope of the present application.

## Claims

1. A compound represented by the following formula (I) or a racemate, a stereoisomer, a tautomer, an isotopically labeled compound, a solvate, a polymorph, a pharmaceutically acceptable salt or a prodrug compound thereof: 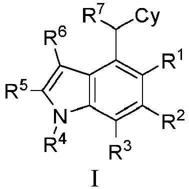
wherein R¹ is selected from halogen, OH, CN, NO₂, and the following groups unsubstituted or optionally substituted with 1, 2 or more R^{a}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄o alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R² is selected from H, halogen, OH, CN, NO₂, and the following groups unsubstituted or optionally substituted with 1, 2 or more R^{b}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R³ is selected from halogen, OH, CN, NO₂, and the following groups unsubstituted or optionally substituted with 1, 2 or more R^{c}: C₁₋₄o alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R⁴ is selected from H, and the following groups unsubstituted or optionally substituted with 1, 2 or more R^{d}: C₁₋₄o alkyl, C₃₋₄₀ cycloalkyl, C₁₋₄₀ alkyl-C(O)-, C₃₋₄₀ cycloalkyl-C(O)-, C₁₋₄₀ alkylS(O)₂-, and C₃₋₄₀ cycloalkyl-C(O)₂-;
R⁵ is selected from H, halogen, OH, CN, NO₂, and the following groups unsubstituted or optionally substituted with 1, 2 or more R^{e}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R⁶ is selected from H, halogen, OH, CN, NO₂, and the following groups unsubstituted or optionally substituted with 1, 2 or more R^{f}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄o alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
R⁷ is selected from hydrogen, OH, CN, and the following groups unsubstituted or optionally substituted with 1, 2 or more R^{g}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
alternatively, R¹ and R⁷, together with atoms to which they are attached, form a 5- to 20-membered cyclic structure that is unsubstituted or optionally substituted with 1, 2 or more R^{h}; wherein the 5- to 20-membered cyclic structure may be selected from, for example, the following groups: C₅₋₂₀ cycloalkenyl, C₆₋₂₀ aryl, 5- to 20-membered heterocyclyl, and 5- to 20-membered heteroaryl;
alternatively, R⁶ and R⁷, together with atoms to which they are attached, form a 5- to 20-membered cyclic structure that is unsubstituted or optionally substituted with 1, 2 or more Rⁱ; wherein the 5- to 20-membered cyclic structure may be selected from, for example, the following groups: C₅₋₂₀ cycloalkenyl, C₆₋₂₀ aryl, 5- to 20-membered heterocyclyl, and 5- to 20-membered heteroaryl;
Cy is selected from the following groups substituted with 1, 2, 3, 4, 5, 6, 7, 8 or more substituents independently selected from R⁸, R⁹, R¹⁰ and R¹¹: C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₃₋₄₀ cycloalkyl-C₁₋₄₀ alkyl-, C₃₋₄₀ cycloalkenyl-C₁₋₄₀ alkyl-, C₃₋₄₀ cycloalkynyl-C₁₋₄₀ alkyl-, C₆₋₂₀ aryl-C₁₋₄₀ alkyl-, 5- to 20-membered heteroaryl-C₁₋₄₀ alkyl-, 3- to 20-membered heterocyclyl-C₁₋₄₀ alkyl-, C₃₋₄₀ cycloalkyl-C₁₋₄₀ alkyl-, C₃₋₄₀ cycloalkenyl-C₁₋₄₀ alkyl-, C₃₋₄₀ cycloalkynyl-C₁₋₄₀ alkyl-, C₆₋₂₀ aryl-C₁₋₄₀ alkyl-, 5- to 20-membered heteroaryl-C₁₋₄₀ alkyl-, and 3- to 20-membered heterocyclyl-C₁₋₄₀ alkyl-, wherein the 3- to 20-membered heterocyclyl in the group Cy comprises 1-5 heteroatoms selected from N, O and S and comprises up to only one N atom;
R⁸ and R⁹ are identical or different, and are each independently selected from H, and the following groups unsubstituted or optionally substituted with 1, 2 or more R^{j}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₃₋₄₀ cycloalkyl-C₁₋₄₀ alkyl-, C₃₋₄₀ cycloalkenyl-C₁₋₄₀ alkyl-, C₃₋₄₀ cycloalkynyl-C₁₋₄₀ alkyl-, C₆₋₂₀ aryl-C₁₋₄₀ alkyl-, 5- to 20-membered heteroaryl-C₁₋₄₀ alkyl-, and 3- to 20-membered heterocyclyl-C₁₋₄₀ alkyl-;
R¹⁰ and R¹¹ are identical or different, and are each independently selected from H, being absent, halogen, OH, CN, NO₂, and the following groups unsubstituted or optionally substituted with 1, 2 or more R^{k}: C₁₋₄o alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂;
alternatively, R⁸ and R⁹, together with atoms to which they are attached, form a 5- to 20-membered cyclic structure that is unsubstituted or optionally substituted with 1, 2 or more R^{j}; wherein the 5- to 20-membered cyclic structure may be selected from, for example, the following groups: C₃₋₂₀ cycloalkyl, C₅₋₂₀ cycloalkenyl, C₆₋₂₀ aryl, 5- to 20-membered heterocyclyl, and 5- to 20-membered heteroaryl;
alternatively, R¹⁰ and R¹¹, together with atoms to which they are attached, form a 5- to 20-membered cyclic structure that is unsubstituted or optionally substituted with 1, 2 or more R^{k}; wherein the 5- to 20-membered cyclic structure may be selected from, for example, the following groups: C₃₋₂₀ cycloalkyl, C₅₋₂₀ cycloalkenyl, C₆₋₂₀ aryl, 5- to 20-membered heterocyclyl, and 5- to 20-membered heteroaryl;
each R^{a}, R^{b}, R^{c}, R^{d}, R^{e}, R^{f}, R^{g}, R^{h}, Rⁱ, R^{j} and R^{k} are identical or different, and are independently selected from H, halogen, OH, CN, NO₂, oxo (=O), thio (=S), and the following groups unsubstituted or optionally substituted with 1, 2 or more R^{p}: C₁₋₄o alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)R¹², -C(O)OR¹³, - OC(O)R¹⁴, -S(O)₂R¹⁵, -S(O)₂OR¹⁶, -OS(O)₂R¹⁷, -B(OR¹⁸)(OR¹⁹), -P(O)(OR²⁰)(OR²¹), and 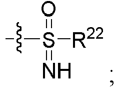
each R^{p} is identical or different, and is independently selected from H, halogen, OH, CN, NO₂, oxo (=O), thio (=S), and the following groups unsubstituted or optionally substituted with 1, 2 or more R^{q}: C₁₋₄o alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclothio, NH₂, -C(O)R¹²¹, -COOR¹³¹, -OC(O)R¹⁴¹, -S(O)₂R¹⁵¹, -S(O)₂OR¹⁶¹, - OS(O)₂R¹⁷¹, -B(OR¹⁸¹)(OR¹⁹¹), -P(O)(OR²⁰¹)(OR²¹¹), and 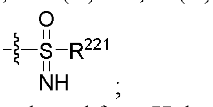
each R^{q} is identical or different, and is independently selected from H, halogen, OH, CN, NO₂, oxo (=O), thio (=S), C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)C₁₋₄₀ alkyl, -C(O)NH₂, -C(O)NHC₁₋₄₀ alkyl, -C(O)-NH-OH, -COOC₁₋₄₀ alkyl, -COOH, -OC(O)C₁₋₄₀ alkyl, -OC(O)H, -S(O)₂C₁₋₄₀ alkyl, S(O)₂H, -S(O)₂OC₁₋₄₀ alkyl, -OS(O)₂C₁₋₄₀ alkyl, -P(O)(OH)₂, -B(OH)₂, and 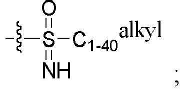
R¹², R¹³, R¹⁴, R¹⁵, R¹⁶, R¹⁷, R¹⁸, R¹⁹, R²⁰, R²¹, R¹²¹, R¹³¹, R¹⁴¹, R¹⁵¹, R¹⁶¹, R¹⁷¹, R¹⁸¹, R¹⁹¹, R²⁰¹, R²¹¹, R¹²², R¹³², R¹⁴², R¹⁵², R¹⁶², R¹⁷², R¹⁸², R¹⁹², R²⁰² and R²¹² are identical or different, and are each independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, and NH₂.

2. The compound represented by formula (I) or the racemate, the stereoisomer, the tautomer, the isotopically labeled compound, the solvate, the polymorph, the pharmaceutically acceptable salt or the prodrug compound thereof according to claim 1, wherein R¹ is selected from halogen, OH, CN, NO₂, and the following groups unsubstituted or optionally substituted with 1, 2 or more R^{a}: C₁₋₆ alkyl, C₃₋₈ cycloalkyl, C₁₋₆ alkyloxy, C₃₋₈ cycloalkyloxy, and NH₂;
preferably, R² is selected from H, halogen, OH, CN, NO₂, and the following groups unsubstituted or optionally substituted with 1, 2 or more R^{b}: C₁₋₆ alkyl, C₃₋₈ cycloalkyl, C₁₋₆ alkyloxy, C₃₋₈ cycloalkyloxy, and NH₂;
preferably, R³ is selected from halogen, OH, CN, NO₂, and the following groups unsubstituted or optionally substituted with 1, 2 or more R^{c}: C₁₋₆ alkyl, C₃₋₈ cycloalkyl, C₁₋₆ alkyloxy, C₃₋₈ cycloalkyloxy, and NH₂;
preferably, R⁴ is selected from H, and C₁₋₆ alkyl unsubstituted or optionally substituted with 1, 2 or more R^{d};
preferably, R⁵ is selected from H, halogen, OH, CN, NO₂, and the following groups unsubstituted or optionally substituted with 1, 2 or more R^{e}: C₁₋₆ alkyl, C₃₋₈ cycloalkyl, C₁₋₆ alkyloxy, C₃₋₈ cycloalkyloxy, and NH₂;
preferably, R⁶ is selected from H, halogen, OH, CN, NO₂, and the following groups unsubstituted or optionally substituted with 1, 2 or more R^{f}: C₁₋₆ alkyl, C₃₋₈ cycloalkyl, C₁₋₆ alkyloxy, C₃₋₈ cycloalkyloxy, and NH₂;
R⁷ is selected from hydrogen, OH, CN, and the following groups unsubstituted or optionally substituted with 1, 2 or more R^{g}: C₁₋₆ alkyl, C₃₋₈ cycloalkyl, C₁₋₆ alkyloxy, C₃₋₈ cycloalkyloxy, and NH₂;
preferably, R¹ and R⁷, together with atoms to which they are attached, may form the following groups unsubstituted or optionally substituted with 1, 2 or more R^{h}: C₅₋₁₀ cycloalkenyl, C₆₋₁₀ aryl, 5-to 10-membered heterocyclyl, 5- to 10-membered heteroaryl, e.g., C₅₋₆ cycloalkenyl, C₆ aryl, 5- to 6-membered heterocyclyl, and 5- to 6-membered heteroaryl; preferably, the 5- to 6-membered heterocyclyl and 5- to 6-membered heteroaryl comprise, for example, 1, 2, 3, 4, 5 or more heteroatoms selected from O, S and N, wherein N and S may optionally be unoxidized or oxidized to various oxidation forms; preferably, R¹ and R⁷, together with atoms to which they are attached, may form cyclopentyl, cyclohexyl, tetrahydrofuranyl, tetrahydropyranyl or tetrahydrothiopyranyl (wherein a sulfur atom is unoxidized or is oxidized to a -S(O)₂- group) that is fused to the indolyl group in formula (I) and is unsubstituted or optionally substituted with 1, 2 or more R^{h};
preferably, R⁶ and R⁷, together with atoms to which they are attached, may form the following groups unsubstituted or optionally substituted with 1, 2 or more R¹: C₅₋₂₀ cycloalkenyl, C₆₋₂₀ aryl, 5-to 20-membered heterocyclyl, 5- to 20-membered heteroaryl, e.g., C₅₋₆ cycloalkenyl, C₆ aryl, 5- to 6-membered heterocyclyl, and 5- to 6-membered heteroaryl; preferably, the 5- to 6-membered heterocyclyl and 5- to 6-membered heteroaryl comprise, for example, 1, 2, 3, 4, 5 or more heteroatoms selected from O, S and N, wherein N and S may optionally be unoxidized or oxidized to various oxidation forms; preferably, R⁶ and R⁷, together with atoms to which they are attached, may form cyclopentyl, cyclohexyl, tetrahydrofuranyl, tetrahydropyranyl or tetrahydrothiopyranyl (wherein a sulfur atom is unoxidized or is oxidized to a -S(O)₂- group) that is fused to the indolyl group in formula (I) and is unsubstituted or optionally substituted with 1, 2 or more R^{h};
preferably, Cy may be selected from the following groups substituted with 1, 2, 3, 4, 5, 6, 7, 8 or more substituents independently selected from R⁸, R⁹, R¹⁰ and R¹¹: C₃₋₄₀ cycloalkyl, C₆₋₂₀ aryl, 5-to 20-membered heteroaryl, and 3- to 20-membered heterocyclyl, wherein the 3- to 20-membered heterocyclyl in the group Cy comprises 1-5 heteroatoms selected from N, O and S and comprises up to only one N atom;
preferably, Cy may be selected from 3- to 20-membered heterocyclyl substituted with 1, 2, 3, 4, 5, 6, 7 or 8 substituents selected from R⁸, R⁹, R¹⁰ and R¹¹; for example, Cy is selected from 3- to 20-membered heterocyclyl substituted with R⁸, R⁹, R¹⁰ and R¹¹ and optionally further substituted with 1, 2, 3 or 4 substituents independently selected from R⁸, R⁹, R¹⁰ and R¹¹, wherein the 3- to 20-membered heterocyclyl in the group Cy comprises 1-3 heteroatoms selected from N, O and S and comprises up to only one N atom;
preferably, Cy may be selected from the following saturated or unsaturated non-aromatic carbocyclic or heterocyclic ring systems: a 4-, 5-, 6- or 7-membered monocyclic ring system, a 7-, 8-, 9-, 10-, 11- or 12-membered bicyclic (e.g. fused, bridged, or spiro) ring system or a 10-, 11-, 12-, 13-, 14- or 15-membered tricyclic ring system, and the ring systems comprise 1-5 heteroatoms selected from O, S and N and comprise up to only one N atom, wherein the N and S atoms, if present, may optionally be unoxidized or oxidized to various oxidized forms;
preferably, Cy comprises 1 N atom, and optionally comprises 1 or 2 atoms selected from O and S that are present or absent; preferably, when Cy is selected from a bicyclic ring system, the N atom is in a different cyclic structure in the bicyclic ring than the O or S atom;
preferably, Cy comprises up to 2 heteroatoms, one and only one of which is selected from N atom;
preferably, Cy may be selected from the following cyclic groups:
piperidyl;
piperidyl fused to a ring system selected from cyclopropyl, tetrahydrofuranyl, tetrahydropyranyl and phenyl;
aza and/or oxa spiro[2.4], [3.4], [4.4], [2.5], [3.5], [4.5] or [5.5] cyclic groups; and
aza and/or oxa bicyclo[2.2.1], [2.2.2], [3.2.1], [3.2.2] or [3.3.2] cyclic groups;
preferably, the N atom of Cy is bonded to the C atom shared by the groups Cy and R⁷ of formula (I);
preferably, Cy may be selected from monocyclic, fused and bridged cyclic groups, e.g., the following groups:
piperidyl; 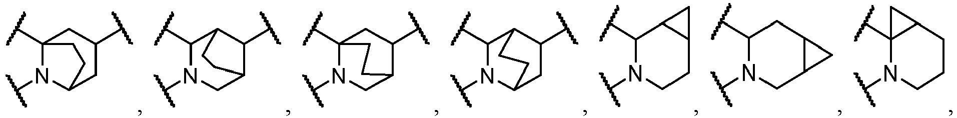 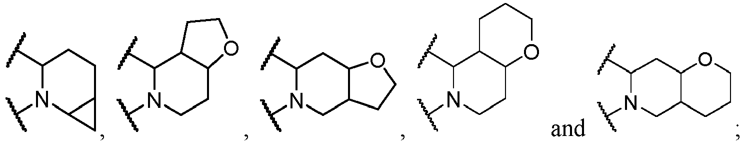 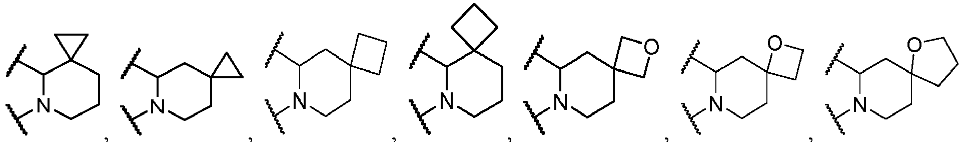 and 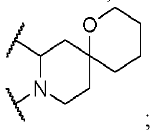 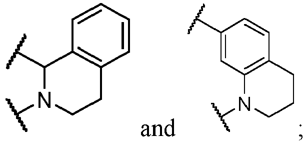
preferably, R⁸ may be selected from the following groups optionally substituted with 1, 2 or more R^{j}: C₆₋₁₀ aryl, 5- to 10-membered heteroaryl, and 3- to 20-membered heterocyclyl, e.g., phenyl, pyridinyl, pyrazinyl, furanyl, pyranyl, benzocyclohexyl, benzocyclopentyl, benzofuranyl, and benzotetrahydrofuranyl;
preferably, R⁹ is identical or different, and is each independently selected from H, and C₁₋₆ alkyl unsubstituted or optionally substituted with 1, 2 or more R^{j};
preferably, R⁸ and R⁹, together with atoms to which they are attached, may form the following groups that are unsubstituted or optionally substituted with 1, 2 or more R^{j}: C₅₋₁₀ cycloalkenyl, C₆₋₁₀ aryl, 5- to 10-membered heterocyclyl, and 5- to 10-membered heteroaryl;
preferably, R¹⁰ and R¹¹ may be identical or different, and are each independently selected from halogen, OH, CN, NO₂, and the following groups unsubstituted or optionally substituted with 1, 2 or more R^{k}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₆ cycloalkyloxy, C₆₋₁₀ aryloxy, 5- to 6-membered heteroaryloxy, 3-to 6-membered heterocyclyloxy, and NH₂;
preferably, R¹⁰ and R¹¹, together with atoms to which they are attached, may form the following groups that are unsubstituted or optionally substituted with 1, 2 or more R^{k}: C₅₋₁₀ cycloalkenyl, C₆₋₁₀ aryl, 5- to 10-membered heterocyclyl, and 5- to 10-membered heteroaryl;
preferably, each R^{j} is identical or different, and is independently selected from the following groups unsubstituted or optionally substituted with 1, 2 or more R^{p}: C₁₋₆ alkyl, C₃₋₈ cycloalkyl, C₆₋₁₀ aryl, 5- to 10-membered heteroaryl, 3- to 10-membered heterocyclyl, C₁₋₆ alkyloxy, C₃₋₈ cycloalkyloxy, C₆₋₁₀ aryloxy, 5- to 10-membered heteroaryloxy, 3- to 10-membered heterocyclyloxy, NH₂, -C(O)R¹², -C(O)OR¹³, -B(OR¹⁸)(OR¹⁹), -P(O)(OR²⁰)(OR²¹), and 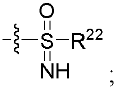
preferably, each R^{k} is identical or different, and is independently selected from halogen, OH, CN, NO₂, and the following groups unsubstituted or optionally substituted with 1, 2 or more R^{p}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₆ cycloalkyloxy, C₆₋₁₀ aryloxy, 5- to 6-membered heteroaryloxy, 3- to 6-membered heterocyclyloxy, and NH₂;
preferably, each R^{p} is identical or different, and is independently selected from H, halogen, OH, and the following groups unsubstituted or optionally substituted with 1, 2 or more R^{q}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₈ cycloalkyloxy, C₆₋₁₀ aryloxy, 5- to 6-membered heteroaryloxy, 3- to 6-membered heterocyclyloxy, NH₂, -C(O)R¹²¹, -C(O)OR¹³¹, -B(OR¹⁸¹)(OR¹⁹¹), -P(O)(OR²⁰¹)(OR²¹¹), and 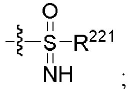
preferably, R⁴ is as defined in claim 1;
preferably, R¹², R¹³, R¹⁸, R¹⁹, R²⁰, R²¹, R¹²¹, R¹³¹, R¹⁸¹, R¹⁹¹, R²⁰¹, R²¹¹ are identical or different, and are each independently selected from H, C₁₋₆ alkyl, C₃₋₈ cycloalkyl, C₆₋₁₀ aryl, 5- to 6-membered heteroaryl, 3- to 6-membered heterocyclyl, and NH₂.

3. The compound or the racemate, the stereoisomer, the tautomer, the isotopically labeled compound, the solvate, the polymorph, the pharmaceutically acceptable salt or the prodrug compound thereof according to claim 1 or 2, wherein the compound has a structure represented by the following formula (I-1) or formula (I-2): 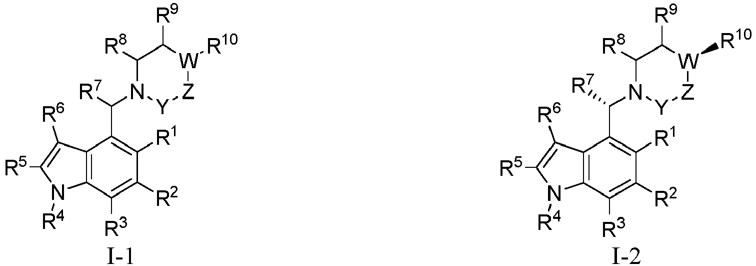
wherein W is selected from CH, O and S;
Y and Z are identical or different, and are each independently selected from CHR¹¹, O and S;
R¹, R², R³, R⁴, R⁵, R⁶, R⁷, R⁸, R⁹, R¹⁰ and R¹¹ are independently as defined in claim 1 or 2; preferably, a carbon-carbon single bond or a carbon-carbon double bond may be formed between W and Z or Z and Y;
preferably, when W is selected from O and S, R¹⁰ is absent;
preferably, when W is selected from CH, R¹⁰ is selected from H, halogen, OH, CN, NO₂, and the following groups unsubstituted or optionally substituted with 1, 2 or more R^{k}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, and NH₂, wherein R^{k} is as defined in claim 1 or 2.

4. The compound or the racemate, the stereoisomer, the tautomer, the isotopically labeled compound, the solvate, the polymorph, the pharmaceutically acceptable salt or the prodrug compound thereof according to claim 3, wherein the compound has a structure represented by the following formula (I-3) or formula (I-4): 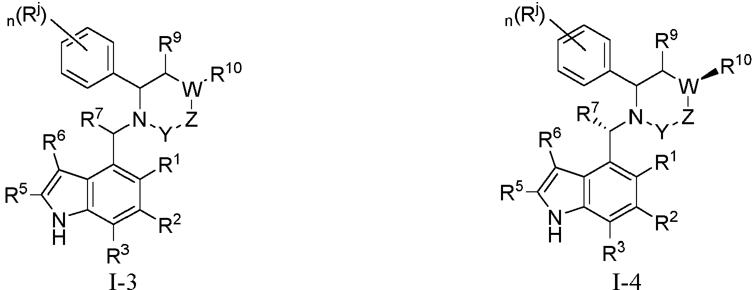
wherein W, Y, Z, R¹, R², R³, R⁵, R⁶, R⁷, R⁹, R¹⁰ and R^{j} are independently as defined in claim 3;
n is selected from 1, 2, 3, 4 and 5;
preferably, n may be selected from 1, 2 and 3;
preferably, each R^{j} may be a substituent at the 2-, 3-, 4- or 5-position of phenyl;
preferably, each R^{j} may be independently selected from the following groups unsubstituted or optionally substituted with 1, 2 or more R^{p}: C₁₋₆ alkyl, NH₂, -C(O)R¹², -C(O)OR¹³, -B(OR¹⁸)(OR¹⁹), -P(O)(OR²⁰)(OR²¹), and 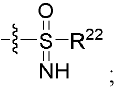
preferably, R¹⁰ is selected from halogen, OH, CN, NO₂, and the following groups unsubstituted or optionally substituted with 1, 2 or more R^{k}: C₁₋₆ alkyl (e.g., methyl, ethyl, propyl, isopropyl, butyl, isobutyl, *tert*-butyl, pentyl, or isopentyl), C₃₋₈ cycloalkyl (e.g., cyclopropyl, cyclobutyl, cyclopentyl, cyclohexyl, cycloheptyl, or cyclooctyl), 3- to 6-membered heterocyclyl (e.g., pyrrolidinyl, imidazolidinyl, piperidyl, piperazinyl, oxetanyl, tetrahydrofuranyl, or tetrahydropyranyl), C₁₋₆ alkyloxy, C₃₋₆ cycloalkyloxy, 3- to 6-membered heterocyclyloxy, and NH₂;
preferably, each R^{k} is identical or different, and is independently selected from halogen, OH, CN, NO₂, and the following groups unsubstituted or optionally substituted with 1, 2 or more R^{p}: C_{1- 6} alkyl (e.g., methyl, ethyl, propyl, isopropyl, butyl, isobutyl, *tert*-butyl, pentyl, or isopentyl), C₃₋₈ cycloalkyl (e.g., cyclopropyl, cyclobutyl, cyclopentyl, cyclohexyl, cycloheptyl, or cyclooctyl), C₆₋₁₀ aryl (e.g., phenyl), 5- to 6-membered heteroaryl (e.g., pyrrolyl, pyridinyl, pyrazinyl, imidazolyl, or triazolyl), 3- to 6-membered heterocyclyl (e.g., pyrrolidinyl, imidazolidinyl, piperidyl, piperazinyl, oxetanyl, tetrahydrofuranyl, or tetrahydropyranyl), C₁₋₆ alkyloxy, C₃₋₆ cycloalkyloxy, C₆₋₁₀ aryloxy, 5- to 6-membered heteroaryloxy, and 3- to 6-membered heterocyclyloxy;
preferably, each R^{p} is identical or different, and is independently selected from H, halogen (F, Cl, Br or I), OH, and the following groups unsubstituted or optionally substituted with 1, 2 or more R^{q}: C₁₋₆ alkyl, C₃₋₈ cycloalkyl, C₆₋₁₀ aryl, 5- to 6-membered heteroaryl, 3- to 6-membered heterocyclyl, C₁₋₆ alkyloxy, C₃₋₈ cycloalkyloxy, C₆₋₁₀ aryloxy, 5- to 6-membered heteroaryloxy, 3-to 6-membered heterocyclyloxy, and NH₂;
preferably, 1, 2, 3 or more H atoms in the compound and a substituent thereof (e.g., methyl and ethyl) may be optionally replaced with its isotope (e.g., D) to form groups such as CD₃ and C₂D₅.

5. The compound or the racemate, the stereoisomer, the tautomer, the isotopically labeled compound, the solvate, the polymorph, the pharmaceutically acceptable salt or the prodrug compound thereof according to any one of claims 1-4, wherein the compound may be selected from the following compounds:

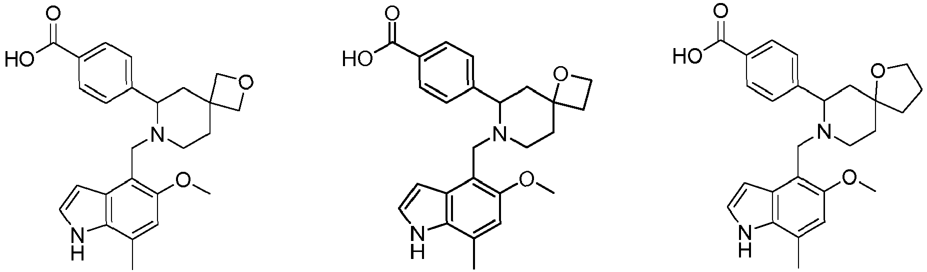

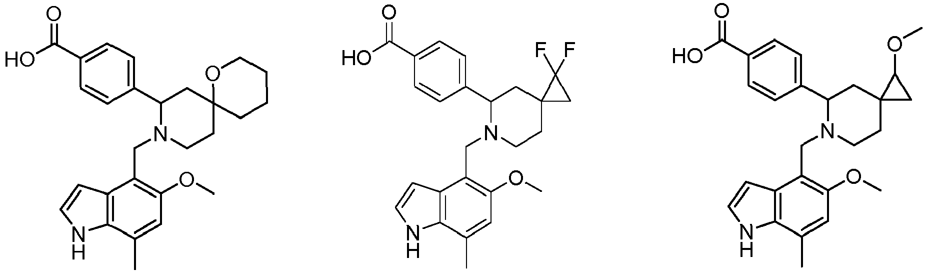

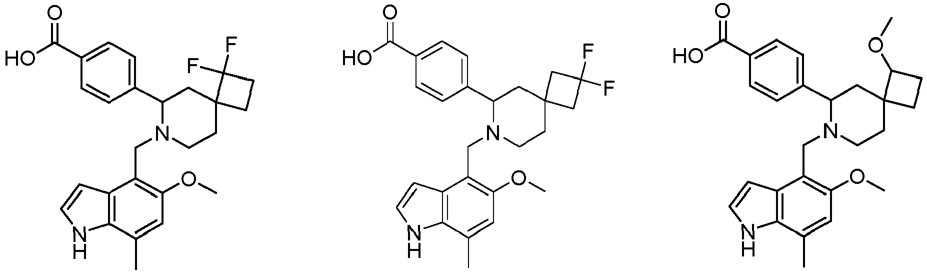

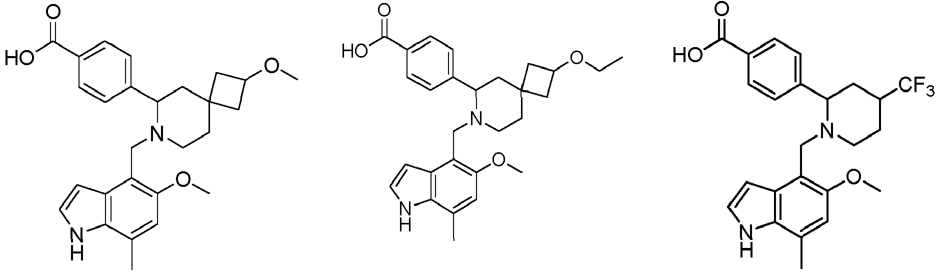

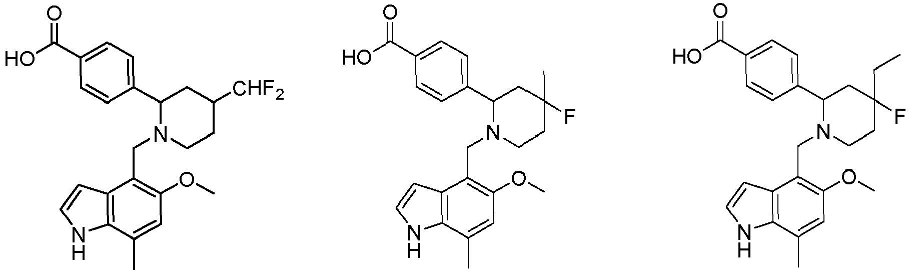

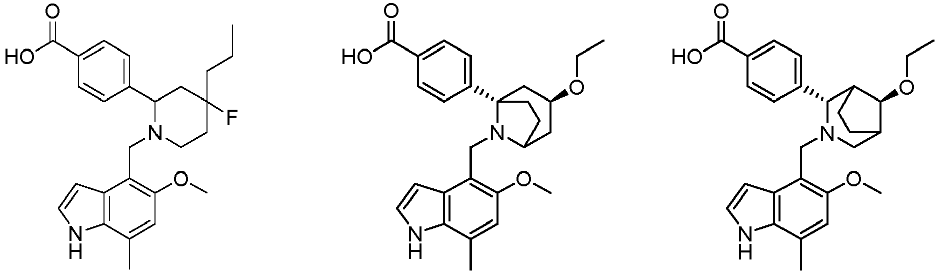

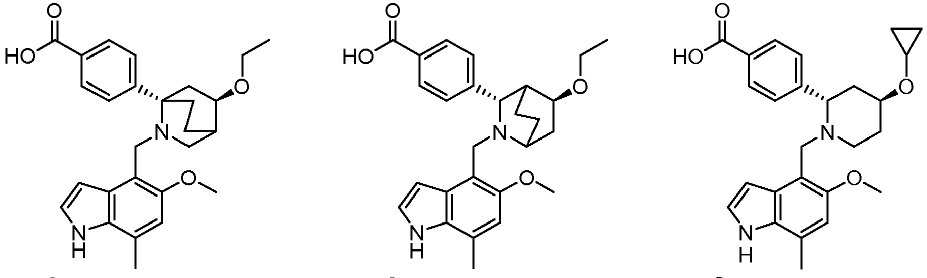

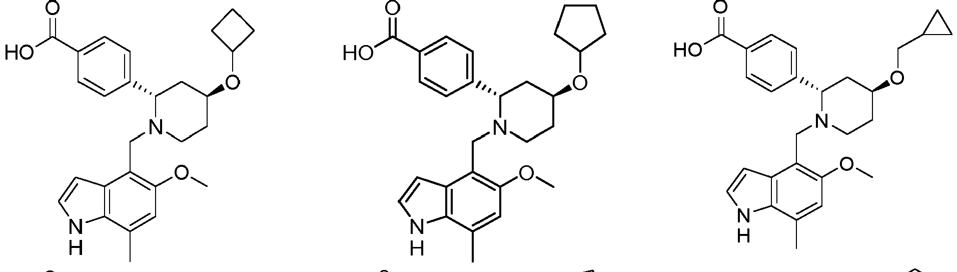

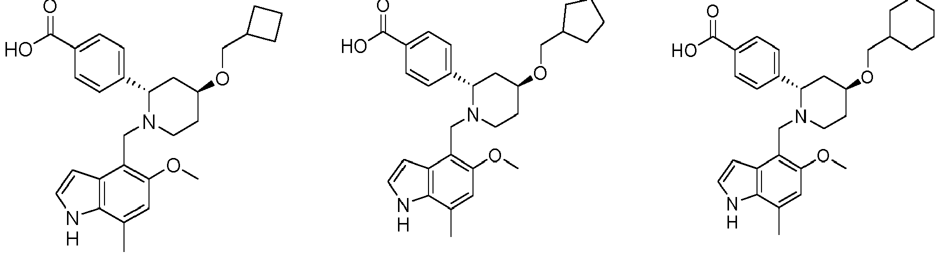

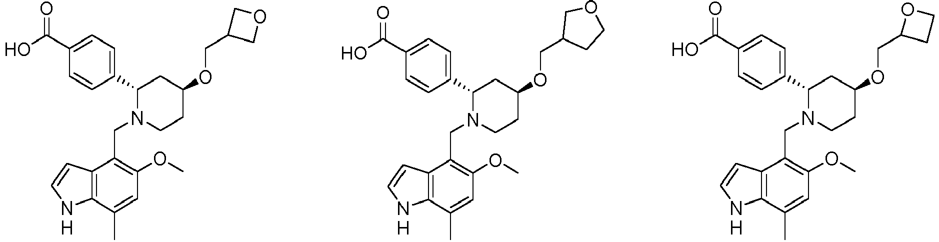

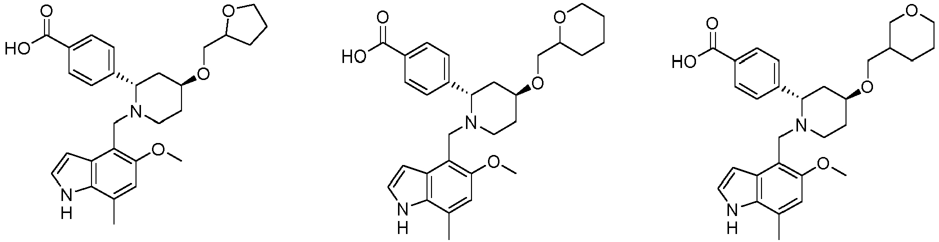

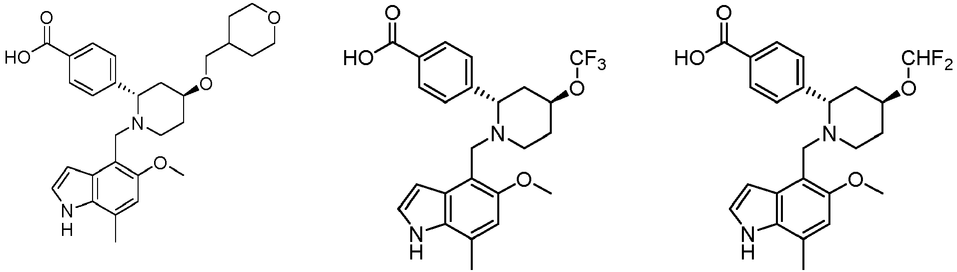

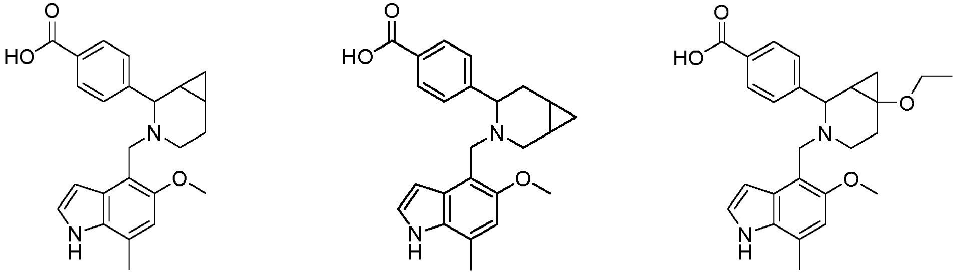

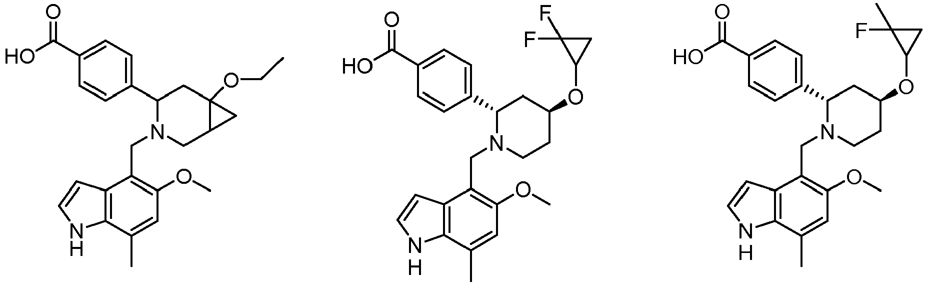

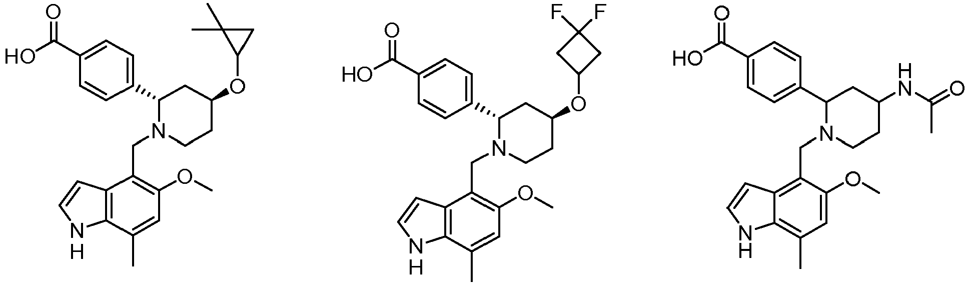

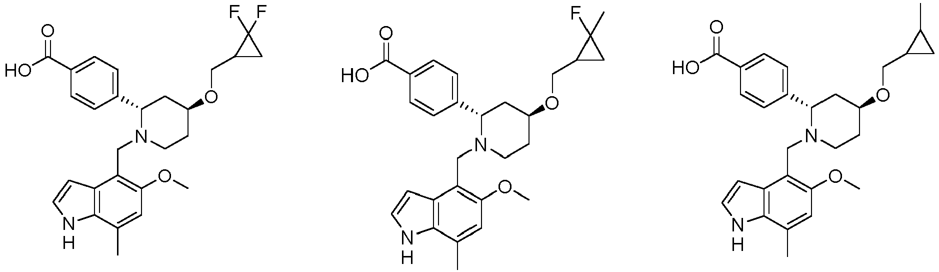

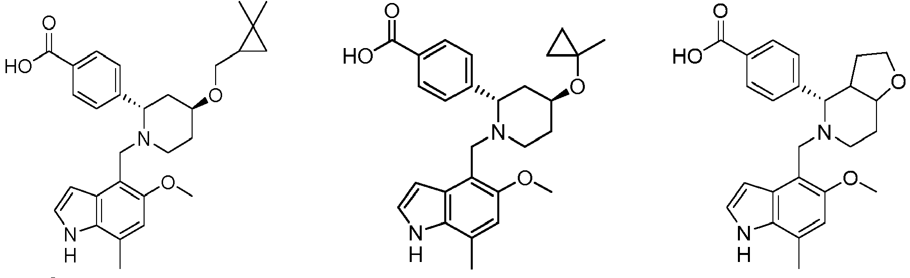

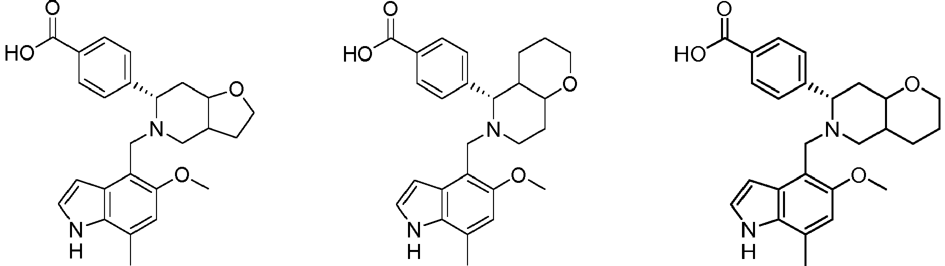

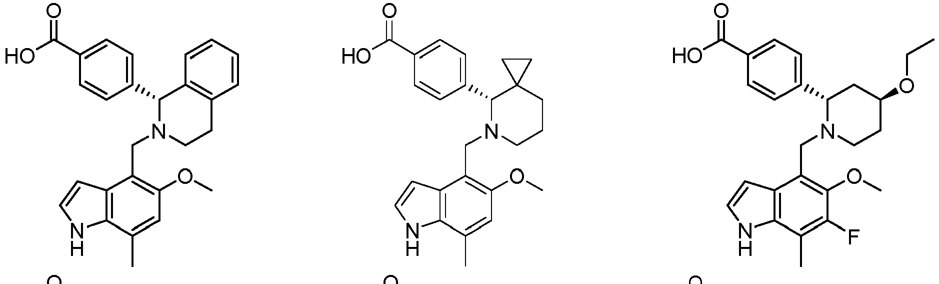

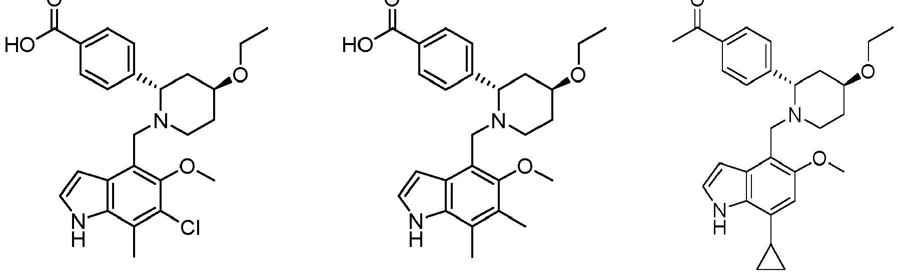

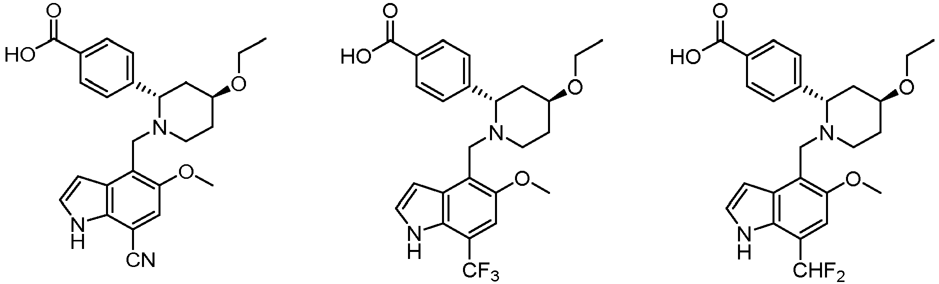

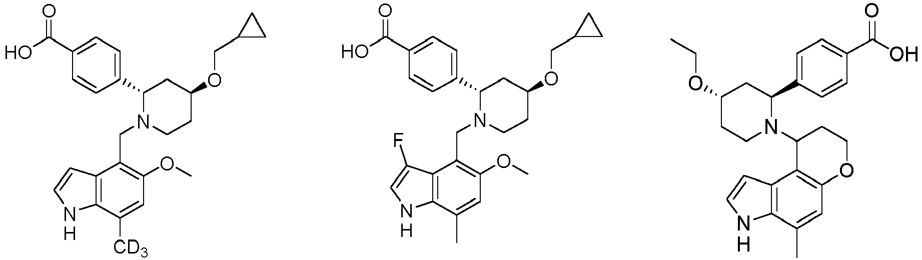

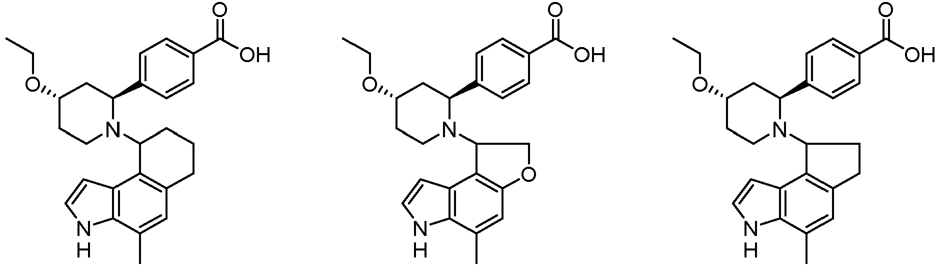

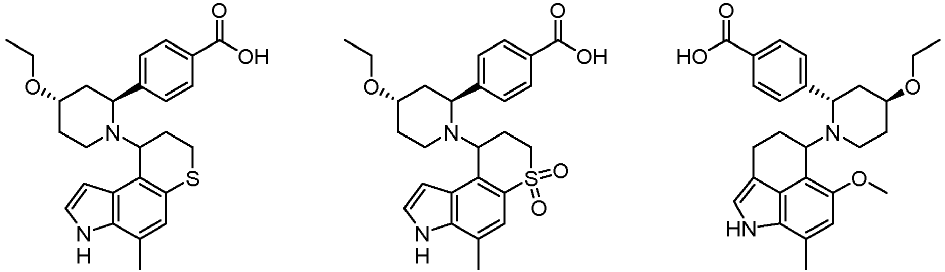

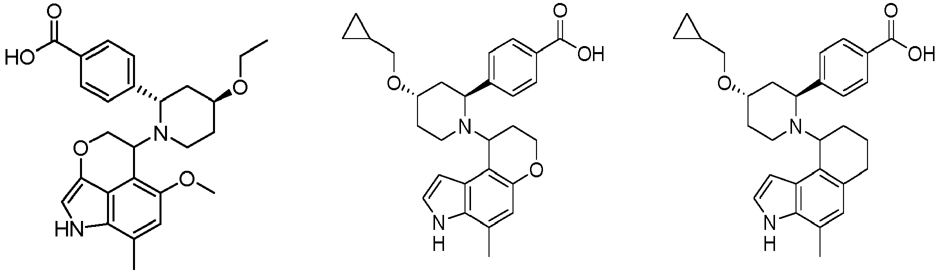

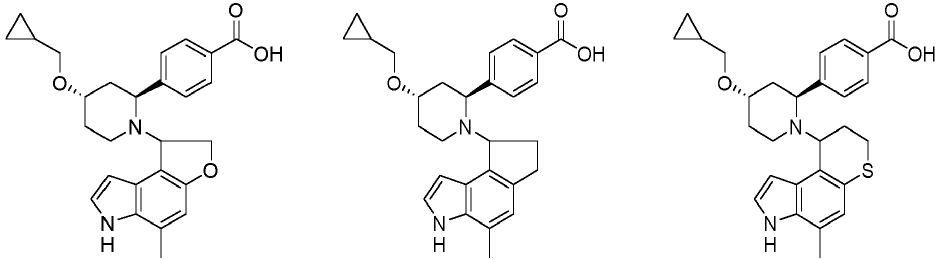

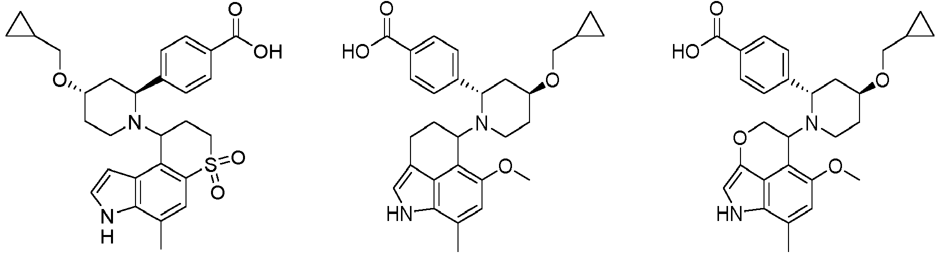

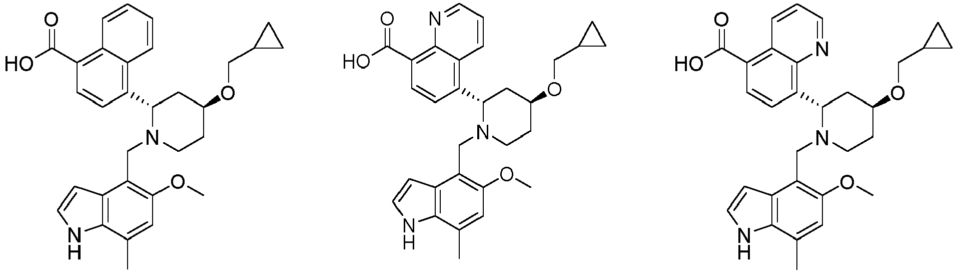

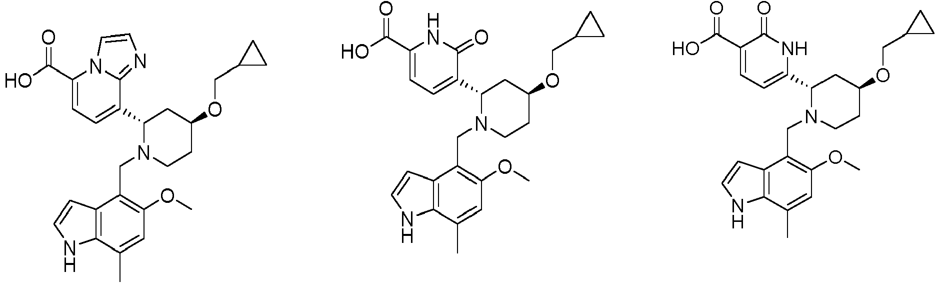

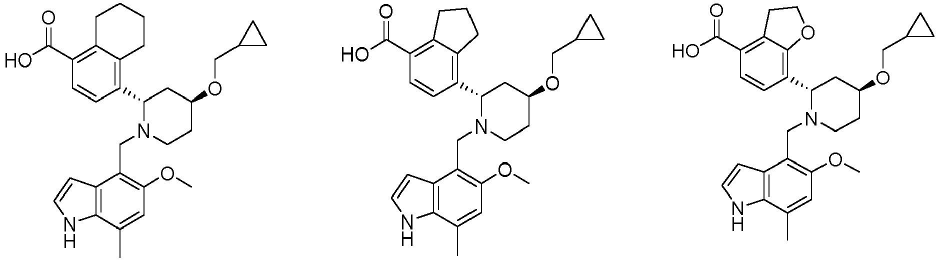

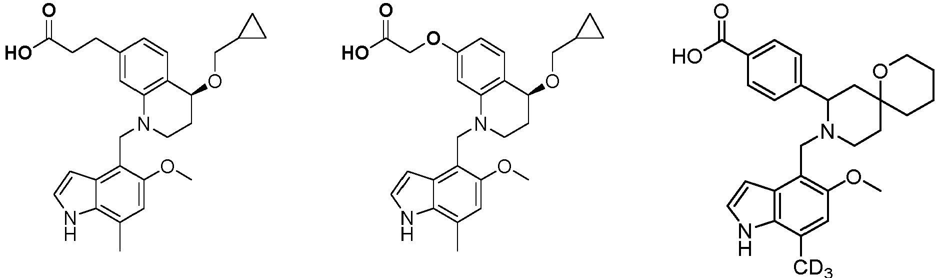

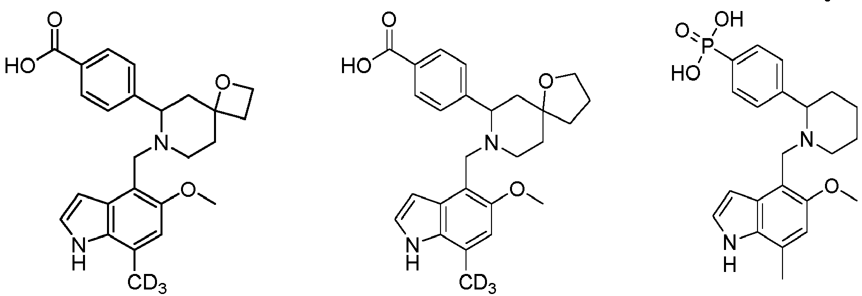

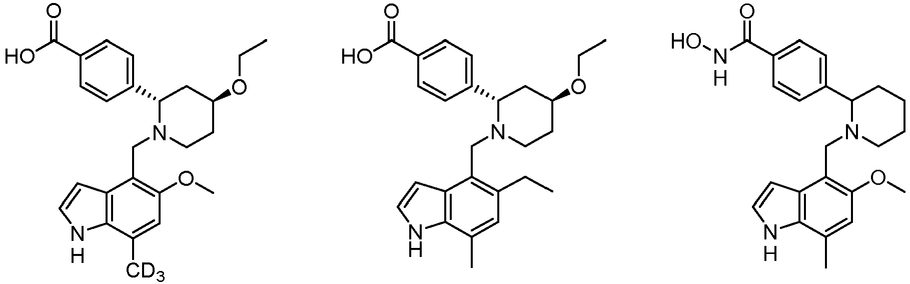

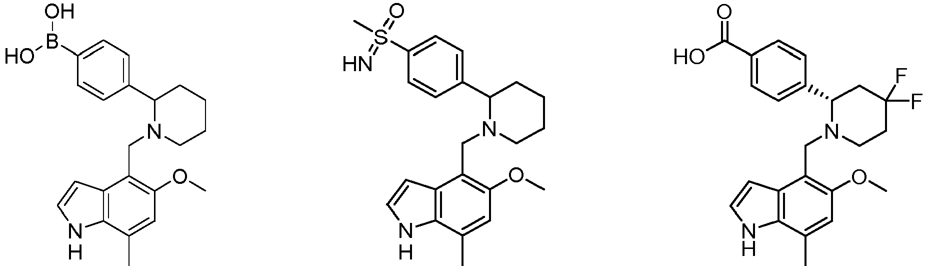

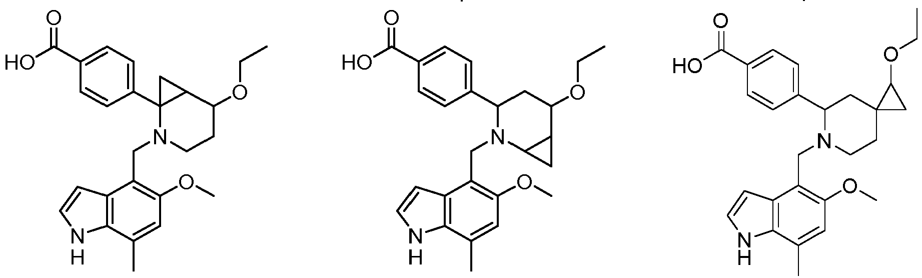

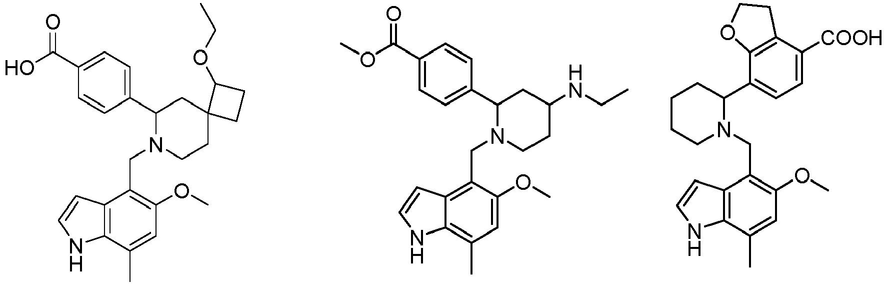

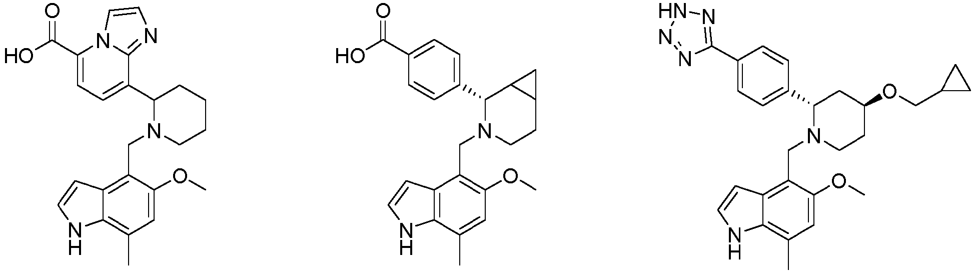

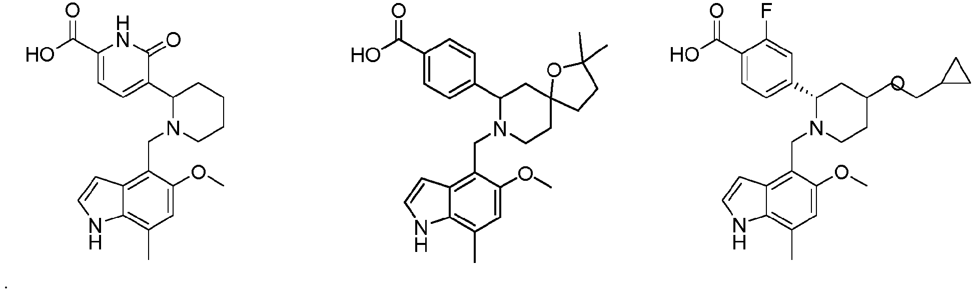

6. A compound represented by the following formula (IV): 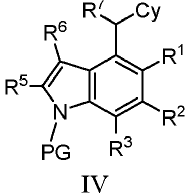
wherein PG is a protective group; PG may be selected from amino protective groups; wherein a suitable PG may be selected from C₁₋₄₀ alkyl and C₆₋₂₀ aryl C₁₋₄₀ alkyl-, e.g., *tert*-butyl, isopropyl, benzyl, *tert*-butoxycarbonyl (Boc), 2-biphenyl-2-propoxycarbonyl, benzyloxycarbonyl, fluorenylmethoxycarbonyl (Fmoc) and trifluoroacetyl;
R¹, R², R³, R⁵, R⁶, R⁷ and Cy are independently as defined in any one of claims 1-5.

7. A preparation method for the compound or the racemate, the stereoisomer, the tautomer, the isotopically labeled compound, the solvate, the polymorph, the pharmaceutically acceptable salt or the prodrug compound thereof according to any one of claims 1-5, comprising reacting a compound of the following formula (IV) as a starting material to give a compound of the following formula (Ia), i.e. the compound represented by formula (I) in which R⁴ is H: 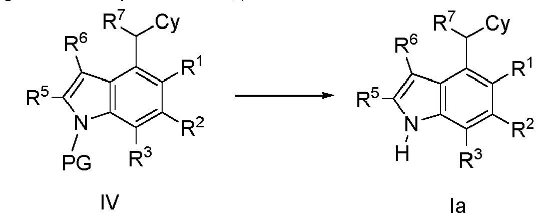
and optionally, further reacting the compound of formula (Ia) with R⁴-L¹ to give a compound represented by formula (I) in which R⁴ is a group according to any one of claims 1-5 other than H; L¹ is a leaving group, e.g., OH, F, Cl, Br, I, or halogenated C₁₋₄₀ alkyl;
wherein PG, R¹, R², R³, R⁵, R⁶, R⁷ and Cy are independently as defined in any one of claims 1-6;
according to an embodiment of the present disclosure, the compound of formula (IV) is reacted under a condition for removing the protective group PG to give the compound of formula (I);
preferably, a preparation method for the compound represented by formula (IV) comprises reacting a compound of the following formula (II) with a compound of the following formula (III) to give the compound represented by formula (IV): 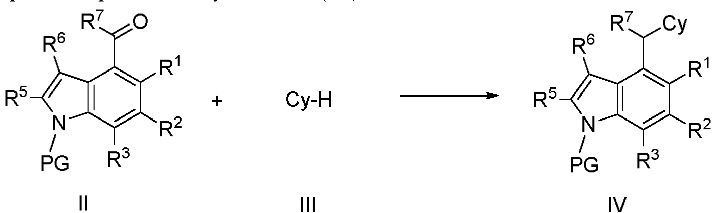
wherein PG, R¹, R², R³, R⁵, R⁶, R⁷ and Cy are independently as defined in any one of claims 1-6;
preferably, the preparation method may be carried out in the presence of a solvent such as an organic solvent; for example, the organic solvent may be selected from at least one of the following: alcohols such as methanol, ethanol, isopropanol and n-butanol; ethers such as ethyl propyl ether, *n-*butyl ether, anisole, phenetole, cyclohexylmethyl ether, dimethyl ether, diethyl ether, dimethyl glycol, diphenyl ether, dipropyl ether, diisopropyl ether, di-*n*-butyl ether, diisobutyl ether, diisoamyl ether, ethylene glycol dimethyl ether, isopropyl ethyl ether, methyl *tert*-butyl ether, tetrahydrofuran, methyltetrahydrofuran, dioxane, dichlorodiethyl ether, and polyethers of ethylene oxide and/or propylene oxide; aliphatic, cycloaliphatic or aromatic hydrocarbons such as pentane, hexane, heptane, octane, nonane, and those that may be substituted with a fluorine or chlorine atom, such as methylene chloride, dichloromethane, trichloromethane, tetrachloromethane, fluorobenzene, chlorobenzene or dichlorobenzene; cyclohexane, methylcyclohexane, petroleum ether, octane, benzene, toluene, chlorobenzene, bromobenzene, and xylene; and esters such as methyl acetate, ethyl acetate, butyl acetate, isobutyl acetate and dimethyl carbonate, dibutyl carbonate or ethylene carbonate;
preferably, the preparation method may be carried out in the presence of a reducing agent;
wherein the reducing agent is used for reducing a carbon-nitrogen double bond, and may be selected from sodium borohydride, potassium borohydride, lithium borohydride, sodium borohydride acetate, sodium cyanoborohydride and lithium aluminum hydride.

8. A pharmaceutical composition comprising a therapeutically effective amount of at least one selected from the compound and the racemate, the stereoisomer, the tautomer, the isotopically labeled compound, the solvate, the polymorph, the pharmaceutically acceptable salt and the prodrug compound thereof according to any one of claims 1-5.

9. A method for treating a disease associated with activation of the complement alternative pathway, comprising administering to a patient a prophylactically or therapeutically effective amount of at least one selected from the compound and the racemate, the stereoisomer, the tautomer, the isotopically labeled compound, the solvate, the polymorph, the pharmaceutically acceptable salt and the prodrug compound thereof according to any one of claims 1-5;
preferably, the disease associated with activation of the complement alternative pathway comprises a disease selected from paroxysmal nocturnal hemoglobinuria (PNH), primary glomerulonephritis (IgAN), membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), diabetic retinopathy (DR), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory diseases and cardiovascular diseases and the like.

10. Use of the compound represented by formula (IV) according to claim 6 for the preparation of the compound or the racemate, the stereoisomer, the tautomer, the isotopically labeled compound, the solvate, the polymorph, the pharmaceutically acceptable salt or the prodrug compound thereof according to any one of claims 1-5.
